# EUROPEAN PATENT SPECIFICATION

(11) **EP 2 805 705 B1**
(45) Date of publication and mention of the grant of the patent: **09.11.2016**
(21) Application number: 14168586.7
(22) Date of filing: 16.05.2014
(51) Int. Cl.: A61J 1/00, A61K 31/4439

(54) **Packaging with one or more administration units comprising a sodium salt of (R)-3-[6-amino-pyridin-3-yl]-2-(1-cyclohexyl-1 H-imidazol-4-yl)-propionic acid**
Verpackung enthaltend Verabreichungseinheiten von Natriumsalzen der (R)-3-[6-amino-pyridin-3-yl]-2-(1-cyclohexyl-1 H-imidazol-4-yl)-Propionsäure
Emballage comprenant des unités d'administration des sels de sodium de l'acide (R)-3-[6-amino-pyridin-3-yl]-2-(1-cyclohexyl-1 H-imidazol-4-yl)-propionique

(30) Priority: 23.05.2013 EP 13169049; 30.05.2013 EP 13169940; 30.05.2013 EP 13169941; 30.05.2013 EP 13169942; 13.06.2013 EP 13171930; 13.06.2013 EP 13171933
(43) Date of publication of application: 26.11.2014
(73) Proprietor: IP Gesellschaft für Management mbH, 50931 Köln (DE)
(72) Inventor: Trinius, Frank, 50931 Köln (DE)

(56) References cited:
- WO-A1-03/013526
- WO-A1-2005/105781
- WO-A1-2013/076178
- WO-A1-2013/076179

## Description

### CROSSREFERENCES

The present application claims priorities of European patent application no. 13169049.7, filed on 23 May 2013; European patent application no. 13169940.7, filed on 30 May 2013; European patent application no. 13169941.5, filed on 30 May 2013; European patent application no. 13169942.3, filed on 30 May 2013; European patent application no. 13171930.4, filed on 13 June 2013; and European patent application no. 13171933.8, filed on 13 June 2013.

### BACKGROUND ARTS

Many pharmaceuticals are marketed without any packaging. In various countries, small drug shops are a common source of medicine where stocking of prescription-only medicines and unpackaged tablets is the norm (Goodman C et al., Health Policy Plan. 2007 Nov;22(6):393-403). Furthermore, self-medication is common practice in various countries. For example, the most frequently proposed drugs to treat male urethritis are: ampicillin 250-mg capsules (44%); oxytetracyline 250-mg capsules (24%); and cotrimoxazole 450-mg tablets (12%), and these drugs are frequently sold unpackaged (Sow PS et al., Int J Infect Dis. 2002 Jun;6(2):108-12).

In Germany and other European countries, numerous pharmaceuticals are marketed in packaging wherein every single administration unit is individually packaged in a single packaging. Examples include but are not limited to Frubiase® Calcium (Boehringer Ingelheim) marketed as a liquid administration unit that is individually packaged in a glass ampoule; Orthomol Immun® Direktgranulat (Orthomol) marketed as administration unit in granulate form that is individually packaged in a bag; Orthomol Vital m® (Orthomol) marketed as a liquid administration unit that is individually packaged in a bottle having a lid containing an individually packaged tablet; Vitasprint® (Pfizer) and Celestamine® (MSD Sharp & Dohme) each marketed as a liquid administration unit that is individually packaged in a bottle; Alka-Seltzer® (Bayer) marketed as effervescent tablet that is individually packaged in a bag; Aspirin® Complex (Bayer), Aspirin® Effect (Bayer), Helmex® (Infectopharm), Monuril® Granulat (Pierre Fabre Pharma) each marketed as administration unit in granulate form that individually packaged in a bag; ellaOne® (HRA Pharma), Levonelle® (Bayer Schering Pharma), Pidana® (HRA Pharma), Fimgata® (Pfizer), and Canesten® Gyn once (Bayer) each marketed as a single tablet that is individually packaged in a blister. Furthermore, numerous single-to-use syringes are marketed individually packaged.

It is an object of the invention to provide administration units containing drugs that have advantages compared to conventional administration units and conventional drugs, respectively. It is also an object of the invention to provide packaging containing administration units containing drugs that have advantages compared to conventional packaging. These objects have been achieved by the present invention.

### SUMMARY OF THE INVENTION

The present invention relates to different drugs (active pharmaceutical ingredients) and certain forms of said drugs, such as stereoisomers, salts and/or polymorphs thereof. The present invention covers various aspects that are numbered (i), (ii), (iii), (iv), (v), and (vi). Aspects (i), (ii), (iii), (iv), (v), and (vi) are separate from one another. As aspects (iii) and (iv) relate to the same parent compound, {drug3} and {drug4}, respectively, these aspects are related to one another.

The present invention relates to an administration unit comprising (i) {drugla}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug31}, {drug3m}, {drug3n}, or {drug30}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}; a packaging comprising one or more of such administration units; a method for manufacturing such an administration unit; a method for manufacturing such a packaging; and particles of (i) {drugla}; (ii) {drug2a}, {drug2b}, {drug2c}, {drug2d}, or {drug2e}; (iii) {drug3a}, {drug3b}, {drug3c}, {drug3d}, {drug3e}, (drug3f), {drug3g}, {drug3h}, {drug3i}, {drug3j}, {drug3k}, {drug31}, {drug3m}, {drug3n}, or {drug30}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or(vi) {drug6a}.

Certain embodiments of the invention are also disclosed in WO2013/072327, WO2013/076177, WO2013/076178, WO2013/076179, WO2013/083553, and WO2013/084150, respectively. Any embodiment disclosed in any of these references is incorporated herein by reference as a preferred embodiment of the invention.

### GENERAL DEFINITIONS

To avoid unnecessary repetitions, the description refers to the following abbreviations the meaning of which is defined hereinafter:
Aspect (i) of the present invention relates to Boceprevir, also referred to as compound of the formula (I), 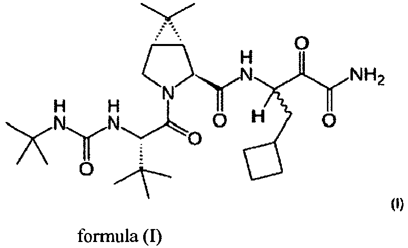 or a physiologically acceptable salt thereof, for use in the treatment of congestive heart failure, myocardial infarction, edema, diabetic complications, diuretic, abbreviated as '(drug1)'. In particular, the present invention relates to solid form of Boceprevir or a physiologically acceptable salt thereof for use in the treatment of congestive heart failure, myocardial infarction, edema, diabetic complications, diuretic, abbreviated as '{drug1a}'. {drug1a} is a specific form falling within the definition of {drug1}. Aspect (i) of the invention is separate from aspects (ii), (iii), (iv), (v), and (vi) of the invention. Thus, all embodiments of {drug1a} are only related to {drug1}, but neither to {drug2}, nor to {drug3}, nor to {drug4}, nor to {drug5}, nor to {drug6}.
Aspect (ii) of the present invention relates to naphthalene-1,5-disulfonic acid salt of (R)-3-(6-amino-pyridin-3-yl)-2-(1-cyclohexyl-1 H-imidazol-4-yl)-propionic acid-ethylester according to formula (II) 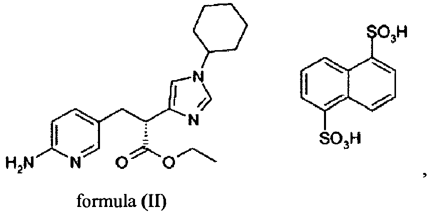 abbreviated as '{ drug2}'. In particular, the present invention relates to crystalline form of naphthalene-1,5-disulfonic acid salt of (R)-3-(6-amino-pyridin-3-yl)-2-(1-cyclohexyl- H-imidazol-4-yl)-propionic acide-ethylester, abbreviated as '{drug2a}'; a crystalline salt of naphthalene-1,5-disulfonic acid with (R)-3-(6-amino-pyridin-3-yl)-2-(1-cyclohexyl-1 H-imidazol-4-yl)-propionic acid ethyl ester of formula (II), characterized in that the measured with CuKa radiation having a XRPD reflection intensity at a high angle position of 22.48 degrees 2-theta ± 0.2 degrees 2 theta, abbreviated as '{drug2b}'; a crystalline salt of naphthalene-1,5-disulfonic acid with (R)-3-(6-amino-pyridin-3-yl)-2-(1-cyclohexyl-1 H-imidazol-4-yl)-propionic acid ethyl ester of formula (II), characterized in that the measured with CuKa radiation having XRPD peaks at the following 2-theta values at the angular positions: 7.35 and 22.48 respectively ± 0.2 degrees 2 theta, abbreviated as '{drug2c}'; a crystalline salt of naphthalene-1,5-disulfonic acid with (R)-3-(6-amino-pyridin-3-yl)-2-(1-cyclohexyl-1 H-imidazol-4-yl)-propionic acid ethyl ester of formula (II), characterized in that the measured with CuKa radiation having XRPD peaks at the following 2-theta values at the angular positions: 7.35; 11.4; 13.69; 14.96; 17.49; 19.3 and 22.48, respectively ± 0.2 degrees 2 theta, abbreviated as '{drug2d}'; and a crystalline salt of naphthalene-1,5-disulfonic acid with (R)-3-(6-amino-pyridin-3-yl)-2-(1-cyclohexyl-1 H-imidazol-4-yl)-propionic acid ethyl ester of formula (II) characterized by a Raman spectrum comprising characteristic bands at the following wavelengths [cm-¹]: 2936; 1570; 1401; 1352; 998 and 856, respectively ± 2 cm⁻¹, abbreviated as '{drug2e}'. {drug2a}, {drug2b}, {drug2c}, {drug2d}, and {drug2e} are specific forms falling within the definition of {drug2}. Aspect (ii) of the invention is separate from aspects (i), (iii), (iv), (v), and (vi) of the invention. Thus, all embodiments of {drug2a}, {drug2b}, {drug2c}, {drug2d}, and {drug2e}, respectively, are only related to {drug2}, but neither to {drug1}, nor to {drug3}, nor to {drug4}, nor to {drug5}, nor to {drug6}.
Aspect (iii) of the present invention relates to sodium salt of (R)-3-[6-amino-pyridin-3-yl]-2-(1-cyclohexyl-1 H-imidazol-4-yl)-propionic acid having the structure illustrated in Formula (III): 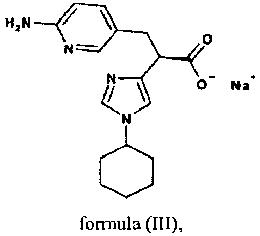 abbreviated as '{drag3}'. In particular, the present invention relates to crystalline form of sodium anhydrate salt of (R)-3-[6-amino-pyridin-3-yl]-2-(1-cyclohexyl-1 H-imidazol-4-yl)-propionic acid, abbreviated as '{drug3a}'; to monohydrate salt of (R)-3-[6-amino-pyridin-3-yl]-2-(1-cyclohexyl-1 H-imidazol-4-yl)-propionic acid, abbreviated as '{drug3b}'; to a sodium salt of (R)-3-[6-amino-pyridin-3-yl]-2-(1-cyclohexyl-1H-imidazol-4-yl)-propionic acid of Formula (III") as a hydrate 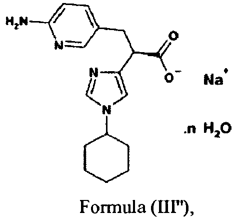 wherein n has the value from 0.7 to 1.4, or from 0.8 to 1.2, or from 0.9 to 1.1, abbreviated as '{drug3c}'; to a crystalline sodium monohydrate salt of (R)-3-[6-amino-pyridin-3-yl]-2-(1-cyclohexyl-1 H-imidazol-4-yl)-propionic acid wherein the crystalline salt exhibits in an X-ray powder diffraction pattern measured in transmission with CuKal radiation at room temperature a characteristic reflection at degrees 2 theta of 22.2, 16.5 and 6.3, each time ± 0.2 degrees 2 theta, abbreviated as '{drug3d}'; to a crystalline sodium monohydrate salt of (R)-3-[6-amino-pyridin-3-yl]-2-(1-cyclohexyl-1H-imidazol-4-yl)-propionic acid wherein the crystalline salt exhibits in an X-ray powder diffraction pattern measured in transmission with CuKal radiation at room temperature a characteristic reflection at degrees 2 theta of 22.2, 21.8, 18.1, 16.5, 8.2 and 6.3, each time ± 0.2 degrees 2 theta, abbreviated as '{drug3e}'; to a crystalline sodium monohydrate salt of (R)-3-[6-amino-pyridin-3-yl]-2-(1-cyclohexyl-1 H-imidazol-4-yl)-propionic acid wherein the crystalline salt exhibits in an X-ray powder diffraction pattern measured in transmission with CuKal radiation at room temperature a characteristic reflection at degrees 2 theta of 24.0, 22.2, 21.8, 18.8, 18.1, 16.8, 16.5, 16.0, 8.2 and 6.3, each time ± 0.2 degrees 2 theta, abbreviated as '{drug3f}'; to a crystalline sodium monohydrate salt of (R)-3-[6-amino-pyridin-3-yl]-2-(1-cyclohexyl-1 H-imidazol-4-yl)-propionic acid characterized by its Raman spectrum having the following values as characteristic wave number of the Raman shift (cm⁻¹) and the relative intensities shown in brackets: 818 (31), 864 (100), 1054 (24), 1284 (26), 1561 (37), and 1613 (27), each time ± 4 cm⁻¹, abbreviated as '{drug3g}'; to a crystalline sodium monohydrate salt of (R)-3-[6-amino-pyridin-3-yl]-2-(1-cyclohexyl-1 H-imidazol-4-yl)-propionic acid characterized by its Raman spectrum having the following values as characteristic wave number of the Raman shift (cm⁻¹) and the relative intensities shown in brackets: 657 (10), 818 (31), 864 (100), 981 (15), 1030 (18), 1054 (24), 1137 (10), 1216 (21), 1266 (20), 1284 (26), 1309 (16), 1351 (10), 1363 (19), 1407 (16), 1445 (20), 1561 (37) and 1613 (27), each time ± 4 cm⁻¹, abbreviated as '{drug3h}'; to a crystalline sodium monohydrate salt of (R)-3-[6-amino-pyridin-3-yl]-2-(1-cyclohexyl-1H-imidazol-4-yl)-propionic acid characterized by its Raman spectrum having the following values (cm⁻¹) and the relative intensities shown in brackets: 431 (3), 484 (3), 539 (2), 566 (6), 657 (10), 759 (8), 798 (2), 818 (31), 864 (100), 882 (8), 920 (6), 968 (9), 981 (15), 999 (3), 1030 (18), 1054 (24), 1077 (6), 1137 (10), 1186 (8), 1202 (6), 1216 (21), 1242 (4), 1266 (20), 1284 (26), 1309 (16), 1351 (10), 1363(19), 1407(16), 1445(20), 1495 (3), 1561 (37), 1613 (27), each time ± 4 cm⁻¹, abbreviated as '{drug3i}'; to a crystalline sodium anhydrate salt of (R)-3-[6-amino-pyridin-3-yl]-2-(1-cyclohexyl-1 H-imidazol-4-yl)-propionic acid wherein the crystalline salt exhibits in an X-ray powder diffraction pattern measured in transmission with CuKal radiation at 110°C a characteristic reflection at degrees 2 theta of 21.4, 16.8 and 6.1, each time ± 0.2 degrees 2 theta, abbreviated as '{dntg3j}'; to a crystalline sodium anhydrate salt of (R)-3-[6-amino-pyridin-3-yl]-2-(1-cyclohexyl-1H-imidazol-4-yl)-propionic acid wherein the crystalline salt exhibits in an X-ray powder diffraction pattern measured in transmission with CuKal radiation at 110°C a characteristic reflection at degrees 2 theta of 22.4, 21.4, 16.8, 16.4, 8.6 and 6.1, each time ± 0.2 degrees 2 theta, abbreviated as '{drug3k}'; to a crystalline sodium anhydrate salt of (R)-3-[6-amino-pyridin-3-yl]-2-(1-cyclohexyl-1H-imidazol-4-yl)-propionic acid wherein the crystalline salt exhibits in an X-ray powder diffraction pattern measured in transmission with CuKal radiation at 110°C a characteristic reflection at degrees 2 theta of 22.4, 21.4, 18.7, 18.4, 18.2, 18.0, 17.5, 16.8, 16.4, 14.0, 8.6 and 6.1, each time ± 0.2 degrees 2 theta, abbreviated as '{drug31}'; to a crystalline sodium anhydrate salt of (R)-3-[6-amino-pyridin-3-yl]-2-(1-cyclohexyl-1 H-imidazol-4-yl)-propionic acid characterized by its Raman spectrum having the following values as characteristic wave number of the Raman shift (cm⁻¹) and the relative intensities shown in brackets: 1604 (46), 1552 (43), 1447 (43), 1281 (46), 1262 (43), 861 (100), and 818 (49), each time ± 4 cm⁻¹, abbreviated as '{drug3m}'; to a crystalline sodium anhydrate salt of (R)-3-[6-amino-pyridin-3-yl]-2-(1-cyclohexyl-1 H-imidazol-4-yl)-propionic acid characterized by its Raman spectrum having the following values as characteristic wave number of the Raman shift (cm⁻¹) and the relative intensities shown in brackets: 1604 (46), 1552 (43), 1447 (43), 1365 (31), 1349 (34), 1281 (46), 1262 (43), 1051 (37), 861 (100), and 818 (49), each time ± 4 cm⁻¹, abbreviated as '{drug3n}'; and to a crystalline sodium anhydrate salt of (R)-3-[6-amino-pyridin-3-yl]-2-(1-cyclohexyl-1H-imidazol-4-yl)-propionic acid characterized by its Raman spectrum having the following values (cm⁻¹) and the relative intensities are shown in brackets: 1604 (46), 1552 (43), 1502 (9), 1447 (43), 1398 (24), 1365 (31), 1349 (34), 1328 (24), 1302 (25), 1281 (46), 1262 (43), 1216 (22), 1189 (10), 1174 (9), 1156 (10), 1140 (16), 1074 (11), 1051 (37), 1029 (25), 1003, (13), 976 (17), 964 (12), 920 (6), 895 (10), 861 (100), 818 (49), 791 (6), 759 (10), 731 (6), 702 (4), 682 (5), 648 (19), 570 (3), 531 (6), 512 (4), 486 (9), 447 (9), 418 (10), 402 (7), each time ± 4 cm⁻¹, abbreviated as '{drug3o}'. {drug3a}, {drug3b}, {drug3c}, {drug3d}, {drug3e}, {drug3f}, {drug3g}, {drug3h}, {drug3i}, {drug3j}, {drug3k}, {drug31}, {drug3m}, {drug3n}, and {drug3o} are specific forms falling within the definition of {drug3}. Aspect (iii) of the invention is separate from aspects (i), (ii), (iv), (v), and (vi) of the invention. As aspects (iii) and (iv) relate to the same parent compound, {drug3} and {drug4}, respectively, these aspects are related to one another. Thus, all embodiments of {drug3a}, {drug3b}, {drug3c}, {drug3d}, {drug3e}, {drug3f}, {drug3g}, {drug3h}, {drug3i}, {drug3j}, {drug3k}, {drug31}, {drug3m}, {drug3n}, and {drug3o}, respectively, are only related to {drug3} and in part also to {drug4}, but neither to {drug1}, nor to {drug2}, nor to {drug5}, nor to {drug6}.
Aspect (iv) of the present invention relates to sodium salt of (R)-3-[6-amino-pyridin-3-yl]-2-(1-cyclohexyl-1H-imidazol-4-yl)-propionic acid according to Formula (IV): 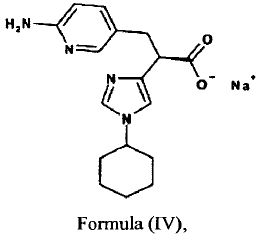 abbreviated as '{drug4}'. In particular, the present invention relates to a sodium dihydrate salt of (R)-3-[6-amino-pyridin-3-yl]-2-(1-cyclohexyl-1 H-imidazol-4-yl)-propionic acid, which is in a crystalline form or in at least partially crystalline form, abbreviated as '{drug4a}'; to a sodium salt of (R)-3-[6-amino-pyridin-3-yl]-2-(1-cyclohexyl-1H-imidazol-4-yl)-propionic acid according to Formula (IV"), 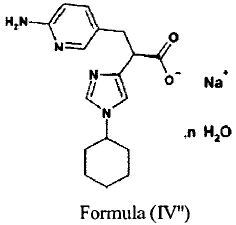 wherein n has the value wherein n has the value from 1.5 to 2.8, or from 1.8 to 2.3, or from 2.0 to 2.2, abbreviated as '{drug4b}'; to a crystalline sodium salt of (R)-3-[6-amino-pyridin-3-yl]-2-(1-cyclohexyl-1H-imidazol-4-yl)-propionic acid wherein the crystalline salt exhibits in an X-ray powder diffraction pattern measured in transmission with CuKcH radiation at room temperature a characteristic reflection at degrees 2 theta of 20.0, 14.6 and 8.6, each time ± 0.2 degrees 2 theta, abbreviated as '{drug4c}'; to a crystalline sodium salt of (R)-3-[6-amino-pyridin-3-yl]-2-(1-cyclohexyl-1H-imidazol-4-yl)-propionic acid wherein the crystalline salt exhibits in an X-ray powder diffraction pattern measured in transmission with CuKal radiation at room temperature a characteristic reflection at degrees 2 theta of 20.0, 19.2, 18.8, 17.8, 14.6 and 8.6, each time ± 0.2 degrees 2 theta, abbreviated as '{drug4d}'; to a crystalline sodium salt of (R)-3-[6-amino-pyridin-3-yl]-2-(1-cyclohexyl-1H-imidazol-4-yl)-propionic acid wherein the crystalline salt exhibits in an X-ray powder diffraction pattern measured in transmission with CuKcH radiation at room temperature a characteristic reflection at degrees 2 theta of 32.5, 23.9, 20.0, 19.2, 18.8, 17.8, 16.1, 15.6, 14.6 and 8.6, each time ± 0.2 degrees 2 theta, abbreviated as '{drug4e}'; to a crystalline sodium dihydrate salt of (R)-3-[6-amino-pyridin-3-yl]-2-(1-cyclohexyl-1H-imidazol-4-yl)-propionic acid characterized by its Raman spectrum having the following values (cm⁻¹) as characteristic wave number of the Raman shift and the relative intensities are shown in brackets: 1565 (75), 1328 (52), 1270 (57), 1054 (53), 858 (100), and 819 (51), each time ± 4 cm⁻¹, abbreviated as '{drug4f}'; to a crystalline sodium dihydrate salt of (R)-3-[6-amino-pyridin-3-yl]-2-(1-cyclohexyl-1 H-imidazol-4-yl)-propionic acid characterized by its Raman spectrum having the following values (cm⁻¹) as characteristic wave number of the Raman shift and the relative intensities are shown in brackets: 1565 (75), 1328 (52), 1304 (31), 1270 (57), 1054 (53), 1032 (34), 951 (34), 858 (100), and 819 (51), each time ± 4 cm⁻¹, abbreviated as '{drug4g}'; and to a crystalline sodium dihydrate salt of (R)-3-[6-amino-pyridin-3-yl]-2-(1-cyclohexyl-1H-imidazol-4-yl)-propionic acid characterized by its Raman spectrum having the following values (cm⁻¹) as characteristic wave number of the Raman shift and the relative intensities are shown in brackets: 1635 (23), 1616 (27), 1565 (75), 1504 (8), 1450 (24), 1440 (23), 1399 (19), 1383 (13), 1367 (15), 1355 (16), 1328 (52), 1304 (31), 1296 (21), 1287 (21), 1270 (57), 1252 (12), 1221 (24), 1211 (13), 1185 (10), 1162 (8), 1138 (18), 1083 (7), 1054 (53), 1032 (34), 1003 (12), 973 (5), 951 (34), 938 (14), 916 (14) 898 (3), 858 (100), 819 (51), 790 (5), 749 (15), 714(10), 691 (3), 656 (25), 616 (3), 577 (2), 538 (2), 510 (5), 469 (11), 447 (11), and 427 (12), each time ± 4 cm⁻¹, abbreviated as '{drug4h}'. {drug4a}, {drug4b}, {drug4c}, {drug4d}, {drug4e}, {drug4f}, {drug4g}, and {drug4h} are specific forms falling within the definition of {drug4}. Aspect (iv) of the invention is separate from aspects (i), (ii), (iii), (v), and (vi) of the invention. As aspects (iii) and (iv) relate to the same parent compound, {drug3} and {drug4}, respectively, these aspects are related to one another. Thus, all embodiments of {drug4a}, {drug4b}, {drug4c}, {drug4d}, {drug4e}, {drug4f}, {drug4g}, and {drug4h}, respectively, are only related to {drug4} and in part also to {drug3}, but neither to {drug1}, nor to {drug2}, nor to {drug5}, nor to {drug6}.
Aspect (v) of the present invention relates to 2-(2-Methylamino-pyrimidin-4-yl)-1H-indole-5-carboxylic acid [(S)-1-carbamoyl-2-(phenyl-pyrimidin-2-yl-amino)-ethyl]-amide of the formula (V), 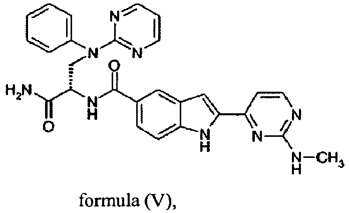 abbreviated as '{drug5}'. In particular, the present invention relates to polymorph of 2-(2-Methylamino-pyrimidin-4-yl)-1 H-indole-5-carboxylic acid [(S)-1-carbamoyl-2-(phenyl-pyrimidin-2-yl-amino)-ethyl]-amide, abbreviated as '{drug5a}'; to polymorph 1 ({drug5b}) of 2-(2-methylamino-pyrimidin-4-yl)-1 H-indole-5-carboxylic acid [(S)-1-carbamoyl-2-(phenyl-pyrimidin-2-yl-amino)-ethyl]-amide which has characteristic reflections in an X-ray powder d iff ractog ram using CuKal (CuK-alphal) radiation in reflection mode at 2Theta (20) angles in degree [°] of 14.9 ± 0.2, 19.4 ± 0.2, 19.7 ± 0.2, 20.0 ± 0.2, 22.3 ± 0.2, 25.0 ± 0.2; preferably of 10.4 ± 0.2, 14.9 ± 0.2, 17.5° ± 0.2,18.0 ± 0.2, 19.4 ± 0.2, 19.7 ± 0.2, 20.0 ± 0.2, 21.0 ± 0.2, 22.3 ± 0.2, 25.0 ± 0.2, abbreviated as '{drug5b}'; to polymorph 2 ({drug5c}) of 2-(2-methylamino-pyrimidin-4-yl)-1 H-indole-5-carboxylic acid [(S)-1-carbamoyl-2-(phenyl-pyrimidin-2-yl-amino)-ethyl]-amide which has characteristic reflections in an X-ray powder diffractogram using CuKal (CuK-alphal) radiation in reflection mode at 2Theta (20) angles in degree [°] of 5.8 ± 0.2,6.7 ± 0.2, 9.3 ± 0.2, 11.2 ± 0.2, 19.4 ± 0.2, 22.1 ± 0.2; preferably of 5.8 ± 0.2, 6.7 ± 0.2, 9.3 ± 0.2, 9.9 ± 0.2, 11.2 ± 0.2, 16.5 ± 0.2, 18.1 ± 0.2, 19.4 ± 0.2, 22.1 ± 0.2, abbreviated as '{drug5c}'; to polymorph 3 ({drug5d}) of 2-(2-methylamino-pyrimidin-4-yl)-1 H-indole-5-carboxylic acid [(S)-1-carbamoyl-2-(phenyl-pyrimidin-2-yl-amino)-ethyl]-amide which has characteristic reflections in an X-ray powder diffractogram using CuKal (CuK-alphal) radiation in reflection mode at 2Theta (20) angles in degree [°] of 15.2 ± 0.2, 15.9 ± 0.2, 17.3 ± 0.2, 19.2 ± 0.2, 22.2 ± 0.2, 25.3 ± 0.2; preferably of 13.9 ± 0.2, 15.2 ± 0.2, 15.9 ± 0.2, 17.3 ± 0.2, 19.2 ± 0.2, 22.2 ± 0.2, 24.5 ± 0.2, 25.3 ± 0.2, abbreviated as '{drug5d}'; to hydrate 1 ({drug5e}) of 2-(2-methylamino-pyrimidin-4-yl)-1 H-indole-5-carboxylic acid [(S)-1-carbamoyl-2-(phenyl-pyrimidin-2-yl-amino)-ethyl]-amide which has characteristic reflections in an X-ray powder diffractogram using CuKal (CuK-alpha1) radiation in reflection mode at 2Theta (20) angles in degree [°] of 5.3 ± 0.2, 13.5 ± 0.2, 17.9 ± 0.2, 19.5 ± 0.2, 21.5 ± 0.2, 24.9 ±0.2; preferably of 5.3 ± 0.2, 11.1 ± 0.2, 13.5 ± 0.2, 17.9 ± 0.2, 19.5 ± 0.2, 21.5 ± 0.2, 23.5 ± 0.2, 24.9 ± 0.2, 28.2 ± 0.2, abbreviated as '{drug5e}'; and to hydrate 2 ({drug5f}) of 2-(2-methylamino-pyrimidin-4-yl)-1 H-indole-5-carboxylic acid [(S)-1-carbamoyl-2-(phenyl-pyrimidin-2-yl-amino)-ethyl]-amide which has characteristic reflections in an X-ray powder diffractogram using CuKal (CuK-alpha1) radiation in reflection mode at 2Theta (20) angles in degree [°] of 2.9 ± 0.2, 5.3 ± 0.2, 8.3 ± 0.2, 11.5 ± 0.2, 17.1 ± 0.2, 22.8 ± 0.2; preferably of 2.9 ± 0.2, 5.3 ± 0.2, 6.2 ± 0.2, 8.3 ± 0.2, 9.5 ± 0.2, 11.5 ± 0.2, 14.0 ± 0.2, 17.1 ± 0.2, 18.0 ± 0.2, 22.8 ± 0.2, abbreviated as '{drug5f}'. {drug5a}, {drug5b}, {drug5c}, {drug5d}, {drug5e}, and {drug5f} are specific forms falling within the definition of {drug5}. Aspect (v) of the invention is separate from aspects (i), (ii), (iii), (iv), and (vi) of the invention. Thus, all embodiments of {drug5a}, {drug5b}, {drug5c}, {drug5d}, {drug5e}, and {drug5f}, respectively, are only related to {drug5}, but neither to {drug1}, nor to {drug2}, nor to {drug3}, nor to {drug4}, nor to {drug6}.
Aspect (vi) of the present invention relates to (2S)-2-amino-3-hydroxy-N-[2-methoxy-2-[(1Z)-2-(3,4,5-trimethoxyphenyl)ethenyl] phenyl] propanamide of formula (VI) 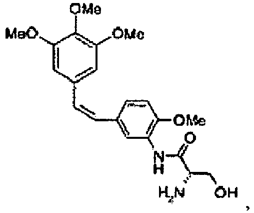 abbreviated as '{drug6}'. In particular, the present invention relates to crystal form I of (2S)-2-amino-3-hydroxy-N-[2-methoxy-2-[(1Z)-2-(3,4,5-trimethoxyphenyl)ethenyl] phenyl] propanamide, preferably having at least one of the following characteristics: (a) an X-ray (λCu) powder diffraction pattern with peaks at about 3.6, 7.1, 10.5, 11.2, 12.2 and 17.9 ± 0.2 degrees 2-theta; and/or (b) a melting point (onset) around 104°C, abbreviated as '{drug6a}'. {drug6a} is a specific form falling within the definition of {drug6}. Aspect (vi) of the invention is separate from aspects (i), (ii), (iii), (iv), and (v) of the invention. Thus, all embodiments of {drug6a} are only related to {drug6}, but neither to {drug1}, nor to {drug2}, nor to {drug3}, nor to {drug4}, nor to {drug5}.

Summarizing therefore the following abbreviations have the following meanings:
Aspect i :
   - {drug1} =: Boceprevir of formula (I) 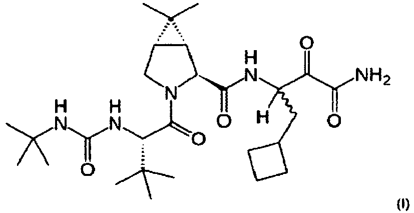 or a physiologically acceptable salt thereof, for use in the treatment of congestive heart failure, myocardial infarction, edema, diabetic complications, diuretic;
   - {drug1a} =: solid form of Boceprevir or a physiologically acceptable salt thereof for use in the treatment of congestive heart failure, myocardial infarction, edema, diabetic complications, diuretic.
Aspect (ii):
   - {drug2} =: naphthalene-1,5-disulfonic acid salt of (R)-3-(6-amino-pyridin-3-yl)-2-(1-cyclohexyl-1 H-imidazol-4-yl)-propionic acid-ethylester according to formula **(II)** 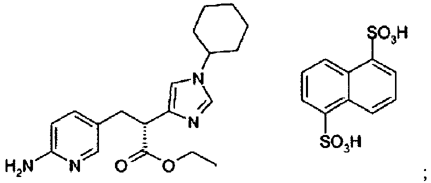
   - {drug2a} =: crystalline form of naphthalene-1,5-disulfonic acid salt of (R)-3-(6-amino-pyridin-3-yl)-2-(1-cyclohexyl-1H-imidazol-4-yl)-propionic acide-ethylester;
   - {drug2b} =: crystalline salt of naphthalene-1,5-disulfonic acid with (R)-3-(6-amino-pyridin-3-yl)-2-(1-cyclohexyl-1H-imidazol-4-yl)-propionic acid ethyl ester of formula (II), characterized in that the measured with CuKa radiation having a XRPD reflection intensity at a high angle position of 22.48 degrees 2-theta ± 0.2 degrees 2 theta;
   - {drug2c} =: crystalline salt of naphthalene-1,5-disulfonic acid with (R)-3-(6-amino-pyridin-3-yl)-2-(1-cyclohexyl-1 H-imidazol-4-yl)-propionic acid ethyl ester of formula (II), characterized in that the measured with CuKa radiation having XRPD peaks at the following 2-theta values at the angular positions: 7.35 and 22.48 respectively ± 0.2 degrees 2 theta;
   - {drug2d} =: crystalline salt of naphthalene-1,5-disulfonic acid with (R)-3-(6-amino-pyridin-3-yl)-2-(1-cyclohexyl-1H-imidazol-4-yl)-propionic acid ethyl ester of formula (II), characterized in that the measured with CuKa radiation having XRPD peaks at the following 2-theta values at the angular positions: 7.35; 11.4; 13.69; 14.96; 17.49; 19.3 and 22.48, respectively ± 0.2 degrees 2 theta; and
   - {drug2e} =: crystalline salt of naphthalene-1,5-disulfonic acid with (R)-3-(6-amino-pyridin-3-yl)-2-(1-cyclohexyl-1 H-imidazol-4-yl)-propionic acid ethyl ester of formula (II) characterized by a Raman spectrum comprising characteristic bands at the following wavelengths [cm⁻¹]: 2936; 1570; 1401; 1352; 998 and 856, respectively ± 2 cm⁻¹.
Aspect (iii):
   - {drug3} =: sodium salt of (R)-3-[6-amino-pyridin-3-yl]-2-(1-cyclohexyl-1H-imidazol-4-yl)-propionic acid according to Formula (III): 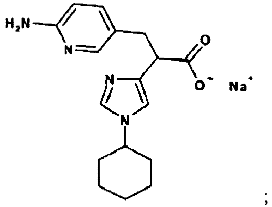
   - {dmg3a} =: crystalline form of sodium anhydrate salt of (R)-3-[6-amino-pyridin-3-yl]-2-(1-cyclohexyl-1H-imidazol-4-yl)-propionic acid;
   - {drug3b} =: monohydrate salt of (R)-3-[6-amino-pyridin-3-yl]-2-(1-cyclohexyl-1H-imidazol-4-yl)-propionic acid;
   - {drug3c} =: sodium salt of (R)-3-[6-amino-pyridin-3-yl]-2-(1-cyclohexyl-1H-imidazol-4-yl)-propionic acid of Formula (III") as a hydrate 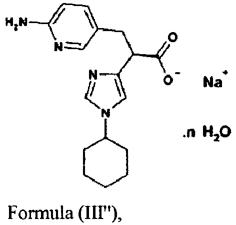 wherein n has the value from 0.7 to 1.4, or from 0.8 to 1.2, or from 0.9 to 1.1;
   - {drug3d}: = crystalline sodium monohydrate salt of (R)-3-[6-amino-pyridin-3-yl]-2-(-cyclohexyl-1H-imidazol-4-yl)-propionic acid wherein the crystalline salt exhibits in an X-ray powder diffraction pattern measured in transmission with CuKal radiation at room temperature a characteristic reflection at degrees 2 theta of 22.2, 16.5 and 6.3, each time ± 0.2 degrees 2 theta;
   - {drug3e}: = crystalline sodium monohydrate salt of (R)-3-[6-amino-pyridin-3-yl]-2-(1-cyclohexyl-1H-imidazol-4-yl)-propionic acid wherein the crystalline salt exhibits in an X-ray powder diffraction pattern measured in transmission with CuKal radiation at room temperature a characteristic reflection at degrees 2 theta of 22.2, 21.8, 18.1, 16.5, 8.2 and 6.3, each time ± 0.2 degrees 2 theta;
   - {drug3f}: = crystalline sodium monohydrate salt of (R)-3-[6-amino-pyridin-3-yl]-2-(1-cyclohexyl-1H-imidazol-4-yl)-propionic acid wherein the crystalline salt exhibits in an X-ray powder diffraction pattern measured in transmission with CuKal radiation at room temperature a characteristic reflection at degrees 2 theta of 24.0, 22.2,21.8, 18.8, 18.1, 16.8, 16.5, 16.0, 8.2 and 6.3, each time ± 0.2 degrees 2 theta;
   - {drug3g}: = crystalline sodium monohydrate salt of (R)-3-[6-amino-pyridin-3-yl]-2-(1-cyclohexyl-1H-imidazol-4-yl)-propionic acid characterized by its Raman spectrum having the following values as characteristic wave number of the Raman shift (cm⁻¹) and the relative intensities shown in brackets: 818 (31), 864 (100), 1054 (24), 1284 (26), 1561 (37), and 1613 (27), each time ± 4 cm⁻¹];
   - {drug3h}: = crystalline sodium monohydrate salt of (R)-3-[6-amino-pyridin-3-yl]-2-(1-cyclohexyl-1H-imidazol-4-yl)-propionic acid characterized by its Raman spectrum having the following values as characteristic wave number of the Raman shift (cm⁻¹) and the relative intensities shown in brackets: 657 (10), 818 (31), 864 (100), 981 (15), 1030 (18), 1054 (24), 1137 (10), 1216 (21), 1266 (20), 1284 (26), 1309 (16), 1351 (10), 1363 (19), 1407 (16), 1445 (20), 1561 (37) and 1613 (27), each time ± 4 cm⁻¹;
   - {drug3i}: = crystalline sodium monohydrate salt of (R)-3-[6-amino-pyridin-3-yl]-2-(1-cyclohexyl-1H-imidazol-4-yl)-propionic acid characterized by its Raman spectrum having the following values (cm⁻¹) and the relative intensities shown in brackets: 431 (3), 484 (3), 539 (2), 566 (6), 657 (10), 759 (8), 798 (2), 818 (31), 864 (100), 882 (8), 920 (6), 968 (9), 981 (15), 999 (3), 1030 (18), 1054 (24), 1077 (6), 1137 (10), 1186 (8), 1202 (6), 1216 (21), 1242 (4), 1266 (20), 1284 (26), 1309 (16), 1351 (10), 1363 (19), 1407 (16), 1445 (20), 1495 (3), 1561 (37), 1613 (27), each time ± 4 cm⁻¹;
   - {drug3j} =: crystalline sodium anhydrate salt of (R)-3-[6-amino-pyridin-3-yl]-2-(1-cyclohexyl-1H-imidazol-4-yl)-propionic acid wherein the crystalline salt exhibits in an X-ray powder diffraction pattern measured in transmission with CuKal radiation at 110°C a characteristic reflection at degrees 2 theta of 21.4, 16.8 and 6.1, each time ± 0.2 degrees 2 theta;
   - {drug3k} =: crystalline sodium anhydrate salt of (R)-3-[6-amino-pyridin-3-yl]-2-(1-cyclohexyl-1H-imidazol-4-yl)-propionic acid wherein the crystalline salt exhibits in an X-ray powder diffraction pattern measured in transmission with CuKal radiation at 110°C a characteristic reflection at degrees 2 theta of 22.4, 21.4, 16.8, 16.4, 8.6 and 6.1, each time ± 0.2 degrees 2 theta;
   - {drug31} =: crystalline sodium anhydrate salt of (R)-3-[6-amino-pyridin-3-yl]-2-(1-cyclohexyl-1H-imidazol-4-yl)-propionic acid wherein the crystalline salt exhibits in an X-ray powder diffraction pattern measured in transmission with CuKal radiation at 110°C a characteristic reflection at degrees 2 theta of 22.4, 21.4, 18.7, 18.4, 18.2, 18.0, 17.5, 16.8, 16.4, 14.0, 8.6 and 6.1, each time ± 0.2 degrees 2 theta;
   - {drug3m} =: crystalline sodium anhydrate salt of (R)-3-[6-amino-pyridin-3-yl]-2-(1-cyclohexyl-1H-imidazol-4-yl)-propionic acid characterized by its Raman spectrum having the following values as characteristic wave number of the Raman shift (cm⁻¹) and the relative intensities shown in brackets: 1604 (46), 1552 (43), 1447 (43), 1281 (46), 1262 (43), 861 (100), and 818 (49), each time ± 4 cm⁻¹;
   - {drug3n} =: crystalline sodium anhydrate salt of (R)-3-[6-amino-pyridin-3-yl]-2-(1-cyclohexyl-1H-imidazol-4-yl)-propionic acid characterized by its Raman spectrum having the following values as characteristic wave number of the Raman shift (cm⁻¹) and the relative intensities shown in brackets: 1604 (46), 1552 (43), 1447 (43), 1365 (31), 1349 (34), 1281 (46), 1262 (43), 1051 (37), 861 (100), and 818 (49), each time ± 4 cm⁻¹; and
   - {drug3o} =: crystalline sodium anhydrate salt of (R)-3-[6-amino-pyridin-3-yl]-2-(1-cyclohexyl-1H-imidazol-4-yl)-propionic acid characterized by its Raman spectrum having the following values (cm⁻¹) and the relative intensities are shown in brackets: 1604 (46), 1552 (43), 1502 (9), 1447 (43), 1398 (24), 1365 (31), 1349 (34), 1328 (24), 1302 (25), 1281 (46), 1262 (43), 1216 (22), 1189 (10), 1174 (9), 1156 (10), 1140 (16), 1074 (11), 1051 (37), 1029 (25), 1003, (13), 976 (17), 964 (12), 920 (6), 895 (10), 861 (100), 818 (49), 791 (6), 759 (10), 731 (6), 702 (4), 682 (5), 648 (19), 570 (3), 531 (6), 512 (4), 486 (9), 447 (9), 418 (10), 402 (7), each time ± 4 cm⁻¹.
Aspect (iv):
   - {drug4} =: sodium salt of (R)-3-[6-amino-pyridin-3-yl]-2-(1-cyclohexyl-1H-imidazol-4-yl)-propionic acid according to Formula (IV): 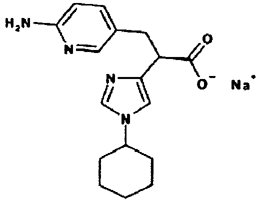
   - {drug4a} =: sodium dihydrate salt of (R)-3-[6-amino-pyridin-3-yl]-2-(1-cyclohexyl-1H-imidazol-4-yl)-propionic acid, which is in a crystalline form or in at least partially crystalline form;
   - {drug4b} =: sodium salt of (R)-3-[6-amino-pyridin-3-yl]-2-(1-cyclohexyl-1H-imidazol-4-yl)-propionic acid according to Formula (IV"), 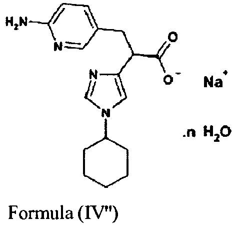 wherein n has the value wherein n has the value from 1.5 to 2.8, or from 1.8 to 2.3, or from 2.0 to 2.2;
   - {drug4c} =: crystalline sodium salt of (R)-3-[6-amino-pyridin-3-yl]-2-(1-cyclohexyl-1H-imidazol-4-yl)-propionic acid wherein the crystalline salt exhibits in an X-ray powder diffraction pattern measured in transmission with CuKcH radiation at room temperature a characteristic reflection at degrees 2 theta of 20.0, 14.6 and 8.6, each time ± 0.2 degrees 2 theta;
   - {drug4d} =: crystalline sodium salt of (R)-3-[6-amino-pyridin-3-yl]-2-(1-cyclohexyl-1H-imidazol-4-yl)-propionic acid wherein the crystalline salt exhibits in an X-ray powder diffraction pattern measured in transmission with CuKcH radiation at room temperature a characteristic reflection at degrees 2 theta of 20.0, 19.2, 18.8, 17.8, 14.6 and 8.6, each time ± 0.2 degrees 2 theta;
   - {drug4e} =: crystalline sodium salt of (R)-3-[6-amino-pyridin-3-yl]-2-(1H-imidazol-4-yl)-propionic acid wherein the crystalline salt exhibits in an X-ray powder diffraction pattern measured in transmission with CuKcH radiation at room temperature a characteristic reflection at degrees 2 theta of 32.5, 23.9, 20.0, 19.2, 18.8, 17.8, 16.1, 15.6, 14.6 and 8.6, each time ± 0.2 degrees 2 theta;
   - {drug4f} =: crystalline sodium dihydrate salt of (R)-3-[6-amino-pyridin-3-yl]-2-(1-cyclohexyl-1H-imidazol-4-yl)-propionic acid characterized by its Raman spectrum having the following values (cm⁻¹) as characteristic wave number of the Raman shift and the relative intensities are shown in brackets: 1565 (75), 1328 (52), 1270 (57), 1054 (53), 858 (100), and 819 (51), each time ± 4 cm⁻¹;
   - {drug4g} =: crystalline sodium dihydrate salt of (R)-3-[6-amino-pyridin-3-yl]-2-(1-cyclohexyl-1H-imidazol-4-yl)-propionic acid characterized by its Raman spectrum having the following values (cm⁻¹) as characteristic wave number of the Raman shift and the relative intensities are shown in brackets: 1565 (75), 1328 (52), 1304 (31), 1270 (57), 1054 (53), 1032 (34), 951 (34), 858 (100), and 819 (51), each time ± 4 cm⁻¹; and
   - {drug4h} =: crystalline sodium dihydrate salt of (R)-3-[6-amino-pyridin-3-yl]-2-(1-cyclohexyl-1H-imidazol-4-yl)-propionic acid characterized by its Raman spectrum having the following values (cm⁻¹) as characteristic wave number of the Raman shift and the relative intensities are shown in brackets: 1635 (23), 1616 (27), 1565 (75), 1504 (8), 1450 (24), 1440 (23), 1399 (19), 1383 (13), 1367 (15), 1355 (16), 1328 (52), 1304 (31), 1296 (21), 1287 (21), 1270 (57), 1252 (12), 1221 (24), 1211 (13), 1185 (10), 1162 (8), 1138 (18), 1083 (7), 1054 (53), 1032 (34), 1003 (12), 973 (5), 951 (34), 938 (14), 916 (11) 898 (3), 858 (100), 819 (51), 790 (5), 749 (15), 714 (10), 691 (3), 656 (25), 616 (3), 577 (2), 538 (2), 510 (5), 469 (11), 447 (11), and 427 (12), each time ± 4 cm⁻¹.
Aspect (v):
   - {drug5} =: 2-(2-Methylamino-pyrimidin-4-yl)-1H-indole-5-carboxylic acid [(S)-1-carbamoyl-2-(phenyl-pyrimidin-2-yl-amino)-ethyl]-amide of the formula (V), 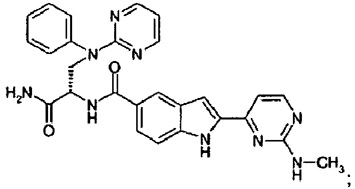
   - {drug5a} =: polymorph of 2-(2-Methylamino-pyrimidin-4-yl)-1H-indole-5-carboxylic acid [(S)-1-carbamoyl-2-(phenyl-pyrimidin-2-yl-amino)-ethyl]-amide;
   - {drug5b} =: polymorph 1 of 2-(2-methylamino-pyrimidin-4-yl)-1H-indole-5-carboxylic acid [(S)-1-carbamoyl-2-(phenyl-pyrimidin-2-yl-amino)-ethyl]-amide which has characteristic reflections in an X-ray powder d iff ractog ram using CuKal (CuK-alphal) radiation in reflection mode at 2Theta (20) angles in degree [°] of 14.9 ± 0.2, 19.4 ± 0.2, 19.7 ± 0.2, 20.0 ± 0.2, 22.3 ± 0.2, 25.0 ± 0.2; preferably of 10.4 ± 0.2, 14.9 ± 0.2, 17.5° ± 0.2, 18.0 ± 0.2, 19.4 ± 0.2, 19.7 ± 0.2, 20.0 ± 0.2, 21.0 ± 0.2, 22.3 ± 0.2, 25.0 ± 0.2;
   - {drug5c} =: polymorph 2 of 2-(2-methylamino-pyrimidin-4-yl)-1 H-indole-5-carboxylic acid [(S)-1-carbamoyl-2-(phenyl-pyrimidin-2-yl-amino)-ethyl]-amide which has characteristic reflections in an X-ray powder diffractogram using CuKal (CuK-alphal) radiation in reflection mode at 2Theta (20) angles in degree [°] of 5.8 ± 0.2, 6.7 ± 0.2, 9.3 ± 0.2, 11.2 ± 0.2, 19.4 ± 0.2, 22.1 ± 0.2; preferably of 5.8 ± 0.2, 6.7 ± 0.2, 9.3 ± 0.2, 9.9 ± 0.2, 11.2 ± 0.2, 16.5 ± 0.2, 18.1 ± 0.2, 19.4 ± 0.2, 22.1 ± 0.2;
   - {drug5d} =: polymorph 3 of 2-(2-methylamino-pyrimidin-4-yl)-1H-indole-5-carboxylic acid [(S)-1-carbamoyl-2-(phenyl-pyrimidin-2-yl-amino)-ethyl]-amide which has characteristic reflections in an X-ray powder diffractogram using CuKal (CuK-alpha1) radiation in reflection mode at 2Theta (20) angles in degree [°] of 15.2 ± 0.2, 15.9 ± 0.2, 17.3 ± 0.2.19.2 ± 0.2. 22.2 ± 0.2, 25.3 ± 0.2; preferably of 13.9 ± 0.2, 15.2 ± 0.2, 15.9 ± 0.2, 17.3 ± 0.2, 19.2 ± 0.2, 22.2 ± 0.2,24.5 ± 0.2, 25.3 ± 0.2;
   - {drug5e} =: hydrate 1 of 2-(2-methylamino-pyrimidin-4-yl)-1 H-indole-5-carboxylic acid [(S)-1-carbamoyl-2-(phenyl-pyrimidin-2-yl-amino)-ethyl]-amide which has characteristic reflections in an X-ray powder diffractogram using CuKal (CuK-alphal) radiation in reflection mode at 2Theta (20) angles in degree [°] of 5.3 ± 0.2, 13.5 ± 0.2, 17.9 ± 0.2, 19.5 ± 0.2, 21.5 ± 0.2, 24.9 ± 0.2; preferably of 5.3 ± 0.2, 11.1 ± 0.2, 13.5 ± 0.2, 17.9 ± 0.2, 19.5 ± 0.2, 21.5 ± 0.2, 23.5 ± 0.2, 24.9 ± 0.2, 28.2 ± 0.2;
   - {drug5f} =: hydrate 1 of 2-(2-methylamino-pyrimidin-4-yl)-1H-indole-5-carboxylic acid [(S)-1-carbamoyl-2-(phenyl-pyrimidin-2-yl-amino)-ethyl]-amide which has characteristic reflections in an X-ray powder diffractogram using CuKal (CuK-alphal) radiation in reflection mode at 2Theta (20) angles in degree [°] of 2.9 ± 0.2, 5.3 ± 0.2, 8.3 ± 0.2, 11.5 ± 0.2, 17.1 ± 0.2, 22.8 ± 0.2; preferably of 2.9 ± 0.2, 5.3 ± 0.2, 6.2 ± 0.2, 8.3 ± 0.2, 9.5 ± 0.2, 11.5 ± 0.2, 14.0 ± 0.2, 17.1 ± 0.2, 18.0 ± 0.2, 22.8 ± 0.;
Aspect (vi):
   - {drug6} =: (2S)-2-amino-3-hydroxy-N-[2-methoxy-2-[(1Z)-2-(3,4,5-trimethoxyphenyl)ethenyl] phenyl] propanamide of formula (VI) 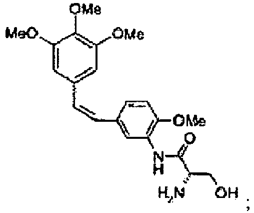 and
   - {drug6a} =: crystal form I of (2S)-2-amino-3-hydroxy-N-[2-methoxy-2-[(1Z)-2-(3,4,5-trimethoxyphenyl)ethenyl] phenyl] propanamide, preferably having at least one of the following characteristics: (a) an X-ray (λCu) powder diffraction pattern with peaks at about 3.6, 7.1, 10.5, 11.2, 12.2 and 17.9 ±0.2 degrees 2-theta; and/or (b) a melting point (onset) around 104°C.

### SPECIFIC INORMATION CONCERNING ASPECT (I) OF THE INVENTION

Aspect (i) of the invention relates to an administration unit comprising a compound of the formula (I) for use in the treatment of congestive heart failure, myocardial infarction, edema, diabetic complications, diuretic ({drug1}) and/or to a solid form of a compound of the formula (I) for use in the treatment of congestive heart failure, myocardial infarction, edema, diabetic complications, diuretic ({drug1a}). The invention relates to a form of a compound of the formula (I) for use in the treatment of congestive heart failure, myocardial infarction, edema, diabetic complications, diuretic ({drug1}), namely to solid form of a compound of the formula (I) for use in the treatment of congestive heart failure, myocardial infarction, edema, diabetic complications, diuretic ({drug1a}), which is useful for treating congestive heart failure, myocardial infarction, edema, diabetic complications, diuretic. A compound of the formula (I) for use in the treatment of congestive heart failure, myocardial infarction, edema, diabetic complications, diuretic ({drug1}) and/or avsolid form of a compound of the formula (I) for use in the treatment of congestive heart failure, myocardial infarction, edema, diabetic complications, diuretic ({drug1a}) is described in WO2013/072327, which is incorporated by reference. A compound of the formula (I) for use in the treatment of congestive heart failure, myocardial infarction, edema, diabetic complications, diuretic ({drug1}) is described in WO2013/072327: 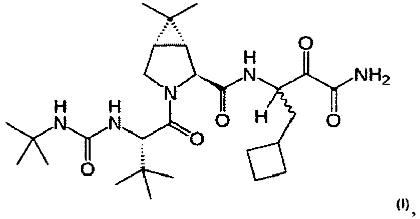 including also in any of its stereoisomeric forms or a mixture of stereoisomeric forms in any ratio or a physiologically acceptable salt thereof or a physiologically acceptable solvate. The compound of formula (I) is known as Boceprevir (IUPAC name: (1R,2S,5S)-N-[(2E)-4-amino-1-cyclobutyl-3,4-dioxobutan-2-yl)]-3-{(2S)-2-[(tert-butylcarbamoyl)amino]-3,3-dimethylbutanoyl}-6,6-dimethyl-3-azabicyclo[3.1.0]hexane-2-carboxamide) and is an inhibitor of HCV NS3/4a protease and intended for the treatment of hepatitis C virus infection. Boceprevir and related compounds and their preparation are described in WO0208244, WO03062265, US7012066, US7772178 and WO2006113942. The invention relates to an administration unit comprising a compound of the formula (I) for use in the treatment of congestive heart failure, myocardial infarction, edema, diabetic complications, diuretic ({drug1}) and/or solid form of a compound of the formula (I) for use in the treatment of congestive heart failure, myocardial infarction, edema, diabetic complications, diuretic ({drug1a}). The administration unit according to the invention is useful for treating various diseases and disorders which include but are not limited to congestive heart failure, myocardial infarction, edema, diabetic complications, diuretic. Preferred embodiments of the administration unit are a compound of the formula (I) for use in the treatment of congestive heart failure, myocardial infarction, edema, diabetic complications, diuretic ({drug1}) and solid form of a compound of the formula (I) for use in the treatment of congestive heart failure, myocardial infarction, edema, diabetic complications, diuretic ({drug1a}) are described in WO2013/072327 and are repeated hereinafter: The present invention relates to the use of Boceprevir and related compounds in atherosclerosis, heart failure, diseases, liver diseases or inflammatory diseases. The present invention relates to the use of the compounds of the formula I,

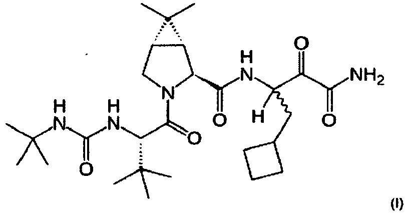

The compound of formula (I) is known as Boceprevir (IUPAC name: (1R,2S,5S)-N-[(2E)-4-amino-1-cyclobutyl-3,4-dioxobutan-2-yl)]-3-{(2S)-2-[(tert-butylcarbamoyl)amino]-3,3-dimethylbutanoyl}-6,6-dimethyl-3-azabicyclo[3.1.0]hexane-2-carboxamide) and is an inhibitor of HCV NS3/4a protease and intended for the treatment of hepatitis C virus infection. Boceprevir and related compounds and their preparation are described in WO0208244, WO03062265, US7012066, US7772178 and WO2006113942. It has been found that the compound of formula (I) showed to inhibit the protease cathepsin A, and is therefore useful for the treatment of diseases such as atherosclerosis, heart failure, renal diseases, liver diseases or inflammatory diseases, for example. Cathepsin A (EC = 3.4.16.5; gene symbol CTSA) is a protease also known as lysosomal carboxypeptidase A or protective protein. It belongs to a family of serine carboxypeptidases which contains only two other mammalian representatives, retinoid-inducible serine carboxypeptidase and vitellogenic carboxypeptidase-like protein. Within the cell cathepsin A resides in lysosomes where it forms a high molecular weight complex with beta-galactosidase and neuraminidase. The interaction of cathepsin A with these glycosidases is essential for their correct routing to the lysosome and protects them from intralysosomal proteolysis. A deficiency of cathepsin A resulting from various mutations in the ctsa gene leads to a secondary deficiency of beta-galactosidase and neuraminidase that is manifest as the autosomal recessive lysosomal storage disorder galactosialidosis (cf. A. d'Azzo et al., in "The Metabolic and Molecular Bases of Inherited Disease", vol. 2 (1995), 2835-2837). The majority of identified mutations in ctsa are missense mutations affecting the folding or the stability of the protein. None of them was shown to occur in the active site of the enzyme (G. Rudenko et al., Proc. Natl. Acad. Sci. USA 95 (1998), 621-625). Accordingly, the lysosomal storage disorder can be corrected with catalytically inactive cathepsin A mutants (N. J. Galjart et al., J. Biol. Chem. 266 (1991), 14754-14762). The structural function of cathepsin A is therefore separable from its catalytic activity. This is also underscored by the observation that in contrast to mice deficient in the ctsa gene, mice carrying a catalytically inactivating mutation in the ctsa gene do not develop signs of the human disease galactosialidosis (R. J. Rottier et al., Hum. Mol. Genet. 7 (1998), 1787-1794; V. Seyrantepe et al., Circulation 117 (2008), 1973-1981). Cathepsin A displays carboxypeptidase activity at acidic pH and deamidase and esterase activities at neutral pH against various naturally occurring bioactive peptides. In vitro studies have indicated that cathepsin A converts angiotensin I to angiotensin 1-9 and bradykinin to bradykinin 1-8, which is the ligand for the bradykinin B1 receptor. It hydrolyzes endothelin-1, neurokinin and oxytocin, and deamidates substance P (cf. M. Hiraiwa, Cell. Mol. Life Sci. 56 (1999), 894-907). High cathepsin A activity has been detected in urine, suggesting that it is responsible for tubular bradykinin degradation (M. Saito et al., Int. J. Tiss. Reac. 17 (1995), 181-190). However, the enzyme can also be released from platelets and lymphocytes and is expressed in antigen-presenting cells where it might be involved in antigen processing (W. L. Hanna et al., J. Immunol. 153 (1994), 4663-4672; H. Ostrowska, Thromb. Res. 86 (1997), 393-404; M. Reich et al., Immunol. Lett, (online Nov. 30, 2009)). Immunohistochemistry of human organs revealed prominent expression in renal tubular cells, bronchial epithelial cells, Leydig's cells of the testis and large neurons of the brain (O. Sohma et al., Pediatr. Neurol. 20 (1999), 210-214). It is upregulated during differentiation of monocytes to macrophages (N. M. Stamatos et al., FEBS J. 272 (2005), 2545-2556). Apart from structural and enzymatic functions, cathepsin A has been shown to associate with neuraminidase and an alternatively spliced beta-galactosidase to form the cell-surface laminin and elastin receptor complex expressed on fibroblasts, smooth muscle cells, chondroblasts, leukocytes and certain cancer cell types (A. Hinek, Biol. Chem. 377 (1996), 471-480). The importance of cathepsin A for the regulation of local bradykinin levels has been demonstrated in animal models of hypertension. Pharmacological inhibition of cathepsin A activity increased renal bradykinin levels and prevented the development of salt-induced hypertension (H. Ito et al., Br. J. Pharmacol. 126 (1999), 613-620). This could also be achieved by antisense oligonucleotides suppressing the expression of cathepsin A (I. Hajashi et al., Br. J. Pharmacol. 131 (2000), 820-826). Besides in hypertension, beneficial effects of bradykinin have been demonstrated in various further cardiovascular diseases and other diseases (cf. J. Chao et al., Biol. Chem. 387 (2006), 665-75; P. Madeddu et al., Nat. Clin. Pract. Nephrol. 3 (2007), 208-221). Key indications of cathepsin A inhibitors therefore include atherosclerosis, heart failure, cardiac infarction, cardiac hypertrophy, vascular hypertrophy, left ventricular dysfunction, in particular left ventricular dysfunction after myocardial infarction, renal diseases such as renal fibrosis, renal failure and kidney insufficiency; liver diseases such as liver fibrosis and liver cirrhosis, diabetes complications such as nephropathy, as well as organ protection of organs such as the heart and the kidney. As indicated above, cathepsin A inhibitors can prevent the generation of the bradykinin B1 receptor ligand bradykinin 1-8 (M. Saito et al., Int. J. Tiss. Reac. 17 (1995), 181-190). This offers the opportunity to use cathepsin A inhibitors for the treatment of pain, in particular neuropathic pain, and inflammation, as has been shown for bradykinin B1 receptor antagonists (cf. F. Marceau et al., Nat. Rev. Drug Discov. 3 (2004), 845-852). Cathepsin A inhibitors can further be used as anti-platelet agents as has been demonstrated for the cathepsin A inhibitor ebelactone B, a propiolactone derivative, which suppresses platelet aggregation in hypertensive animals (H. Ostrowska et al., J. Cardiovasc. Pharmacol. 45 (2005), 348-353). Further, like other serine proteases such as prostasin, elastase or matriptase, cathepsin A can stimulate the amiloride-sensitive epithelial sodium channel (ENaC) and is thereby involved in the regulation of fluid volumes across epithelial membranes (cf. C. Planes et al., Curr. Top. Dev. Biol. 78 (2007), 23-46). Thus, respiratory diseases can be ameliorated by the use of cathepsin A inhibitors, such as cystic fibrosis, chronic bronchitis, chronic obstructive pulmonary disease, asthma, respiratory tract infections and lung carcinoma. Cathepsin A modulation in the kidney could be used to promote diuresis and thereby induce a hypotensive effect. Besides for the above-mentioned compound ebelactone B, an inhibitory effect on cathepsin A has been found for certain dipeptidic phenylalanine derivatives which are described in JP 2005/145839. There is a need for further compounds which inhibit cathepsin A and offer an opportunity for the treatment of the mentioned diseases and further diseases in which cathepsin A plays a role. The present invention satisfies this need by providing the oxygen-substituted 3-heteroaroylamino-propionic acid derivatives of the formula (I) defined below. Certain compounds in which a 3-heteroaroylamino-propionic acid moiety can be present, have already been described. For example, in WO 2006/076202 amine derivatives, which modulate the activity of steroid nuclear receptors, are described which carry on the nitrogen atom of the amine function a heteroaroyl group and a further group which is defined very broadly. In US 2004/0072802 broadly-defined beta-amino acid derivatives are described which carry an acyl group on the beta-amino group and are inhibitors of matrix metalloproteases and/or tumor necrosis factor. In WO 2009/080226 and WO 2009/080227, which relate to antagonists of the platelet ADP receptor P2Y12 and inhibit platelet aggregation, pyrazoloylamino-substituted carboxylic acid derivatives are described which, however, additionally carry a carboxylic acid derivative group on the carbon atom carrying the pyrazoloylamino group. Other pyrazoloylamino-substituted compounds, in which the nitrogen atom of the amino group is connected to a ring system and which are inhibitors of the blood clotting enzymes factor Xa and/or factor Vila, are described in WO 2004/056815. The present invention comprises the use of all stereoisomeric forms of the compounds of the formula I, for example all enantiomers and diastereomers including cis/trans isomers. The invention likewise comprises mixtures of two or more stereoisomeric forms, for example mixtures of enantiomers and/or diastereomers including cis/trans isomers, in all ratios. Physiologically acceptable salts, including pharmaceutically utilizable salts, of the compounds of the formula (I) generally comprise a nontoxic salt component. They can contain inorganic or organic salt components. Such salts can be formed, for example, from compounds of the formula (I) which contain an acidic group, for example a carboxylic acid group (hydroxycarbonyl group, HO-C(O)-), and nontoxic inorganic or organic bases. Suitable bases are, for example, alkali metal compounds or alkaline earth metal compounds, such as sodium hydroxide, potassium hydroxide, sodium carbonate or sodium hydrogencarbonate, or ammonia, organic amino compounds and quaternary ammonium hydroxides. Reactions of compounds of the formula (I) with bases for the preparation of the salts are in general carried out according to customary procedures in a solvent or diluent. Examples of salts of acidic groups thus are sodium, potassium, magnesium or calcium salts or ammonium salts which can also carry one or more organic groups on the nitrogen atom. Compounds of the formula (I) which contain a basic, i.e. protonatable, group, for example an amino group or a basic heterocycle, can be present in the form of their acid addition salts with physiologically acceptable acids, for example as salt with hydrogen chloride, hydrogen bromide, phosphoric acid, sulfuric acid, acetic acid, benzoic acid, methanesulfonic acid, p-toluenesulfonic acid, which in general can be prepared from the compounds of the formula (I) by reaction with an acid in a solvent or diluent according to customary procedures. If the compounds of the formula (I) simultaneously contain an acidic and a basic group in the molecule, the invention also includes internal salts (betaines, zwitterions) in addition to the salt forms mentioned. The present invention also comprises all salts of the compounds of the formula (I) which, because of low physiological tolerability, are not directly suitable for use as a pharmaceutical, but are suitable as intermediates for chemical reactions or for the preparation of physiologically acceptable salts, for example by means of anion exchange or cation exchange. The present invention also comprises all solvates of the compounds of the formula (I) and their salts, including physiologically acceptable solvates, such as hydrates, i.e. adducts with water, and adducts with alcohols like (Ci-C4)-alkanols, as well as active metabolites of compounds of the formula (I) and prodrugs of the compounds of the formula I, i.e. compounds which in vitro may not necessarily exhibit pharmacological activity but which in vivo are converted into pharmacologically active compounds of the formula I, for example compounds which are converted by metabolic hydrolysis into a compound of the formula I, such as compounds in which a carboxylic acid group is present in esterified form or in the form of an amide. The compounds of the formula (I) inhibit the protease cathepsin A as can be demonstrated in the pharmacological test described below and in other tests which are known to a person skilled in the art. The compounds of the formula (I) and their physiologically acceptable salts and solvates therefore are valuable pharmaceutical active compounds. The compounds of the formula (I) and their physiologically acceptable salts and solvates can be used for the treatment of cardiovascular diseases such as heart failure including systolic heart failure, diastolic heart failure, diabetic heart failure and heart failure with preserved ejection fraction, cardiomyopathy, myocardial infarction, left ventricular dysfunction including left ventricular dysfunction after myocardial infarction, cardiac hypertrophy, myocardial remodeling including myocardial remodeling after infarction or after cardiac surgery, valvular heart diseases, vascular hypertrophy, vascular remodeling including vascular stiffness, hypertension including pulmonary hypertension, portal hypertension and systolic hypertension, atherosclerosis, peripheral arterial occlusive disease (PAOD), restenosis, thrombosis and vascular permeability disorders, ischemia and/or reperfusion damage including ischemia and/or reperfusion damage of the heart and ischemia and/or reperfusion damage of the retina, inflammation and inflammatory diseases such as rheumatoid arthritis and osteoarthritis, renal diseases such as renal papillary necrosis and renal failure including renal failure after ischemia/reperfusion, pulmonary diseases such as cystic fibrosis, chronic bronchitis, chronic obstructive pulmonary disease (COPD), asthma, acute respiratory dystress syndrome (ARDS), respiratory tract infections and lung carcinoma, immunological diseases, diabetic complications including diabetic nephropathy and diabetic cardiomyopathy, fibrotic diseases such as pulmonary fibrosis including idiopathic lung fibrosis, cardiac fibrosis, vascular fibrosis, perivascular fibrosis, renal fibrosis including renal tubulointerstitial fibrosis, fibrosing skin conditions including keloid formation, collagenosis and scleroderma, and liver fibrosis, liver diseases such as liver cirrhosis, pain such as neuropathic pain, diabetic pain and inflammatory pain, macular degeneration, neurodegenerative diseases or psychiatric disorders, or for cardioprotection including cardioprotection after myocardial infarction and after cardiac surgery, or for renoprotection, for example. The compounds of the formula (I) and their physiologically acceptable salts and solvates can be used as diuretic (stand-alone treatment or in combination with established diuretics). Another embodiment is a compound of the formula (I) in any of its stereoisomeric forms or a mixture of stereoisomeric forms in any ratio or a physiologically acceptable salt thereof or a physiologically acceptable solvate for use in the treatment of congestive heart failure, cardiomyopathy, myocardial infarction, left ventricular dysfunction, valvular heart diseases, hypertension, atherosclerosis, peripheral arterial occlusive disease, restenosis, vasvular permeability disorders, treatment of edema, thrombosis, osteoarthritis, renal failure, cystic fibrosis, chronic bronchitis, chronic obstructive pulmonary disease, diabetic complications, fibrotic diseases, pain, ischemia or reperfusion damage, or for cardioprotection or renoprotection or as a diuretic (standalone treatment or in combination with established diuretics). Another embodiment is a compound of the formula (I) in any of its stereoisomeric forms or a mixture of stereoisomeric forms in any ratio or a physiologically acceptable salt thereof or a physiologically acceptable solvate for use in the treatment of congestive heart failure. Another embodiment is a compound of the formula (I) in any of its stereoisomeric forms or a mixture of stereoisomeric forms in any ratio or a physiologically acceptable salt thereof or a physiologically acceptable solvate for use in the treatment of myocardial infarction. Another embodiment is a compound of the formula (I) in any of its stereoisomeric forms or a mixture of stereoisomeric forms in any ratio or a physiologically acceptable salt thereof or a physiologically acceptable solvate for use in the treatment of edema. Another embodiment is a compound of the formula (I) in any of its stereoisomeric forms or a mixture of stereoisomeric forms in any ratio or a physiologically acceptable salt thereof or a physiologically acceptable solvate for use in the treatment of diabetic complications. Another embodiment is a compound of the formula (I) in any of its stereoisomeric forms or a mixture of stereoisomeric forms in any ratio or a physiologically acceptable salt thereof or a physiologically acceptable solvate for use as a diuretic (stand-alone treatment or in combination with established diuretics). Another embodiment is a pharmaceutical composition of a compound of the formula (I) in any of its stereoisomeric forms or a mixture of stereoisomeric forms in any ratio or a physiologically acceptable salt thereof or a physiologically acceptable solvate for use in the treatment of congestive heart failure. Another embodiment is a pharmaceutical composition of a compound of the formula (I) in any of its stereoisomeric forms or a mixture of stereoisomeric forms in any ratio or a physiologically acceptable salt thereof or a physiologically acceptable solvate for use in the treatment of myocardial infarction. Another embodiment is a pharmaceutical composition of a compound of the formula (I) in any of its stereoisomeric forms or a mixture of stereoisomeric forms in any ratio or a physiologically acceptable salt thereof or a physiologically acceptable solvate for use in the treatment of edema. Another embodiment is a pharmaceutical composition of a compound of the formula (I) in any of its stereoisomeric forms or a mixture of stereoisomeric forms in any ratio or a physiologically acceptable salt thereof or a physiologically acceptable solvate for use in the treatment of diabetic complications. Another embodiment is a pharmaceutical composition of a compound of the formula (I) in any of its stereoisomeric forms or a mixture of stereoisomeric forms in any ratio or a physiologically acceptable salt thereof or a physiologically acceptable solvate for use as a diuretic (stand-alone treatment or in combination with established diuretics). The treatment of diseases is to be understood as meaning both therapy of existing pathological changes or malfunctions of the organism or of existing symptoms with the aim of relief, alleviation or cure, and the prophylaxis or prevention of pathological changes or malfunctions of the organism or of symptoms in humans or animals which are susceptible thereto and are in need of such a prophylaxis or prevention, with the aim of a prevention or suppression of their occurrence or of an attenuation in the case of their occurrence. For example, in patients who on account of their disease history are susceptible to myocardial infarction, by means of the prophylactic or preventive medicinal treatment the occurrence or re-occurrence of a myocardial infarction can be prevented or its extent and sequelae decreased, or in patients who are susceptible to attacks of asthma, by means of the prophylactic or preventive medicinal treatment such attacks can be prevented or their severity decreased. The treatment of diseases can occur both in acute cases and in chronic cases. The efficacy of the compounds of the formula (I) can be demonstrated in the pharmacological test described below and in other tests which are known to a person skilled in the art. The compounds of the formula (I) and their physiologically acceptable salts and solvates can therefore be used in animals, in particular in mammals and specifically in humans, as a pharmaceutical or medicament on their own, in mixtures with one another or in the form of pharmaceutical compositions. A subject of the present invention also are the compounds of the formula (I) and their physiologically acceptable salts and solvates for use as a pharmaceutical, as well as pharmaceutical compositions and medicaments which comprise an efficacious dose of at least one compound of the formula (I) and/or a physiologically acceptable salt thereof and/or solvate thereof as an active ingredient and a pharmaceutically acceptable carrier, i.e. one or more pharmaceutically innocuous, or nonhazardous, vehicles and/or excipients, and optionally one or more other pharmaceutical active compounds. A subject of the present invention furthermore are the compounds of the formula (I) and their physiologically acceptable salts and solvates for use in the treatment of the diseases mentioned above or below, including the treatment of any one of the mentioned diseases, for example the treatment of heart failure, myocardial infarction, cardiac hypertrophy, diabetic nephropathy, diabetic cardiomyopathy, cardiac fibrosis, or ischemia and/or reperfusion damage, or for cardioprotection, the use of the compounds of the formula (I) and their physiologically acceptable salts and solvates for the manufacture of a medicament for the treatment of the diseases mentioned above or below, including the treatment of any one of the mentioned diseases, for example the treatment of heart failure, myocardial infarction, cardiac hypertrophy, diabetic nephropathy, diabetic cardiomyopathy, cardiac fibrosis, or ischemia and/or reperfusion damage, or for cardioprotection, wherein the treatment of diseases comprises their therapy and prophylaxis as mentioned above, as well as their use for the manufacture of a medicament for the inhibition of cathepsin A. A subject of the invention also are methods for the treatment of the diseases mentioned above or below, including the treatment of any one of the mentioned diseases, for example the treatment of heart failure, myocardial infarction, cardiac hypertrophy, diabetic nephropathy, diabetic cardiomyopathy, cardiac fibrosis, or ischemia and/or reperfusion damage, or for cardioprotection, which comprise administering an efficacious amount of at least one compound of the formula (I) and/or a physiologically acceptable salt thereof and/or solvate thereof to a human or an animal which is in need thereof. The compounds of the formula (I) and pharmaceutical compositions and medicaments comprising them can be administered enterally, for example by oral, sublingual or rectal administration, parenterally, for example by intravenous, intramuscular, subcutaneous or intraperitoneal injection or infusion, or by another type of administration such as topical, percutaneous, transdermal, intraarticular or intraocular administration. The compounds of the formula (I) and their physiologically acceptable salts and solvates can also be used in combination with other pharmaceutical active compounds, wherein in such a combination use the compounds of the formula (I) and/or their physiologically acceptable salts and/or solvates and one or more other pharmaceutical active compounds can be present in one and the same pharmaceutical composition or in two or more pharmaceutical compositions for separate, simultaneous or sequential administration. Examples of such other pharmaceutical active compounds are diuretics, aquaretics, angiotensin converting enzyme (ACE) inhibitors, angiotensin receptor blockers, renin inhibitors, beta blockers, digoxin, aldosterone antagonists, NO donors, nitrates, hydralazines, ionotropes, vasopressin receptor antagonists, soluble guanylate cyclase activators, statins, peroxisome proliferator-activated receptor-alpha (PPAR-cc) activators, peroxisome proliferator-activated receptor-gamma (PPAR-γ) activators, rosiglitazone, pioglitazone, metformin, sulfonylureas, glucagon-like peptide 1 (GLP-1) agonists, dipeptidyl peptidase IV (DPPIV) inhibitors, insulins, anti-arrhythmics, endothelin receptor antagonists, calcium antagonists, phosphodiesterase inhibitors, phosphodiesterase type 5 (PDE5) inhibitors, factor II/factor I la inhibitors, factor IX/factor IXa inhibitors, factor X/factor Xa inhibitors, factor Xlll/factor XI I la inhibitors, heparins, glycoprotein llb/llla antagonists, P2Y12 receptor antagonists, clopidogrel, coumarins, cyclooxygenase inhibitors, acetylsalicylic acid, RAF kinase inhibitors and p38 mitogen-activated protein kinase inhibitors. A subject of the present invention also is the said combination use of any one or more of the compounds of the formula (I) disclosed herein and their physiologically acceptable salts and solvates, with any one or more, for example one or two, of the mentioned other pharmaceutical active compounds. The pharmaceutical compositions and medicaments according to the invention normally contain from about 0.5 to about 90 percent by weight of compounds of the formula (I) and/or physiologically acceptable salts and/or solvates thereof, and an amount of active ingredient of the formula (I) and/or its physiologically acceptable salt and/or solvate which in general is from about 0.2 mg to about 1.5 g, particularly from about 0.2 mg to about 1 g, more particularly from about 0.5 mg to about 0.5 g, for example from about 1 mg to about 0.3 g, per unit dose. Depending on the kind of the pharmaceutical composition and other particulars of the specific case, the amount may deviate from the indicated ones. The production of the pharmaceutical compositions and medicaments can be carried out in a manner known per se. For this, the compounds of the formula (I) and/or their physiologically acceptable salts and/or solvates are mixed together with one or more solid or liquid vehicles and/or excipients, if desired also in combination with one or more other pharmaceutical active compounds such as those mentioned above, and brought into a suitable form for dosage and administration, which can then be used in human medicine or veterinary medicine. As vehicles, which may also be looked upon as diluents or bulking agents, and excipients suitable organic and inorganic substances can be used which do not react in an undesired manner with the compounds of the formula I. As examples of types of excipients, or additives, which can be contained in the pharmaceutical compositions and medicaments, lubricants, preservatives, thickeners, stabilizers, disintegrants, wetting agents, agents for achieving a depot effect, emulsifiers, salts, for example for influencing the osmotic pressure, buffer substances, colorants, flavorings and aromatic substances may be mentioned. Examples of vehicles and excipients are water, vegetable oils, waxes, alcohols such as ethanol, isopropanol, 1,2-propanediol, benzyl alcohols, glycerol, polyols, polyethylene glycols or polypropylene glycols, glycerol triacetate, polyvinylpyrrolidone, gelatin, cellulose, carbohydrates such as lactose or starch like corn starch, sodium chloride, stearic acid and its salts such as magnesium stearate, talc, lanolin, petroleum jelly, or mixtures thereof, for example saline or mixtures of water with one or more organic solvents such as mixtures of water with alcohols. For oral and rectal use, pharmaceutical forms such as, for example, tablets, film-coated tablets, sugar-coated tablets, granules, hard and soft gelatin capsules, suppositories, solutions, including oily, alcoholic or aqueous solutions, syrups, juices or drops, furthermore suspensions or emulsions, can be used. For parenteral use, for example by injection or infusion, pharmaceutical forms such as solutions, for example aqueous solutions, can be used. For topical use, pharmaceutical forms such as ointments, creams, pastes, lotions, gels, sprays, foams, aerosols, solutions or powders can be used. Further suitable pharmaceutical forms are, for example, implants and patches and forms adapted to inhalation. The compounds of the formula (I) and their physiologically acceptable salts can also be lyophilized and the obtained lyophilizates used, for example, for the production of injectable compositions. In particular for topical application, also liposomal compositions are suitable. The pharmaceutical compositions and medicaments can also contain one or more other active ingredients and/or, for example, one or more vitamins. As usual, the dosage of the compounds of the formula (I) depends on the circumstances of the specific case and is adjusted by the physician according to the customary rules and procedures. It depends, for example, on the compound of the formula (I) administered and its potency and duration of action, on the nature and severity of the individual syndrome, on the sex, age, weight and the individual responsiveness of the human or animal to be treated, on whether the treatment is acute or chronic or prophylactic, or on whether further pharmaceutical active compounds are administered in addition to a compound of the formula I. Normally, in the case of administration to an adult weighing about 75 kg, a dose from about 0.1 mg to about 100 mg per kg per day, in particular from about 1 mg to about 20 mg per kg per day, for example from about 1 mg to about 10 mg per kg per day (in each case in mg per kg of body weight), is administered. The daily dose can be administered in the form of a single dose or divided into a number of individual doses, for example two, three or four individual doses. The administration can also be carried out continuously, for example by continuous injection or infusion. Depending on the individual behavior in a specific case, it may be necessary to deviate upward or downward from the indicated dosages. Besides as a pharmaceutical active compound in human medicine and veterinary medicine, the compounds of the formula (I) can also be employed as an aid in biochemical investigations or as a scientific tool or for diagnostic purposes, for example in in-vitro diagnoses of biological samples, if an inhibition of cathepsin A is intended. The compounds of the formula (I) and their salts can also be used as intermediates, for example for the preparation of further pharmaceutical active substances. The following examples illustrate the invention. Pharmacological tests:
A) Cathepsin A inhibitory activity: Recombinant human cathepsin A (residues 29-480, with a C-terminal 10-His tag; R&D Systems, # 1049-SE) was proteolytically activated with recombinant human cathepsin L (R&D Systems, # 952-CY). Briefly, cathepsin A was incubated at 10 g/ml with cathepsin L at 1 g/ml in activation buffer (25 mM 2-(morpholin-4-yl)-ethanesulfonic acid (MES), pH 6.0, containing 5 mM dithiothreitol (DTT)) for 15 min at 37 °C. Cathepsin L activity was then stopped by the addition of the cysteine protease inhibitor E-64 (N-(trans-epoxysuccinyl)-L-leucine-4-guanidinobutylamide; Sigma-Aldrich, # E3132; dissolved in activation buffer/DMSO) to a final concentration of 10 µM. The activated cathepsin A was diluted in assay buffer (25 mM MES, pH 5.5, containing 5 mM DTT) and mixed with the test compound (dissolved in assay buffer containing (v/v) 3 % DMSO) or, in the control experiments, with the vehicle in a multiple assay plate. After incubation for 15 min at room temperature, as substrate then bradykinin carrying an N-terminal ®Bodipy FL (4,4-difluoro-5,7-dimethyl-4-bora-3a,4a-diaza-s-indacene-3-propionyl) label (JPT Peptide Technologies GmbH; dissolved in assay buffer) was added to the mixture. The final concentration of cathepsin A was 833 ng/ml and the final concentration of labeled bradykinin 2 µM. After incubation for 15 min at room temperature the reaction was stopped by the addition of stop buffer (130 mM 2-(4-(2-hydroxy-ethyl)-piperazin-1-yl)-ethanesulfonic acid, pH 7.4, containing (v/v) 0.013 % ®Triton X-100, 0.13 % Coating Reagent 3 (Caliper Life Sciences), 6.5 % DMSO and 20 µM ebelactone B (Sigma, # E0886)). Uncleaved substrate and product were then separated by a microfluidic capillary electrophoresis on a LabChip® 3000 Drug Discovery System (12-Sipper-Chip; Caliper Life Sciences) and quantified by determination of the respective peak areas. Substrate turnover was calculated by dividing product peak area by the sum of substrate and product peak areas, and the enzyme activity and the inhibitory effect of the test compound thus quantified. From the percentage of inhibition of cathepsin A activity observed with the test compound at several concentrations, the inhibitory concentration IC5o, i.e. the concentration which effects 50 % inhibition of enzyme activity was, calculated. Boceprevir of formula (I) showed an IC50 of 1 µM.
B) In vivo antihypertrophic and renoprotective activity: The in vivo pharmacological activity of the compounds of the invention can be investigated, for example, in the model of DOCA-salt sensitive rats with unilateral nephrectomy. Briefly, in this model unilateral nephrectomy of the left kidney (UNX) is performed on Sprague Dawley rats of 150 g to 200 g of body weight. After the operation as well as at the beginning of each of the following weeks 30 mg/kg of body weight of DOCA (desoxycorticosterone acetate) are administered to the rats by subcutaneous injection. The nephrectomized rats treated with DOCA are supplied with drinking water containing 1 % of sodium chloride (UNX/DOCA rats). The UNX/DOCA rats develop high blood pressure, endothelial dysfunction, myocardial hypertrophy and fibrosis as well as renal dysfunction. In the test group (UNX/DOCA Test) and the placebo group (UNX/DOCA Placebo), which consist of randomized UNX/DOCA rats, the rats are treated orally by gavage in two part administrations at 6 a.m. and 6 p.m. with the daily dose of the test compound (for example 10 mg/kg of body weight dissolved in vehicle) or with vehicle only, respectively. In a control group (control), which consists of animals which have not been subjected to UNX and DOCA administration, the animals receive normal drinking water and are treated with vehicle only. After five weeks of treatment, systolic blood pressure (SBP) and heart rate (HR) are measured non-invasively via the tail cuff method. For determination of albuminuria and creatinine, 24 h urine is collected on metabolic cages. Endothelial function is assessed in excised rings of the thoracic aorta as described previously (W. Linz et al., JRAAS (Journal of the renin-angiotensin-aldosterone system) 7 (2006), 155-161). As a measure of myocardial hypertrophy and fibrosis, heart weight, left ventricular weight and the relation of hydroxyproline and proline are determined in excised hearts. Very preferred embodiments of the administration unit and for this application are the claims described also in WO2013/072327 and are repeated as preferred items 1 to 14 hereinafter:
   Item 1. A compound of the formula (I) 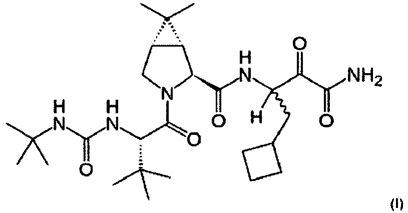 in any of its stereoisomeric forms or a mixture of stereoisomeric forms in any ratio or a physiologically acceptable salt thereof or a physiologically acceptable solvate for use in the treatment of heart failure, congestive heart failure, cardiomyopathy, myocardial infarction, left ventricular dysfunction, cardiac hypertrophy, valvular heart diseases, hypertension, atherosclerosis, peripheral arterial occlusive disease, restenosis, vasvular permeability disorders, treatment of edema, thrombosis, rheumatoid arthritis, osteoarthritis, renal failure, cystic fibrosis, chronic bronchitis, chronic obstructive pulmonary disease, asthma, immunological diseases, diabetic complications, fibrotic diseases, pain, ischemia or reperfusion damage or neurodegenerative diseases, or for cardioprotection or renoprotection or as a diuretic (stand-alone treatment or in combination with established diuretics).
   Item 2. A pharmaceutical composition of a compound of the formula (I) 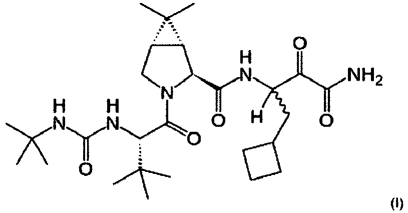 in any of its stereoisomeric forms or a mixture of stereoisomeric forms in any ratio or a physiologically acceptable salt thereof or a physiologically acceptable solvate for use in the treatment of heart failure, congestive heart failure, cardiomyopathy, myocardial infarction, left ventricular dysfunction, cardiac hypertrophy, valvular heart diseases, hypertension, atherosclerosis, peripheral arterial occlusive disease, restenosis, vasvular permeability disorders, treatment of edema, thrombosis, rheumatoid arthritis, osteoarthritis, renal failure, cystic fibrosis, chronic bronchitis, chronic obstructive pulmonary disease, asthma, immunological diseases, diabetic complications, fibrotic diseases, pain, ischemia or reperfusion damage or neurodegenerative diseases, or for cardioprotection or renoprotection or as a diuretic (stand-alone treatment or in combination with established diuretics).
   Item 3. A compound of the formula (I) according to item 1 in any of its stereoisomeric forms or a mixture of stereoisomeric forms in any ratio or a physiologically acceptable salt thereof or a physiologically acceptable solvate for use in the treatment of congestive heart failure, cardiomyopathy, myocardial infarction, left ventricular dysfunction, valvular heart diseases, hypertension, atherosclerosis, peripheral arterial occlusive disease, restenosis, vasvular permeability disorders, treatment of edema, thrombosis, osteoarthritis, renal failure, cystic fibrosis, chronic bronchitis, chronic obstructive pulmonary disease, diabetic complications, fibrotic diseases, pain, ischemia or reperfusion damage or for cardioprotection or renoprotection or as a diuretic (standalone treatment or in combination with established diuretics).
   Item 4. A pharmaceutical composition of a compound of the formula (I) according to item 2 in any of its stereoisomeric forms or a mixture of stereoisomeric forms in any ratio or a physiologically acceptable salt thereof or a physiologically acceptable solvate for use in the treatment of congestive heart failure, cardiomyopathy, myocardial infarction, left ventricular dysfunction, valvular heart diseases, hypertension, atherosclerosis, peripheral arterial occlusive disease, restenosis, vasvular permeability disorders, treatment of edema, thrombosis, osteoarthritis, renal failure, cystic fibrosis, chronic bronchitis, chronic obstructive pulmonary disease, diabetic complications, fibrotic diseases, pain, ischemia or reperfusion damage, or for cardioprotection or renoprotection or as a diuretic (stand-alone treatment or in combination with established diuretics).
   Item 5. A compound of the formula (I) according to item 1 in any of its stereoisomeric forms or a mixture of stereoisomeric forms in any ratio or a physiologically acceptable salt thereof or a physiologically acceptable solvate for use in the treatment of congestive heart failure.
   Item 6. A compound of the formula (I) according to item 1 in any of its stereoisomeric forms or a mixture of stereoisomeric forms in any ratio or a physiologically acceptable salt thereof or a physiologically acceptable solvate for use in the treatment of myocardial infarction.
   Item 7. A compound of the formula (I) according to item 1 in any of its stereoisomeric forms or a mixture of stereoisomeric forms in any ratio or a physiologically acceptable salt thereof or a physiologically acceptable solvate for use in the treatment of edema.
   Item 8. A compound of the formula (I) according to item 1 in any of its stereoisomeric forms or a mixture of stereoisomeric forms in any ratio or a physiologically acceptable salt thereof or a physiologically acceptable solvate for use in the treatment of diabetic complications.
   Item 9. A compound of the formula (I) according to item 1 in any of its stereoisomeric forms or a mixture of stereoisomeric forms in any ratio or a physiologically acceptable salt thereof or a physiologically acceptable solvate for use as a diuretic (stand-alone treatment or in combination with established diuretics).
   Item 10. A pharmaceutical composition of a compound of the formula (I) according to item 2 in any of its stereoisomeric forms or a mixture of stereoisomeric forms in any ratio or a physiologically acceptable salt thereof or a physiologically acceptable solvate for use in the treatment of congestive heart failure.
   Item 11. A pharmaceutical composition of a compound of the formula (I) according to item 2 in any of its stereoisomeric forms or a mixture of stereoisomeric forms in any ratio or a physiologically acceptable salt thereof or a physiologically acceptable solvate for use in the treatment of myocardial infarction.
   Item 12. A pharmaceutical composition of a compound of the formula (I) according to item 2 in any of its stereoisomeric forms or a mixture of stereoisomeric forms in any ratio or a physiologically acceptable salt thereof or a physiologically acceptable solvate for use in the treatment of edema.
   Item 13. A pharmaceutical composition of a compound of the formula (I) according to item 2 in any of its stereoisomeric forms or a mixture of stereoisomeric forms in any ratio or a physiologically acceptable salt thereof or a physiologically acceptable solvate for use in the treatment of diabetic complications.
   Item 14. A pharmaceutical composition of a compound of the formula (I) according to item 2 in any of its stereoisomeric forms or a mixture of stereoisomeric forms in any ratio or a physiologically acceptable salt thereof or a physiologically acceptable solvate for use as a diuretic (stand-alone treatment or in combination with established diuretics).

### SPECIFIC INORMATION CONCERNING ASPECT (II) OF THE INVENTION

Aspect (ii) of the invention relates to a salt of (R)-3-(6-amino-pyridin-3-yl)-2-(1-cyc1ohexyl-1 H-imidazol-4-yl)-propionic acid ethyl ester. The invention relates to a solid form of naphthalene-1,5-disulfonic acid salt of (R)-3-(6-amino-pyridin-3-yl)-2-(1-cyclohexyl-1 H-imidazol-4-yl)-propionic acid ethylester ({drug2}), namely to crystalline form of naphthalene-1,5-disulfonic acid salt of (R)-3-(6-amino-pyridin-3-yl)-2-(1-cyclohexyl-1H-imidazol-4-yl)-propionic acid ethylester ({drug2a}), which is useful for treating von myocardial infarct, angina pectoris and other forms of the acute coronal syndrome, stroke, peripheral vascular disorders, deep venous thrombosis, lung emboli, embolic or thrombotic events. Crystalline form of naphthalene-1,5-disulfonic acid salt of (R)-3-(6-amino-pyridin-3-yl)-2-(1-cyclohexyl-1H-imidazol-4-yl)-propionic acid ethylester ({drug2a}) is described in WO2013/076177, which is incorporated by reference. As used herein, crystalline form of naphthalene-1,5-disulfonic acid salt of (R)-3-(6-amino-pyridin-3-yl)-2-(1-cyclohexyl-1H-imidazol-4-yl)-propionic acid ethylester ({drug2a}) is a crystalline form of naphthalene-1,5-disulfonic acid salt of (R)-3-(6-amino-pyridin-3-yl)-2-(1-cyclohexyl-1H-imidazol-4-yl)-propionic acid ethylester as characterized and obtained according to WO2013/076177: The invention relates to the salt of the naphthalene-1,5-disulfonic acid with (R)-3-(6-amino-pyridin-3-yl)-2-(1-cyclohexyl-1H-imidazol-4-yl)-propionic acid ethyl ester and the naphthalene-1,5-disulfonic acid salt of (R)-3-(6-amino-pyridin-3-yl)-2-(1-cyclohexyl-1 H-imidazol-4-yl)-propionic acid ethylester which in a crystalline form or is at least partially present in a crystalline form, a process for their production, medicaments containing these compounds and their use, wherein the structure is represented by the following structural formula of the formula (II): 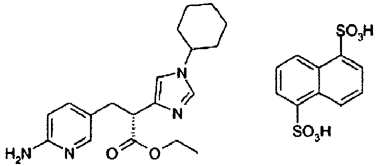 3-(6-amino-pyridin-3-yl)-2-(1-cyclohexyl-1H-imidazol-4-yl)-propionate hydrochloride, and its pharmacological properties are already described in the international application PCT/EP2005/003630 (WO2005/105781). The hydrochloride salts of 3-(6-amino-pyridin-3-yl)-2-(1-cyclohexyl-1H-imidazol-4-yl)-propionic acid ethylester or (R)-3-(6-amino-pyridin-3-yl)-2-(1-cyclohexyl-1 H-imidazol-4-yl) propionate, have the disadvantage that they are amorphous and cannot be obtained in crystalline form. The above hydrochloride salts cannot be purified by crystallization and therefore are thus hardly useful for use as an active ingredient in pharmaceutical compositions for the legislature precisely defined purity of the ingredients are prescribed. The armorphous hydrochloride salts are little suited, because of their physical characteristics and their handling the pharmaceutical preparation of pharmaceutical compositions such as tablets. Further, (R)-3-(6-amino-pyridin-3-yl)-2-(1-cyclohexyl-1H-imidazol-4-yl)-propionic acid ethyl ester is not chemically stable at elevated temperature and humidity. It comes to the chemical degradation of the compound and the enantiomeric purity of the chiral compound (R)-3-(6-amino-pyridin-3-yl)-2-(1-cyclohexyl-1H-imidazol-4-yl) propionate take over time quickly. The object of the present invention is therefore to provide the compound (R)-3-(6-amino-pyridin-3-yl)-2-(1-cyclohexyl-1H-imidazol-4-yl) propionate in a suitable form that is easy to clean and has an improved stability at elevated temperature and humidity. The present invention thus relates to a salt of the naphthalene-1,5-disulfonic acid with (R)-3-(6-amino-pyridin-3-yl)-2-(1-cyclohexyl-1H-imidazol-4-yl)-propionic acid ethyl ester, which may be in crystalline form or in a partially crystalline form. The present invention also relates to the salt of the compound of formula (II) 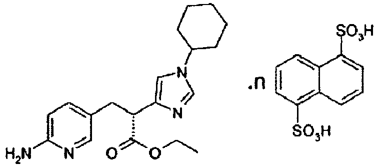 wherein n has a value from 0.5 to 1.8, and n has a molar ratio of (R)-3-(6-amino-pyridin-3-yl)-2-(1-cycloheacyl-1 H-imidazol-4-yl)-propionic acid ethyl ester indicates to naphthalene-1,5-disulfonic acid. Preferred are salts of the compound of formula (II), wherein n has a value from 0.8 to 1.3. Preferred are salts of the compound of formula (II), wherein n has a value of 0.95 to 1.05. The salt of the invention, especially the salt of the compound of formula (H) is at least partially, preferably completely crystalline. By providing the crystalline salts of the invention in particular completely the compound of formula (II), the active ingredient (R)-3-(6-amino-pyridin-3-yl)-2-(1-cyclohexyl-1H-imidazol-4-yl)-propionic acid ethyl ester, can
- be easily cleaned (eg by recrystallisation);
- have a defined purity that is necessary for drug approval;
- can be easily detected and identified by conventional methods such as XRPD (X-ray Powder Diffraction), melting point, IR (infrared spectrum), and it has
- a reproducible physical quality
- an improved chemical stability and
- an improved chiral stability.

Crystalline substances are normally more stable than amorphous substances. This will avoid problems with the degradation of the active ingredients and occurring degradation products. The amorphous form of an active substance may still contain undesired solvent content. These are difficult to remove, as a rule, it cannot be recrystallized. The amorphous form is higher in energy than the crystalline form. This can lead to the random pattern of the molecular distribution of the amorphous form that rearranges spontaneously releasing energy and dissipates some of the energy. This can lead to a change in the effect of the drug, and that is not directly visible on a measurable parameter of the active ingredient. A significant impact on the reliability of the active ingredient and thus a risk to the patient is the result. Only a defined crystalline substance allows a reliable and reproducible formulation with reproducible bioavailability. A further embodiment of the invention comprises a crystalline salt of formula (II), characterized in that the measured with CuKa radiation having a XRPD reflection intensity at a high angle position of 22.48 degrees 2-theta ± 0.2 degrees 2 theta ({drug2b}). A further embodiment of the invention comprises a crystalline salt of formula (II), characterized in that the measured with CuKa radiation having XRPD peaks at the following 2-theta values at the angular positions: 7.35 and 22.48 respectively ± 0.2 degrees 2 theta ({drug2c}). A further embodiment of the invention comprises a crystalline salt of formula (II), characterized in that the measured with CuKa radiation having XRPD peaks at the following 2-theta values at the angular positions: 7.35; 11.4; 13.69; 14.96; 17.49; 19.3 and 22.48, respectively ± 0.2 degrees 2 theta (drug2d}) The selection of the characteristic reflections was due to the level of intensity. An example of a XRPD is depicted in Figure 1 of WO2013/076177. The embodiment of the invention can also contain, depending on the relative humidity, a small amount of water; the water content is from about 0.1% to 2.0%. The percentages refer to the weight. This water can be calculated from the crystalline salt of the formula (II) when heated to completely remove as far as possible above 100°C. XRPD measurements at different temperatures show essentially unchanged spectra. Alternatively, the crystalline salts of naphthalene-1,5-disulfonic acid with (R)-3-(6-amino-pyridin-3-yl)-2-(1-cyclohexyl-1H-imidazol-4-yl)-propionic acid ethyl ester of formula (II) by a Raman spectrum characterized. This can be determined by characteristic bands at the following wavelengths [cm⁻¹]: 2936; 1570; 1401; 1352; 998 and 856, respectively ± 2 cm⁻¹ ({drug2e}). An example of a Raman spectrum is shown in Figure 2 of WO2013/076177. The term "polymorphism "means that the individual compounds may exist in more than one form or crystal structure. Different polymorphs are solids, which are characterized by the same molecular formula, but can have different physical properties. The term "amorphous" solid compounds are referred to that at all angular positions have no reflections in the XRPD spectrum, which can be clearly distinguished from each other in their intensities. The term "intensity "be the set of reflections measured respectively., The term "characteristic bands "in the Raman spectrum bands are referred to, which can be clearly assigned to the crystal form. Figure 1 of WO2013/076177 shows a XRPD spectrum of the crystalline salt of naphthalene-1,5-disulfonic acid with (R)-3-(6-amino-pyridin-3-yl)-2-(1-cyclohexyl-1H-imidazole-4-yl)-propionic acid ethyl ester of formula (II), measured in transmission with CuKa radiation at room temperature (x-axis angle of diffraction 2theta (20) [°], Y axis: relative intensity). Figure 2 of WO2013/076177 shows the Raman spectrum of the crystalline salt of naphthalene-1,5-disulfonic acid with (R)-3-(6-amino-pyridin-3-yl)-2-(1-cyclohexyl-1H-imidazole-4-yl)-propionic acid ethyl ester of formula (II) as measured in the wavelength range of 3500 to 200 cm⁻¹ (X-axis wavelength [cm⁻¹], Y axis: relative intensity). The invention further relates to processes for preparing the compound of formula (II). A process for producing the compound of formula (II) is characterized in that one dissolves naphthalene-1,5-disulfonic acid in a solvent A, and adds a solution of (R)-3-(6-amino-pyridin-3-yl)-2-(1-cyclohexyl-1H-imidazol-4-yl)-propionic acid ethyl ester in a solvent B, then the salt of (R)-3-(6-amino-pyridin-3-yl)-2-(1-cyclohexyl-1H-imidazol-4-yl) propionate with naphthalene-1,5-disulfonic acid of the formula (II) is obtained. Optionally, the solvent may be partially or completely removed. The salt obtained is at least partially, preferably completely crystalline. A suitable solvent A is a polar protic or aprotic solvent such as ethanol, acetone, methyl ethyl ketone, acetonitrile, tetrahydrofuran, dimethylsulfoxide, dimethylformamide, N-methylpyrrolidinone, methanol, propanol, butanol or water. Mixtures of one or more of the solvents are also possible. Suitable solvents B are polar protic or aprotic solvents such as ethanol, acetone, methyl ethyl ketone, acetonitrile, tetrahydrofuran, dimethylsulfoxide, dimethylformamide, N-Methylpyrrolidinone, methanol, propanol, butanol or water. Mixtures of one or more of the solvents are also possible. Solvents A and B may be the same or different. The removal of the solvent can take place for example by heating or by passing over or passing a gas. Suitable gases are, for example, air, nitrogen or noble gases such as argon or xenon. Advantageous is the addition of (R)-3-(6-amino-pyridin-3-yl)-2-(1-cyclohexyl-1H-imidazol-4-yl)-propionic acid ethyl ester into a solution of naphthalene-1,5-disulfonic acid, because the possible racemization of (R)-3-(6-amino-pyridin-3-yl)-2-(1-cyclohexyl-1H-imidazol-4-yl)-propionic acid ethyl ester is largely reduced. The molar ratio of naphthalene-1,5-disulfonic acid to (R)-3-(6-amino-pyridin-3-yl)-2-(1-cyclohexyl-1H-imidazol-4-yl)-propionic acid ethyl ester in the process for preparing the compound of formula (II) is from 0.95 to 1.5. Beneficial is a ratio of 1.1 to 1.2. Suitable temperatures in the process for preparing the compound of formula (II) are from -20° C to 60° C or from -10° C to 25° C. The appropriate temperatures depend on the solvent and may be readily determined by one skilled in the art. The invention also relates to the compound of formula (II) as a medicament. The invention also relates to pharmaceuticals which contain a compound of formula (II) together with a pharmaceutically suitable and physiologically tolerated carrier. Due to the pharmacological properties the compounds of the invention are useful for the prevention and treatment of all disorders that are treatable by inhibition of TAFIa. Thus, the compound of formula (II) is suitable both for a prophylactic and for a therapeutic use in humans. They are suitable both for acute treatment as well as for long-term therapy. The compound of formula (II) can be used in patients who are suffering from the discomfort or diseases associated with thrombosis, embolism, hypercoagulability or fibrotic changes. These include myocardial infarction, angina pectoris, and all other forms of acute coronary syndrome, stroke, peripheral vascular disease, deep vein thrombosis, pulmonary embolism, embolic or thrombotic events caused by cardiac arrhythmias, cardiovascular events such as restenosis following revascularization, angioplasty and similar procedures such as stent implantations and bypass operations. Furthermore, the compound of formula (II) may be employed in all procedures leading to contact of blood with foreign surfaces such as dialysis patients and patients with indwelling, the compound of formula (II) can be used to the risk of thrombosis after surgical interventions such as in knee and to reduce hip operations. The compound of formula (II) is suitable for the treatment of patients with disseminated intravascular coagulation, sepsis and other intravascular events associated with inflammation. Furthermore, the compound of formula (II) is suitable for the prophylaxis and treatment of patients with atherosclerosis, diabetes and the metabolic syndrome and its consequences. Disorders of the hemostatic system (eg, fibrin) have been implicated in mechanisms that lead to tumor growth and tumor metastasis; the compound of formula (II) is suitable for slowing down or preventing such processes. Other indications for the use of the compound of formula (II) are fibrotic changes of the lung such as chronic obstructive pulmonary disease, adult respiratory distress syndrome (ARDS) and of the eye such as fibrin deposits after eye surgery. The compound of formula (II) is also suitable for prevention and/or treatment of scarring. The medicaments of the invention can be administered orally, by inhalation, rectally or by transdermal administration or by subcutaneous, intraarticular, intraperitoneal or intravenous injection. Preferred is oral application. A coating of stents or other surfaces which come in contact with blood in the body, with the compound of formula (II) is possible. The invention also relates to a process for preparing a pharmaceutical composition characterized by bringing at least one compound of formula (II) with a pharmaceutically suitable and physiologically acceptable carrier into a suitable dosage form. For the treatment of the above diseases, the compound of formula (II) can be used itself as the compound, but preferably is with a suitable carrier in the form of a pharmaceutical composition. The carrier must, of course, be acceptable in the sense that it is compatible with the other ingredients of the composition and is not harmful for the patient. The carrier may be a solid or a liquid, or both and is preferably formulated with the compound as single dose, for example a tablet, and may contain from 0.05 weight-% to 95 weight-% of the compound of formula (II). Other pharmaceutically active substances may also be present. The pharmaceutical compositions of the invention can be prepared, which consist essentially in the fact that the ingredients are mixed with pharmacologically acceptable carriers and/or excipients according to one of the known pharmaceutical methods. Inventive pharmaceutical compositions are those which are suitable (for example sublingual) for oral and peroral administration, although it is the cheapest mode of administration in each case is dependent on the nature and severity of the condition to be treated. Coated formulations and coated slow-release formulations are in the scope of the invention. Possible are acid and gastric juice-resistant formulations. Suitable enteric coatings include cellulose poylvinylacetatphthalat, hydroxypropylmethylcellulose phthalate and anionic polymers of methacrylic acid and methyl methacrylate. Suitable pharmaceutical compounds for oral administration can exist in separate units, such as capsules, cachets, lozenges or tablets, each containing a predetermined amount of the compound of formula (II); as a powder or granules; as a solution or a suspension in an aqueous or non-aqueous liquid; or as an oil-in-water or water-in-oil emulsion. These compositions may be prepared, as already mentioned, by any suitable pharmaceutical method, which comprises a step in which the active ingredient and the carrier (which may consist of one or more additional ingredients) are brought into contact. In general, the compositions are prepared by uniformly and homogeneously mixing the active ingredient with a liquid and/or finely divided solid carrier, after which the product is shaped if necessary. For example, a tablet can be prepared by mixing a powder or granules of the compound compressing or molding, optionally with one or more additional components. Compressed tablets can be prepared by tableting the compound in free-flowing form such as a powder or granules, optionally mixed with a binder, lubricant, inert diluent and/or a (more) surface active / dispersing agent in a suitable machine. Molded tablets may be made by molding the pulverulent compound, moistened with an inert liquid diluent in a suitable machine. Pharmaceutical compositions suitable for peroral (sublingual) suitable administration comprise suckable tablets which contain a compound of formula (I) with a flavoring, normally sucrose and gum arabic or tragacanth, and pastilles comprising the compound in an inert base such as gelatin and glycerin or sucrose and acacia. Other suitable solid or pharmaceutical preparation forms are, for example, granules, powders, pills, tablets, (micro) capsules, suppositories, syrups, elixirs, suspensions, emulsions, drops or injectable solutions and preparations with protracted release of active ingredients, common in the manufacture of tools such as find excipients, disintegrants, binders, coating agents, swelling agents, lubricants or lubricants, flavorings, sweeteners and solubilizers. Frequently used auxiliaries are magnesium carbonate, titanium dioxide, lactose, mannitol and other sugars, talc, milk protein, gelatin, starch, cellulose and its derivatives, animal and vegetable oils such as fish liver oil, sunflower, groundnut or sesame oil, polyethylene glycol and solvents such as sterile water and monohydric or polyhydric alcohols such as glycerol. Preferably, the pharmaceutical compositions are prepared in unit dosage form and administered, each unit containing as active constituent a certain dose of the compound of the formula (II) according to the invention. For fixed dosage units such as tablets, capsules, coated tablets or suppositories, this dose can be up to about 1000 mg, but preferably about 50 to 300 mg and injection solutions in ampoule form up to approximately 300 mg, but preferably about 10 to 100 mg. For the treatment of an adult weighing about 70 kg patient daily doses of about 2 mg to 1000 mg of the compound of formula (II), preferably from about 50 mg to 500 mg, are indicated. Under certain circumstances, higher or lower daily doses may be appropriate. The administration of the daily dose can be carried out both by single administration in the form of an individual dose unit or else several smaller dose units and also by multiple administrations of subdivided doses at specific intervals. The compound of formula (II) can be used both as monotherapy and in combination or together with antithrombotics (anticoagulants and profibrinolytic other active substances, blood pressure medicines, regulators of blood sugar, lipid-lowering agents and antiarrhythmics platelet aggregation inhibitor), thrombolytics (plasminogen activators any kind). The preparation of some examples is described in detail.

Example 1: Preparation of the crystalline salt of (R)-3-(6-amino-pyridin-3-yl)-2-(1-cyclohexyl-1H-imidazol-4-yl)-propionic acid ethyl ester with naphthalene-1,5-disulfonic acid: 1.50 g (5.20 mmol, 1.2 eq.) naphthalene-1,5-disulfonic acid are dissolved in 15 ml of water. Optionally, a few crystals of the compound of formula (II) may be added. The solution is cooled to 10°C. Then, 1.48 g (4.32 mmol, 1.0 eq.) of (R)-3-(6-amino-pyridin-3-yl)-2-(1-cyclohexyl-1H-imidazole-4-yl)-propionic acid ethyl ester (prepared as described in WO2005/105781), which was previously dissolved in 15 mL of acetone / water 2:3 was added dropwise slowly. The solvents are gradually removed by flowing argon therby slowly forming crystals. The argon flow is stopped and the mixture is stirred for 3 hours (h) at room temperature (20°C to 22°C). The solids are filtered and dried overnight in air. A a yield of 1.83 g (2.90 mmol, 67%) of the crystalline salt of (R)-3-(6-amino-pyridin-3-yl)-2-(1-cyclohexyl-1H-imidazol-4-yl)-propionic acid ethyl ester was obtained with naphthalene-1,5-disulfonic acid as a white solid. The obtained crystalline salt of (R)-3-(6-amino-pyridin-3-yl)-2-(1-cyclohexyl-1H-imidazol-4-yl)-propionic acid ethyl ester with naphthalene-1,5-disulfonic acid is reproduced by the XRPD shown in Fig. 1 of WO2013/076177. The peaks at the following 2 theta values have the largest intensity and the relative intensities are shown in parentheses: 7.35 (40); 9.43 (21); 11.40 (37); 11, 96 (24); 12.70(21); 13.11 (17); 13.43 (14); 13.69(31); 14.96(33); 15.28 (23); 15.53 (22); 16.39(14); 16.68 (14); 17.05 (22); 17.49 (33); 18.09 (27); 18,95 (21); 19.31 (30); 19.88 (26); 20.40 (22); 20.69(1:1); 22.48 (100); 22.85 (17); 23.35 (16); 23.74 (30); 24.14 (32); 25.46 (16); 26.27 (9); 27.09 (10); 27.46 (9); 27.58 (9); 28.03 (13); 28.93 (9); 29.88 (7); 30.20 (8); 31,43 (7); 32.43 (6). The 2 Theta values of the highest intensity are:

| | | | | | | | |
|---|---|---|---|---|---|---|---|
| 2 Theta (+/- 0,2 Grad 2 Theta) | 7,35 | 11,4 | 13,69 | 14,96 | 17,49 | 19,3 | 22,48 |

Due to natural variations in the sample or in the measurement method, the 2 theta values of angular positions can be specified with an accuracy of + /- 0.2 degrees theta. The XRPD measurements are made in a STOE Stadi P transmission diffractometer; radiation source: an CuKa1, λ = 1,5406 A; angular range: 2° to 40° in 2-theta Bragg; step width: 0.033°; step time: 1 s. At room temperature, linear position sensitive detectors are used, dry samples are measured in a flat configuration while suspensions are measured in quartz capillaries, the recorded data is processed with the software WinXPOW V2, 12. Raman spectroscopy: The Raman spectrum is a FT-Raman spectrometer (RFS 100 / S, Bruker) equipped with a 1.5 W NIR laser (Nd: YAG, λ = 1064 nm) and with nitrogen-cooled NIR detector D418-T. The recorded spectrum is processed by the software OPUS 4.2 V, and the reproduced spectrum of the resulting crystalline salt of (R)-3-(6-amino-pyridin-3-yl)-2-(1-cyclohexyl-1 H-imidazol-4-yl) propionate with naphthalene-1,5-disulfonic acid is shown in Fig. 2 of WO2013/076177. In this case characteristic bands can be determined at the following wavelengths [cm⁻¹]: 2936; 1570; 1401; 1352; 998 and 856, respectively ± 2 cm⁻¹. Due to natural variations in the sample or in the measurement method, the characteristic bands can be given at the wavelengths with an accuracy of + /-2 cm⁻¹. Chiral HPLC: (Chiralcel OD-H/84 (250x4.6 mm), heptane: iPrOH: MeCN 15:1: 0.5 +0.1 % DEA, 30° C, 1 ml/min): Rt = 27.01 min. NMR (d6-DMSO, 600 MHz): δ = 1.12 (t, 3H), 1.13 to 1.21 (m, 1 H), 1.29 to 1.39 (m, 2H), 1.57-1.68 (m, 3H), 1.75 to 1.82 (m, 2H) 1.95-2.02 (m, 2H), 3.02 (dd, 1 H), 3.15 (dd, 1 H), 4.05-4.15 (m, 2H), 4.17-4.24 (m, 2H), 6.85-6.89 (m, 1 H), 7.39-7.44 (m, 2H), 7.68-7.72 (m, 2H), 7.73-7.76 (m, 1 H), 7.88-7.99 (m, 4H), 8.86-8.90 (m, 2H), 9.08 (s, 1 H).

Example 2: Salt Sreening: 100 mg of the compound (R)-3-(6-amino-pyridin-3-yl)-2-(1-cyclohexyl-1H-imidazol-4-yl)-propionic acid ethyl ester (hereinafter referred to as API), prepared as described in WO2005/105781, are dissolved in 10 mL acetone in an ultrasonic bath at room temperature. The molar ratio between the H⁺ concentration of the acid and the API is 2 (for details see Table below). In each well of the microtiter plate 68 micro liters of dissolved API are mixed with 40 micro liters of 0.1 normal acid solutions.

| acid | molecular weight | H⁺ Eq. | weight [g] in 100 mL solution | solvent | molarity of acid [mol/L] | molarity of H⁺ [mol/L] |
|---|---|---|---|---|---|---|
| adipic acid | 146.14 | 2 | 0.731 | ethanol | 0.05 | 0.1 |
| benzoic acid | 122.12 | 1 | 1.221 | ethanol | 0.1 | 0.1 |
| succinic acid | 118.09 | 2 | 0.590 | water ethanol 1:1 | 0.05 | 0.1 |
| citric acid | 192.13 | 2 | 0.961 | water ethanol 1:1 | 0.05 | 0.1 |
| acetic acid | 60.05 | 1 | 0.601 | ethanol | 0.1 | 0.1 |
| fumaric acid | 116.08 | 2 | 0.580 | methanol | 0.05 | 0.1 |
| glucuronic acid | 194.14 | 1 | 1.941 | water ethanol 3:1 | 0.1 | 0.1 |
| tartraic acid | 150.09 | 2 | 0.750 | ethanol | 0.05 | 0.1 |
| phosphoric acid | 98.00 | 1 | 0.980 | ethanol | 0.1 | 0.1 |
| malonic acid | 104.06 | 2 | 0.520 | water ethanol 1:1 | 0.05 | 0.1 |
| hydrochloric acid | 36.46 | 1 | 0.365 | ethanol | 0.1 | 0.1 |
| sulfuric acid | 98.08 | 2 | 0.490 | ethanol | 0.05 | 0.1 |

| | | | | | | |
|---|---|---|---|---|---|---|
| Eq. means equivalents | | | | | | |

Experimental procedure: In each well of a 96 well microtiter plate 68 microliters of the dissolved API are pipetted. Then, 40 microliters of the acids dissolved according to the table above are added according to the scheme as shown in the below table. The microtiter plate is shaken at room temperature for 20 min, so that equilibrium is formed in the wells. Evaporation of the solvent is effected at room temperature in a stream of nitrogen for 2 hours. Then, in each case 40 microliters of the solvent are added according to the scheme as shown in the below table. The microtiter plate is shaken at room temperature for 20 min so that equilibrium in the wells is formed. Then, the evaporation of the solvent is carried out at room temperature in a stream of nitrogen for 2 hours. Finally, the XRPD measurement is performed. The results are shown in the below table:

| acid | water | water : methanol 1:1 | methanol | ethanol | 2-propanol | 2-methylpropanol | methylethylketone | butylacetate |
|---|---|---|---|---|---|---|---|---|
| hydrochloric acid | NCP | NCP | NCP | NCP | NCP | NCP | NCP | NCP |
| sulfuric acid | NCP | NCP | NCP | NCP | NCP | NCP | NCP | NCP |
| phosphoric acid | NCP | NCP | NCP | NCP | NCP | NCP | NCP | NCP |
| acetic acid | NCP | NCP | NCP | NCP | NCP | NCP | NCP | NCP |
| tartraic acid | NCP | NCP | NCP | NCP | NCP | NCP | NCP | NCP |
| citric acid | NCP | NCP | NCP | NCP | NCP | NCP | NCP | NCP |
| fumaric acid | NCP | NCP | NCP | NCP | NCP | NCP | NCP | NCP |
| succinic acid | NCP | NCP | NCP | FAC | FAC | FAC | NCP | FAC |
| malonic acid | NCP | NCP | NCP | NCP | NCP | NCP | NCP | NCP |
| adipic acid | FAC | NCP | FAC | FAC | FAC | FAC | FAC | FAC |
| benzoic acid | FAC | NCP | FAC | FAC | FAC | FAC | FAC | FAC |
| glucuronic acid | NCP | NCP | NCP | NCP | NCP | NCP | NCP | NCP |

| | | | | | | | | |
|---|---|---|---|---|---|---|---|---|
| NCP means no crystalline phase; FAC means free acid crystallized | | | | | | | | |

The results show that with the acids and solvents tested, no crystalline salt of (R)-3-(6-amino-pyridin-3-yl)-2-(1-cyclohexyl-1 H-imidazol-4-yl)-propionic acid ethyl ester was found and it is therefore very difficult to find a solid crystalline salt of (R)-3-(6-amino-pyridin-3-yl)-2-(1-cyclohexyl-1 H-imidazol-4-yl)-propionic acid ethyl ester.

Example 3: Physical stability: Representative samples of the crystalline salt of (R)-3-(6-amino-pyridin-3-yl)-2-(1-cyclohexyl-1 H-imidazol-4-yl)-propionic acid ethyl ester with naphthalene-1,5-disulfonic acid as in Example 1 are produced and are stored for 14 days under the conditions (A), (B), (C) and (D): (A) 50°C; (B) 50°C and 80% relative humidity; (C) 80°C; (D) 80°C and 80% relative humidity. Results: The XRPD measurements at the beginning and after 14 days are close to each other. There is only a small loss in mass of thermogravimetris measurements (apparatus TA Instuments: TGA Q500, atmospheric nitrogen; rate 10°C / min) to observe the effect on the disposal of water return the crystals, the mass loss is between 1.01 % and 1.43%.

Example 4: Chiral stability: Representative samples of the crystalline salt of (R)-3-(6-amino-pyridin-3-yl)-2-(1-cyclohexyl-1 H-imidazol-4-yl)-propionic acid ethyl ester with naphthalene-1,5disulfonic acid were prepared as described in example 1 amd stored for 14 days under described conditions (A), (B), (C) and (D): Results:

| storage conditions | (A) | (B) | (C) | (D) |
|---|---|---|---|---|
| enantiomeric purity | 98.8% | 98.8% | 99.0% | 96.3% |

The enantiomeric purity of the sample measured at the beginning of measurement was 99.0%. Under the storage conditions 50°C (A) and 50°C and 80% relative humidity (B) a small increase of 0.2% of the (S)-enantiomers was observed. Under storage conditions 80°C (C), no change can be observed. Under the storage condition 80°C and 80% relative humidity (D) an increase of 2.7% of (S)-enantiomers was are observed. The measurements were made as described in Example 1 by chromatography on a chiral phase, Chiral HPLC (Chiralcel OD-H/84 (250x4.6 mm), heptane: isopropanol: acetonitrile 15:1: 0.5 +0.1% diethylamine (DEA), 30°C, 1 ml / min): Rt = 27.01 min.

### SPECIFIC INORMATION CONCERNING ASPECT (III) OF THE INVENTION

Aspect (iii) of the invention relates to an administration unit comprising crystalline form of sodium anhydrate salt of (R)-3-[6-amino-pyridin-3-yl]-2-(1-cyclohexyl-1H-imidazol-4-yl)-propionic acid and/or monohydrate salt of (R)-3-[6-amino-pyridin-3-yl]-2-(1-cyclohexyl-1H-imidazol-4-yl)-propionic acid. The invention relates to a solid form of sodium salt of (R)-3-[6-amino-pyridin-3-yl]-2-(1-cyclohexyl-1H-imidazol-4-yl)-propionic acid ({drug3}), namely to crystalline form of sodium anhydrate salt of (R)-3-[6-amino-pyridin-3-yl]-2-(1-cyclohexyl-1 H-imidazol-4-yl)-propionic acid ({drug3a}) or monohydrate salt of (R)-3-[6-amino-pyridin-3-yl]-2-(1-cyclohexyl-1 H-imidazol-4-yl)-propionic acid ({drug3b}), which is useful for treating subjects suffering from conditions which can be ameliorated by the administration of an inhibitor of the enzyme TAFIa (activated thrombin-activatable fibrinolysis inhibitor). Crystalline form of sodium anhydrate salt of (R)-3-[6-amino-pyridin-3-yl]-2-(1-cyclohexyl-1 H-imidazol-4-yl)-propionic acid ({drug3a}) and/or monohydrate salt of (R)-3-[6-amino-pyridin-3-yl]-2-(1-cyclohexyl-1 H-imidazol-4-yl)-propionic acid ({drug3b}) are described in WO2013/076178, which is incorporated by reference. As used herein, crystalline form of sodium anhydrate salt of (R)-3-[6-amino-pyridin-3-yl]-2-(1-cyclohexyl-1 H-imidazol-4-yl)-propionic acid and/or monohydrate salt of (R)-3-[6-amino-pyridin-3-yl]-2-(1-cyclohexyl-1 H-imidazol-4-yl)-propionic acid is a crystalline form of sodium salt of (R)-3-[6-amino-pyridin-3-yl]-2-(1-cyclohexyl-1 H-imidazol-4-yl)-propionic acid as described in WO2013/076178 and repeated hereinafter: The present invention relates to crystalline sodium salts of (R)-3-[6-amino-pyridin-3-yl]-2-(1-cyclohexyl-1 H-imidazol-4-yl)-propionic acid ({drug3}), which are in a crystalline form or in at least partially crystalline form as a monohydrate or anhydrate, as well as a process for the preparation of the same, methods of using such salts to treat subjects suffering from conditions which can be ameliorated by the administration of an inhibitor of the enzyme TAFIa (activated thrombin-activatable fibrinolysis inhibitor), and shows the structure illustrated in Formula (III):

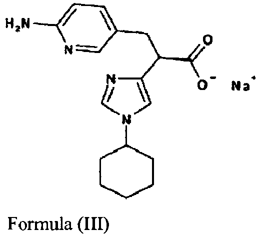

(R)-3-[6-amino-pyridin-3-yl]-2-(1-cyclohexyl-1 H-imidazol-4-yl)-propionic acid as a free acid shows the structure illustrated in Formula (III'):

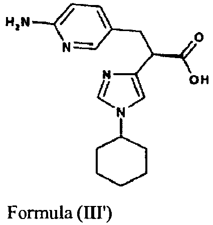

(R)-3-[6-amino-pyridin-3-yl]-2-(1-cyclohexyl-1 H-imidazol-4-yl)-propionic acid use in the preparation of a medicament for treating a patient suffering from, or subject to, conditions which can be ameliorated by the administration of an inhibitor of the enzyme TAFIa (activated thrombin-activatable fibrinolysis inhibitor), has been disclosed in WO 2005/105781. The enzyme TAFIa is produced for example through thrombin activation from the thrombin-activatable fibrinolysis inhibitor zymogen (TAFI). The enzyme TAFI is also referred to as plasma procarboxypeptidase B, procarboxypeptidase U or procarboxypeptidase R and is a proenzyme similar to carboxypeptidase B (L. Bajzar, Arterioscler. Thromb. Vase. Biol. 2000, pages 2511-2518). During formation of a clot, thrombin is generated as the final product of the coagulation cascade and induces conversion of soluble plasma fibrinogen to an insoluble fibrin matrix. At the same time, thrombin activates the endogenous fibrinolysis inhibitor TAFI. Activated TAFI (TAFIa) is thus produced during thrombus formation and lysis from the zymogen TAFI through the action of thrombin; thrombomodulin in a complex with thrombin increases this effect about 1250-fold. TAFIa cleaves basic amino acids at the carboxy end of fibrin fragments. The loss of carboxy-terminal lysines as binding sites for plasminogen then leads to inhibition of fibrinolysis. Efficient inhibitors of TAFIa prevent the loss of these high-affinity lysine binding sites for plasminogen and, in this way, assist endogenous fibrinolysis by plasmin: TAFIa inhibitors have profibrinolytic effects. In order to maintain hemostasis in the blood, mechanisms which lead to the clotting of blood and to the breaking up of clots have developed; these are in equilibrium. If a disturbed equilibrium favors coagulation, fibrin is produced in larger quantities, so that pathological processes of thrombus formation may lead to serious pathological states in humans. Just like excessive coagulation may lead to serious pathological states caused by thrombosis, an antithrombotic treatment entails the risk of unwanted bleeding through disturbance of the formation of a necessary hemostatic plug. Inhibition of TAFIa increases endogenous fibrinolysis-without influencing coagulation and platelet aggregation-i.e. the disturbed equilibrium is shifted in favor of fibrinolysis. It is thus possible both to counter the buildup of a clinically relevant thrombus, and to increase the lysis of a pre-existing clot. On the other hand, buildup of a hemostatic plug is not impaired, so that a hemorrhagic diathesis is probably not to be expected (Bouma et al., J. Thrombosis and Haemostasis, 1, 2003, pages 1566-1574). Inhibitors of TAFIa have already been described in the International Applications WO03/013526 and WO2005/105781. Processes for preparation are disclosed in WO2010/130718. 3-[6-amino-pyridin-3-yl]-2-(1-cyclohexyl-1 H-imidazol-4-yl)-propionic acid bishydrochloride salt and its pharmacological activities have been disclosed in WO2005/105781. The bishydrochloride salt of 3-[6-amino-pyridin-3-yl]-2-(1-cyclohexyl-1 H-imidazol-4-yl)-propionic acid and the free acid of 3-[6-amino-pyridin-3-yl]-2-(1-cyclohexyl-1 H-imidazol-4-yl)-propionic acid have the disadvantage to occur as amorphous solids. Thus, said hydrochloride acid salts as well as said free acid cannot be purified by crystallization. Additionally, it was found that the free acid of 3-[6-amino-pyridin-3-yl]-2-(1-cyclohexyl-1 H-imidazol-4-yl)-propionic acid is hygroscopic. Further it was found that (R)-3-[6-amino-pyridin-3-yl]-2-(1-cyclohexyl-1 H-imidazol-4-yl)-propionic acid is chemically not stable under elevated temperature and humidity. A degradation of the molecule could be observed and the enantiomeric purity of (R)-3-[6-amino-pyridin-3-yl]-2-(1-cyclohexyl-1 H-imidazol-4-yl)-propionic acid is reduced within a short time. Therefore, (R)-3-[6-amino-pyridin-3-yl]-2-(1-cyclohexyl-1 H-imidazol-4-yl)-propionic acid is not suitable for the development in a pharmaceutical formulation. Hygroscopicity is the ability of a substance to attract and hold water molecules from the surrounding environment through either absorption or adsorption with the adsorbing or absorbing material becoming physically 'changed,' somewhat, increase in volume, stickiness, or other physical characteristic changes of the material as water molecules become 'suspended' between the material's molecules in the process. Therefore hygroscopic compounds are generally very unfavorable for use in solid pharmaceutical compositions. It is an object of the present invention to find crystalline salts of (R)-3-[6-amino-pyridin-3-yl]-2-(1-cyclohexyl-1 H-imidazol-4-yl)-propionic acid with reduced absorption or adsorption of water molecules from the surrounding environment and are chemically stable. It has been found that crystalline sodium salts of (R)-3-[6-amino-pyridin-3-yl]-2-(1-cyclohexyl-1 H-imidazol-4-yl)-propionic acid have favorable reduced absorption or adsorption of water molecules from the surrounding environment and are chemically stable. In one embodiment the present invention relates to a sodium salt of (R)-3-[6-amino-pyridin-3-yl]-2-(1-cyclohexyl-1 H-imidazol-4-yl)-propionic acid, which is in a crystalline form or in at least partially crystalline form and shows the structure illustrated in Formula (III):

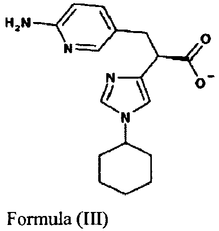

In one embodiment the present invention relates to a sodium anhydrate salt of (R)-3-[6-amino-pyridin-3-yl]-2-(1-cyclohexyl-1 H-imidazol-4-yl)-propionic acid ({drug3a}), which is in a crystalline form or in at least partially crystalline form. In another embodiment the invention relates to a sodium monohydrate salt of (R)-3-[6-amino-pyridin-3-yl]-2-(1-cyclohexyl-1 H-imidazol-4-yl)-propionic acid, which is in a crystalline form or in at least partially crystalline form ({drug3b}). Polymorphism is the ability of a single compound to exist in more than one form or crystal structure. Different polymorphs represent distinct solids sharing the same molecular formula, yet each polymorph may have distinct physical properties. A single compound may give rise to a variety of polymorphic forms wherein each form may have different and distinct physical properties, such as different solubility profiles, different thermodynamic stability, different crystallization behavior, different filterability, different melting point temperatures and/or different X-ray diffraction peaks. The difference in the physical properties of different polymorphic forms results from different orientation and intermolecular interactions of adjacent molecules in the solid. Polymorphic forms of a compound can be distinguished by X-ray diffraction and by other methods such as, infrared spectroscopy or Raman spectroscopy. "Amorphous" means a solid that exhibits in an X-ray powder diffraction pattern measured in transmission with CuKal radiation at room temperature no characteristic reflections at degrees 2 theta which can be separated from each other by their diffraction angle or specific degree 2 theta. In another embodiment the invention relates to a sodium salt of Formula (III") as a hydrate 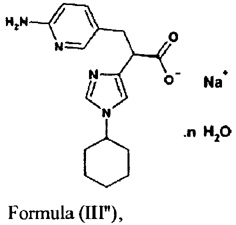 wherein n has the value from 0.7 to 1.4 ({drug3c}) and wherein n is the molar relation between water and (R)-3-[6-amino-pyridin-3-yl]-2-(1-cyclohexyl-1 H-imidazol-4-yl)-propionic acid. In another embodiment the invention relates to a sodium salt of (R)-3-[6-amino-pyridin-3-yl]-2-(1-cyclohexyl-1 H-imidazol-4-yl)-propionic acid, wherein n has the value from 0.8 to 1.2 or from 0.9 to 1.1. In another embodiment the invention relates to a crystalline sodium monohydrate salt of (R)-3-[6-amino-pyridin-3-ylj-2-(1-cyclohexyl-1 H-imidazol-4-yl)-propionic acid wherein the crystalline salt exhibits in an X-ray powder diffraction pattern measured in transmission with CuKa1 radiation at room temperature a characteristic reflection at degrees 2 theta of 22.2, 16.5 and 6.3, each time ± 0.2 degrees 2 theta ({drug3d}). In another embodiment the invention relates to a crystalline sodium monohydrate salt of (R)-3-[6-amino-pyridin-3-yl]-2-(1-cyclohexyl-1 H-imidazol-4-yl)-propionic acid wherein the crystalline salt exhibits in an X-ray powder diffraction pattern measured in transmission with CuKa1 radiation at room temperature a characteristic reflection at degrees 2 theta of 22.2, 21.8, 18.1, 16.5, 8.2 and 6.3, each time ± 0.2 degrees 2 theta ({drug3e}). In another embodiment the invention relates to a crystalline sodium monohydrate salt of (R)-3-[6-amino-pyridin-3-yl]-2-(1-cyclohexyl-1 H-imidazol-4-yl)-propionic acid wherein the crystalline salt exhibits in an X-ray powder diffraction pattern measured in transmission with CuKa1 radiation at room temperature a characteristic reflection at degrees 2 theta of 24.0, 22.2, 21.8, 18.8, 18.1, 16.8, 16.5, 16.0, 8.2 and 6.3, each time ± 0.2 degrees 2 theta ({drug3f}). The selection of characteristic reflections was determined by the number of reflections at a specified degree 2 theta. In another embodiment the crystalline sodium monohydrate salt of (R)-3-[6-amino-pyridin-3-yl]-2-(1-cyclohexyl-1 H-imidazol-4-yl)-propionic acid may also be characterized by its X-ray powder diffraction pattern substantially by the one shown in Figure 1 of WO2013/076178 which has been obtained using CuKa1 radiation in transmission mode, wherein the intensities of the reflections depicted in the Figure as well as those of the reflections specified above are not a prerequisite, but may vary. In another embodiment the crystalline sodium monohydrate salt of (R)-3-[6-amino-pyridin-3-yl]-2-(-cyclohexyl-1 H-imidazol-4-yl)-propionic acid may also be characterized by its Raman spectrum. The Raman spectrum was recorded on RXN1-Dispersive Raman spectrometer equipped with PhAT-probe head (for solids) and 785 nm diode laser (400 mW) from Kaiser Optical Systems, Ltd., USA; Software Details; Aquisition: Holograms, Kaiser; Polynomial spline background correction performed in Software: OPUS 6.5 (from Bruker Optics, Ettlingen, Germany). The characteristic wave number of the Raman shift has the following values (cm⁻¹) and the relative intensities are shown in brackets: 818 (31), 864 (100), 1054 (24), 1284 (26), 1561 (37), and 1613 (27), each time ± 4 cm⁻¹ ({drug3g}). In another embodiment the crystalline sodium monohydrate salt of (R)-3-[6-amino-pyridin-3-yl]-2-(1-cyclohexyl-1 H-imidazol-4-yl)-propionic acid may also be characterized by its Raman spectrum. The characteristic wave number of the Raman shift has the following values (cm⁻¹) and the relative intensities are shown in brackets: 657 (10), 818 (31), 864 (100), 981 (15), 1030 (18), 1054 (24), 1137 (10), 1216 (21), 1266 (20), 1284 (26), 1309 (16), 1351 (10), 1363 (19), 1407 (16), 1445 (20), 1561 (37) and 1613 (27), each time ± 4 cm⁻¹ ({drug3h}). The selection of characteristic wave number was determined by the number of reflections at a specified wave number. In another embodiment the crystalline sodium monohydrate salt of (R)-3-[6-amino-pyridin-3-yl]-2-(-cyclohexyl-1 H-imidazol-4-yl)-propionic acid may also be characterized by its Raman spectrum pattern, wherein the Raman shift is characterized by the following values (cm⁻¹) and the relative intensities are shown in brackets: 431 (3), 484 (3), 539 (2), 566 (6), 657 (10), 759 (8), 798 (2), 818 (31), 864 (100), 882 (8), 920 (6), 968 (9), 981 (15), 999 (3), 1030 (18), 1054 (24), 1077 (6), 1137 (10), 1186 (8), 1202 (6), 1216 (21), 1242 (4), 1266 (20), 1284 (26), 1309 (16), 1351 (10), 1363 (19), 1407 (16), 1445 (20), 1495 (3), 1561 (37), 1613 (27), each time ± 4 cm⁻¹ ({drug3i}). The intensities of the Raman shift wave numbers depicted above as well as those of the wave numbers specified above are not a prerequisite, but may vary. The crystalline sodium monohydrate salt of (R)-3-[6-amino-pyridin-3-yl]-2-(1-cyclohexyl-1 H-imidazol-4-yl)-propionic acid may also be characterized by its crystal lattice parameters which have been determined by indexing its powder pattern. The crystalline sodium monohydrate salt crystallizes in the monoclinic crystal system with a = 14.109(3) A, b = 5.8202(13) A, c = 10.912(3) A, a = 90.00°, β = 97.958(11)°, γ = 90.00°, volume = 887.5(5) A3. Moreover, crystalline sodium monohydrate salt of (R)-3-[6-amino-pyridin-3-yl]-2-(1-cyclohexyl-1 H-imidazol-4-yl)-propionic acid may also be characterized by its dynamic vapor sorption (DVS) water vapor sorption and desorption isotherms measured at 25°C. As shown in the examples the sorption and desorption isotherms between 30% relative humidity and 0.4% relative humidity are almost the same (low water uptake or water desorption) wherein a water uptake of 4.0% at 70% relative humidity (RH), 11.9% at 80% RH, 38.2% at 90% RH and 67.4% at 95% RH takes place. Figure 1 of WO2013/076178 -X-ray powder diffraction pattern of crystalline sodium monohydrate salt of (R)-3-[6-amino-pyridin-3-yl]-2-(1-cyclohexyl-1 H-imidazol-4-yl)-propionic acid, measured in transmission mode with CuKa1 radiation at room temperature (x-axis: diffraction angle 2theta (20) [°]; y-axis: relative intensity. Figure 2 of of WO2013/076178 -X-ray powder diffraction pattern of crystalline sodium anhydrate salt of (R)-3-[6-amino-pyridin-3-yl]-2-(1-cyclohexyl-1 H-imidazol-4-yl)-propionic acid, measured in transmission mode with CuKa1 radiation at 110°C (x-axis: diffraction angle 2theta (20) [°]; y-axis: relative intensity. The sodium salt of (R)-3-[6-amino-pyridin-3-yl]-2-(1-cyclohexyl-1 H-imidazol-4-yl)-propionic acid may be prepared by dissolving of (R)-3-[6-amino-pyridin-3-yl]-2-(1-cyclohexyl-1 H-imidazol-4-yl)-propionic acid in acetone or aqueous-acetone mixture or other suitable solvents to which the aqueous sodium base such as sodium hydroxide, disodium carbonate, sodium hydrogen carbonate or sodium ethanolate or a mixture thereof is added. Under stirring, the mixture can be heated to about 55°C yielding a clear solution and subsequently cooling to about 1 °C yields a precipitate. The precipitate obtained can be filtered, washed with water and dried under reduced pressure. A useful molar relation between a sodium base such as sodium hydroxide and (R)-3-[6-amino-pyridin-3-yl]-2-(1-cyclohexyl-1 H-imidazol-4-yl)-propionic acid is from 1 to 2. A further useful molar relation between sodium hydroxide and (R)-3-[6-amino-pyridin-3-yl]-2-(1-cyclohexyl-1 H-imidazol-4-yl)-propionic acid is from 1.2 to 1.5. In general, the crystalline sodium monohydrate salt of (R)-3-[6-amino-pyridin-3-yl]-2-(1-cyclohexyl-1 H-imidazol-4-yl)-propionic acid may also be prepared by dissolving of (R)-3-[6-amino-pyridin-3-yl]-2-(1-cyclohexyl-1 H-imidazol-4-yl)-propionic acid in a suitable solvent such as an alcohol, e.g. methanol, ethanol, 2-propanol; and can be obtained by crystallizing or recrystallizing compound of Formula (III), starting from a solution of compound of Formula (III) or from a suspension of compound of Formula (III) or from solid compound of Formula (III). A solution of compound of Formula (III), or a suspension of compound of Formula (III), may have been obtained at the end of the chemical synthesis of compound of Formula (III), or it may have been obtained by dissolving or suspending previously synthesized crude compound of Formula (III). The term "crude compound of Formula (III)"comprises any form of compound of Formula (III), e.g. the material directly obtained from chemical synthesis, a distinct crystalline form or amorphous material of the compound of Formula (III). More specifically, the crystalline salt of Formula (III) of the invention can be obtained by
(a) providing a solution or suspension of compound of Formula (III), for example by dissolving or suspending crude compound Formula (III) in a suitable solvent such as an alcohol, e.g. methanol, ethanol, 2-propanol; or a mixture thereof, wherein a solution of compound of Formula (III) generally is a clear solution and may optionally have been filtered, (b) maintaining, heating, cooling or concentrating the solution or suspension with or without agitation such as stirring, to form crystals of a desired distinct crystalline form of Formula (III"), and (c) isolating the distinct crystalline salt of Formula (III"). The processes for preparing crystalline forms and solvates of compound of Formula (III") can be performed with conventional equipment and according to standard procedures. For example, concentrating of a solution or suspension in step
(b) may be done by distilling off solvent partially or totally at atmospheric pressure or at reduced pressure. Isolating of a crystalline form or solvate in step (c) may be done by any conventional technique such as filtration or vacuum filtration or centrifugation. Isolating may also comprise drying, e.g. by applying elevated temperatures and/or reduced pressure, for example at moderately reduced pressure at about room temperature, i.e. a temperature of about 18 °C to about 55 °C, for example about 20 °C, or at about 55 °C. In a preferred embodiment, the solution or suspension may be seeded in step (b) to promote crystallization. Seeding is preferably done with a small amount of the crystalline salt of Formula (III") already prepared.

In another embodiment the invention relates to a crystalline sodium anhydrate salt of (R)-3-[6-amino-pyridin-3-yl]-2-(1-cyclohexyl-1 H-imidazol-4-yl)-propionic acid wherein the crystalline salt exhibits in an X-ray powder diffraction pattern measured in transmission with CuKa1 radiation at 110°C a characteristic reflection at degrees 2 theta of 21.4, 16.8 and 6.1, each time ± 0.2 degrees 2 theta ({drug3j}). In another embodiment the invention relates to a crystalline sodium anhydrate salt of (R)-3-[6-amino-pyridin-3-yl]-2-(1-cyclohexyl-1 H-imidazol-4-yl)-propionic acid wherein the crystalline salt exhibits in an X-ray powder diffraction pattern measured in transmission with CuKa1 radiation at 110°C a characteristic reflection at degrees 2 theta of 22.4, 21.4, 16.8, 16.4, 8.6 and 6.1, each time ± 0.2 degrees 2 theta ({drug3k}). In another embodiment the invention relates to a crystalline sodium anhydrate salt of (R)-3-[6-amino-pyridin-3-yl]-2-(1-cyclohexyl-1 H-imidazol-4-yl)-propionic acid wherein the crystalline salt exhibits in an X-ray powder diffraction pattern measured in transmission with CuKa1 radiation at 110°C a characteristic reflection at degrees 2 theta of 22.4, 21.4, 18.7, 18.4, 18.2, 18.0, 17.5, 16.8, 16.4, 14.0, 8.6 and 6.1, each time ± 0.2 degrees 2 theta ({drug31}). The selection of characteristic reflections was determined by the number of reflections at a specified degree 2 theta. In another embodiment the crystalline sodium anhydrate salt of (R)-3-[6-amino-pyridin-3-yl]-2-(1-cyclohexyl-1 H-imidazol-4-yl)-propionic acid may also be characterized by its X-ray powder diffraction pattern substantially by the one shown in Figure 2 of WO2013/076178, which has been obtained using CUKa radiation in transmission mode, wherein the intensities of the reflections depicted in the Figure as well as those of the reflections specified above are not a prerequisite, but may vary. In another embodiment the crystalline sodium anhydrate salt of (R)-3-[6-amino-pyridin-3-yl]-2-(1-cyclohexyl-1 H-imidazol-4-yl)-propionic acid may also be characterized by its Raman spectrum. The characteristic wave number of the Raman shift has the following values (cm⁻¹) and the relative intensities are shown in brackets: 1604 (46), 1552 (43), 1447 (43), 1281 (46), 1262 (43), 861 (100), and 818 (49), each time ± 4 cm⁻¹ ({drug3m}). In another embodiment the crystalline sodium anhydrate salt of (R)-3-[6-amino-pyridin-3-yl]-2-(1-cyclohexyl-1 H-imidazol-4-yl)-propionic acid may also be characterized by its Raman spectrum. The characteristic wave number of the Raman shift has the following values (cm⁻¹) and the relative intensities are shown in brackets: 1604 (46), 1552 (43), 1447 (43), 1365 (31), 1349 (34), 1281 (46), 1262 (43), 1051 (37), 861 (100), and 818 (49), each time ± 4 cm⁻¹ ({drug3n}). The selection of characteristic wave number was determined by the number of reflections at a specified wave number. In another embodiment the crystalline sodium anhydrate salt of (R)-3-[6-amino-pyridin-3-yl]-2-(1-cyclohexyl-1 H-imidazol-4-yl)-propionic acid may also be characterized by its Raman spectrum pattern, wherein the Raman shift is characterized by the following values (cm⁻¹) and the relative intensities are shown in brackets: 1604 (46), 1552 (43), 1502 (9), 1447 (43), 1398 (24), 1365 (31), 1349 (34), 1328 (24), 1302 (25), 1281 (46), 1262 (43), 1216 (22), 1189 (10), 1174 (9), 1156 (10), 1140 (16), 1074 (11), 1051 (37), 1029 (25), 1003, (13), 976 (17), 964 (12), 920 (6), 895 (10), 861 (100), 818 (49), 791 (6), 759 (10), 731 (6), 702 (4), 682 (5), 648 (19), 570 (3), 531 (6), 512 (4), 486 (9), 447 (9), 418 (10), 402 (7), each time ± 4 cm⁻¹ ({drug3o}). The intensities of the Raman shift wave numbers depicted above as well as those of the wave numbers specified above are not a prerequisite, but may vary. The crystalline sodium anhydrate salt of (R)-3-[6-amino-pyridin-3-yl]-2-(1-cyclohexyl-1 H-imidazol-4-yl)-propionic acid may also be characterized by its crystal lattice parameters which have been determined by indexing its powder pattern. The crystalline sodium anhydrate salt crystallizes in the triclinic crystal system at 110°C with a = 14.570(14) A, b = 5.769(5) A, c = 10.462(9) A, a = 93.87(5)°, β = 97.24(4)°, γ = 91.33(5)°, volume = 870.0(19) A3. In general, the crystalline sodium anhydrate salt of (R)-3-[6-amino-pyridin-3-yl]-2-(1-cyclohexyl-1 H-imidazol-4-yl)-propionic acid may also be prepared by storage of already prepared crystalline sodium monohydrate salt of (R)-3-[6-amino-pyridin-3-yl]-2-(1-cyclohexyl-1 H-imidazol-4-yl)-propionic acid at a relative humidity of 0.2 % or heating said monohydrate crystals at temperatures from 90 °C to 120 °C. The sodium monohydrate or anhydrate salts of (R)-3-[6-amino-pyridin-3-yl]-2-(1-cyclohexyl-1 H-imidazol-4-yl)-propionic acid (in the following compound of Formula (III)) of the present invention may be useful in the prophylaxis and therapy of all disorders which can be treated by inhibition of TAFIa. Thus, the compound of Formula (III) is suitable both for a prophylactic and for a therapeutic use in humans and is suitable both for an acute treatment and for a long-term therapy. The compound of Formula (III) can be employed in patients suffering from impairments of wellbeing or diseases associated with thromboses, embolisms, hypercoagulability or fibrotic changes. These include myocardial infarction, angina pectoris and all other types of acute coronary syndrome, stroke, peripheral vascular disorders, deep vein thrombosis, pulmonary embolism, embolic or thrombotic events caused by cardiac arrhythmias, cardiovascular events such as restenosis following revascularization, angioplasty and similar procedures such as stent implantations and bypass operations. The compound of Formula (III) can additionally be employed in all procedures leading to contact of the blood with foreign surfaces such as, for example, for dialysis patients and patients with indwelling catheters. The compound of Formula (III) can be employed to reduce the risk of thrombosis after surgical procedures such as knee and hip joint operations. The compound of Formula (III) may be suitable for the treatment of patients with disseminated intravascular coagulation, sepsis and other intravascular events associated with an inflammation and is additionally suitable for the prophylaxis and treatment of patients with atherosclerosis, diabetes and the metabolic syndrome and its sequelae. The compound of Formula (III) may further be used to inhibit fibrotic changes of the lung such as chronic obstructive lung disease, adult respiratory distress syndrome (ARDS) and of the eye such as fibrin deposits after eye operations. The compound of Formula (III) may also be suitable for the prevention and/or treatment of scar formation. The invention also relates to a process for producing a pharmaceutical composition, which comprises making a suitable dosage form from a compound of the Formula (III) with a pharmaceutically suitable and physiologically tolerated carrier and, where appropriate, further suitable active ingredients, additives or excipients. Suitable solid or pharmaceutical formulations are, for example, granules, powder, coated tablets, tablets, (micro)capsules, suppositories, syrups, solutions, suspensions, emulsions, drops or injectable solutions, and products with protrated release of active ingredient, in the production of which normally physiologically suitable aids such as carriers, disintegrants, binders, coating agents, swelling agents, glidants or lubricants, flavorings, sweeteners and solubilizers are used. Excipients which are frequently used and which may be mentioned are magnesium carbonate, titanium dioxide, lactose, mannitol and other sugars, talc, milk protein, gelatin, starch, cellulose and its derivatives, animal and vegetable oils such as fish liver oil, sunflower, peanut or sesame oil, polyethylene glycol and solvents such as, for example, sterile water and monohydric or polyhydric alcohols such as glycerol. The pharmaceutical products are preferably produced and administered in dosage units, where each unit comprises as active ingredient a particular dose of the compound of the Formula (III). In the case of solid dosage units such as tablets, capsules, coated tablets or suspensions, this dose can be up to about 1000 mg, but preferably about 50 to 300 mg and, in the case of injection solutions in ampoule form, up to about 300 mg but preferably about 10 to 100 mg. The daily doses indicated for the treatment of an adult patient weighing about 70 kg are, depending on the activity of the compound of Formula (III), from about 2 mg to 1000 mg of active ingredient, preferably about 50 mg to 500 mg. However, in some circumstances, higher or lower daily doses may also be appropriate. The compound of Formula (III) according to the invention may be administered as frequently as necessary to obtain the desired therapeutic effect. Some patients may respond rapidly to a higher or lower dose and may find much weaker maintenance doses adequate. For other patients, it may be necessary to have long-term treatments at the rate of about 1 to about 4 doses per day, in accordance with the physiological requirements of each particular patient. Generally, the active product may be administered orally about 1 to about 4 times per day. It goes without saying that, for other patients, it may be necessary to prescribe not more than one or two doses per day. The medicaments of the invention can be administered by oral, inhalational, rectal or transdermal administration or by subcutaneous, intraarticular, intraperitoneal or intravenous injection. Intravenous injection is preferred. As would be apparent to a person of ordinary skill in the art, once armed with the teachings of the present disclosure, when dissolved, crystalline sodium salt of Formula (III) loses its crystalline structure, and is therefore considered to be a solution of sodium salt of Formula (III). All forms of the present invention, however, may be used for the preparation of liquid formulations in which crystalline sodium salt of Formula (III) may be, for example, dissolved or suspended. In addition, the crystalline sodium salt of Formula (III) may be incorporated into solid formulations. The following non-limiting examples illustrate the inventors' preferred methods for preparing and using the sodium salt of Formula (III) of the present invention.

### Example 1: Preparation of (R)-3-[6-amino-pyridin-3-yl]-2-(-cyclohexyl-1 H-imidazol-4-yl)-propionic acid free acid:

a) (E)-2-(1-cyclohexyl-1 H-imidazol-4-yl)-3-hydroxy-but-2-enedioic acid diethyl ester: 54 g (0.23 mol) (1-Cyclohexyl-1 H-imidazol-4-yl)-acetic acid ethyl ester were dissolved in 300 ml absolute ethanol. Diethyl oxalate (77 ml, 0.57 mmol), followed by sodium ethylate (23 g, 0.34 mol) were added successively thereby the temperature rose to 35°C. The mixture was allowed to cool slowly down to 25°C within one hour. The solution was concentrated and poured into 1 liter (I) phosphate buffer (pH = 6.2, c = 0.8 M). The yellow-brown solid was filtered and washed with 300 ml of water. The solid was then dried under reduced pressure at 60 °C. The dry residue was digested with 300 ml of preferably tert.-butyl methyl ether (MTBE) at 40 °C for 2 hours, cooled to RT, filtered and dried on air to gave 53 g (0.16 mol, 69%) (E)-2-(1-cyclohexyl-1 H-imidazol-4-yl)-3-hydroxy-but-2-enedioic acid diethyl ester as an off white solid. 1H-NMR (400 MHz, d6-DMSO): δ = 1.17 (t, J = 7.1 Hz, 3H), 1.23 (t, J = 7.2 Hz, 3H), 1.20-1.29 (m, 1 H), 1.30-1.44 (m, 2H), 1.61-1.77 (m, 3H), 1.78-1.88 (m, 2H), 1.98-2.07 (m, 2H), 4.03 (q, J = 7.1 Hz, 2H), 4.10 (q, J = 7.1 Hz, 2H), 4.22-4.31 (m, 1 H), 7.49 (d, J = 1.8 Hz, 1 H), 8.57 (d, J = 1.7 Hz, 1 H), 13.4-14.1 (bs, 1 H); HPLC: tR = 1.10 min (YMC J' sphere ODS H 80 20x2.1 mm, 4 pm, A: H2O+0.05% TFA, B: MeCN, 4%→ 95% B in 2 min, 1 ml/min, 30 °C); Mass (ES+) (Ci7H24N2O5): calculated. 366, found 337 [M+H]+, Melting point (Mp): >150°C decomposition, (tert-butyl methyl ether; MTBE).
b) 3-(6-tert-Butoxycarbonylamino-pyridin-3-yl)-2-(1-cyclohexyl-1 H-imidazol-4-yl)-propionic acid ethyl ester: The compound prepared according to process step a) (5.0 g, 14.9 mmol) was suspended in 80 ml tetrahydrofuran (THF) and lithium hexamethyldisilazide (LiHMDS) (16.4 ml, 16.4 mmol, 1.0 M in THF) was added at room temperature (RT). The mixture was stirred for 20 min, tetra-(n)-butylammonium iodide (catalytic) was added, followed by (5-bromomethyl-pyridin-2-yl)-carbamic acid tert-butyl ester (4.48 g, 15.6 mmol). After one hour water (20 ml), ethyl acetate (AcOEt; 50 ml) were added and the pH-value was adjusted to 10.5 with saturated K₂CO₃. The mixture was allowed stirring for 90 min. The phases were separated and the aqueous layer was extracted with AcOEt (2x 20 ml). The combined organic layers were washed with brine (20 ml), dried with MgSO4 and concentrated to yield a red-orange crude product. The residue was treated with 50 ml MTBE and stirred for 30 min at 60°C. After cooling to RT the solid was filtered and washed with cold MTBE to yield the title compound (3.1 g, 7.0 mmol, 47%) as a yellowish solid. 1H-NMR (500 MHz, d6-DMSO): δ = 1.05 (t, J = 7.1 Hz, 3H), 1.13-1.24 (m, 1 H), 1.29-1.40 (m, 2H), 1.46 (s, 9H), 1.53-1.67 (m, 3H), 1.74-1.82 (m, 2H), 1.88-1.96 (m, 2H), 3.76-3.81 (m, 1 H), 3.91-4.05 (m, 3H), 7.05 (s, 1 H), 7.48 (dd, J = 8.6, 1.9 Hz, 1 H), 7.58 (s, 1 H), 7.64 (d, J = 8.6 Hz, 1 H), 7.99 (d, J = 1.9 Hz, 1 H), 9.62 (s, 1 H); HPLC: tR = 1.00 min (YMC J' sphere ODS H 80 20x2.1 mm, 4 pm, A: H2O+0.05% TFA, B: MeCN, 4%→ 95% B in 2 min, 1 ml/min, 30°C); Mass (ES+) (C2 H34N4O4): calculated. 442, found 443 [M+H]+, Mp: 161-163°C (MTBE).
c) 3-(6-amino-pyhdin-3-yl)-2-(1-cyclohexyl-1 H-imidazol-4-yl)-propionic acid ethyl ester: 39.0 g (88.1 mmol) 3-(6-tert-butoxycarbonylamino-pyridin-3-yl)-2-(1-cyclohexyl-1 H-imidazol-4-yl)-propionic acid ethyl ester as prepared in process step b) were suspended in 200 ml ethanol (EtOH) at RT. The mixture was saturated with HCl and was then refluxed for 2 h. The mixture was concentrated and the residue was treated with saturated aqueous K₂CO₃ (pH 9.0). The aqueous layer was extracted with AcOEt (3x 200 ml). The combined organic layers were washed with brine (100 ml), dried with MgSO₄ and concentrated to yield the title compound (30 g, 87.6 mmol, 99%) as brown oil. HPLC: tR = 0.69 min (YMC J' sphere ODS H 80 20x2.1 mm, 4 pm, A: H2O+0.05% TFA, B: MeCN, 4%→ 95% B in 2 min, 1 ml/min, 30°C); Mass (ES+) (Ci9H26N4O2): calculated. 342, found 343 [M+H]+.
d) (R)-3-(6-amino-pyridin-3-yl)-2-(1-cyclohexyl-1 H-imidazol-4-yl)-propionic acid ethyl ester: The two enantiomers of 3-(6-amino-pyridin-3-yl)-2-(1-cyclohexyl-1 H-imidazol-4-yl)-propionic acid ethyl ester were separated by chiral chromatography: Batches containing 540 g of 3-(6-amino-pyridin-3-yl)-2-(1-cyclohexyl-1 H-imidazol-4-yl)-propionic acid ethyl ester were filled into a column containing 20 kg of Chiralpack AD 20 µm (Ciral Technologies Europe, France) as a chiral stationary phase. Ethanol was pumped through the column until no more (S)-3-(6-amino-pyridin-3-yl)-2-(1-cyclohexyl-1 H-imidazol-4-yl)-propionic acid ethyl ester was eluted. Then a mixture containing ethanol with 0.4 % of trifluoroacetic acid were pumped through the column and fractions containing (R)-3-(6-amino-pyridin-3-yl)-2-(1-cyclohexyl-1 H-imidazol-4-yl)-propionic acid ethyl ester were collected. The received title compound showed a purity of 91.7 % and an ee of 99.2 %. The purity was determined by the chromatography described in Example 8.
e) ((R)-3-[6-amino-pyridin-3-yl]-2-(1-cyclohexyl-1 H-imidazol-4-yl)-propionic acid ethyl ester, naphtalene 1,5-disulfonic acid salt: 1.50 g (5.20 mmol, 1.2 equivalents) naphthalene-1,5-disulfonic acid were dissolved in 15 ml water. The received solution was cooled to 10°C. 1.48 g (4.32 mmol, 1.0 equivalents) (R)-3-(6-amino-pyridin-3-yl)-2-(1-cyclohexyl-1 H-imidazol-4-yl)-propionic acid ethyl ester as prepared in process step d) were dissolved in 15 ml of a acetone/water mixture 2: 3 and slowly added to the naphthalene-1, 5-disulfonic acid solution. The solvents were slowly evaporated by an argon gas stream until first crystals occurred. The argon gas stream is stopped and the suspension was stirred for three hours at RT (20°C to 22°C). The residues were filtered off and dried over night by air. 1.83 g (2.90 mmol, 67%) of the crystalline (R)-3-(6-amino-pyridin-3-yl)-2-(1-cyclohexyl-1 H-imidazol-4-yl)-propionic acid ethyl ester 1,5-naphthyldisulfonic acid salt was received as a white solid.
f) 60 g (R)-3-[6-amino-pyridin-3-yl]-2-(1-cyclohexyl-1 H-imidazol-4-yl)-propionic acid ethyl ester, naphtalene 1,5-disulfonic acid salt as prepared in process step e) were stirred overnight in 480 ml 6 N hydrochloric acid. The solvent of the reaction mixture was evaporated to dryness. The dry residue was dissolved in 222 ml of water and given on a Dowex 50WX8-200 ion exchange resin. The ion exchanger was washed with water until no 1,5-naphthyldisulfonate was detected in the water after the washing process. The product was then eluted from the ion exchanger with 2.5% ammonium hydroxide solution. The product fractions were collected and the solvent of these fractions was distilled off. The obtained solid was dissolved in 500 ml water and subsequently lyophilized. 26.8 g light yellowish powder were obtained (91 % yield).

Example 2: Preparation of sodium monohydrate salt of (R)-3-[6-amino-pyridin-3-yl]-2-(1-cyclohexyl-1 H-imidazol-4-yl)-propionic acid by salt screening on a microreactor plate: 300 µl of the (R)-3-[6-amino-pyridin-3-yl]-2-(1-cyclohexyl-1 H-imidazol-4-yl)-propionic acid solution of the compound prepared in Example 1 (stock solution: 250 mg (R)-3-[6-amino-pyridin-3-yl]-2-(1-cyclohexyl-1 H-imidazol-4-yl)-propionic acid of the compound prepared in Example 1 free acid in 30 ml methanol) were dispensed into a microreactor plate well at 18°C. 90 µl aqueous NaOH solution (1 equivalent OH⁻ ions) were given into the well at 18°C. The microreactor plate was then covered by a lid and shaken for two hours on a vortexer at a temperature of 25°C. Subsequently the solvent was evaporated in a stream of nitrogen for 5 hours at 25°C. Afterwards 200 (µl of water/methanol mixture (1:4) were added at 18°C. The microreactor plate was then covered by a lid and shaken for 5 hours on a vortexer at a temperature of 25°C. Then the lid was cooled to 2°C for 5 hours to facilitate the nucleation of crystalline salts. After removal of the lid the solvents were allowed to evaporate at 25°C for 5 hours in a stream of nitrogen.

Example 3: Preparation of (R)-3-[6-amino-pyridin-3-yl]-2-(1-cyclohexyl-1 H-imidazol-4-yl)-propionic acid sodium monohydrate salt by evaporative crystallization: 8.0 g (R)-3-[6-amino-pyridin-3-yl]-2-(1-cyclohexyl-1 H-imidazol-4-yl)-propionic acid as prepared in Example 1 were dissolved in 106 g ethanol at 25°C. The solution was heated to 53°C during 30 min. Within this time frame 14.4g NaOH solution (7.5 w/w-%) were dosed into the solution. After reaching 55°C the solution was cooled in 2 h to 0°C. Subsequently the solvent was evaporated, giving a solid crystalline product (Yield: 68%), which was identified by X-ray diffraction and thermal analysis as monohydrate sodium salt of SAR126119 (Form A).

Example 4: Differential scanning calorimetry (DSC) of (R)-3-[6-amino-pyridin-3-yl]-2-(1-cyclohexyl-1 H-imidazol-4-yl)-propionic acid sodium monohydrate salt: The DSC curve of (R)-3-[6-amino-pyridin-3-yl]-2-(1-cyclohexyl-1 H-imidazol-4-yl)-propionic acid sodium salt monohydrate as prepared in Example 3 was measured on a Q1000 from TA instruments in closed pan with the following method: Equilibrate at-30°C, Ramp 10 K/min to 300°C, Amount of the sample as prepared in Example 3: 7.2mg. The DSC thermogram of the crystalline material of Example 3 shows two endothermic events in range from 10°C to 130°C, which corresponds to the loss of water.

Example 5: Thermogravimetric analysis (TGA) of (R)-3-[6-amino-pyridin-3-yl]-2-(1-cyclohexyl-1 H-imidazol-4-yl)-propionic acid sodium salt monohydrate: The TGA diagram of (R)-3-[6-amino-pyridin-3-yl]-2-(1-cyclohexyl-1 H-imidazol-4-yl)-propionic acid sodium salt monohydrate as prepared in Example 3 was recorded on TA Instruments TGA Q500 equipment in nitrogen atmosphere with the following method: Equilibrate at 25°C, Ramp: 10 K/min to 350°, Amount of the sample: 10-20mg, Sample purge 25 ml/min. The TGA thermogram of the crystalline sample of Example 3 showed 4.9% weight loss between 26 °C and 150°C, which corresponds to about 1 mol of water of (R)-3-[6-amino-pyridin-3-yl]-2-(1-cyclohexyl-1 H-imidazol-4-yl)-propionic acid sodium salt monohydrate.

Example 6: X-ray powder diffraction (XRPD) of (R)-3-[6-amino-pyridin-3-yl]-2-(1-cyclohexyl-1 H-imidazol-4-yl)-propionic acid sodium salt monohydrate: X-ray powder diffraction was performed with a Stoe Stadi-P transmission diffractometer using CuKa1 radiation (wavelength is 1.54060 Angstrom) and a linear position sensitive detector. Unless stated otherwise, X-ray powder diffraction was performed at room temperature. Samples were investigated in flat preparation. The measured data were evaluated and plotted with the Software WinXPOW V2.12. The observed X-ray powder diffraction pattern of (R)-3-[6-amino-pyridin-3-yl]-2-(1-cyclohexyl-1 H-imidazol-4-yl)-propionic acid sodium salt monohydrate as prepared in Example 3 is displayed in the Figure 1 of WO2013/076178. The 20 (2theta) angles in°(degree) are specified as the number of characteristic reflections. The 2theta angles in degree have the following values in Figure 1 of WO2013/076178 and the relative intensities are shown in brackets: 6.33 (67), 8.19 (40), 9.61 (7), 14.11 (9), 16.03 (15), 16.50 (100), 16.75 (17), 17.30 (10), 18.04 (30), 18.42 (6), 18.83 (22), 19.04 (9), 19.69 (8), 19.84 (9), 20.81 (6), 21.80 (32), 22.17 (76), 22.44 (14), 22.70 (12), 23.41 (11), 23.96 (18), 24.48 (12), 24.69 (10), 25.49 (5), 25.69 (5), 26.37 (6), 26.68 (12), 26.95 (5), 27.90 (4), 28.42 (4), 29.10 (8), 29.41 (5), 29.81 (5), 30.43 (5), 30.70 (4), 31.07 (6), 31.84 (6), 32.37 (7), 33.05 (8), 33.27 (5).

Example 7: Dynamic vapor sorption (DVS) of (R)-3-[6-amino-pyridin-3-yl]-2-(1-cyclohexyl-1 H-imidazol-4-yl)-propionic acid sodium salt monohydrate: Moisture sorption/desorption isotherms were recorded on a SPS11-10µ from Projekt Messtechnik (Ulm, Germany). One cycle was run at 25 °C, in which the sample was first stepwise decreased to 0% RH and then the relative humidity was stepwise increased from 0 to 95% and the weight of the sample was measured. Typical total measurement times for both cycles were about 20 to 30 hours. The measured data for the (R)-3-[6-amino-pyridin-3-yl]-2-(1-cyclohexyl-1 H-imidazol-4-yl)-propionic acid sodium salt monohydrate as prepared in Examples 2 or 3 are shown in the following table:

**DVS Isotherm at 25 °C:**

| | Target RH (%) | Change In Mass (%) Sorption | Change In Mass (%) Desorption |
|---|---|---|---|
| Cycle 1 | 30.1 | | 0.1 |
| | 20.0 | | -0.1 |
| | 10.0 | | -0.6 |
| | 0.2 | | -4.4 |
| | 10.0 | -3.7 | |
| | 20.0 | -0.2 | |
| | 30.0 | 0.2 | |
| | 40.0 | 0.6 | |
| | 50.0 | 0.9 | |
| | 60.0 | 1.5 | |
| | 70.0 | 4.0 | |
| | 75.0 | 8.2 | |
| | 80.1 | 11.9 | |
| | 85.1 | 17.7 | |
| | 90.0 | 38.2 | |
| | 94.9 | 67.4 | |

"RH"means relative humidity; the relative humidity of an air-water mixture is defined as the ratio of the partial pressure of water vapor in the mixture to the saturated vapor pressure of water at a prescribed temperature. DVS showed desorption of - 4.4% water at 0.2% RH accompanied by a phase transition from the monohydrate phase into the anhydrate phase (detected by Raman spectroscopy and XRPD). Upon rehydration at 20% RH the original monohydrate phase was regained. The monohydrate phase of (R)-3-[6-amino-pyridin-3-yl]-2-(1-cyclohexyl-1 H-imidazol-4-yl)-propionic acid sodium salt monohydrate showed high water uptake of 4.0% at 70% RH, 11.9% at 80% RH, 38.2% at 90% RH and 67.4% at 95% RH for the (R)-3-[6-amino-pyridin-3-yl]-2-(1-cyclohexyl-1 H-imidazol-4-yl)-propionic acid sodium salt monohydrate.

Example 8: Preparation of (R)-3-[6-amino-pyridin-3-yl]-2-(1-cyclohexyl-1 H-imidazol-4-yl)-propionic acid sodium anhydrate salt by drying (R)-3-[6-amino-pyridin-3-yl]-2-(1-cyclohexyl-1 H-imidazol-4-yl)-propionic acid sodium monohydrate salt at low humidity: The anhydrate sodium salt of (R)-3-[6-amino-pyridin-3-yl]-2-(1-cyclohexyl-1 H-imidazol-4-yl)-propionic acid sodium was prepared by storage of the (R)-3-[6-amino-pyridin-3-yl]-2-(1-cyclohexyl-1 H-imidazol-4-yl)-propionic acid sodium monohydrate salt in a SPS11-10µ device from Projekt Messtechnik (Ulm, Germany) at 25°C and 0.2% relative humidity. The crystalline phase of the anhydrate was identified by Raman spectroscopy.

Example 9: Preparation of (R)-3-[6-amino-pyridin-3-yl]-2-(1-cyclohexyl-1 H-imidazol-4-yl)-propionic acid sodium anhydrate salt by heating (R)-3-[6-amino-pyridin-3-yl]-2-(1-cyclohexyl-1 H-imidazol-4-yl)-propionic acid sodium monohydrate salt above 90°C: The anhydrate sodium salt of (R)-3-[6-amino-pyridin-3-yl]-2-(-cyclohexyl-1 H-imidazol-4-yl)-propionic acid was prepared by heating of the (R)-3-[6-amino-pyridin-3-yl]-2-(1-cyclohexyl-1 H-imidazol-4-yl)-propionic acid sodium monohydrate salt on a hot stage device from Linkam Scientific Instruments (UK) to a temperature of 120°C. The crystalline phase of the anhydrate was identified by Raman spectroscopy and XRPD.

Example 10: X-ray powder diffraction (XRPD) of (R)-3-[6-amino-pyridin-3-yl]-2-(1-cyclohexyl-1 H-imidazol-4-yl)-propionic acid sodium salt anhydrate: X-ray powder diffraction was performed with a Stoe Stadi-P transmission diffractometer using CuKa1 radiation (wavelength is 1.54060 Angstrom) and a linear position sensitive detector. Unless stated otherwise, X-ray powder diffraction was performed at 110 °C. Samples were investigated in flat preparation. The measured data were evaluated and plotted with the Software WinXPOW V2.12. The observed X-ray powder diffraction pattern of (R)-3-[6-amino-pyridin-3-yl]-2-(1-cyclohexyl-1 H-imidazol-4-yl)-propionic acid sodium salt anhydrate as prepared in Examples 8 and 9 is displayed in the Figure 2 of WO2013/076178. The 20 (2theta) angles in°(degree) are specified as the number of characteristic reflections. The 2theta angles in degree have the following values in Figure 2 of WO2013/076178 and the relative intensities are shown in brackets: 6.13 (91), 8.57 (64), 11.19 (22), 14.06 (36), 15.43 (27), 15.87 (31), 16.43 (55), 16.80 (89), 17.15 (30), 17.47 (45), 18.01 (41), 18.19 (41), 18.43 (41), 18.69 (35), 18.96 (32), 19.35 (32), 19.78 (30), 20.06 (35), 20.99 (30), 21.42 (100), 22.39 (64), 22.86 (32), 23.59 (39), 24.04 (33), 24.54 (23), 25.42 (22), 25.79 (24), 26.35 (24), 26.89 (24), 27.19 (24), 28.25 (20), 28.72 (16), 28.84 (19), 29.66 (18), 30.21 (18), 30.69 (19), 30.80 (17), 31.15 (16), 32.15 (17), 33.30 (19), 34.27 (16), 34.46 (15), 35.09 (16).

Example 11: Differential scanning calorimetry (DSC) of (R)-3-[6-amino-pyridin-3-yl]-2-(1-cyclohexyl-1 H-imidazol-4-yl)-propionic acid sodium anhydrate salt: The DSC curve was measured on a Mettler Toledo DSC 821 e type instrument in punched pan with the following method: Equilibrate at 25°C, Ramp: 10 K/min to 350°C, Amount of the sample prepared in Example 3: 2-3mg. The DSC thermogram of a crystalline sample of the compound as prepared in Example 3 showed two endothermic events in range of 80°C to 130°C corresponding to the loss of water. The formed sodium anhydrate salt form above 130 °C shows a melting endotherm with onset of 266.1 °C.

Example 12: DVS, Comparison Experiment: (R)-3-[6-amino-pyridin-3-yl]-2-(1-cyclohexyl-1 H-imidazol-4-yl)-propionic acid as prepared in Example 1 was used for the measurement. The measurement was performed as disclosed in Example 7 with (R)-3-[6-amino-pyridin-3-yl]-2-(1-cyclohexyl-1 H-imidazol-4-yl)-propionic acid (free acid, amorphous) and the received data are shown in the following table: DVS Isotherm at 25 °C

| | Target RH (%) | Change In Mass (%) Sorption | Change In Mass (%) Desorption |
|---|---|---|---|
| Cycle 1 | 30.1 | | 1.7 |
| | 20.0 | | 1.1 |
| | 10.1 | | 0.3 |
| | 0.4 | | -1.6 |
| | 10.0 | -0.5 | |
| | 20.0 | 0.5 | |
| | 30.0 | 1.9 | |
| | 40.1 | 3.9 | |
| | 50.0 | 6.6 | |
| | 60.1 | 9.6 | |
| | 70.0 | 13.4 | |
| | 75.0 | 15.0 | |
| | 80.1 | 16.9 | |
| | 85.1 | 20.1 | |
| | 89.9 | 25.1 | |
| | 95.0 | 32.3 | |

DVS shows a strong water uptake of 3.9% at 40% RH, 6.6% at 50% RH, 9.6% at 60% RH, 13.4% at 70% RH, 16.9% at 80% RH, 25.1 % at 90% RH and 32.3% at 95% RH for (R)-3-[6-amino-pyridin-3-yl]-2-(1-cyclohexyl-1 H-imidazol-4-yl)-propionic acid (free acid, amorphous).

### SPECIFIC INORMATION CONCERNING ASPECT (IV) OF THE INVENTION

Aspect (iv) of the invention relates to an administration unit comprising crystalline form of sodium salt of (R)-3-[6-amino-pyridin-3-yl]-2-(1-cyclohexyl-1 H-imidazol-4-yl)-propionic acid. The invention relates to a solid form of sodium salt of (R)-3-[6-amino-pyridin-3-yl]-2-(1-cyclohexyl-1 H-imidazol-4-yl)-propionic acid, namely to crystalline form of sodium salt of (R)-3-[6-amino-pyridin-3-yl]-2-(1-cyclohexyl-1 H-imidazol-4-yl)-propionic acid, which is useful for treating subjects suffering from conditions which can be ameliorated by the administration of an inhibitor of the enzyme TAFIa (activated thrombin-activatable fibrinolysis inhibitor). Crystalline form of sodium salt of (R)-3-[6-amino-pyridin-3-yl]-2-(1-cyclohexyl-1 H-imidazol-4-yl)-propionic acid is described in WO2013/076179, which is incorporated by reference. As used herein, crystalline form of sodium salt of (R)-3-[6-amino-pyridin-3-yl]-2-(1-cyclohexyl-1 H-imidazol-4-yl)-propionic acid is a crystalline form of sodium salt of (R)-3-[6-amino-pyridin-3-yl]-2-(1-cyclohexyl-1 H-imidazol-4-yl)-propionic acid as described in WO2013/076179 and repeated hereinafter: The present invention relates to a sodium salt of (R)-3-[6-amino-pyridin-3-yl]-2-(1-cyclohexyl-1 H-imidazol-4-yl)-propionic acid ({drug4}), and to a sodium salt of (R)-3-[6-amino-pyridin-3-yl]-2-(1-cyclohexyl-1 H-imidazol-4-yl)-propionic acid which is in a crystalline form or in at least partially crystalline form, as well as a process for the preparation of the same, methods of using such salt to treat subjects suffering from conditions which can be ameliorated by the administration of an inhibitor of the enzyme TAFIa (activated thrombin-activatable fibrinolysis inhibitor), and shows the structure illustrated in Formula (IV):

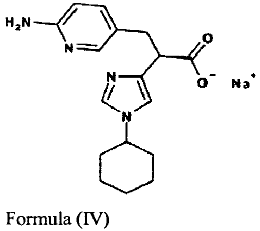

### (R)-3-[6-amino-pyridin-3-yl]-2-(1-cyclohexyl-1 H-imidazol-4-yl)-propionic acid as a free acid shows the structure illustrated in Formula (IV'):

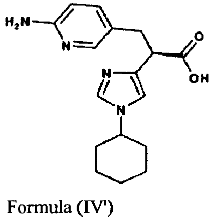

(R)-3-[6-amino-pyridin-3-yl]-2-(1-cyclohexyl-1 H-imidazol-4-yl)-propionic acid use in the preparation of a medicament for treating a patient suffering from, or subject to, conditions which can be ameliorated by the administration of an inhibitor of the enzyme TAFIa (activated thrombin-activatable fibrinolysis inhibitor), has been disclosed in WO 2005/105781. The enzyme TAFIa is produced for example through thrombin activation from the thrombin-activatable fibrinolysis inhibitor zymogen (TAFI). The enzyme TAFI is also referred to as plasma procarboxypeptidase B, procarboxypeptidase U or procarboxypeptidase R and is a proenzyme similar to carboxypeptidase B (L. Bajzar, Arterioscler. Thromb. Vase. Biol. 2000, pages 2511-2518). During formation of a clot, thrombin is generated as the final product of the coagulation cascade and induces conversion of soluble plasma fibrinogen to an insoluble fibrin matrix. At the same time, thrombin activates the endogenous fibrinolysis inhibitor TAFI. Activated TAFI (TAFIa) is thus produced during thrombus formation and lysis from the zymogen TAFI through the action of thrombin; thrombomodulin in a complex with thrombin increases this effect about 1250-fold. TAFIa cleaves basic amino acids at the carboxy end of fibrin fragments. The loss of carboxy-terminal lysines as binding sites for plasminogen then leads to inhibition of fibrinolysis. Efficient inhibitors of TAFIa prevent the loss of these high-affinity lysine binding sites for plasminogen and, in this way, assist endogenous fibrinolysis by plasmin: TAFIa inhibitors have profibrinolytic effects. In order to maintain hemostasis in the blood, mechanisms which lead to the clotting of blood and to the breaking up of clots have developed; these are in equilibrium. If a disturbed equilibrium favors coagulation, fibrin is produced in larger quantities, so that pathological processes of thrombus formation may lead to serious pathological states in humans. Just like excessive coagulation may lead to serious pathological states caused by thrombosis, an antithrombotic treatment entails the risk of unwanted bleeding through disturbance of the formation of a necessary hemostatic plug. Inhibition of TAFIa increases endogenous fibrinolysis-without influencing coagulation and platelet aggregation-i.e. the disturbed equilibrium is shifted in favor of fibrinolysis. It is thus possible both to counter the buildup of a clinically relevant thrombus, and to increase the lysis of a pre-existing clot. On the other hand, buildup of a hemostatic plug is not impaired, so that a hemorrhagic diathesis is probably not to be expected (Bouma et al., J. Thrombosis and Haemostasis, 1, 2003, pages 1566-1574). Inhibitors of TAFIa have already been described in the International Applications WO03/013526 and WO2005/105781. Processes for preparation are disclosed in WO2010/130718. 3-[6-amino-pyridin-3-yl]-2-(1-cyclohexyl-1 H-imidazol-4-yl)-propionic acid bishydrochloride salt and its pharmacological activities have been disclosed in WO2005/105781. The bishydrochloride salt of 3-[6-amino-pyridin-3-yl]-2-(1-cyclohexyl-1 H-imidazol-4-yl)-propionic acid and the free acid of 3-[6-amino-pyridin-3-yl]-2-(1-cyclohexyl-1 H-imidazol-4-yl)-propionic acid have the disadvantage to occur as amorphous solids. Thus, said hydrochloride acid salts as well as said free acid cannot be purified by crystallization. Additionally, it was found that the free acid of 3-[6-amino-pyridin-3-yl]-2-(1-cyclohexyl-1 H-imidazol-4-yl)-propionic acid is hygroscopic. Further it was found that (R)-3-[6-amino-pyridin-3-yl]-2-(1-cyclohexyl-1 H-imidazol-4-yl)-propionic acid is chemically not stable under elevated temperature and humidity. A degradation of the molecule could be observed and the enantiomeric purity of (R)-3-[6-amino-pyridin-3-yl]-2-(1-cyclohexyl-1 H-imidazol-4-yl)-propionic acid is reduced within a short time. Therefore, (R)-3-[6-amino-pyridin-3-yl]-2-(1-cyclohexyl-1 H-imidazol-4-yl)-propionic acid is not suitable for the development in a pharmaceutical formulation. Hygroscopicity is the ability of a substance to attract and hold water molecules from the surrounding environment through either absorption or adsorption with the adsorbing or absorbing material becoming physically 'changed,' somewhat, increase in volume, stickiness, or other physical characteristic changes of the material as water molecules become 'suspended' between the material's molecules in the process. Therefore hygroscopic compounds are generally very unfavorable for use in solid pharmaceutical compositions. It is an object of the present invention to find a salt of (R)-3-[6-amino-pyridin-3-yl]-2-(1-cyclohexyl-1 H-imidazol-4-yl)-propionic acid with reduced absorption or adsorption of water molecules from the surrounding environment and which is chemically stable. It has been found that a sodium salt of (R)-3-[6-amino-pyridin-3-yl]-2-(1-cyclohexyl-1 H-imidazol-4-yl)-propionic acid has favorable reduced absorption or adsorption of water molecules from the surrounding environment and is chemically stable.

In one embodiment the present invention relates to a sodium salt of (R)-3-[6-amino-pyridin-3-yl]-2-(1-cyclohexyl-1 H-imidazol-4-yl)-propionic acid ({drug4}) and shows the structure illustrated in Formula (IV):

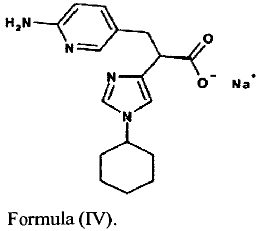

In another embodiment the invention relates to a sodium salt of (R)-3-[6-amino-pyridin-3-yl]-2-(-cyclohexyl-1 H-imidazol-4-yl)-propionic acid, which is in a crystalline form or in at least partially crystalline form ({drug4a}). In one embodiment the present invention relates to a sodium salt of (R)-3-[6-amino-pyridin-3-yl]-2-(1-cyclohexyl-1 H-imidazol-4-yl)-propionic acid and shows the structure illustrated in Formula (IV). In another embodiment the invention relates to a sodium dihydrate salt of (R)-3-[6-amino-pyridin-3-yl]-2-(1-cyclohexyl-1 H-imidazol-4-yl)-propionic acid, which is in a crystalline form or in at least partially crystalline form ({drug4a}). Polymorphism is the ability of a single compound to exist in more than one form or crystal structure. Different polymorphs represent distinct solids sharing the same molecular formula, yet each polymorph may have distinct physical properties. A single compound may give rise to a variety of polymorphic forms wherein each form may have different and distinct physical properties, such as different solubility profiles, different thermodynamic stability, different crystallization behavior, different filterability, different melting point temperatures and/or different X-ray diffraction peaks. The difference in the physical properties of different polymorphic forms results from different orientation and intermolecular interactions of adjacent molecules in the solid. Polymorphic forms of a compound can be distinguished by X-ray diffraction and by other methods such as, infrared spectroscopy or Raman spectroscopy. "Amorphous"means a solid that exhibits in an X-ray powder diffraction pattern measured in transmission with CuKa1 radiation at room temperature no characteristic reflections at degrees 2 theta which can be separated from each other by their diffraction angle or specific degree 2 theta. In another embodiment the invention relates to a sodium salt of Formula (IV") as a hydrate 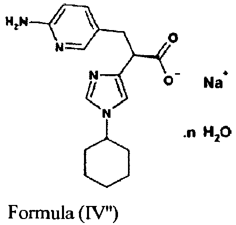 wherein n has the value from 1.5 to 2.8 ({drug4b}) and wherein n is the molar relation between water and (R)-3-[6-amino-pyridin-3-yl]-2-(1-cyclohexyl-1 H-imidazol-4-yl)-propionic acid. In another embodiment the invention relates to a sodium salt of (R)-3-[6-amino-pyridin-3-yl]-2-(1-cyclohexyl-1 H-imidazol-4-yl)-propionic acid, wherein n has the value from 1.8 to 2.3 or from 2.0 to 2.2. In another embodiment the invention relates to a crystalline sodium salt of (R)-3-[6-amino-pyridin-3-yl]-2-(1-cyclohexyl-1 H-imidazol-4-yl)-propionic acid wherein the crystalline salt exhibits in an X-ray powder diffraction pattern measured in transmission with CuKcH radiation at room temperature a characteristic reflection at degrees 2 theta of 20.0, 14.6 and 8.6, each time ± 0.2 degrees 2 theta ({drug4c}). In another embodiment the invention relates to a crystalline sodium salt of (R)-3-[6-amino-pyridin-3-yl]-2-(1-cyclohexyl-1 H-imidazol-4-yl)-propionic acid wherein the crystalline salt exhibits in an X-ray powder diffraction pattern measured in transmission with CuKcH radiation at room temperature a characteristic reflection at degrees 2 theta of 20.0, 19.2, 18.8, 17.8, 14.6 and 8.6, each time ± 0.2 degrees 2 theta ({drug4d}). In another embodiment the invention relates to a crystalline sodium salt of (R)-3-[6-amino-pyridin-3-yl]-2-(-cyclohexyl-1 H-imidazol-4-yl)-propionic acid wherein the crystalline salt exhibits in an X-ray powder diffraction pattern measured in transmission with CuKcH radiation at room temperature a characteristic reflection at degrees 2 theta of 32.5, 23.9, 20.0, 19.2, 18.8, 17.8, 16.1, 15.6, 14.6 and 8.6, each time ± 0.2 degrees 2 theta ({drug4e}). The selection of characteristic reflections was determined by the number of reflections at a specified degree 2 theta. In another embodiment the crystalline sodium dihydrate salt of (R)-3-[6-amino-pyridin-3-yl]-2-(1-cyclohexyl-1 H-imidazol-4-yl)-propionic acid may also be characterized by its X-ray powder diffraction pattern substantially by the one shown in Figure 1 of WO2013/076179, which has been obtained using CuKa1 radiation in transmission mode, wherein the intensities of the reflections depicted in the Figure of WO2013/076179 as well as those of the reflections specified above are not a prerequisite, but may vary. The crystalline sodium salt of (R)-3-[6-amino-pyridin-3-yl]-2-(1-cyclohexyl-1 H-imidazol-4-yl)-propionic acid may also be characterized by its crystal lattice parameters which have been determined by indexing its powder pattern. The crystalline sodium salt of Formula (IV") crystallizes in the monoclinic crystal system with a = 10.6769(3) A, b = 6.8336(2) A, c = 13.5530(3) A, a = 90.00°, β = 104.3710(10)°, γ = 90.00°, volume = 957.9 A3.

In another embodiment the crystalline sodium dihydrate salt of (R)-3-[6-amino-pyridin-3-yl]-2-(1-cyclohexyl-1 H-imidazol-4-yl)-propionic acid may also be characterized by its Raman spectrum. The Raman spectrum was recorded on RXN1-Dispersive Raman spectrometer equipped with PhAT-probe head (for solids) and 785 nm diode laser (400 mW) from Kaiser Optical Systems, Ltd., USA; Software Details; Aquisition: Holograms, Kaiser; Polynomial spline background correction performed in Software: OPUS 6.5 (from Bruker Optics, Ettlingen, Germany). The characteristic wave number of the
Raman shift has the following values (cm⁻¹) and the relative intensities are shown in brackets: 1565 (75), 1328 (52), 1270 (57), 1054 (53), 858 (100), and 819 (51), each time ± 4 cm⁻¹ ({drug4f}). In another embodiment the crystalline sodium dihydrate salt of (R)-3-[6-amino-pyridin-3-yl]-2-(1-cyclohexyl-1 H-imidazol-4-yl)-propionic acid may also be characterized by its Raman spectrum. The characteristic wave number of the Raman shift has the following values (cm⁻¹) and the relative intensities are shown in brackets: 1565 (75), 1328 (52), 1304 (31), 1270 (57), 1054 (53), 1032 (34), 951 (34), 858(100), and 819 (51), each time ± 4 cm⁻¹ ({drug4g}). The selection of characteristic wave number was determined by the number of reflections at a specified wave number. In another embodiment the crystalline sodium dihydrate salt of (R)-3-[6-amino-pyridin-3-yl]-2-(1-cyclohexyl-1 H-imidazol-4-yl)-propionic acid may also be characterized by its Raman spectrum pattern, wherein the Raman shift is characterized by the following values (cm⁻¹) and the relative intensities are shown in brackets: 1635 (23), 1616 (27), 1565 (75), 1504 (8), 1450 (24), 1440 (23), 1399 (19), 1383 (13), 1367 (15), 1355 (16), 1328 (52), 1304 (31), 1296 (21), 1287 (21), 1270 (57), 1252 (12), 1221 (24), 1211 (13), 1185 (10), 1162 (8), 1138 (18), 1083 (7), 1054 (53), 1032 (34), 1003 (12), 973 (5), 951 (34), 938 (14), 916 (11) 898 (3), 858 (100), 819 (51), 790 (5), 749 (15), 714 (10), 691 (3), 656 (25), 616 (3), 577 (2), 538 (2), 510 (5), 469 (11), 447 (11), and 427 (12), each time ± 4 cm⁻¹ ({drug4h}). The intensities of the Raman shift wave numbers depicted above as well as those of the wave numbers specified above are not a prerequisite, but may vary. Moreover, crystalline sodium salt of (R)-3-[6-amino-pyridin-3-yl]-2-(1-cyclohexyl-1 H-imidazol-4-yl)-propionic acid may also be characterized by its dynamic vapor sorption (DVS) water vapor sorption and desorption isotherms measured at 25°C. As shown in the examples the sorption and desorption isotherms between 30% relative humidity and 0.4% relative humidity are almost the same (low water uptake or water desorption) wherein a moderate water uptake of 2.1 % at 80% relative humidity (RH), and 2.7% at 85% RH, and strong water uptake of 18.8% at 90% RH, and 51.8% at 95% RH takes place. Figure 1 of WO2013/076179-X-ray powder diffraction pattern of crystalline sodium dihydrate salt of (R)-3-[6-amino-pyridin-3-yl]-2-(1-cyclohexyl-1 H-imidazol-4-yl)-propionic acid, measured in transmission mode with CuKa1 radiation at room temperature (x-axis: diffraction angle 2theta (20) [°]; y-axis: relative intensity. The sodium salt of (R)-3-[6-amino-pyridin-3-yl]-2-(1-cyclohexyl-1 H-imidazol-4-yl)-propionic acid may be prepared by dissolving of (R)-3-[6-amino-pyridin-3-yl]-2-(1-cyclohexyl-1 H-imidazol-4-yl)-propionic acid in acetone or aqueous-acetone mixture or other suitable solvents to which the aqueous sodium base such as sodium hydroxide, disodium carbonate, sodium hydrogen carbonate or sodium ethanolate or a mixture thereof is added. Under stirring, the mixture can be heated to about 55°C yielding a clear solution and subsequently cooling to about 1°C yields a precipitate. The precipitate obtained can be filtered, washed with water and dried under reduced pressure. A useful molar relation between a sodium base such as sodium hydroxide and (R)-3-[6-amino-pyridin-3-yl]-2-(1-cyclohexyl-1 H-imidazol-4-yl)-propionic acid is from 1 to 2. A further useful molar relation between sodium hydroxide and (R)-3-[6-amino-pyridin-3-yl]-2-(1-cyclohexyl-1 H-imidazol-4-yl)-propionic acid is from 1.2 to 1.5. In general, the crystalline sodium salt of (R)-3-[6-amino-pyridin-3-yl]-2-(1-cyclohexyl-1 H-imidazol-4-yl)-propionic acid may also be prepared by dissolving of (R)-3-[6-amino-pyridin-3-yl]-2-(1-cyclohexyl-1 H-imidazol-4-yl)-propionic acid in acetone or aqueous-acetone mixture and can be obtained by crystallizing or recrystallizing compound of Formula (IV), starting from a solution of compound of Formula (IV) or from a suspension of compound of Formula (IV) or from solid compound of Formula (IV). A solution of compound of Formula (IV), or a suspension of compound of Formula (IV), may have been obtained at the end of the chemical synthesis of compound of Formula (IV), or it may have been obtained by dissolving or suspending previously synthesized crude compound of Formula (IV). The term "crude compound of Formula (IV)"comprises any form of compound of Formula (IV), e.g. the material directly obtained from chemical synthesis, a distinct crystalline form or amorphous material of the compound of Formula (IV). More specifically, the crystalline salt of Formula (IV") of the invention can be obtained by
(a) providing a solution or suspension of compound of Formula (IV), for example by dissolving or suspending crude compound Formula (IV) in an acetone-water mixture; wherein a solution of compound of Formula (IV) generally is a clear solution and may optionally have been filtered,
(b) maintaining, heating, cooling and/or concentrating the solution or suspension and/or adding one or more components of said acetone-water mixture, with or without agitation such as stirring, to form crystals of a desired distinct crystalline form of Formula (IV"), and
(c) isolating the distinct crystalline salt of Formula (IV").

The processes for preparing crystalline forms and solvates of compound of Formula (IV") can be performed with conventional equipment and according to standard procedures. For example, concentrating of a solution or suspension in step (b) may be done by distilling off solvent partially or totally at atmospheric pressure or at reduced pressure. Isolating of a crystalline form or solvate in step (c) may be done by any conventional technique such as filtration or vacuum filtration or centrifugation. Isolating may also comprise drying, e.g. by applying elevated temperatures and/or reduced pressure, for example at moderately reduced pressure at about room temperature, i.e. a temperature of about 18 °C to about 55 °C, for example about 20 °C, or at about 55 °C. Useful mixtures of water in acetone in process step (a) contain from 5 w/w-% to 15 w/w-% water in acetone. Further useful mixtures contain from 8 w/w-% to 12 w/w-% water in acetone. In a preferred embodiment, the solution or suspension may be seeded in step (b) to promote crystallization. Seeding is preferably done with a small amount of the crystalline salt of Formula (IV") already prepared. The sodium salt of (R)-3-[6-amino-pyridin-3-yl]-2-(1-cyclohexyl-1 H-imidazol-4-yl)-propionic acid (in the following compound of Formula (IV)) of the present invention may be useful in the prophylaxis and therapy of all disorders which can be treated by inhibition of TAFIa. Thus, the compound of Formula (IV) is suitable both for a prophylactic and for a therapeutic use in humans and is suitable both for an acute treatment and for a long-term therapy. The compound of Formula (IV) can be employed in patients suffering from impairments of wellbeing or diseases associated with thromboses, embolisms, hypercoagulability or fibrotic changes. These include myocardial infarction, angina pectoris and all other types of acute coronary syndrome, stroke, peripheral vascular disorders, deep vein thrombosis, pulmonary embolism, embolic or thrombotic events caused by cardiac arrhythmias, cardiovascular events such as restenosis following revascularization, angioplasty and similar procedures such as stent implantations and bypass operations. The compound of Formula (IV) can additionally be employed in all procedures leading to contact of the blood with foreign surfaces such as, for example, for dialysis patients and patients with indwelling catheters. The compound of Formula (IV) can be employed to reduce the risk of thrombosis after surgical procedures such as knee and hip joint operations. The compound of Formula (IV) may be suitable for the treatment of patients with disseminated intravascular coagulation, sepsis and other intravascular events associated with an inflammation and is additionally suitable for the prophylaxis and treatment of patients with atherosclerosis, diabetes and the metabolic syndrome and its sequelae. The compound of Formula (IV) may further be used to inhibit fibrotic changes of the lung such as chronic obstructive lung disease, adult respiratory distress syndrome (ARDS) and of the eye such as fibrin deposits after eye operations. The compound of Formula (IV) may also be suitable for the prevention and/or treatment of scar formation. The invention also relates to a process for producing a pharmaceutical composition, which comprises making a suitable dosage form from a compound of the Formula (IV) with a pharmaceutically suitable and physiologically tolerated carrier and, where appropriate, further suitable active ingredients, additives or excipients. Suitable solid or pharmaceutical formulations are, for example, granules, powder, coated tablets, tablets, (micro)capsules, suppositories, syrups, solutions, suspensions, emulsions, drops or injectable solutions, and products with protrated release of active ingredient, in the production of which normally physiologically suitable aids such as carriers, disintegrants, binders, coating agents, swelling agents, glidants or lubricants, flavorings, sweeteners and solubilizers are used. Excipients which are frequently used and which may be mentioned are magnesium carbonate, titanium dioxide, lactose, mannitol and other sugars, talc, milk protein, gelatin, starch, cellulose and its derivatives, animal and vegetable oils such as fish liver oil, sunflower, peanut or sesame oil, polyethylene glycol and solvents such as, for example, sterile water and monohydric or polyhydric alcohols such as glycerol. The pharmaceutical products are preferably produced and administered in dosage units, where each unit comprises as active ingredient a particular dose of the compound of the Formula (IV). In the case of solid dosage units such as tablets, capsules, coated tablets or suspensions, this dose can be up to about 1000 mg, but preferably about 50 to 300 mg and, in the case of injection solutions in ampoule form, up to about 300 mg but preferably about 10 to 100 mg. The daily doses indicated for the treatment of an adult patient weighing about 70 kg are, depending on the activity of the compound of Formula (IV), from about 2 mg to 1000 mg of active ingredient, preferably about 50 mg to 500 mg. However, in some circumstances, higher or lower daily doses may also be appropriate. The compound of Formula (IV) according to the invention may be administered as frequently as necessary to obtain the desired therapeutic effect. Some patients may respond rapidly to a higher or lower dose and may find much weaker maintenance doses adequate. For other patients, it may be necessary to have long-term treatments at the rate of about 1 to about 4 doses per day, in accordance with the physiological requirements of each particular patient. Generally, the active product may be administered orally about 1 to about 4 times per day. It goes without saying that, for other patients, it may be necessary to prescribe not more than one or two doses per day. The medicaments of the invention can be administered by oral, inhalational, rectal or transdermal administration or by subcutaneous, intraarticular, intraperitoneal or intravenous injection. Intravenous injection is preferred. As would be apparent to a person of ordinary skill in the art, once armed with the teachings of the present disclosure, when dissolved, crystalline sodium salt of Formula (IV) loses its crystalline structure, and is therefore considered to be a solution of sodium salt of Formula (IV). All forms of the present invention, however, may be used for the preparation of liquid formulations in which crystalline sodium salt of Formula (IV) may be, for example, dissolved or suspended. In addition, the crystalline sodium salt of Formula (IV) may be incorporated into solid formulations. The following non-limiting examples illustrate the inventors' preferred methods for preparing and using the sodium salt of Formula (IV) of the present invention.

### Example 1: Preparation of (R)-3-[6-amino-pyridin-3-yl]-2-(1-cyclohexyl-1 H-imidazol-4-yl)-propionic acid free acid

a) (E)-2-(1-Cyclohexyl-1 H-imidazol-4-yl)-3-hydroxy-but-2-enedioic acid diethyl ester: 54 g (0.23 mol) (1-Cyclohexyl-1 H-imidazol-4-yl)-acetic acid ethyl ester were dissolved in 300 ml absolute Ethanol. Diethyl oxalate (77 ml, 0.57 mmol), followed by sodium ethylate (23 g, 0.34 mol) were added successively thereby the temperature rose to 35°C. The mixture was allowed to cool slowly down to 25°C within one hour. The solution was concentrated and poured into 1 liter (I) phosphate buffer (pH = 6.2, c = 0.8 M). The yellow-brown solid was filtered and washed with 300 ml of water. The solid was then dried under reduced pressure at 60 °C. The dry residue was digested with 300 ml of preferably tert.-butyl methyl ether (MTBE) at 40 °C for 2 hours, cooled to RT, filtered and dried on air to gave 53 g (0.16 mol, 69%) (E)-2-(1-Cyclohexyl-1 H-imidazol-4-yl)-3-hydroxy-but-2-enedioic acid diethyl ester as an off white solid. 1H-NMR(400 MHz, d6-DMSO): δ = 1.17 (t, J = 7.1 Hz, 3H), 1.23 (t, J = 7.2 Hz, 3H), 1.20-1.29 (m, 1 H), 1.30-1.44 (m, 2H), 1.61-1.77 (m, 3H), 1.78-1.88 (m, 2H), 1.98-2.07 (m, 2H), 4.03 (q, J = 7.1 Hz, 2H), 4.10 (q, J = 7.1 Hz, 2H), 4.22-4.31 (m, 1 H), 7.49 (d, J = 1.8 Hz, 1 H), 8.57 (d, J = 1.7 Hz, 1 H), 13.4-14.1 (bs, 1 H); HPLC: tR = 1.10 min (YMC J' sphere ODS H 80 20x2.1 mm, 4 pm, A: H2O+0.05% TFA, B: MeCN, 4%→ 95% B in 2 min, 1 ml/min, 30 °C); Mass (ES+) (Ci7H24N2O5): calculated. 366, found 337 [M+H]+, Melting point (Mp): >150°C decomposition, (tert-butyl methyl ether; MTBE).
b) 3-(6-terf-Butoxycarbonylamino-pyridin-3-yl)-2-(1-cyclohexyl-1 H-imidazol-4-yl)-propionic acid ethyl ester: The compound prepared according to process step a) (5.0 g, 14.9 mmol) was suspended in 80 ml Tetrahydrofuran (THF) and Lithium hexamethyldisilazide (LiHMDS) (16.4 ml, 16.4 mmol, 1.0 M in THF) was added at room temperature (RT). The mixture was stirred for 20 min, Tetra-(n)-butylammonium iodide (catalytic) was added, followed by (5-Bromomethyl-pyridin-2-yl)-carbamic acid tert-butyl ester (4.48 g, 15.6 mmol). After one hour water (20 ml), Ethyl acetate (AcOEt; 50 ml) were added and the pH-value was adjusted to 10.5 with saturated K2CO3. The mixture was allowed stirring for 90 min. The phases were separated and the aqueous layer was extracted with AcOEt (2x 20 ml). The combined organic layers were washed with brine (20 ml), dried with MgSO4 and concentrated to yield a red-orange crude product. The residue was treated with 50 ml MTBE and stirred for 30 min at 60°C. After cooling to RT the solid was filtered and washed with cold MTBE to yield the title compound (3.1 g, 7.0 mmol, 47%) as a yellowish solid. 1 H-NMR (500 MHz, d6-DMSO): δ = 1.05 (t, J = 7.1 Hz, 3H), 1.13-1.24 (m, 1 H), 1.29-1.40 (m, 2H), 1.46 (s, 9H), 1.53-1.67 (m, 3H), 1.74-1.82 (m, 2H), 1.88-1.96 (m, 2H), 3.76-3.81 (m, 1 H), 3.91-4.05 (m, 3H), 7.05 (s, 1 H), 7.48 (dd, J = 8.6, 1.9 Hz, 1 H), 7.58 (s, 1 H), 7.64 (d, J = 8.6 Hz, 1 H), 7.99 (d, J = 1.9 Hz, 1 H), 9.62 (s, 1 H); HPLC: tR = 1.00 min (YMC J' sphere ODS H 80 20x2.1 mm, 4 pm, A: H2O+0.05% TFA, B: MeCN, 4%→ 95% B in 2 min, 1 ml/min, 30°C); Mass (ES+) (C2 H34N4O4): calculated. 442, found 443 [M+H]+, Mp: 161-163°C (MTBE).
c) 3-(6-amino-pyridin-3-yl)-2-(1-cyclohexyl-1 H-imidazol-4-yl)-propionic acid ethyl ester: 39.0 g (88.1 mmol) 3-(6-terf-Butoxycarbonylamino-pyridin-3-yl)-2-(1-cyclohexyl-1 H-imidazol-4-yl)-propionic acid ethyl ester as prepared in process step b) were suspended in 200 ml Ethanol (EtOH) at RT. The mixture was saturated with HCl and was then refluxed for 2 h. The mixture was concentrated and the residue was treated with saturated aqueous K2CO3 (pH 9.0). The aqueous layer was extracted with AcOEt (3x 200 ml). The combined organic layers were washed with brine (100 ml), dried with MgSO4 and concentrated to yield the title compound (30 g, 87.6 mmol, 99%) as brown oil. HPLC: tR = 0.69 min (YMC J' sphere ODS H 80 20x2.1 mm, 4 pm, A: H2O+0.05% TFA, B: MeCN, 4%→ 95% B in 2 min, 1 ml/min, 30°C); Mass (ES+) (Ci9H26N4O2): calculated. 342, found 343 [M+H]+.
d) (R)-3-(6-amino-pyhdin-3-yl)-2-(1-cyclohexyl-1 H-imidazol-4-yl)-propionic acid ethyl ester: The two enantiomers of 3-(6-amino-pyridin-3-yl)-2-(1-cyclohexyl-1 H-imidazol-4-yl)-propionic acid ethyl ester were separated by chiral chromatography: Batches containing 540 g of 3-(6-amino-pyridin-3-yl)-2-(1-cyclohexyl-1 H-imidazol-4-yl)-propionic acid ethyl ester were filled into a column containing 20 kg of Chiralpack AD 20 µlτ 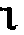 (Ciral Technologies Europe, France) as a chiral stationary phase. Ethanol was pumped through the column until no more (S)-3-(6-amino-pyridin-3-yl)-2-(1-cyclohexyl-1 H-imidazol-4-yl)-propionic acid ethyl ester was eluted. Then a mixture containing ethanol with 0.4 % of trifluoroacetic acid were pumped through the column and fractions containing (R)-3-(6-amino-pyridin-3-yl)-2-(1-cyclohexyl-1 H-imidazol-4-yl)-propionic acid ethyl ester were collected. The received title compound showed a purity of 91.7 % and an ee of 99.2 %. The purity was determined by the chromatography described in Example 8.
e) ((R)-3-[6-amino-pyridin-3-yl]-2-(1-cyclohexyl-1 H-imidazol-4-yl)-propionic acid ethyl ester, naphtalene 1,5-disulfonic acid salt: 1.50 g (5.20 mmol, 1.2 equivalents) Naphthalene-1,5-disulfonic acid were dissolved in 15 ml water. The received solution was cooled to 10°C. 1.48 g (4.32 mmol, 1.0 equivalents) (R)-3-(6-amino-pyiidin-3-yi)-2-(I-cyclohexyl-1 H-imidazol-4-yl)-propionic acid ethyl ester as prepared in process step d) were dissolved in 15 mL of a acetone/water mixture 2: 3 and slowly added to the naphthalene-1, 5-disulfonic acid solution. The solvents were slowly evaporated by an argon gas stream until first crystals occurred. The argon gas stream is stopped and the suspension was stirred for three hours at RT (20°C to 22°C). The residues were filtered off and dried over night by air. 1.83 g (2.90 mmol, 67%) of the crystalline (R)-3-(6-amino-pyridin-3-yl)-2-(1-cyclohexyl-1 H-imidazol-4-yl)-propionic acid ethyl ester 1,5-naphthyldisulfonic acid salt was received as a white solid.
f) 60 g (R)-3-[6-amino-pyridin-3-yl]-2-(1-cyclohexyl-1 H-imidazol-4-yl)-propionic acid ethyl ester, naphtalene 1,5-disulfonic acid salt as prepared in process step e) were stirred overnight in 480 mL 6 N hydrochloric acid. The solvent of the reaction mixture was evaporated to dryness. The dry residue was dissolved in 222 mL of water and given on a Dowex 50WX8-200 ion exchange resin. The ion exchanger was washed with water until no 1,5-naphthyldisulfonate was detected in the water after the washing process. The product was then eluted from the ion exchanger with 2.5% ammonium hydroxide solution. The product fractions were collected and the solvent of these fractions was distilled off. The obtained solid was dissolved in 500 ml water and subsequently lyophilized. 26.8 g light yellowish powder were obtained (91 % yield).

Example 2: Preparation of sodium salt of (R)-3-[6-amino-pyridin-3-yl]-2-(1-cyclohexyl-1 H-imidazol-4-yl)-propionic acid and crystallization: 400 mg (R)-3-[6-amino-pyridin-3-yl]-2-(1-cyclohexyl-1 H-imidazol-4-yl)-propionic acid of the compound prepared in Example 1 were dissolved in 5.3 g acetone and 742 mg aqueous sodium hydroxide solution (10.1 w/w-%) and heated to 53°C and subsequently cooled to 0°C. The solid crystalline product was isolated by filtration and dryed at 40°C. 376 mg dry crude material were obtained (yield: 80%).

Example 3: Preparation of (R)-3-[6-amino-pyridin-3-yl]-2-(1-cyclohexyl-1 H-imidazol-4-yl)-propionic acid sodium salt, crystallization and seeded re-crystallization: 30.4 g (R)-3-[6-amino-pyridin-3-yl]-2-(1-cyclohexyl-1 H-imidazol-4-yl)-propionic acid of the compound prepared in Example 1 were dissolved in 507.1 g acetone and 56.5 g aqueous sodium hydroxide solution (10.3 w/w-%) at 55°C and subsequently cooled to 1 °C. The solid crystalline product was isolated by filtration and dryed at 50°C. 32.9 g dry crude material were obatiend (yield: 91 %, enantiomeric excess: 94.5%). 37.9 g of crude product dissolved in 594 g acetone-water mixture (12 w/w-% water in acetone) at 55°C. After cooling to 50°C a fine filtration was performed using a chacoal filter BECO-ACF07. The clear solution after filtration was then linearily cooled to-5°C and seeded with 190 mg crystals of prepared in Example 2 at 40°C. During the cooling phase 833 g acetone were dosed into the crystallization suspension. The solid product was then isolated by filtration, washed twice with 50 ml ice-cold acetone and dryed at 50°C. 31.3 g product were obtained (yield: 82.5%, enantiomeric excess: 99.6%). NMR and elementary data confirmed the received salt as a 1:1 ratio ofNa+ and (R)-3-[6-amino-pyridin-3-yl]-2-(1-cyclohexyl-1 H-imidazol-4-yl)-propionate dihydrate and shows the structure illustrated in the following schema:

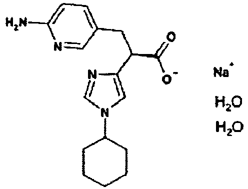

Assignment of the 1H NMR spectrum of (R)-3-[6-amino-pyridin-3-yl]-2-(1-cyclohexyl-1 H-imidazol-4-yl)-propionic acid sodium salt acquired on a Bruker DRX 400 spectrometer at T=295 K, with resonance frequency of 400.1 MHz (The sample of (R)-3-[6-amino-pyridin-3-yl]-2-(1-cyclohexyl-1 H-imidazol-4-yl)-propionic acid sodium salt was dissolved in DMSO-d6; DMSO means dimethyl sulfoxide):

| **Chemical shift (ppm)** | **Multiplicity** | **Integrated intensity** | **Assignment** | **Coupling constant (Hz)** |
|---|---|---|---|---|
| 1.17 | m | 1 | 3-Hₐ | |
| 1.34 | m | 2 | 2-Hₐ, 4-Hₐ | |
| 1.56 | m | 3 | 1-Hₐ, 5-Hₐ | |
| 1.63 | | | 3-H_{b} | |
| 1.77 | m | 2 | 2-H_{b}, 4-H_{b} | |
| 1.90 | m | 2 | 1-H_{b}, 5-H_{b} | |
| 2.64 | dd | 1 | 14-Hₐ | ²J_{14-Ha, 14-Hb} = 13.9 |
| | | | | ³J_{12-H, 14-Ha} = 6.3 |
| 2.90 | dd | 1 | 14-H_{b} | ²J_{14-Ha, 14-Hb} = 13.9 |
| | | | | ³J_{12-H, 14-Hb} = 8.6 |
| 3.22 | dd | 1 | 12-H | ³J_{12-H, 14-Ha} = 6.3 |
| | | | | ³J_{12-H, 14-Hb} = 8.6 |

| **Chemical shift (ppm)** | **Multiplicity** | **Integrated intensity** | **Assignment** | **Coupling constant (Hz)** |
|---|---|---|---|---|
| 3.89 | m | 1 | 6-H | |
| 5.48 | s | 2 | 21-H₂ | |
| 6.27 | d | 1 | 17-H | ³J_{16-H, 17-H} = 8.5 |
| 6.83 | d | 1 | 11-H | ⁴J_{8-H, 11-H} = 1.2 |
| 7.14 | dd | 1 | 16-H | ³J_{16-H, 17-H} = 8.5 |
| | | | | ⁴J_{16-H, 20-H} = 2.3 |
| 7.44 | d | 1 | 8-H | ⁴J_{8-H, 11-H} = 1.2 |
| 7.66 | d | 1 | 20-H | ⁴J_{16-H, 20-H} = 2.3 |

Elemental Analysis Results of (R)-3-[6-amino-pyridin-3-yl]-2-(1-cyclohexyl-1 H-imidazol-4-yl)-propionic acid sodium salt Carbon, hydrogen, nitrogen and sulphur contents were measured on a Carlo Erba model 11 10 elemental analyzer using a standard combustion method. The chloride and sodium content were measured on Dionex ICS-3000 ion chromatograph.

| **Element** | **%Calculated¹** | **% Found** |
|---|---|---|
| C | 6.77 | 6.62 |
| H | 54.83 | 54.03 |
| N | 15.04 | 14.81 |
| Na⁺ | 6.17 | 6.6 |
| S | - | <0.1 |
| Cl⁻ | - | 0.84 |

| | | |
|---|---|---|
| ¹Calculated from the molecular formula C₁₇H₂₅N₄O₄Na | | |

Example 4: Differential scanning calorimetry (DSC): The DSC curve was measured on a Mettler Toledo DSC 821 e type instrument in punched pan with the following method: Equilibrate at 25°C, Ramp: 10 K/min to 350°C, Amount of the sample (Example 3): 2-3mg. The DSC thermogram of a crystalline sample of the compound as prepared in Example 3 showed two endothermic events in range of 80°C to 130°C corresponding to the loss of water. The formed anhydrate form showed a melting endotherm with onset of 284.1 °C.

Example 5: Thermogravimetric analysis (TGA): The TGA diagram is recorded on TA Instruments TGA Q500 equipment in nitrogen atmosphere with the following method: Equilibrate at 25°C, Ramp: 10 K/min to 350°, Amount of the sample: 10-20mg, Sample purge 25 ml/min. The TGA thermo gram of the crystalline sample of the compound as prepared in Example 3 shows 9.5-10.2% weight loss between 25-120°C, which corresponds to about 2 mol of water of (R)-3-[6-amino-pyridin-3-yl]-2-(1-cyclohexyl-1 H-imidazol-4-yl)-propionic acid sodium salt dihydrate form.

Example 6: X-ray powder diffraction (XRPD): X-ray powder diffraction was performed with a Stoe Stadi-P transmission diffractometer using CuKa1 radiation (wavelength is 1.54060 Angstrom) and a linear position sensitive detector. Unless stated otherwise, X-ray powder diffraction was performed at room temperature. Samples were investigated in flat preparation. The measured data were evaluated and plotted with the Software WinXPOW V2.12.The observed X-ray powder diffraction pattern of (R)-3-[6-amino-pyridin-3-yl]-2-(1-cyclohexyl-1 H-imidazol-4-yl)-propionic acid sodium salt dihydrate as prepared in Example 3 is displayed in the Figure 1 of WO2013/076179. The 20 (2theta) angles in°(degree) are specified as the number of characteristic reflections. The 2theta angles in degree have the following values in Figure 1 of WO2013/076179 and the relative intensities are shown in brackets: 6.76 (24), 8.57 (100), 9.49 (11), 12.16 (10), 13.51 (7), 14.09 (7), 14.64 (68), 15.56 (21), 16.10 (22), 16.81 (7), 17.17 (15), 17.80 (34), 18.77 (31), 19.20 (45), 19.98 (75), 20.31 (17), 21.29 (17), 21.58 (16), 22.03 (9), 23.09 (6), 23.89 (23), 24.17 (13), 25.04 (5), 25.86 (8), 26.10 (9), 26.62 (6), 26.98 (6), 27.36 (10), 27.50 (8), 27.81 (17), 28.30 (20), 28.88 (11), 29.04 (5), 29.22 (5), 29.50 (9), 29.77 (18), 30.22 (4), 30.55 (14), 31.29 (19), 31.74 (10), 32.19 (7), 32.50 (27), 33.07 (14), 33.63 (6), 33.97 (9).

Example 7: Dynamic vapor sorption (DVS): Moisture sorption/desorption isotherms were recorded on a SPS11-10µ from Projekt Messtechnik (Ulm, Germany). One cycle was run at 25 °C, in which the sample was first stepwise decreased to 0% Room humidity (RH) and then the relative humidity was stepwise increased from 0 to 95% and the weight of the sample was measured. Typical total measurement times for both cycles were about 20 to 30 hours. The measured data for the (R)-3-[6-amino-pyridin-3-yl]-2-(1-cyclohexyl-1 H-imidazol-4-yl)-propionic acid sodium salt dihydrate as prepared in Example 3 are shown in the following table:

**DVS isotherm at 25°C:**

| | Target RH (%) | Change In Mass (%) Sorption | Change In Mass (%) Desorption |
|---|---|---|---|
| Cycle 1 | 30.1 | | 0.0 |
| | 20.1 | | 0.0 |
| | 10.0 | | 0.0 |
| | 0.3 | | -0.1 |
| | 10.0 | 0.0 | |
| | 20.0 | 0.0 | |
| | 30.0 | 0.0 | |
| | 40.0 | 0.0 | |
| | 50.0 | 0.0 | |
| | 60.0 | 0.1 | |
| | 70.1 | 0.71 | |
| | 75.0 | 0.8 | |
| | 80.0 | 2.1 | |
| | 85.1 | 2.7 | |
| | 90.1 | 18.8 | |
| | 94.9 | 51.8 | |

"RH"means relative humidity; the relative humidity of an air-water mixture is defined as the ratio of the partial pressure of water vapor in the mixture to the saturated vapor pressure of water at a prescribed temperature. DVS shows a water uptake of 2.1 % at 80% RH and 51.8% at 95% RH for the (R)-3-[6-amino-pyridin-3-yl]-2-(1-cyclohexyl-1 H-imidazol-4-yl)-propionic acid sodium salt dihydrate.

Example 8: Chiral stability: Representative samples of the crystalline sodium salt of (R)-3-[6-amino-pyridin-3-yl]-2-(1-cyclohexyl-1 H-imidazol-4-yl)-propionic acid sodium salt dihydrate as prepared in Example 3 were stored as a solid under the conditions (A) and (B): (A) 40 °C and 75 % RH 2 weeks; (B) 40 °C and 75 % RH 4 weeks: Results are shown in the following table:

| Storage condition | (A) | (B) |
|---|---|---|
| Enantiomeric excess | 99.6 | 99.5 % |

The enantiomeric excess of the measured samples was at the beginning 99.6 % of (R)-3-[6-amino-pyridin-3-yl]-2-(1-cyclohexyl-1 H-imidazol-4-yl)-propionic acid sodium salt dehydrate. Under the storage conditions the samples remain stable. Only after 4 weeks under the condition of 40 °C and 75 % RH the enantiomeric excess of 3-[6-amino-pyridin-3-yl]-2-(1-cyclohexyl-1 H-imidazol-4-yl)-propionic acid sodium salt dihydrate decreased slightly to a value of 99.5%. The purity was determined by chiral HPLC on a HPLC system containing a Quaternary HPLC pump (e.g. HP 1100), an auto sampler, an UV absorbance detector (e.g. DAD Agilent Technologies or other adequate HPLC-System) and a computer data acquisition system with suitable software (e.g. Dionex-Chromeleon). A Zorbax SB_CN (4.6 x 100 mm, 3.5 µlτ 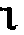 ), column was used as stationary phase and two mobile phases A (975 mL water, 25 mL methanol, 1 mL perchloric acid) and B (975 mL methanol, 25 mL water) were used as mobile phases. The flow rate was set to a value of 1.0 mL/minute and the oven temperature was set to 15°C. Detection was performed by absorbance of the compounds at 236 nm (UV) (bandwidth: 4 nm) and reference wavelength was set to 450 nm (bandwidth: 50 nm). Typical total run times were in the range of about 10 minutes. For sample preparation 20 mg of the substance were accurately weighed into a 10 ml volumetric flask and dissolved and diluted to volume with solvent (acetonitrile/water 90:10 VN). Afterwards 1 ml of this solution was transferred to a 10 mL volumetric flask and then diluted to volume with water. For the HPLC analysis 5 µl of the sample were injected into the system. Gradient used: 0 min (time elapsed) 0% mobile phase B, 1 min 0% mobile phase B, 4 min 53% mobile phase B, 6 min 95% mobile phase B, 7 min 95% mobile phase B, 7.5 min 0% mobile phase B and after 10 min 0% mobile phase B. Enanatiomeric excess was determined by chiral HPLC on a HPLC system containing a Quaternary HPLC pump (e.g. HP 1100), an auto sampler, an UV absorbance detector (e.g. DAD Agilent Technologies or other adequate HPLC-System) and a computer data acquisition system with suitable software (e.g. Dionex-Chromeleon). A Chirobiotic R, Supelco (Astec) (4.6 x 250mm; 5µηη) column was used as stationary phase and a solution of 800 mL water, 1200 mL acetonitrile and 1.0 g ammoniumacetate was used as mobile phase. The flow rate was set to a value of 1.5 mL/minute and the oven temperature was set to 45°C. Detection was performed by absorbance of the compounds at 236 nm (UV) (bandwidth: 8 nm) and reference wavelength was set to 450 nm (bandwidth: 50 nm). Typical total run times were in the range of about 15 minutes. For sample preparation 50 mg of the substance were accurately weighed into a 10 mL volumetric flask and dissolved and diluted to volume with solvent (acetonitrile/water 50:50). For the HPLC analysis 5 µí of the sample were injected into the system.
Gradient used: Isograt (15 minutes 100% mobile phase A).

Example 9: DVS, Comparison Experiment: (R)-3-[6-amino-pyridin-3-yl]-2-(1-cyclohexyl-1 H-imidazol-4-yl)-propionic acid as prepared in Example 1 was used for the measurement. The measurement was performed as disclosed in Example 7 with (R)-3-[6-amino-pyridin-3-yl]-2-(1-cyclohexyl-1 H-imidazol-4-yl)-propionic acid (free acid, amorphous) and the received data are shown in the following table:

**DVS Isotherm at 25 °C**

| | Target RH (%) | Change In Mass (%) Sorption | Change In Mass (%) Desorption |
|---|---|---|---|
| Cycle 1 | 30.1 | | 1.7 |
| | 20.0 | | 1.1 |
| | 10.1 | | 0.3 |
| | 0.4 | | -1.6 |
| | 10.0 | -0.5 | |
| | 20.0 | 0.5 | |
| | 30.0 | 1.9 | |
| | 40.1 | 3.9 | |
| | 50.0 | 6.6 | |
| | 60.1 | 9.6 | |
| | 70.0 | 13.4 | |
| | 75.0 | 15.0 | |
| | 80.1 | 16.9 | |
| | 85.1 | 20.1 | |
| | 89.9 | 25.1 | |
| | 95.0 | 32.3 | |

DVS shows a strong water uptake of 3.9% at 40% RH, 6.6% at 50% RH, 9.6% at 60% RH, 13.4% at 70% RH, 16.9% at 80% RH, 25.1 % at 90% RH and 32.3% at 95% RH for (R)-3-[6-amino-pyridin-3-yl]-2-(1-cyclohexyl-1 H-imidazol-4-yl)-propioni acid (free acid, amorphous).

Example 10: Chiral stability; Comparison Experiment: (R)-3-[6-amino-pyridin-3-yl]-2-(1-cyclohexyl-1 H-imidazol-4-yl)-propionic acid as prepared in Example 1 was used for the measurement. Representative samples of the compound (R)-3-[6-amino-pyridin-3-yl]-2-(1-cyclohexyl-1 H-imidazol-4-yl)-propionic acid as prepared in Example 1 were stored as a solid under the conditions (A) and (B) as defined in Example 8. Results are shown in the following table:

| Storage condition | (A) | (B) |
|---|---|---|
| Enantiomeric excess | 94.9% | 91.6 % |

The enantiomeric excess of the measured samples was at the beginning 98.2 % of (R)-3-[6-amino-pyridin-3-yl]-2-(1-cyclohexyl-1 H-imidazol-4-yl)-propionic acid and under the storage conditions (A) and (B) showed degradation to the (S)-enantiomer at 75% RH. The enantiomeric excess dropped down to a value of 94.9% at storage condition (A) and 9L6% at storage condition (B), respectively. The purity was determined by the chromatography described in Example 8.

### SPECIFIC INORMATION CONCERNING ASPECT (V) OF THE INVENTION

### Aspect (v) of the invention relates to an administration unit comprising polymorph of compound (V)

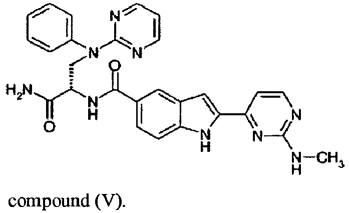

The invention relates to a solid form of compound (V), namely to polymorph of compound (V), which is useful for treating diseases related to IκB kinase (1-kappa-B kinase, IKK) and/or osteoarthritis or pain. Polymorph of compound (V) is described in WO2013/083553, which is incorporated by reference. Compound (V) is 2-(2-Methylamino-pyrimidin-4-yl)-1 H-indole-5-carboxylic acid [(S)-1-carbamoyl-2-(phenyl-pyrimidin-2-yl-amino)-ethyl]-amide of the formula (V), 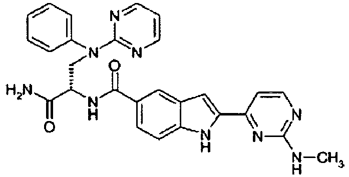 which is also abbreviated herein as "compound (V)" ({drug5}). As used herein, polymorph of compound (V) are crystalline and/or hydrated forms of compound (V) as described in WO2013/083553, which is cited hereinafter. Polymorph of compound (V) ({drug5a}) can be obtained as described in WO2013/083553, which is cited hereinafter: The present invention relates to polymorphs and hydrates of 2-(2-methylamino-pyrimidin-4-yl)-1 H-indole-5-carboxylic acid [(S)-1-carbamoyl-2-(phenyl-pyrimidin-2-yl-amino)-ethyl]-amide, processes for their preparation and their use, in particular in pharmaceutical compositions. 2-(2-Methylamino-pyrimidin-4-yl)-1H-indole-5-carboxylic acid [(S)-1-carbamoyl-2-(phenyl-pyrimidin-2-yl-amino)-ethyl]-amide of the formula (V), 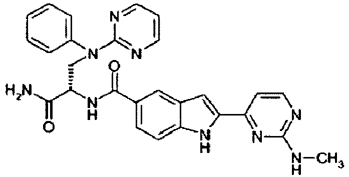 which is also abbreviated herein as "compound (V)" ({drug5}), is a pharmaceutical active compound which inhibits IκB kinase (1-kappa-B kinase, IKK) and is useful for the treatment of various diseases such as osteoarthritis or pain, for example, as is described in WO 2004/022553, US 7285560, WO 2004/022057 and US 7462638, for example. However, data concerning crystalline forms, polymorphs or hydrates of 2-(2-methylamino-pyrimidin-4-yl)-1 H-indole-5-carboxylic acid [(S)-1-carbamoyl-2-(phenyl-pyrimidin-2-yl-amino)-ethyl]-amide are not disclosed in the prior art. Polymorphism is the ability of a compound to exist in more than one crystalline form or crystal structure. Different polymorphs represent distinct solids sharing the same molecular formula, yet each polymorph may have distinct physical properties. A specific compound may give rise to a variety of polymorphic forms wherein each form has different and distinct physical properties, such as different solubility profiles, different thermodynamic stability, different crystallization behavior, different filterability, different melting point temperatures and/or different X-ray diffraction patterns. The difference in the physical properties of different polymorphic forms results from different orientation and intermolecular interactions of adjacent molecules in the solid. Polymorphic forms of a compound can be distinguished by X-ray diffraction and by other methods such as infrared spectroscopy or Raman spectroscopy, for example. These statements apply likewise to hydrates, i.e. solid addition compounds of a compound with water, which are a specific form of solvates and which may form when a compound (V) is in contact with water, for example when crystallizing a compound (V) in the presence of water. However, as acknowledged by the person skilled in the art, the presence of different crystalline polymorphic forms or hydrates of a compound cannot be foreseen. Neither the existence of polymorphic forms or hydrates nor the number of polymorphic forms or hydrates can be foreseen. Also the conditions under which crystallization takes place to give a specific form, and the characteristics of the polymorphic forms and hydrates cannot be predicted. Since properties such as the solubility and stability and consequently the suitability for use and storage of each polymorphic form and hydrate may vary, identifying the existence of polymorphic forms and solvates such as hydrates is essential for providing pharmaceuticals with increased storage stability or suitable solubility profiles, for example. Thus, it is desirable to investigate all solid state forms of a drug substance, including polymorphic forms and hydrates. Accordingly, it was the object of the present invention to provide solid forms of 2-(2-methylamino-pyrimidin-4-yl)-1 H-indole-5-carboxylic acid [(S)-1-carbamoyl-2-(phenyl-pyrimidin-2-yl-amino)-ethyl]-amide, in particular forms which have a favorable property profile or are useful in the preparation of the compound. This object was attained by providing polymorphs and hydrates which are selected from the series consisting of polymorph 1 ({drug5b}), polymorph 2 ({drug5c}), polymorph 3 ({drug5d}), hydrate 1 ({drug5e}) and hydrate 2 ({drug5f}) of 2-(2-methylamino-pyrimidin-4-yl)-1 H-indole-5-carboxylic acid [(S)-1-carbamoyl-2-(phenyl-pyrimidin-2-yl-amino)-ethyl]-amide and any mixture thereof, which polymorphs and hydrates have favorable properties with respect to stability, solubility, processability, hygroscopicity, flowability, filterability or crystallization rate, for example, wherein polymorph 1 ({drug5b}), polymorph 2 ({drug5c}), polymorph 3 ({drug5d}), hydrate 1 ({drug5e}) and hydrate 2 ({drug5f}) may be characterized by any of their data given herein. The data used to characterize the polymorph and hydrates of the present invention were obtained as described below. In the context of the present invention, polymorph, polymorphic form, hydrate etc. refers to a polymorph, polymorphic form or hydrate of 2-(2-methylamino-pyrimidin-4-yl)-1 H-indole-5-carboxylic acid [(S)-1-carbamoyl-2-(phenyl-pyrimidin-2-yl-amino)-ethyl]-amide. Terms like "polymorph", "polymorphic form", "phase"and "crystalline phase"may be used interchangeably herein. One embodiment of the present invention relates to a crystalline form of 2-(2-methylamino-pyrimidin-4-yl)-1 H-indole-5-carboxylic acid [(S)-1-carbamoyl-2-(phenyl-pyrimidin-2-yl-amino)-ethyl]-amide which is selected from the series consisting of polymorph 1 ({drug5b}), polymorph 2 ({drug5c}), polymorph 3 ({drug5d}) and any mixture thereof, wherein polymorph 1 ({drug5b}), polymorph 2 ({drug5c}) and polymorph 3 ({drug5d}) may be characterized by any of their data given herein. One embodiment of the present invention relates to a crystalline form of 2-(2-methylamino-pyrimidin-4-yl)-1 H-indole-5-carboxylic acid [(S)-1-carbamoyl-2-(phenyl-pyrimidin-2-yl-amino)-ethyl]-amide which is selected from the series consisting of hydrate 1 and hydrate 2 and any mixture thereof, wherein hydrate 1 ({drug5e}) and hydrate 2 ({drug5f}) may be characterized by any of their data given herein.

One embodiment of the present invention relates to polymorph 1 ({drug5b}) of 2-(2-methylamino-pyrimidin-4-yl)-1 H-indole-5-carboxylic acid [(S)-1-carbamoyl-2-(phenyl-pyrimidin-2-yl-amino)-ethyl]-amide which has characteristic reflections in an X-ray powder d iff ractog ram using CuKa1 (CuK-alphal) radiation in reflection mode at 2Theta (20) angles in degree [°] of 14.9 ± 0.2, 19.4 ± 0.2, 19.7 ± 0.2, 20.0 ± 0.2, 22.3 ± 0.2, 25.0 ± 0.2. One embodiment of the present invention relates to polymorph 1 ({drug5b}) of 2-(2-methylamino-pyrimidin-4-yl)-1 H-indole-5-carboxylic acid [(S)-1-carbamoyl-2-(phenyl-pyrimidin-2-yl-amino)-ethyl]-amide which has characteristic reflections in an X-ray powder diffractogram using CuKa1 (CuK-alpha1) radiation in reflection mode at 2Theta (20) angles in degree [°] of 10.4 ± 0.2, 14.9 ± 0.2, 17.5° ± 0.2, 18.0 ± 0.2, 19.4 ± 0.2, 19.7 ± 0.2, 20.0 ± 0.2, 21.0 ± 0.2, 22.3 ± 0.2, 25.0 ± 0.2. One embodiment of the present invention relates to polymorph 1 ({drug5b}) of 2-(2-methylamino-pyrimidin-4-yl)-1 H-indole-5-carboxylic acid [(S)-1-carbamoyl-2-(phenyl-pyrimidin-2-yl-amino)-ethyl]-amide which has any one or more of the characteristic reflections in an X-ray powder diffractogram using CuKa1 radiation in reflection mode which are given above or below herein. One embodiment of the present invention relates to polymorph 1 ({drug5b}) of 2-(2-methylamino-pyrimidin-4-yl)-1 H-indole-5-carboxylic acid [(S)-1-carbamoyl-2-(phenyl-pyrimidin-2-yl-amino)-ethyl]-amide which has an X-ray powder diffraction pattern substantially as the one shown in Figure 1 of WO2013/083553 which has been obtained using CuKa1 radiation in reflection mode, wherein the exact relative intensities of the reflections depicted in Figure 1 of WO2013/083553 are not a prerequisite, but may vary and represent another embodiment of the invention. Polymorph 1 ({drug5b}) of 2-(2-methylamino-pyrimidin-4-yl)-1 H-indole-5-carboxylic acid [(S)-1-carbamoyl-2-(phenyl-pyrimidin-2-yl-amino)-ethyl]-amide may also be characterized by its melting characteristics such as its melting point determined by differential scanning calorimetry (DSC) with an onset temperature of 253 ± 1 °C and/or a peak temperature of 257 ± 1 °C (heating rate 10 °C/minute). Polymorph 1 ({drug5b}) of 2-(2-methylamino-pyrimidin-4-yl)-1 H-indole-5-carboxylic acid [(S)-1-carbamoyl-2-(phenyl-pyrimidin-2-yl-amino)-ethyl]-amide is thermodynamically most stable polymorph of 2-(2-methylamino-pyrimidin-4-yl)-1 H-indole-5-carboxylic acid [(S)-1-carbamoyl-2-(phenyl-pyrimidin-2-yl-amino)-ethyl]-amide at temperatures above about 20 °C, as is confirmed in phase conversion experiments, for example. Therefore, in comparison to the other polymorphs, polymorph 1 ({drug5b}) is particularly suitable when a high stability is desired. Polymorph 1 ({drug5b}) is further characterized by a low hygroscopicity of about 0.9 % water uptake at a high relative humidity of 80 %. In view of its stability and low hygroscopicity, polymorph 1 ({drug5b}) is particularly suitable for storage of 2-(2-methylamino-pyrimidin-4-yl)-1 H-indole-5-carboxylic acid [(S)-1-carbamoyl-2-(phenyl-pyrimidin-2-yl-amino)-ethyl]-amide. Polymorph 1 is further characterized by a low solubility in water, which allows an easy realization of a depot effect with respect to the pharmacological activity of 2-(2-methylamino-pyrimidin-4-yl)-1 H-indole-5-carboxylic acid [(S)-1-carbamoyl-2-(phenyl-pyrimidin-2-yl-amino)-ethyl]-amide by administering a suitable pharmaceutical formulation containing solid polymorph 1 ({drug5b}). Therefore, polymorph 1 ({drug5b}) is particularly suitable for use in pharmaceutical compositions, or in medicaments, which are designed to have a long duration of action.

One embodiment of the present invention relates to polymorph 2 ({drug5c}) of 2-(2-methylamino-pyrimidin-4-yl)-1 H-indole-5-carboxylic acid [(S)-1-carbamoyl-2-(phenyl-pyrimidin-2-yl-amino)-ethyl]-amide which has characteristic reflections in an X-ray powder diffractogram using CuKal (CuK-alphal) radiation in reflection mode at 2Theta (20) angles in degree [°] of 5.8 ± 0.2, 6.7 ± 0.2, 9.3 ± 0.2, 11.2 ± 0.2, 19.4 ± 0.2, 22.1 ± 0.2. One embodiment of the present invention relates to polymorph 2 ({drug5c}) of 2-(2-methylamino-pyrimidin-4-yl)-1 H-indole-5-carboxylic acid [(S)-1-carbamoyl-2-(phenyl-pyrimidin-2-yl-amino)-ethyl]-amide which has characteristic reflections in an X-ray powder diffractogram using CuKal (CuK-alphal) radiation in reflection mode at 2Theta (20) angles in degree [°] of 5.8 ± 0.2, 6.7 ± 0.2, 9.3 ± 0.2, 9.9 ± 0.2, 11.2 ± 0.2, 16.5 ± 0.2, 18.1 ± 0.2, 19.4 ± 0.2, 22.1 ± 0.2. One embodiment of the present invention relates to polymorph 2 ({drug5c}) of 2-(2-methylamino-pyrimidin-4-yl)-1 H-indole-5-carboxylic acid [(S)-1-carbamoyl-2-(phenyl-pyrimidin-2-yl-amino)-ethyl]-amide which has any one or more of the characteristic reflections in an X-ray powder diffractogram using CuKal radiation in reflection mode which are given above or below herein. One embodiment of the present invention relates to polymorph 2 ({drug5c}) of 2-(2-methylamino-pyrimidin-4-yl)-1 H-indole-5-carboxylic acid [(S)-1-carbamoyl-2-(phenyl-pyrimidin-2-yl-amino)-ethyl]-amide which has an X-ray powder diffraction pattern substantially as the one shown in Figure 2 of WO2013/083553 which has been obtained using CuKal radiation in reflection mode, wherein the exact relative intensities of the reflections depicted in Figure 2 of WO2013/083553 are not a prerequisite, but may vary and represent another embodiment of the invention. Polymorph 2 ({drug5c}) of 2-(2-methylamino-pyrimidin-4-yl)-1 H-indole-5-carboxylic acid [(S)-1-carbamoyl-2-(phenyl-pyrimidin-2-yl-amino)-ethyl]-amide may also be characterized by its behavior in differential scanning calorimetry (DSC), where it gives rise to an endothermic peak with an onset temperature of 222 ± 1 °C and/or a peak temperature of 225 ± 1 °C, followed by an endothermic peak with an onset temperature of 248 ± 1 °C and/or a peak temperature of 251 ± 1 °C (heating rate 10 °C/minute). Polymorph 2 ({drug5c}) of 2-(2-methylamino-pyrimidin-4-yl)-1 H-indole-5-carboxylic acid [(S)-1-carbamoyl-2-(phenyl-pyrimidin-2-yl-amino)-ethyl]-amide has a higher solubility than polymorph 1 ({drug5b}) and can more easily be purified, and is advantageously isolated in the preparation of 2-(2-methylamino-pyrimidin-4-yl)-1 H-indole-5-carboxylic acid [(S)-1-carbamoyl-2-(phenyl-pyrimidin-2-yl-amino)-ethyl]-amide if a further purification is desired, for example by crystallization from, or slurrying with, a suitable solvent system. Polymorph 2 ({drug5c}) is further characterized by a low hygroscopicity of about 0.4 % water uptake at a high relative humidity of 80 %. It is furthermore suitable for use in pharmaceutical compositions, or medicaments, in case a higher solubility is advantageous for the desired pharmacological effect of 2-(2-methylamino-pyrimidin-4-yl)-1 H-indole-5-carboxylic acid [(S)-1-carbamoyl-2-(phenyl-pyrimidin-2-yl-amino)-ethyl]-amide.

One embodiment of the present invention relates to polymorph 3 ({drug5d}) of 2-(2-methylamino-pyrimidin-4-yl)-1 H-indole-5-carboxylic acid [(S)-1-carbamoyl-2-(phenyl-pyrimidin-2-yl-amino)-ethyl]-amide which has characteristic reflections in an X-ray powder diffractogram using CuKal (CuK-alpha1) radiation in reflection mode at 2Theta (20) angles in degree [°] of 15.2 ± 0.2, 15.9 ± 0.2, 17.3 ± 0.2, 19.2 ± 0.2, 22.2 ± 0.2, 25.3 ± 0.2. One embodiment of the present invention relates to polymorph 3 ({drug5d}) of 2-(2-methylamino-pyrimidin-4-yl)-1 H-indole-5-carboxylic acid [(S)-1-carbamoyl-2-(phenyl-pyrimidin-2-yl-amino)-ethyl]-amide which has characteristic reflections in an X-ray powder diffractogram using CuKal (CuK-alphal) radiation in reflection mode at 2Theta (20) angles in degree [°] of 13.9 ± 0.2, 15.2 ± 0.2, 15.9 ± 0.2, 17.3 ± 0.2, 19.2 ± 0.2, 22.2 ± 0.2, 24.5 ± 0.2, 25.3 ± 0.2. One embodiment of the present invention relates to polymorph 3 ({drug5d}) of 2-(2-methylamino-pyrimidin-4-yl)-1 H-indole-5-carboxylic acid [(S)-1-carbamoyl-2-(phenyl-pyrimidin-2-yl-amino)-ethyl]-amide which has any one or more of the characteristic reflections in an X-ray powder diffractogram using CuKal radiation in reflection mode which are given above or below herein. One embodiment of the present invention relates to polymorph 3 ({drug5d}) of 2-(2-methylamino-pyrimidin-4-yl)-1 H-indole-5-carboxylic acid [(S)-1-carbamoyl-2-(phenyl-pyrimidin-2-yl-amino)-ethyl]-amide which has an X-ray powder diffraction pattern substantially as the one shown in Figure 3 of WO2013/083553 which has been obtained using CuKal radiation in reflection mode, wherein the exact relative intensities of the reflections depicted in Figure 3 of WO2013/083553 are not a prerequisite, but may vary and represent another embodiment of the invention. Polymorph 3 ({drug5d}) of 2-(2-methylamino-pyrimidin-4-yl)-1 H-indole-5-carboxylic acid [(S)-1-carbamoyl-2-(phenyl-pyrimidin-2-yl-amino)-ethyl]-amide can be obtained from hydrate 1 ({drug5e}) of 2-(2-methylamino-pyrimidin-4-yl)-1 H-indole-5-carboxylic acid [(S)-1-carbamoyl-2-(phenyl-pyrimidin-2-yl-amino)-ethyl]-amide described in the following, and again converted into hydrate 1 ({drug5e}), and like hydrate 1 ({drug5e}) is of use in processes for the purification of 2-(2-methylamino-pyrimidin-4-yl)-1 H-indole-5-carboxylic acid [(S)-1-carbamoyl-2-(phenyl-pyrimidin-2-yl-amino)-ethyl]-amide, for example by crystallization from, or slurrying with, a suitable solvent system.

One embodiment of the present invention relates to hydrate 1 ({drug5e}) of 2-(2-methylamino-pyrimidin-4-yl)-1 H-indole-5-carboxylic acid [(S)-1-carbamoyl-2-(phenyl-pyrimidin-2-yl-amino)-ethyl]-amide which has characteristic reflections in an X-ray powder diffractogram using CuKal (CuK-alphal) radiation in reflection mode at 2Theta (20) angles in degree [°] of 5.3 ± 0.2, 13.5 0.2, 17.9 ± 0.2, 19.5 ± 0.2, 21.5 ± 0.2, 24.9 ± 0.2. One embodiment of the present invention relates to hydrate 1 ({drug5e}) of 2-(2-methylamino-pyrimidin-4-yl)-1 H-indole-5-carboxylic acid [(S)-1-carbamoyl-2-(phenyl-pyrimidin-2-yl-amino)-ethyl]-amide which has characteristic reflections in an X-ray powder diffractogram using CuKal (CuK-alphal) radiation in reflection mode at 2Theta (20) angles in degree [°] of 5.3 ± 0.2, 11.1 ± 0.2, 13.5 ± 0.2, 17.9 ± 0.2, 19.5 ± 0.2, 21.5 ± 0.2, 23.5 ± 0.2, 24.9 ± 0.2, 28.2 ± 0.2. One embodiment of the present invention relates to hydrate 1 ({drug5e}) of 2-(2-methylamino-pyrimidin-4-yl)-1 H-indole-5-carboxylic acid [(S)-1-carbamoyl-2-(phenyl-pyrimidin-2-yl-amino)-ethyl]-amide which has any one or more of the characteristic reflections in an X-ray powder diffractogram using CuKa radiation in reflection mode which are given above or below herein. One embodiment of the present invention relates to hydrate 1 ({drug5e}) of 2-(2-methylamino-pyrimidin-4-yl)-1 H-indole-5-carboxylic acid [(S)-1-carbamoyl-2-(phenyl-pyrimidin-2-yl-amino)-ethyl]-amide which has an X-ray powder diffraction pattern substantially as the one shown in Figure 4 of WO2013/083553 which has been obtained using CuKal radiation in reflection mode, wherein the exact relative intensities of the reflections depicted in Figure 4 of WO2013/083553 are not a prerequisite, but may vary and represent another embodiment of the invention. Hydrate 1 ({drug5e}) of 2-(2-methylamino-pyrimidin-4-yl)-1 H-indole-5-carboxylic acid [(S)-1-carbamoyl-2-(phenyl-pyrimidin-2-yl-amino)-ethyl]-amide may also be characterized by its crystal parameters which have been determined by single crystal structure analysis. Hydrate 1 ({drug5e}) crystallizes in the orthorhombic space group P2i2i2i with the following cell characteristics: Z = 4, a = 8.18650(10) A, b = 9.97420(10) A, c = 32.8707(2) A, α = β = Y = 90.00° (at 20 °C). Hydrate 1 ({drug5e}) of 2-(2-methylamino-pyrimidin-4-yl)-1 H-indole-5-carboxylic acid [(S)-1-carbamoyl-2-(phenyl-pyrimidin-2-yl-amino)-ethyl]-amide may also be characterized by its behavior in differential scanning calorimetry (DSC), where it gives rise to a broad endothermic peak between room temperature and about 100 °C, which can be associated with the loss of water, a sharper endothermic peak at about 170 °C, an exothermic peak at about 190 °C, and a melting peak at about 253 °C (heating rate 10 °C/minute). In one embodiment of the invention, hydrate 1 ({drug5e}) of 2-(2-methylamino-pyrimidin-4-yl)-1 H-indole-5-carboxylic acid [(S)-1-carbamoyl-2-(phenyl-pyrimidin-2-yl-amino)-ethyl]-amide contains 2 ± 0.3 mol of water, in another embodiment 2 ± 0.1 mol of water, in another embodiment about 2 mol of water, per mol of 2-(2-methylamino-pyrimidin-4-yl)-1 H-indole-5-carboxylic acid [(S)-1-carbamoyl-2-(phenyl-pyrimidin-2-yl-amino)-ethyl]-amide, which latter water content was determined by thermogravimetric analysis, for example. Hydrate 1 ({drug5e}) may thus be termed 2-(2-methylamino-pyrimidin-4-yl)-1 H-indole-5-carboxylic acid [(S)-1-carbamoyl-2-(phenyl-pyrimidin-2-yl-amino)-ethyl]-amide dihydrate. Hydrate 1 ({drug5e}) of 2-(2-methylamino-pyrimidin-4-yl)-1 H-indole-5-carboxylic acid [(S)-1-carbamoyl-2-(phenyl-pyrimidin-2-yl-amino)-ethyl]-amide has a higher solubility than polymorph 1 ({drug5b}) and can more easily be purified, and is advantageously isolated in the preparation of 2-(2-methylamino-pyrimidin-4-yl)-1 H-indole-5-carboxylic acid [(S)-1-carbamoyl-2-(phenyl-pyrimidin-2-yl-amino)-ethyl]-amide if a further purification is desired, for example by crystallization from, or slurrying with, a suitable solvent system. Hydrate 1 ({drug5e}) is further characterized by a low hygroscopicity. It is furthermore suitable for use in pharmaceutical compositions, or medicaments, in case a higher solubility is advantageous for the desired pharmacological effect of 2-(2-methylamino-pyrimidin-4-yl)-1 H-indole-5-carboxylic acid [(S)-1-carbamoyl-2-(phenyl-pyrimidin-2-yl-amino)-ethyl]-amide.

One embodiment of the present invention relates to hydrate 2 ({drug5f}) of 2-(2-methylamino-pyrimidin-4-yl)-1 H-indole-5-carboxylic acid [(S)-1-carbamoyl-2-(phenyl-pyrimidin-2-yl-amino)-ethyl]-amide which has characteristic reflections in an X-ray powder diffractogram using CuKal (CuK-alphal) radiation in reflection mode at 2Theta (2Θ) angles in degree [°] of 2.9 ± 0.2, 5.3 ± 0.2, 8.3 ± 0.2, 11.5 ± 0.2, 17.1 ± 0.2, 22.8 ± 0.2. One embodiment of the present invention relates to hydrate 2 ({drug5f}) of 2-(2-methylamino-pyrimidin-4-yl)-1 H-indole-5-carboxylic acid [(S)-1-carbamoyl-2-(phenyl-pyrimidin-2-yl-amino)-ethyl]-amide which has characteristic reflections in an X-ray powder diffractogram using CuKal (CuK-alphal) radiation in reflection mode at 2Theta (20) angles in degree [°] of 2.9 ± 0.2, 5.3 ± 0.2, 6.2 ± 0.2, 8.3 ± 0.2, 9.5 ± 0.2, 11.5 ± 0.2, 14.0 ± 0.2, 17.1 ± 0.2, 18.0 ± 0.2, 22.8 ± 0.2. One embodiment of the present invention relates to hydrate 2 ({drug5f}) of 2-(2-methylamino-pyrimidin-4-yl)-1 H-indole-5-carboxylic acid [(S)-1-carbamoyl-2-(phenyl-pyrimidin-2-yl-amino)-ethyl]-amide which has any one or more of the characteristic reflections in an X-ray powder diffractogram using CuKal radiation in reflection mode which are given above or below herein. One embodiment of the present invention relates to hydrate 2 ({drug5f}) of 2-(2-methylamino-pyrimidin-4-yl)-1 H-indole-5-carboxylic acid [(S)-1-carbamoyl-2-(phenyl-pyrimidin-2-yl-amino)-ethyl]-amide which has an X-ray powder diffraction pattern substantially as the one shown in Figure 5 of WO2013/083553 which has been obtained using CuKal radiation in reflection mode, wherein the exact relative intensities of the reflections depicted in Figure 5 of WO2013/083553 are not a prerequisite, but may vary and represent another embodiment of the invention. Hydrate 2 ({drug5f}) of 2-(2-methylamino-pyrimidin-4-yl)-1 H-indole-5-carboxylic acid [(S)-1-carbamoyl-2-(phenyl-pyrimidin-2-yl-amino)-ethyl]-amide may also be characterized by its behavior in differential scanning calorimetry (DSC), where it gives rise to broad endothermic peaks centered around 99 °C and 128 °C, a small endothermic peak at 146 °C, an exothermic peak at 215 °C, and a melting peak around 250 °C (heating rate 10 °C/minute). In one embodiment of the invention, hydrate 2 ({drug5f}) of 2-(2-methylamino-pyrimidin-4-yl)-1 H-indole-5-carboxylic acid [(S)-1-carbamoyl-2-(phenyl-pyrimidin-2-yl-amino)-ethyl]-amide contains 6 ± 0.3 mol of water, in another embodiment 6 ± 0.1 mol of water, in another embodiment about 6 mol of water, per mol of 2-(2-methylamino-pyrimidin-4-yl)-1 H-indole-5-carboxylic acid [(S)-1-carbamoyl-2-(phenyl-pyrimidin-2-yl-amino)-ethyl]-amide, which latter water content was determined by thermogravimetric analysis, for example. Hydrate 2 ({drug5f}) may thus be termed 2-(2-methylamino-pyrimidin-4-yl)-1 H-indole-5-carboxylic acid [(S)-1-carbamoyl-2-(phenyl-pyrimidin-2-yl-amino)-ethyl]-amide hexahydrate. Hydrate 2 ({drug5f}) of 2-(2-methylamino-pyrimidin-4-yl)-1 H-indole-5-carboxylic acid [(S)-1-carbamoyl-2-(phenyl-pyrimidin-2-yl-amino)-ethyl]-amide has a higher solubility than polymorph 1 ({drug5b}) and can more easily be purified, and is advantageously isolated in the preparation of 2-(2-methylamino-pyrimidin-4-yl)-1 H-indole-5-carboxylic acid [(S)-1-carbamoyl-2-(phenyl-pyrimidin-2-yl-amino)-ethyl]-amide if a further purification is desired, for example by crystallization from, or slurrying with, a suitable solvent system. It is furthermore suitable for use in pharmaceutical compositions, or medicaments, in case a higher solubility is advantageous for the desired pharmacological effect of 2-(2-methylamino-pyrimidin-4-yl)-1 H-indole-5-carboxylic acid [(S)-1-carbamoyl-2-(phenyl-pyrimidin-2-yl-amino)-ethyl]-amide.

One embodiment of the present invention, which is based on the properties of the polymorphs and hydrates such as their solubility specified above, relates to a process for purifying 2-(2-methylamino-pyrimidin-4-yl)-1 H-indole-5-carboxylic acid [(S)-1-carbamoyl-2-(phenyl-pyrimidin-2-yl-amino)-ethyl]-amide, wherein at least one of polymorph 2 ({drug5c}), polymorph 3 ({drug5d}), hydrate 1 ({drug5e}) and hydrate 2 ({drug5f}) of 2-(2-methylamino-pyrimidin-4-yl)-1 H-indole-5-carboxylic acid [(S)-1-carbamoyl-2-(phenyl-pyrimidin-2-yl-amino)-ethyl]-amide or a mixture of any of them is isolated in the course of the preparation and purification of 2-(2-methylamino-pyrimidin-4-yl)-1 H-indole-5-carboxylic acid [(S)-1-carbamoyl-2-(phenyl-pyrimidin-2-yl-amino)-ethyl]-amide. The present invention further relates to the use of a polymorph or a hydrate of 2-(2-methylamino-pyrimidin-4-yl)-1 H-indole-5-carboxylic acid [(S)-1-carbamoyl-2-(phenyl-pyrimidin-2-yl-amino)-ethyl]-amide according to the present invention, selected from polymorphs 1 ({drug5b}), 2 ({drug5c}) and 3 ({drug5d}) and hydrates 1 ({drug5e}) and 2 ({drug5f}), or a mixture of polymorphs and hydrates comprising at least one of them, as a pharmaceutical or medicament, which use is based on the pharmacological activity of compound (V) already described in detail in WO 2004/022553, US 7285560, WO 2004/022057 and US 7462638, for example. One embodiment of the invention relates to a polymorph selected from polymorphs 1 ({drug5b}) and 2 ({drug5c}), or a mixture of polymorphs comprising at least one of polymorphs 1 ({drug5b}) and 2 ({drug5c}), for use as a pharmaceutical or medicament. One embodiment of the invention relates to polymorph 1 ({drug5b}), or a mixture of polymorphs comprising at least polymorph 1 ({drug5b}), for use as a pharmaceutical or medicament. One embodiment of the invention relates to a hydrate selected from hydrates 1 ({drug5e}) and ({drug5f}), or a mixture of polymorphs and/or hydrates comprising at least one of hydrates 1 ({drug5e}) and 2 ({drug5f}), for use as pharmaceutical or medicament. One embodiment of the present invention relates to a pharmaceutical composition which comprises at least one polymorph or hydrate of 2-(2-methylamino-pyrimidin-4-yl)-1 H-indole-5-carboxylic acid [(S)-1-carbamoyl-2-(phenyl-pyrimidin-2-yl-amino)-ethyl]-amide according to the present invention, selected from polymorphs 1 ({drug5b}), 2 ({drug5c}) and 3 ({drug5d}) and hydrates 1 ({drug5e}) and 2 ({drug5f}), or a mixture of polymorphs and hydrates comprising at least one of them, and one or more pharmaceutically acceptable excipients, i.e. inactive substances such as diluents and other auxiliaries, and which can be employed when using compound (V) as a pharmaceutical or medicament in human medicine or veterinary medicine. One embodiment of the invention relates to a pharmaceutical composition which comprises at least one of polymorphs 1 ({drug5b}) and 2 ({drug5c}), or a mixture of polymorphs comprising at least one of polymorphs 1 ({drug5b}) and 2 ({drug5c}), and one or more pharmaceutically acceptable excipients. One embodiment of the invention relates to a pharmaceutical composition which comprises polymorph 1 ({drug5b}), or a mixture of polymorphs comprising at least polymorph 1 ({drug5b}), and one or more pharmaceutically acceptable excipients. One embodiment of the invention relates to a pharmaceutical composition which comprises a hydrate selected from hydrates 1 ({drug5e}) and 2 ({drug5f}), or a mixture of polymorphs and/or hydrates comprising at least one of hydrates 1 ({drug5e}) and 2 ({drug5f}), and one or more pharmaceutically acceptable excipients. If desired, the pharmaceutical compositions according to the invention can also contain one or more other suitable pharmacologically active compounds. The pharmaceuticals, medicaments and pharmaceutical compositions according to the invention can generally be administered by means of oral, inhalative, rectal or transdermal administration or by means of subcutaneous, intraarticular, intraperitoneal or intravenous injection, for example. One embodiment of the invention furthermore relates to a process for preparing a pharmaceutical composition which comprises bringing at least one polymorph or hydrate of 2-(2-methylamino-pyrimidin-4-yl)-1 H-indole-5-carboxylic acid [(S)-1-carbamoyl-2-(phenyl-pyrimidin-2-yl-amino)-ethyl]-amide according to the present invention, selected from polymorphs 1 ({drug5b}), 2 ({drug5c}) and 3 ({drug5d}) and hydrates 1 ({drug5e}) and 2 ({drug5f}), together with one or more pharmaceutically acceptable and physiologically tolerated excipients and, if desired, one or more other suitable pharmacologically active compounds into a suitable form for administration and dosage. Examples of pharmaceutical compositions are granules, powders, sugar-coated tablets, tablets, (micro)capsules, suppositories, syrups, juices, suspensions, emulsions, drops or injectable solutions, and also preparations with protracted active compound release, in the preparation of which customary auxiliary substances, such as carrier substances, disintegrants, binders, coating agents, swelling agents, glidants or lubricants, flavorings, sweeteners and solubilizers, are used. Frequently employed auxiliary substances which may be mentioned, are magnesium carbonate, titanium dioxide, lactose, mannitol and other sugars, talc, milk protein, gelatin, starch, cellulose and its derivatives, animal and vegetable oils, such as cod liver oil, sunflower oil, groundnut oil or sesame oil, polyethylene glycol and solvents, such as sterile water and monohydric or polyhydric alcohols, such as glycerol. The pharmaceutical compositions are preferably produced and administered in dosage units, with each unit containing, as the active constituent, a particular dose of at least one polymorph or hydrate according to the invention. In the case of solid dosage units, such as tablets, capsules, sugar-coated tablets or suppositories, this dose can be up to about 1000 mg, preferably from about 50 mg to about 300 mg, and, in the case of injection solutions in ampoule form, up to about 300 mg, preferably from about 1 mg to about 100 mg. The dosage, which is employed when treating a subject, in particular a mammal, specifically a human, with compound (V) in the form of one or more polymorphs and/or hydrates according to the invention and which is effective for obtaining the desired therapeutic or prophylactic result, varies and is determined by the physician in view of the particulars of the specific case. As is known in the art, the dosage depends on a variety of factors such as, for example, the severity of the condition being treated, general health, the route of administration, body weight, gender, diet, time and route of administration, the desired duration of treatment, rates of absorption and excretion, combination with other drugs, and others. Because of the pharmacological properties of compound (V) which are described in WO 2004/022553, US 7285560, WO 2004/022057 and US 7462638, for example, the polymorphs and hydrates according to the invention can be used for the prophylaxis and therapy of all those diseases whose course involves an increased activity of IκB kinase, for example, chronic diseases of the locomotory apparatus, such as inflammatory, immunologically or metabolism-mediated acute and chronic arthritis, arthropathies, rheumatoid arthritis, or degenerative joint diseases such as osteoarthroses, spondyloses, diabetes Type II, inflammatory bowel disease, loss of cartilage following joint trauma or a relatively long period of joint immobilization following meniscus or patella injuries or ligament ruptures, or diseases of the connective tissue, such as collagenoses and periodontal diseases, myalgias and disturbances of bone metabolism, or diseases which are due to overexpression of tumor necrosis factor alpha (TNFcc) or an increased concentration of TNFcc, such as cachexia, multiple sclerosis, craniocerebral trauma, Crohn's disease and intestinal ulcers, or diseases such as atherosclerosis, stenoses, ulceration, Alzheimer's diseases, muscle breakdown, cancer diseases (potentiation of treatment with cytotoxic agents), cardiac infarction, gout, sepsis, septic shock, endotoxic shock, viral infections such as flu, hepatitis, HIV infections, AIDS, or diseases caused by adenoviruses or herpes viruses, parasitic infections such as malaria or leprosy, fungal or yeast infections, meningitis, chronic inflammatory lung diseases such as chronic bronchitis or asthma, acute respiratory distress syndrome, acute synovitis, tuberculosis, psoriasis, diabetes, treatment of acute or chronic rejection reactions on the part of the organ recipient against the transplanted organ, chronic graft-versus-host diseases and inflammatory vascular diseases, and they can further be used for the treatment of pain including acute pains and chronic pains, for example pain associated with inflammatory processes or osteoarthritis. Examples of chronic pains which can be treated, are chronic musculoskeletal diseases, such as back pains, pains associated with menstrual bleeding, pains associated with osteoarthritis or rheumatoid arthritis, pains associated with intestinal inflammation, pains associated with cardiac muscle inflammation, pains associated with multiple sclerosis, pains associated with neuritis, pains associated with carcinomas and sarcomas, pains associated with AIDS, pains associated with chemotherapy, amputation pain, trigeminus neuralgia, headaches, such as migraine cephalalgia, or neuropathic pains, such as post-herpes zoster neuralgia. Examples of acute pains which can be treated, are pains following injuries, post-operative pains, pains in association with an acute attack of gout, or acute pains following jaw-bone surgery interventions. One embodiment of the present invention relates to a polymorph or a hydrate of 2-(2-methylamino-pyrimidin-4-yl)-1 H-indole-5-carboxylic acid [(S)-1-carbamoyl-2-(phenyl-pyrimidin-2-yl-amino)-ethyl]-amide according to the present invention, selected from polymorphs 1 ({drug5b}), 2 ({drug5c}) and 3 ({drug5d}) and hydrates 1 ({drug5e}) and 2 ({drug5f}), for use in the treatment of a disease whose course involves an increased activity of IκB kinase, including the use in the treatment of any one or more of the diseases mentioned herein, for example in the treatment of osteoarthritis or pain, as well as the use of such polymorph or hydrate for the manufacture of a medicament for the treatment of a disease whose course involves an increased activity of IκB kinase, including the treatment of any one or more of the diseases mentioned herein, for example the treatment of osteoarthritis or pain, as well as a method for the treatment of a disease whose course involves an increased activity of IκB kinase, including the treatment of any one or more of the diseases mentioned herein, for example the treatment of osteoarthritis or pain, which method comprises administering to a subject in need thereof an effective amount of a polymorph or a hydrate of 2-(2-methylamino-pyrimidin-4-yl)-1 H-indole-5-carboxylic acid [(S)-1-carbamoyl-2-(phenyl-pyrimidin-2-yl-amino)-ethyl]-amide according to the present invention. One embodiment of the present invention relates to such polymorph of 2-(2-methylamino-pyrimidin-4-yl)-1 H-indole-5-carboxylic acid [(S)-1-carbamoyl-2-(phenyl-pyrimidin-2-yl-amino)-ethyl]-amide for use in the treatment of a disease, as well as such use for the manufacture of a medicament, as well as such method for the treatment of a disease, wherein the polymorph is selected from polymorphs 1 ({drug5b}) and 2({drug5c}), or a mixture of polymorphs comprising at least one of polymorphs 1 ({drug5b}) and 2 ({drug5c}). One embodiment of the present invention relates to such polymorph of 2-(2-methylamino-pyrimidin-4-yl)-1 H-indole-5-carboxylic acid [(S)-1-carbamoyl-2-phenyl-pyrimidin-2-yl-amino)-ethyl]-amide for use in the treatment of a disease, as well as such use for the manufacture of a medicament, as well as such method for the treatment of a disease, wherein the polymorph is polymorph 1 ({drug5b}), or a mixture of polymorphs comprising at least polymorph 1 ({drug5b}). One embodiment of the present invention relates to such hydrate of 2-(2-methylamino-pyrimidin-4-yl)-1 H-indole-5-carboxylic acid [(S)-1-carbamoyl-2-(phenyl-pyrimidin-2-yl-amino)-ethyl]-amide for use in the treatment of a disease, as well as such use for the manufacture of a medicament, as well as such method for the treatment of a disease, wherein the hydrate is selected from hydrates 1 ({drug5e}) and 2 ({drug5f}), or a mixture of polymorphs and/or hydrates comprising at least one of hydrates 1 ({drug5e}) and 2 ({drug5f}). The present invention further relates to processes for the preparation of the polymorphs and hydrates of 2-(2-methylamino-pyrimidin-4-yl)-1 H-indole-5-carboxylic acid [(S)-1-carbamoyl-2-(phenyl-pyrimidin-2-yl-amino)-ethyl]-amide according to the invention, selected from polymorphs 1 ({drug5b}), 2 ({drug5c}) and 3 ({drug5d}) and hydrates 1 ({drug5e}) and 2 ({drug5f}). In general, the polymorphs and hydrates of the invention can be obtained by crystallizing or recrystallizing compound (V) under suitable conditions for the respective polymorph or hydrate, starting from a solution of compound (V) or from a suspension of compound (V) or from solid compound (V). A solution or a suspension of compound (V) for use in the preparation of a desired polymorph or hydrate may directly be obtained at the end of the chemical synthesis of compound (V), or it may be obtained by dissolving or suspending previously obtained compound (V) present in the form of another polymorph or hydrate or a mixture of polymorphs and/or hydrates, which may not be characterized with respect to its crystal properties. Such compound (V) may be termed "crude compound (V)". More specifically, the polymorphs and hydrates of the invention may be obtained by providing a solution or suspension of compound (V), for example by dissolving or suspending crude compound (V) in a suitable solvent or solvent mixture, maintaining, heating, cooling and/or concentrating the solution or suspension and/or adding one or more further solvents and/or adding seed crystals of the desired polymorph or hydrate, with or without agitation such as stirring, to form a precipitate of crystals of a desired polymorph or hydrate or to allow the formation of the desired polymorph or hydrate, and isolating the desired polymorph or hydrate. Seeding with a small amount of the desired polymorph or hydrate is a preferred procedure for promoting crystallization of the desired form. The preparation of the polymorphs and hydrates of compound (V) can be performed with conventional equipment and according to standard procedures. For example, concentrating a solution or suspension may be done by distilling off solvent partially or totally at atmospheric pressure or at reduced pressure. Isolating a polymorph or hydrate may be done by any conventional technique such as filtration or vacuum filtration or centrifugation. Isolating may also include washing of the initially separated solid and/or drying, for example at room temperature and atmospheric pressure or elevated temperature and/or reduced pressure. Compound (V), i.e. 2-(2-methylamino-pyrimidin-4-yl)-1 H-indole-5-carboxylic acid [(S)-1-carbamoyl-2-(phenyl-pyrimidin-2-yl-amino)-ethyl]-amide, can be prepared as described in WO 2004/022553 and US 7285560, for example, and detailed further below herein. In brief, for the synthesis of compound (V) 2-[bis-(tert-butoxycarbonyl)]amino-3-(phenyl-pyrimidin-2-yl-amino)propionic acid methyl ester, which is available from 2-[di-(tert-butoxycarbonyl)amino]acrylic acid methyl ester and 2-anilino-pyrimidine as described in WO 2004/022553 and US 7285560, for example, is deprotected by treatment with an acid such as hydrochloric acid, and the obtained racemic 2-amino-3-(phenyl-pyrimidin-2-yl-amino)propionic acid methyl ester separated into the enantiomers by preparative high pressure liquid chromatography on a chiral phase under standard conditions. The obtained (S)-2-amino-3-(phenyl-pyrimidin-2-yl-amino)propionic acid methyl ester is reacted with 2-(2-methylaminopyrimidin-4-yl)-1 H-indole-5-carboxylic acid, whose synthesis is described in WO 2004/022553 and US 7285560, for example, by means of a coupling agent such as N,N,N',N'-tetramethyl-O-(7-azabenzotriazol-1-yl)-uronium hexafluorophosphate (HATU) in the presence of a base like diisopropylethylamine to give (S)-2-{[2-(2-methylaminopyrimidin-4-yl)-1 H-indole-5-carbonyl]amino}-3-(phenyl-pyrimidin-2-yl-amino)propionic acid methyl ester, which in the last step is reacted with ammonia to give 2-(2-methylamino-pyrimidin-4-yl)-1 H-indole-5-carboxylic acid [(S)-1-carbamoyl-2-(phenyl-pyrimidin-2-yl-amino)-ethyl]-amide, which may also be named as (S)-2-{[2-(2-methylaminopyrimidin-4-yl)-1 H-indole-5-carbonyl]amino}-3-(phenyl-pyrimidin-2-yl-amino)propionamide, for example. If the last step in the aforesaid synthesis of 2-(2-methylamino-pyrimidin-4-yl)-1 H-indole-5-carboxylic acid [(S)-1-carbamoyl-2-(phenyl-pyrimidin-2-yl-amino)-ethyl]-amide is performed by reacting (S)-2-{[2-(2-methylaminopyrimidin-4-yl)-1 H-indole-5-carbonyl]amino}-3-(phenyl-pyrimidin-2-yl-amino)propionic acid methyl ester with a less than saturated solution of ammonia in methanol, such as a 7N solution of ammonia in methanol, at room temperature, i.e. at about 20 to 25 °C, for a longer period of time such as three days, and the precipitated solid is directly isolated by filtration, polymorph 1 of 2-(2-methylamino-pyrimidin-4-yl)-1 H-indole-5-carboxylic acid [(S)-1-carbamoyl-2-(phenyl-pyrimidin-2-yl-amino)-ethyl]-amide is obtained. One embodiment of the present invention thus relates to a process for preparing polymorph 1 of 2-(2-methylamino-pyrimidin-4-yl)-1 H-indole-5-carboxylic acid [(S)-1-carbamoyl-2-(phenyl-pyrimidin-2-yl-amino)-ethyl]-amide, which comprises reacting (S)-2-{[2-(2-methylaminopyrimidin-4-yl)-1 H-indole-5-carbonyl]amino}-3-(phenyl-pyrimidin-2-yl-amino)propionic acid methyl ester with a solution of ammonia in methanol, for example a 7N solution, at room temperature for a sufficient period of time, for example about three days, and isolating the precipitated solid. Instead of by reacting (S)-2-amino-3-(phenyl-pyrimidin-2-yl-amino)propionic acid methyl ester with 2-(2-methylaminopyrimidin-4-yl)-1 H-indole-5-carboxylic acid and subsequently converting the ester group into the amide group by reaction with ammonia, 2-(2-methylamino-pyrimidin-4-yl)-1 H-indole-5-carboxylic acid [(S)-1-carbamoyl-2-(phenyl-pyrimidin-2-yl-amino)-ethyl]-amide can also be prepared according to other synthetic procedures, for example by reacting 2-(2-methylamino-pyrimidin-4-yl)-1 H-indole-5-carboxylic acid, or a salt thereof which renders the addition of a base unnecessary, such as the sodium salt of 2-(2-methylamino-pyrimidin-4-yl)-1 H-indole-5-carboxylic acid whose synthesis is described in WO 2006/128585 and US 2008/0214813, for example, with (S)-2-amino-3-(phenyl-pyrimidin-2-yl-amino)-propionamide, whose synthesis is described in PCT/EP2011/063504, for example, in the presence of a coupling agent, such as 1-(3-dimethylaminopropyl)-3-ethylcarbodiimide hydrochloride, optionally in the presence of a base, as is detailed further below herein. If the aforesaid synthesis of 2-(2-methylamino-pyrimidin-4-yl)-1 H-indole-5-carboxylic acid [(S)-1-carbamoyl-2-(phenyl-pyrimidin-2-yl-amino)-ethyl]-amide from (S)-2-amino-3-(phenyl-pyrimidin-2-yl-amino)-propionamide is performed in a mixture of tetrahydrofuran and N-methylpyrrolidin-2-one as solvent at room temperature, i.e. at about 20 to 25 °C, and for work-up the reaction mixture is diluted with water and the precipitated solid is directly isolated by filtration, hydrate 1 of 2-(2-methylamino-pyrimidin-4-yl)-1 H-indole-5-carboxylic acid [(S)-1-carbamoyl-2-(phenyl-pyrimidin-2-yl-amino)-ethyl]-amide is obtained. One embodiment of the present invention thus relates to a process for preparing hydrate 1 of 2-(2-methylamino-pyrimidin-4-yl)-1 H-indole-5-carboxylic acid [(S)-1-carbamoyl-2-(phenyl-pyrimidin-2-yl-amino)-ethyl]-amide, which comprises reacting 2-(2-methylamino-pyrimidin-4-yl)-1 H-indole-5-carboxylic acid or a salt thereof with (S)-2-amino-3-(phenyl-pyrimidin-2-yl-amino)-propionamide in the presence of a coupling agent, for example 1-(3-dimethylaminopropyl)-3-ethylcarbodiimide hydrochloride, optionally in the presence of a base, in a mixture of tetrahydrofuran and N-methylpyrrolidin-2-one at room temperature, diluting the reaction mixture with water and isolating the precipitated solid. Hydrate 1 ({drug5e}) of 2-(2-methylamino-pyrimidin-4-yl)-1 H-indole-5-carboxylic acid [(S)-1-carbamoyl-2-(phenyl-pyrimidin-2-yl-amino)-ethyl]-amide can be converted in one or more steps into other crystalline forms of 2-(2-methylamino-pyrimidin-4-yl)-1 H-indole-5-carboxylic acid [(S)-1-carbamoyl-2-(phenyl-pyrimidin-2-yl-amino)-ethyl]-amide, such as polymorphs 1 ({drug5b}), 2 ({drug5c}) and 3 ({drug5d}) and hydrate 2 ({drug5f}). For example, by heating hydrate 1 ({drug5e}) in a suitable solvent, optionally seeding with the desired crystalline form and cooling the mixture, polymorphs 1 ({drug5b}) and 2 ({drug5c}) can be obtained. For the conversion into polymorph 1 ({drug5b}), for example, hydrate 1 ({drug5e}) can be heated in acetone to a temperature of about 35 °C to about reflux temperature, for example to about 40 °C, the mixture seeded with a small amount of polymorph 1 ({drug5b}), kept at about 40 °C for some time, for example about 5 to about 10 hours, such as about 6 hours, cooled to room temperature and the solid isolated. One embodiment of the present invention thus relates to a process for preparing polymorph 1 ({drug5b}) of 2-(2-methylamino-pyrimidin-4-yl)-1 H-indole-5-carboxylic acid [(S)-1-carbamoyl-2-(phenyl-pyrimidin-2-yl-amino)-ethyl]-amide, which comprises heating hydrate 1 ({drug5e}) of 2-(2-methylamino-pyrimidin-4-yl)-1 H-indole-5-carboxylic acid [(S)-1-carbamoyl-2-(phenyl-pyrimidin-2-yl-amino)-ethyl]-amide in acetone in the presence of seed crystals of polymorph 1 ({drug5b}), for example to about 40 °C, for a time period of about 5 to about 10 hours, for example for about 6 hours, cooling the mixture to room temperature and isolating the precipitated solid. For the conversion into polymorph 2 ({drug5c}), hydrate 1 ({drug5e}) can be heated in a mixture of diisopropyl ether and methanol to a temperature of about 60 to about 70 °C, for example to about reflux temperature or to about 68 °C, kept at this temperature for some time, for example about 18 to about 30 hours, such as about 24 hours, cooled to room temperature and the solid isolated. One embodiment of the present invention thus relates to a process for preparing polymorph 2 ({drug5c}) of 2-(2-methylamino-pyrimidin-4-yl)-1 H-indole-5-carboxylic acid [(S)-1-carbamoyl-2-(phenyl-pyrimidin-2-yl-amino)-ethyl]-amide, which comprises heating hydrate 1 ({drug5e}) of 2-(2-methylamino-pyrimidin-4-yl)-1 H-indole-5-carboxylic acid [(S)-1-carbamoyl-2-(phenyl-pyrimidin-2-yl-amino)-ethyl]-amide in a mixture of diisopropyl ether and methanol, for example to about 60 to about 70 °C, for a time period of about 18 to about 30 hours, for example for about 24 hours, cooling the mixture to about 20 to 25 °C and isolating the precipitated solid. In one embodiment of this latter process for preparing polymorph 2 ({drug5c}) of 2-(2-methylamino-pyrimidin-4-yl)-1 H-indole-5-carboxylic acid [(S)-1-carbamoyl-2-(phenyl-pyrimidin-2-yl-amino)-ethyl]-amide, the mixture of diisopropyl ether and methanol contains from about 3 to about 10 %, in another embodiment from about 4 to about 8 %, in another embodiment about 6 %, of methanol by weight. For the conversion into polymorph 3 ({drug5d}), hydrate 1 ({drug5e}) can be dried in substance at elevated temperature and reduced pressure, for example at about 50 to about 70 °C, such as at about 60 °C, and about 20 to about 50 mbar, for example about 30 mbar, for example in a standard drying cabinet, for a time period sufficient for the transformation of the dihydrate into the anhydrous form of polymorph 3 ({drug5d}), for example for about 18 to about 30 hours, such as about 24 hours. One embodiment of the present invention thus relates to a process for preparing polymorph 3 ({drug5d}) of 2-(2-methylamino-pyrimidin-4-yl)-1 H-indole-5-carboxylic acid [(S)-1-carbamoyl-2-(phenyl-pyrimidin-2-yl-amino)-ethyl]-amide, which comprises drying hydrate 1 ({drug5e}) of 2-(2-methylamino-pyrimidin-4-yl)-1 H-indole-5-carboxylic acid [(S)-1-carbamoyl-2-(phenyl-pyrimidin-2-yl-amino)-ethyl]-amide at a temperature of about 50 to about 70 °C and a pressure from about 20 to about 50 mbar for a time period of about 18 to about 30 hours. Polymorph 3 ({drug5d}) can be re-converted into hydrate 1 ({drug5e}) by exposing it to an atmosphere which contains water vapor, for example leaving it uncovered in the air at room temperature, i.e. at about 20 to 25 °C, and atmospheric pressure for a time period sufficient for the transformation of the anhydrous form into the dihydrate, for example for about 15 to about 25 hours, such as about 17 hours. One embodiment of the present invention thus relates to a process for preparing hydrate 1 of 2-(2-methylamino-pyrimidin-4-yl)-1 H-indole-5-carboxylic acid [(S)-1-carbamoyl-2-(phenyl-pyrinnidin-2-yl-annino)-ethyl]-annide, which comprises allowing polymorph 3 of 2-(2-methylamino-pyrimidin-4-yl)-1 H-indole-5-carboxylic acid [(S)-1-carbamoyl-2-(phenyl-pyrimidin-2-yl-amino)-ethyl]-amide to take up water from the surrounding atmosphere at a temperature of about 20 to about 25 °C for a time period of about 15 to about 25 hours. Polymorph 2 ({drug5c}) of 2-(2-methylamino-pyrimidin-4-yl)-1 H-indole-5-carboxylic acid [(S)-1-carbamoyl-2-(phenyl-pyrimidin-2-yl-amino)-ethyl]-amide, which can be obtained from hydrate 1 ({drug5e}) as outlined above, for example, can be converted by heating in a suitable solvent, optionally seeding with the desired crystalline form, and optionally cooling the mixture, into polymorph 1 ({drug5b}) and hydrate 2 ({drug5f}) of 2-(2-methylamino-pyrimidin-4-yl)-1 H-indole-5-carboxylic acid [(S)-1-carbamoyl-2-(phenyl-pyrimidin-2-yl-amino)-ethyl]-amide. For example, for the conversion into polymorph 1 ({drug5b}), polymorph 2 ({drug5c}) can be heated in a mixture of acetone and water to a temperature of about 50 to about 60 °C, for example to about reflux temperature or to about 60 °C, kept at this temperature for some time, for example about 1 to about 5 hours, such as about 2 to about 3 hours, cooled to room temperature, and the solid isolated. One embodiment of the present invention thus relates to a process for preparing polymorph 1 ({drug5b}) of 2-(2-methylamino-pyrimidin-4-yl)-1 H-indole-5-carboxylic acid [(S)-1-carbamoyl-2-(phenyl-pyrimidin-2-yl-amino)-ethyl]-amide, which comprises heating polymorph 2 ({drug5c}) of 2-(2-methylamino-pyrimidin-4-yl)-1 H-indole-5-carboxylic acid [(S)-1-carbamoyl-2-(phenyl-pyrimidin-2-yl-amino)-ethyl]-amide in a mixture of acetone and water, for example to about 50 to about 60 °C, for a time period of about 1 to about 5 hours, for example for about 2 to about 3 hours, cooling the mixture to about 20 to 25 °C and isolating the precipitated solid. In one embodiment of this latter process for preparing polymorph 1 ({drug5b}) of 2-(2-methylamino-pyrimidin-4-yl)-1 H-indole-5-carboxylic acid [(S)-1-carbamoyl-2-(phenyl-pyrimidin-2-yl-amino)-ethyl]-amide, the mixture of acetone and water contains from about 3 to about 6 parts of acetone per part of water, in another embodiment from about 3 to about 5 parts of acetone per part of water, in another embodiment about 4 parts of acetone per part of water, in each case parts by volume. For the conversion into hydrate 2 ({drug5f}), polymorph 2 ({drug5c}) can be heated in a mixture of acetone and water to a temperature of about 30 to about 40 °C, for example to about 40 °C, kept at this temperature for some time, for example about 2 to about 10 hours, such as about 4 hours, cooled to room temperature and the solid isolated. One embodiment of the present invention thus relates to a process for preparing hydrate 2 ({drug5f}) of 2-(2-methylamino-pyrimidin-4-yl)-1 H-indole-5-carboxylic acid [(S)-1-carbamoyl-2-(phenyl-pyrimidin-2-yl-amino)-ethyl]-amide, which comprises heating polymorph 2 ({drug5c}) of 2-(2-methylamino-pyrimidin-4-yl)-1 H-indole-5-carboxylic acid [(S)-1-carbamoyl-2-(phenyl-pyrimidin-2-yl-amino)-ethyl]-amide in a mixture of acetone and water, for example to about 40 °C, for a time period of about 2 to about 10 hours, for example for about 4 hours, cooling the mixture to about 20 to 25 °C, and isolating the precipitated solid. In one embodiment of this process for preparing hydrate 2 ({drug5f}) of 2-(2-methylamino-pyrimidin-4-yl)-1 H-indole-5-carboxylic acid [(S)-1-carbamoyl-2-(phenyl-pyrimidin-2-yl-amino)-ethyl]-amide, the mixture of acetone and water contains from about 1 to about 3 parts of acetone per part of water, in another embodiment about 2 parts of acetone per part of water, in each case parts by volume. Figure 1 of WO2013/083553. X-ray powder diffraction pattern of polymorph 1 ({drug5b}) of 2-(2-methylamino-pyrimidin-4-yl)-1 H-indole-5-carboxylic acid [(S)-1-carbamoyl-2-(phenyl-pyrimidin-2-yl-amino)-ethyl]-amide, measured in reflection mode with CuKal radiation at room temperature; x-axis: diffraction angle 2Theta (degree); y-axis: intensity (counts in arbitrary unit). Figure 2 of WO2013/083553. X-ray powder diffraction pattern of polymorph 2 ({drug5c}) of 2-(2-methylamino-pyrimidin-4-yl)-1 H-indole-5-carboxylic acid [(S)-1-carbamoyl-2-(phenyl-pyrimidin-2-yl-amino)-ethyl]-amide, measured in reflection mode with CuKal radiation at room temperature; x-axis: diffraction angle 2Theta (degree); y-axis: intensity (counts in arbitrary unit). Figure 3 of WO2013/083553. X-ray powder diffraction pattern of polymorph 3 ({drug5d}) of 2-(2-methylamino-pyrimidin-4-yl)-1 H-indole-5-carboxylic acid ((S)-1-carbamoyl-2-(phenyl-pyrimidin-2-yl-amino)-ethyl)-amide, measured in reflection mode with CuKal radiation at room temperature; x-axis: diffraction angle 2Theta (degree); y-axis: intensity (counts in arbitrary unit). Figure 4 of WO2013/083553. X-ray powder diffraction pattern of hydrate 1 ({drug5e}) of 2-(2-methylamino-pyrimidin-4-yl)-1 H-indole-5-carboxylic acid [(S)-1-carbamoyl-2-(phenyl-pyrimidin-2-yl-amino)-ethyl]-amide, measured in reflection mode with CuKal radiation at room temperature; x-axis: diffraction angle 2Theta (degree); y-axis: intensity (counts in arbitrary unit). Figure 5 of WO2013/083553. X-ray powder diffraction pattern of hydrate 2 ({drug5f}) of 2-(2-methylamino-pyrimidin-4-yl)-1 H-indole-5-carboxylic acid [(S)-1-carbamoyl-2-(phenyl-pyrimidin-2-yl-amino)-ethyl]-amide, measured in reflection mode with CuKal radiation at room temperature; x-axis: diffraction angle 2Theta (degree); y-axis: intensity (counts in arbitrary unit). In the following, the formation and characterization of the polymorphs and hydrates of the present invention is described in detail by way of example.

Example 1: Formation of polymorph 1 ({drug5b}) of 2-(2-methylamino-pyrimidin-4-yl)-1 H-indole-5-carboxylic acid [(S)-1-carbamoyl-2-(phenyl-pyrimidin-2-yl-amino)-ethyl]-amide: (a) 2-Amino-3-(phenyl-pyrimidin-2-yl-amino)propionic acid methyl ester: 470 ml of 5N hydrochloric acid were added dropwise to a solution of 100 g (212 mmol) of 2-[bis-(tert-butoxycarbonyl)]amino-3-(phenyl-pyrimidin-2-yl-amino)propionic acid methyl ester in 1000 ml of isopropyl acetate at 15 °C, and the mixture was stirred for 2 h. Then the phases were separated and the pH of the aqueous phase was adjusted to 8.5 by addition of an aqueous solution of ammonia under cooling in an ice bath. The aqueous phase was extracted with ethyl acetate, and the extract washed with a saturated solution of sodium chloride, dried over sodium sulfate and evaporated in vacuo to give 52.8 g (92 %) of 2-amino-3-(phenyl-pyrimidin-2-yl-amino)propionic acid methyl ester as a yellow oil. MS (ESI): m/z = 273 (M++1, 100 %); 1H-NMR (500 MHz, DMSO-d6): δ = 8.31 (d (doublet), J = 4.8 Hz, 2 H), 7.40 (m (multiplet), 2 H), 7.33-7.20 (m, 3 H), 6.70 (t (triplet), J = 4.8 Hz, 1 H), 4.18 (dd (double doublet), J = 9.6 und 14.4 Hz, 1 H), 4.05 (dd, J = 9.5 and 14.3 Hz, 1 H), 3.65 (t, J = 9.6 Hz, 1 H), 3.43 (s (singlet), 3 H), 1.88 ppm (bs (broad singlet), 2 H); HPLC (columm: Chiralcel OJ-H, 250 x 4.6 mm; eluent: n-heptane + 0.1 % diethylamine: ethanol + 0.1 % diethylamine (85:15); flow rate: 1 ml/min; detection wavelength: 285 nm): retention time = 14.6 min (S enantiomer) and 16.4 min (R enantiomer). (b) (R)-2-Amino-3-(phenyl-pyrimidin-2-yl-amino)propionic acid methyl ester and (S)-2-amino-3-(phenyl-pyrimidin-2-yl-amino)propionic acid methyl ester: 10 g of 2-amino-3-(phenyl-pyrimidin-2-yl-amino)propionic acid methyl ester were separated into the enantiomers by preparative HPLC on a chiral phase (column: Chiralpak AS-H, 250 x 30 mm; eluent: n-heptane: ethanol (5:1); flow rate: 30 ml/min; detection wavelength: 257 nm; retention time of the S enantiomer: 12.5 min, retention time of the R enantiomer: 18.1 min). 2.6 g (52 %) of (R)-2-amino-3-(phenyl-pyrimidin-2-yl-amino)propionic acid methyl ester and 2.6 g (52 %) of (S)-2-amino-3-(phenyl-pyrimidin-2-yl-amino)propionic acid methyl ester were obtained. (c) (S)-2-{[2-(2-Methylaminopyrimidin-4-yl)-1 H-indole-5-carbonyl]amino}-3-(phenyl-pyrimidin-2-yl-amino)propionic acid methyl ester: 4.01 g (10.6 mmol) of N,N,N',N'-tetramethyl-O-(7-azabenzotriazol-1-yl)-uronium hexafluorophosphate and 6.82 g (52.8 mmol) of diisopropylethylamine were added to a solution of 2.36 g (8.80 mmol) of 2-(2-methylaminopyrimidin-4-yl)-1 H-indole-5-carboxylic acid in 80 ml of dimethylformamide under cooling with an ice bath, and the mixture was stirred at 0 °C for 1.5 h. Then a solution of 2.40 g (8.80 mmol) of (S)-2-amino-3-(phenyl-pyrimidin-2-yl-amino)propionic acid methyl ester (98.9% ee (enantiomeric excess)) in 22 ml of dimethylformamide was added. The mixture was stirred for 2 h, and then an aqueous solution of sodium hydrogencarbonate and isopropyl acetate were added. The organic phase was separated, washed with a saturated solution of sodium chloride and concentrated in vacuo. The solid that precipitated was filtered off with suction, dried in vacuo and purified by column chromatography (silica gel, dichloromethane: methanol (25:1)). 2.35 g (50%) of (S)-2-{[2-(2-methylaminopyrimidin-4-yl)-1 H-indole-5-carbonyl]amino}-3-(phenyl-pyrimidin-2-yl-amino)propionic acid methyl ester were obtained as a yellowish solid. MS (ESI): m/z = 522 (M+, 100 %); 1H-NMR (600 MHz, DMSO-d6): δ = 11.7 (s, 1 H), 8.78 (m, 1 H), 8.40 (d, J = 4.8 Hz, 2 H), 8.32 (bs, 1 H), 8.05 (s, 1 H), 7.60 (d, J = 8.5 Hz, 1 H), 7.52 (d, J = 8.5 Hz, 1 H), 7.40-7.14 (m, 7 H), 7.00 (bs, 1 H), 6.78 (t, J = 4.8 Hz, 1 H), 4.90 (m, 1 H), 4.68 (dd, J = 6.9 and 9.4 Hz, 1 H), 4.29 (dd, J = 7.1 and 9.3 Hz, 1 H), 3.54 (s, 3 H), 2.95 ppm (bs, 3 H); HPLC (column: Chiralcel OD-H, 250 x 4.6 mm; eluent: n-heptane: ethanol (60:40), flow rate: 1 ml/min, detection wavelength: 254 nm): retention time = 10.1 min (R enantiomer) and 15.0 min (S enantiomer); ee of the S enantiomer = 94.7 %. (d) 2-(2-Methylamino-pyrimidin-4-yl)-1 H-indole-5-carboxylic acid [(S)-1-carbamoyl-2-(phenyl-pyrimidin-2-yl-amino)-ethyl]-amide: A solution of 2.20 g (4.21 mmol) of (S)-2-{[2-(2-methylaminopyrimidin-4-yl)-1 H-indole-5-carbonyl]amino}-3-(phenyl-pyrimidin-2-yl-amino)propionic acid methyl ester (94.7 % ee) in 220 ml of a 7N solution of ammonia in methanol was stirred at room temperature for three days. The solid that precipitated was filtered off with suction and dried in vacuo. 1.75 g (82 %) of polymorph 1 of 2-(2-methylamino-pyrimidin-4-yl)-1 H-indole-5-carboxylic acid [(S)-1-carbamoyl-2-(phenyl-pyrimidin-2-yl-amino)-ethyl]-amide, identified by its X-ray powder diffractogram, were obtained as a colorless solid. MS (ESI): m/z = 507 (M+, 100 %); 1H-NMR (400 MHz, DMSO-d6): δ =11.7 (s, 1 H), 8.40 (d, J = 4.8 Hz, 2 H), 8.34 (m, 2 H), 7.92 (s, 1 H), 7.55 (d, J = 8.5 Hz, 1 H), 7.50 (d, J = 8.5 Hz, 1 H), 7.43-7.10 (m, 9 H), 7.00 (bs, 1 H), 6.77 (t, J = 4.8 Hz, 1 H), 4.80 (m, 1 H), 4.53 (dd, J = 10.0 and 14.4 Hz, 1 H), 4.25 (dd, J = 4.2 and 14.3 Hz, 1 H), 2.96 ppm (bs, 3 H); HPLC (column: Chiralcel OD-H, 250 x 4.6 mm; eluent: n-heptane: ethanol (50:50), flow rate: 1 ml/min, detection wavelength: 238 nm): retention time = 10.2 min (R enantiomer) and 14.5 min (S enantiomer); ee of the S enantiomer = 99.2 %.

Example 2: Formation of hydrate 1 ({drug5e}) of 2-(2-methylamino-pyrimidin-4-yl)-1 H-indole-5-carboxylic acid [(S)-1-carbamoyl-2-(phenyl-pyrimidin-2-yl-amino)-ethyl]-amide 40 ml of tetrahydrofuran and 10 ml of N-methylpyrrolidin-2-one were added to 5 g of (S)-2-amino-3-(phenyl-pyrimidin-2-yl-amino)-propionamide, 5.65 g of 2-(2-methylamino-pyrimidin-4-yl)-1 H-indole-5-carboxylic acid sodium salt and 7 g of 1-(3-dimethylaminopropyl)-3-ethylcarbodiimide hydrochloride at room temperature. The mixture was stirred at room temperature for 2 h. Then 500 ml of water were slowly added. From the resulting solution, a solid precipitated which was filtered off with suction and washed with 50 ml of water and dried at room temperature until constant weight was achieved, to give 7.46 g of hydrate 1 of 2-(2-methylamino-pyrimidin-4-yl)-1 H-indole-5-carboxylic acid [(S)-1-carbamoyl-2-(phenyl-pyrimidin-2-yl-amino)-ethyl]-amide identified by its X-ray powder diffractogram.

Example 3: Formation of hydrate 2 ({drug5f}) of 2-(2-methylamino-pyrimidin-4-yl)-1 H-indole-5-carboxylic acid [(S)-1-carbamoyl-2-(phenyl-pyrimidin-2-yl-amino)-ethyl]-amide 1.52 g of polymorph 2 ({drug5c}) of 2-(2-methylamino-pyrimidin-4-yl)-1 H-indole-5-carboxylic acid [(S)-1-carbamoyl-2-(phenyl-pyrimidin-2-yl-amino)-ethyl]-amide in 20 ml of a mixture of acetone and water (2:1, by volume) was heated to 40 °C and the suspension stirred at this temperature for 4 h. A sample of about 1 ml of the suspension was taken, the solid filtered off with suction and dried at room temperature until constant weight was achieved, to give hydrate 2 ({drug5f}) of 2-(2-methylamino-pyrimidin-4-yl)-1 H-indole-5-carboxylic acid [(S)-1-carbamoyl-2-(phenyl-pyrimidin-2-yl-amino)-ethyl]-amide identified by its X-ray powder diffractogram

Example 4: Formation of polymorph 3 ({drug5d}) of 2-(2-methylamino-pyrimidin-4-yl)-1 H-indole-5-carboxylic acid [(S)-1-carbamoyl-2-(phenyl-pyrimidin-2-yl-amino)-ethyl]-amide Hydrate 1 ({drug5e}) of 2-(2-methylamino-pyrimidin-4-yl)-1 H-indole-5-carboxylic acid [(S)-1-carbamoyl-2-(phenyl-pyrimidin-2-yl-amino)-ethyl]-amide was placed in a vacuum drying cabinet and dried at a pressure of 30 mbar and a temperature of 60 °C for 24 h to give polymorph 3 ({drug5d}) of 2-(2-methylamino-pyrimidin-4-yl)-1 H-indole-5-carboxylic acid [(S)-1-carbamoyl-2-(phenyl-pyrimidin-2-yl-amino)-ethyl]-amide identified by its X-ray powder diffractogram. The water content of the product determined by Karl Fischer titration was 0.09 %.

Example 5: Formation of polymorph 2 ({drug5c}) of 2-(2-methylamino-pyrimidin-4-yl)-1 H-indole-5-carboxylic acid [(S)-1-carbamoyl-2-(phenyl-pyrimidin-2-yl-amino)-ethyl]-amide 16.7 g of diisopropyl ether and 1.0 g of methanol were added to 0.5 g of hydrate 1 ({drug5e}) of 2-(2-methylamino-pyrimidin-4-yl)-1 H-indole-5-carboxylic acid [(S)-1-carbamoyl-2-(phenyl-pyrimidin-2-yl-amino)-ethyl]-amide in a reaction vessel, and the suspension placed in a shaking device (Heidolph Synthesis 1; adjusted to 500 rpm) and heated to reflux at an internal temperature of 68 °C. The mixture was kept at this temperature for 24 h, and allowed to cool to room temperature. The solid was filtered off with suction and air-dried at room temperature for 8 h to give polymorph 2 ({drug5c}) of 2-(2-methylamino-pyrimidin-4-yl)-1 H-indole-5-carboxylic acid [(S)-1-carbamoyl-2-(phenyl-pyrimidin-2-yl-amino)-ethyl]-amide identified by its X-ray powder diffractogram.

Example 6: Formation of hydrate 1 ({drug5e}) of 2-(2-methylamino-pyrimidin-4-yl)-1 H-indole-5-carboxylic acid [(S)-1-carbamoyl-2-(phenyl-pyrimidin-2-yl-amino)-ethyl]-amide Polymorph 3 ({drug5d}) of 2-(2-methylamino-pyrimidin-4-yl)-1 H-indole-5-carboxylic acid [(S)-1-carbamoyl-2-(phenyl-pyrimidin-2-yl-amino)-ethyl]-amide was kept in the air at room temperature for 17 h to give hydrate 1 ({drug5e}) of 2-(2-methylamino-pyrimidin-4-yl)-1 H-indole-5-carboxylic acid [(S)-1-carbamoyl-2-(phenyl-pyrimidin-2-yl-amino)-ethyl]-amide identified by its X-ray powder diffractogram.

Example 7: Formation of polymorph 1 ({drug5b}) of 2-(2-methylamino-pyrimidin-4-yl)-1 H-indole-5-carboxylic acid [(S)-1-carbamoyl-2-(phenyl-pyrimidin-2-yl-amino)-ethyl]-amide: 1.0 g of polymorph 2 ({drug5c}) of 2-(2-methylamino-pyrimidin-4-yl)-1 H-indole-5-carboxylic acid [(S)-1-carbamoyl-2-(phenyl-pyrimidin-2-yl-amino)-ethyl]-amide and 15 ml of a mixture of acetone and water (4:1, by volume) were heated with stirring to an internal temperature of 60 °C. The mixture was kept at this temperature for 2.5 h and then cooled to 24 °C within 1 h The solid was filtered off with suction, washed twice with 5 ml each of water and dried in vacuo at a pressure of 50 mbar and a temperature of 60 °C for 19 h, to give 0.89 g of polymorph 1 ({drug5b}) of 2-(2-methylamino-pyrimidin-4-yl)-1 H-indole-5-carboxylic acid [(S)-1-carbamoyl-2-(phenyl-pyrimidin-2-yl-amino)-ethyl]-amide identified by its X-ray powder diffractogram.

Example 8: Formation of polymorph 1 ({drug5b}) of 2-(2-methylamino-pyrimidin-4-yl)-1 H-indole-5-carboxylic acid [(S)-1-carbamoyl-2-(phenyl-pyrimidin-2-yl-amino)-ethyl]-amide 0.6 g of hydrate 1 ({drug5e}) of 2-(2-methylamino-pyrimidin-4-yl)-1 H-indole-5-carboxylic acid [(S)-1-carbamoyl-2-(phenyl-pyrimidin-2-yl-amino)-ethyl]-amide and 15 ml of acetone in a reaction vessel were placed in a shaking device (Heidolph Synthesis 1; adjusted to 500 rpm). The mixture was seeded with a small amount of polymorph 1 ({drug5b}) of 2-(2-methylamino-pyrimidin-4-yl)-1 H-indole-5-carboxylic acid [(S)-1-carbamoyl-2-(phenyl-pyrimidin-2-yl-amino)-ethyl]-amide, heated to an internal temperature of 40 °C, and kept at this temperature for 6 h. A sample of about 1 ml of the suspension was taken, the solid filtered off and dried at room temperature until constant weight was achieved to give polymorph 1 ({drug5b}) of 2-2-methylamino-pyrimidin-4-yl)-1 H-indole-5-carboxylic acid [(S)-1-carbamoyl-2-(phenyl-pyrimidin-2-yl-amino)-ethyl]-amide identified by its X-ray powder diffractogram.

Analytical Methods: X-ray powder diffraction (XRPD): X-ray powder diffraction measurements were performed with a Bruker AXS D8 Advance diffractometer in reflection mode using CuKal (CuK-alphal) radiation. Unless stated otherwise, X-ray powder diffraction was performed at room temperature. Samples were investigated in a flat preparation. The measured data were visualized and evaluated with the Software EVA 12.0. X-ray powder diffractograms of polymorphs 1 ({drug5b}), 2 ({drug5c}) and 3 ({drug5d}) and hydrates 1 ({drug5e}) and 2 ({drug5f}) of compound (V) are displayed in Figures 1 to 5 of WO2013/083553. The data of observed X-ray reflections are given below in the following form: angle 2Theta (20) in°(degree), followed in brackets by the relative intensity in % of the intensity of the strongest reflection whose intensity was set to 100 % (the intensities are measured in counts in arbitrary unit). The given data are rounded to multiples of 0.1° and 1 %, respectively. As indicated above, the given angles 2Theta may be understood as lying within an margin of ± 0.2°, and the relative intensities may vary to a greater extent depending on the sample and measurement conditions, and the given exact values of the relative intensities are not to be regarded as a prerequisite for the presence of a crystalline form. For a classification, reflections with a relative intensity of more than 60 % of the strongest reflection, for example, may be termed as strong reflections, and reflections with a relative intensity between 30 % and 60 % of the strongest reflection, for example, may be termed as medium strong reflections, and such medium strong reflections and/or strong reflections used to characterize the polymorphs and hydrates of compound (V). XRPD reflections of polymorph 1 ({drug5b}) (2Theta [°], relative intensity [%]): 5.6° (5%), 10.4° (21 %), 11.1°(8%), 11.4° (10%), 12.8° (13%), 13.2° (6%), 13.9° (11 %), 14.2° (13%), 14.9° (100%), 17.2° (8%), 17.5° (19%), 18.0° (19%), 19.4° (35%), 19.7° (67%), 20.0° (55%), 20.4° (10%), 21.0° (20%), 22.3° (96%), 23.3° (15%), 24.1°(7%), 24.6° (21 %), 25.0° (82%), 26.1° (6%), 27.0° (6%), 27.7° (16%), 28.9° (7%), 29.6° (9%), 30.2° (10%), 31.3° (7%), 31.9° (4%), 32.4° (6%), 33.1°(6%); XRPD reflections of polymorph 2 ({drug5c}) (2Theta [°], relative intensity [%]): 5.8° (27%), 6.7° (100%), 8.2° (7%), 9.3° (60%), 9.9° (31 %), 11.2° (65%), 13.5° (9%), 13.8° (8%), 14.3° (15%), 16.5° (25%), 17.0° (10%), 17.7° (21 %), 18.1°(28%), 18.8° (24%), 19.4° (89%), 20.2° (15%), 20.4° (21 %), 22.1°(41%), 24.2° (11 %), 24.6° (8%), 25.5° (16%), 27.9° (12%), 30.0° (10%), 30.6° (8%); XRPD reflections of polymorph 3 ({drug5d}) (2Theta [°], relative intensity [%]): 5.1°(17%), 5.6° (12%), 7.0° (16%), 7.5° (16%), 9.7° (14%), 11.2° (13%), 12.7° (19%), 13.4° (20%), 13.9° (30%), 15.2° (76%), 15.9° (59%), 16.6° (18%), 17.3° (36%), 19.2° (100%), 22.2° (83%), 23.2° (23%), 24.5° (33%), 25.3° (42%), 27.9° (23%), 28.7° (27%); XRPD reflections of hydrate 1 ({drug5e}) (2Theta [°], relative intensity [%]): 5.3° (33%), 9.2° (19%), 10.7° (9%), 11.1°(25%), 12.0° (8%), 13.5° (44%), 14.0° (17%), 16.1°(20%), 17.2° (5%), 17.9° (51 %), 18.5° (12%), 19.5° (69%), 21.0° (12%), 21.5° (100%), 22.4° (11 %), 23.5° (33%), 24.9° (37%), 25.8° (16%), 26.5° (20%), 27.2° (7%), 28.2° (26%), 28.6° (9%), 29.4° (12%), 30.2° (12%), 31.3° (4%), 32.3° (3%), 32.7° (4%), 33.3° (3%); XRPD reflections of hydrate 2 ({drug5f}) (2Theta [°], relative intensity [%]): 2.9° (100%), 5.3° (29%), 6.2° (9%), 8.3° (33%), 9.5° (13%), 11.0° (3%), 11.5° (28%), 12.8° (4%), 14.0° (11 %), 14.7° (4%), 16.1°(3%), 16.8° (4%), 17.1°(14%), 18.0° (11 %), 18.8° (4%), 19.7° (3%), 20.3° (6%), 21.3° (11 %), 21.9° (6%), 22.8° (14%), 23.5° (7%), 24.7° (5%), 25.5° (4%), 26.1°(9%), 27.3° (5%), 28.6° (5%), 29.6° (4%), 30.1°(3%), 31.4° (3%), 31.8° (5%), 34.9° (4%). Crystal structure determination: The crystal structure of hydrate 1 ({drug5e}) of compound (V) was determined by X-ray single crystal structure analysis with a crystal obtained by crystallization from a mixture of acetone and water (4:1) and sealed in a Lindemann-glass capillary. Single crystal X-ray diffraction data were collected on a Bruker/AXS three circle diffractometer, equipped with a SMART APEX area detector, a low temperature device (model LT2) and a copper micro focus generator (IpS), operated at 45 kV/650 mA, and focusing beam Montel multilayer optic with an image focus spot diameter of-250 µm. Data processing was done with the program SAINT+ Release 6.45. Hydrate 1 of compound (V) crystallizes in the orthorhombic space group P2i2i2i. The data of the unit cell are Z = 4, a = 8.18650(10) A, b = 9.97420(10) A, c = 32.8707(2) A, α = β = γ = 90.00°, cell volume = 2684.02(5) A3, molecular formula = C27H25N902■ 2 H2O, calculated density p = 1.345 Mgm"3 (at 20 °C). Differential scanning calorimetry (DSC): DSC measurements were performed with a METTLER DSC822e instrument. 40 µl Al-crucibles with sealed lid and hole were used and the measurements carried out in a nitrogen gas flow of 50 ml/min with a typical heating rate of 10 °C/min, unless stated otherwise. The measured data were evaluated with the software STARe V8.10. For polymorph 1 ({drug5b}) of compound (V), a DSC melting point with an onset temperature of 253 °C and a peak temperature of 257 °C was observed. In the DSC analysis of polymorph 2 ({drug5c}) of compound (V), an endothermic peak with an onset temperature of 222 °C and a peak temperature of 225 °C, followed by an endothermic peak with an onset temperature of 248 °C and a peak temperature of 251 °C was observed. The DSC behavior of hydrates 1 ({drug5e}) and 2 ({drug5f}) of compound (V) is described above. Thermogravimetry (TG): Thermogravimetric analyses were performed with a METTLER TGA851 e instrument. 100 µl Al-crucibles with sealed lid and hole were used and the measurements performed in a nitrogen gas flow of 50 ml/min. The measured data were evaluated with the software STARe V8.10. TG measurements with infra-red (IR) spectroscopic analysis of volatiles were performed with a METTLER TGA851 e instrument as described, which was coupled to a Thermo-Nicolet 380 FT-IR spectrometer equipped with a cell for gas phase measurements. The measured IR spectra were evaluated using the software OMNIC V.7.3. In the TG analyses of polymorph 1 and polymorph 2 of compound (V) until the melting point, insignificant weight losses of 0.2 % and 0.7 %, respectively, were observed. In the TG analysis of hydrate 1 ({drug5e}) of compound (V), a gradual weight loss of 6.4 % was observed between room temperature and 120 °C. FT-IR analysis of the evolved gas proved it to be water. The observed weight loss corresponds to about 1.9 mol of water per mol of 2-(2-methylamino-pyrimidin-4-yl)-1 H-indole-5-carboxylic acid [(S)-1-carbamoyl-2-(phenyl-pyrimidin-2-yl-amino)-ethyl]-amide. In the TG FT-IR analysis of hydrate 2 of compound (V), a weight loss of 17-18 % of water mainly in two steps was observed between room temperature and 140 °C, which corresponds to about 6 mol of water per mol of 2-(2-methylamino-pyrimidin-4-yl)-1 H-indole-5-carboxylic acid [(S)-1-carbamoyl-2-(phenyl-pyrimidin-2-yl-amino)-ethyl]-amide. Dynamic vapor sorption (DVS): For the determination of the hygroscopicity, moisture sorption/desorption isotherms were recorded on a Surface Measurement Systems DVS-1 instrument. Two cycles were run at 25 °C, in which the relative humidity was stepwise increased from 0 % to 95 % and subsequently decreased again to 0 %, and the weight of the sample measured. The data were evaluated with the software DVSWin V.2.15. In the DVS analysis of polymorph 1 ({drug5b}) and polymorph 2 ({drug5c}) of compound (V), in the sorption steps of the two cycles the following weight increases were observed.

| Relative humidity | Polymorph 1 | Polymorph 2 |
|---|---|---|
| 40 % | 0.5 % / 0.5 % | 0.2 % / 0.0 % |
| 60 % | 0.6 % / 0.6 % | 0.3 % / 0.1 % |
| 80 % | 0.9 % / 0.9 % | 0.4 % / 0.3 % |
| 95 % | 1.3% / 1.4 % | 0.8 % / 0.6 % |

Determination of solubility: For the determination of the solubility in a mixture of acetone and water (4:1, by volume), samples of 0.5 mg to 50 mg of the test substances were suspended in a defined amount of the solvent (800 mg to 1200 mg) in screw cap vials with a magnetic stirring bar, which were placed in a temperature-controlled multiple stirring device equipped for transmission measurement (Avantium Crystal 16). With stirring at 700 rpm, the vials were cooled from room temperature to 0 °C and then heated to 60 °C with a heating rate of 3 °C/h, kept at 60 °C for 2 h and cooled again to 0 °C with a cooling rate of 3 °C/h. By means of transmission measurement it was determined whether the sample was dissolved (100 % transmission), or crystals were present. For polymorph 1 ({drug5b}), polymorph 2 ({drug5c}), hydrate 1 ({drug5e}) and hydrate 2 ({drug5f}) of compound (V) the following solubilities in a mixture of acetone and water (4:1, by volume) were determined.

| Substance | Temperature | Solubility [g in 100 g of solvent] |
|---|---|---|
| Polymorph 1 | 36.9 °C | 0.43 |
| Polymorph 1 | 44.7 °C | 0.50 |
| Polymorph 1 | 60.0 °C | 0.85 |
| Polymorph 2 | 46.0 °C | 1.02 |
| Polymorph 2 | 57.5 °C | 2.48 |
| Hydrate 1 | 38.6 °C | 2.11 |
| Hydrate 1 | 49.7 °C | 3.39 |
| Hydrate 1 | 57.4 °C | 5.04 |
| Hydrate 2 | 36.8 °C | 0.50 |
| Hydrate 2 | 46.1 °C | 1.02 |
| Hydrate 2 | 55.9 °C | 2.61 |

Maturation experiments: By maturation experiments (slurry conversion) at 20 °C the relative stability of polymorphs 1 ({drug5b}) and 2 ({drug5c}) and hydrate 1 ({drug5e}) was investigated. A polymorph mixture of 81 mg (Mixture of Polymorph 1 ({drug5b}) and Polymorph 2 ({drug5c})), 83 mg of Polymorph 1 ({drug5b}) and 60 mg of hydrate 1 ({drug5e}) was prepared. The mixture was subjected to XRPD analysis to confirm the presence of all three polymorphs and then distributed into five capillaries. These samples were kept in suspension at 20°C for five days. After X-ray powder diffraction measurement of the suspensions these were dried over night at 40°C and <100mbar and again subjected to X-ray diffraction. Maturation experiments (a) to (e) were performed under the specified conditions, starting with the polymorph mixture described above. (a) The polymorph mixture was suspended in water. After stirring the suspension for five days at 20 °C, the suspension was analyzed via XRPD and a mixture of polymorph 1 ({drug5b}) and hydrate 1 ({drug5e}) was proven. After drying over night at 40°C and <100 mbar the obtained solid was analyzed via XRPD, again, and only polymorph 1 ({drug5b}) was found. (b) The polymorph mixture was suspended in water/methanol 1:1 (vol.: vol.). After stirring the suspension for five days at 20 °C, the suspension was analyzed via XRPD and only polymorph 1 ({drug5b}) was proven. After drying over night at 40°C and <100 mbar the obtained solid was analyzed via XRPD, again, and only polymorph 1 ({drug5b}) was found. (c) The polymorph mixture was suspended in 2-propanol. After stirring the suspension for five days at 20 °C, the suspension was analyzed via XRPD and only polymorph 1 ({drug5b}) was proven. After drying over night at 40°C and <100 mbar the obtained solid was analyzed via XRPD, again, and only polymorph 1 ({drug5b}) was found. (d) The polymorph mixture was suspended in acetone. After stirring the suspension for five days at 20 °C, the suspension was analyzed via XRPD and only polymorph 1 ({drug5b}) was proven. After drying over night at 40°C and <100 mbar the obtained solid was analyzed via XRPD, again, and only polymorph 1 ({drug5b}) was found. (e) The polymorph mixture was suspended in ethylacetate. After stirring the suspension for five days at 20 °C, the suspension was analyzed via XRPD and only polymorph 1 ({drug5b}) was proven. After drying over night at 40°C and <100 mbar the obtained solid was analyzed via XRPD, again, and only polymorph 1 ({drug5b}) was found. In all maturation experiments (a) to (e) the solid completely transformed to polymorph 1 ({drug5b}) after drying. The performed maturation experiments prove that polymorph 1 ({drug5b}) is thermodynamically most stable among polymorphs 1 ({drug5b}), 2 ({drug5c}) and hydrate 1 ({drug5e}).

### SPECIFIC INORMATION CONCERNING ASPECT (VI) OF THE INVENTION

Aspect (vi) of the invention relates to an administration unit comprising crystal form I of a compound according tp formula (VI)

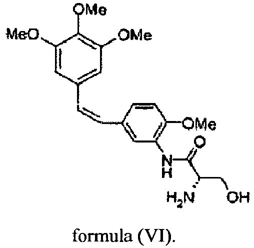

The invention relates to a solid form of formula (VI), namely to crystal form I of formula (VI), which is useful for treating prevention and/or treatment, of cancers and/or tumors. Crystal form I of formula (VI) is described in WO2013/084150, which is incorporated by reference. Formula (VI) is a compound named (2S)-2-ammo-3-hydroxy-N-[2-methoxy-2-[(1Z)-2-(3,4,5-trimethoxyphenyl)ethenyl] phenyl] propanamide or (Z)-N-[2-memoxy-5-[2-(3,4,5 rimethoxyphenyl)vinyl]phenyl]--L-serinamide, a well known combretastatin derivative known under the INN (International Non-proprietary Name) ombrabulin, which has the following structure of formula (VI):

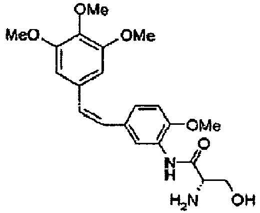

As used herein, crystal form I of formula (VI) is a crystalline form of formula (VI) characterized and described in WO2013/084150. Crystal form I of formula (VI) can as decribed in WO2013/084150 be characterized / obtained by: The present invention relates to a novel crystal form, hereinafter referred to as crystal Form Ï, of (2S)-2-amino-3-hydroxy-N-[2-memoxy-2-[(1Z)-2-(3,4,5-trimethoxyphenyl)ethenyl] phenyl]propanamide. The invention also relates to processes for the preparation of said crystal Form I. pharmaceutical compositions and medicament comprising it, and its therapeutic use more particularly in the prevention and/or treatment, of cancers and/or tumors. Cancer is a disease of humans and animals which is for the most part fatal and which is caused by the uncontrolled growth of endogenous cells. Cancer is the term for the formation of malignant tumors (malignomas) and of neoplasm (tumors or carcinomas) or for the malignant degeneration and disturbed maturation of white blood cells (leukemia, blood cancer). For obvious reasons, there is a permanent need to identify new drugs for preventing and/or treating cancers and/or to increase the efficacy of existing anti-cancer drugs. The present invention relates more particularly to the anti-cancer compound named (2S)-2-ammo-3-hydroxy-N-[2-methoxy-2-[(1Z)-2-(3,4,5-trimethoxyphenyl)ethenyl] phenyl] propanamide or (Z)-N-[2-memoxy-5-[2-(3,4,5 rimethoxyphenyl)vinyl]phenyl]--L-serinamide, a well known combretastatin derivative known under the INN (International Non-proprietary Name) ombrabulin, which has the following structure of formula (VI):

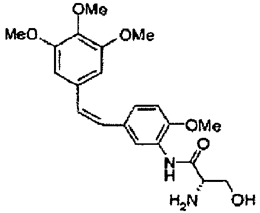

Said compound is particularly known as a vascular disrupting agent (VDA) of tumors. Some stilbene compounds: 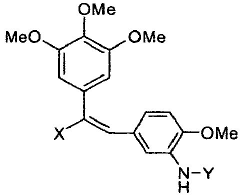 wherein X represents a hydrogen atom or a nitrile group and Y is an amino acid acyl group, as well as a process for their preparation have already been disclosed in EP 733 085, by Monk et al. ("Design, Synthesis, and biological evaluation of combretastatin nitrogen-containing derivatives as inhibitors of tubulin assembly and vascular disrupting agents", Bioorganic & Medicinal Chemistry 1.4 (2006) 3231-3244), in the patent US 6,759,555 and in the patent applications WO2011/067538 and WO2009/118474. Further, US 6,930,205 and US 5,731,353 disclose a process for preparing aminostilbene derivatives of formula (VΓ): 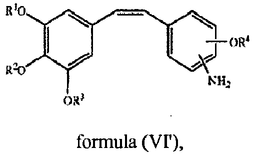 wherein R1, R2, R3 and R4 each independently represents a C1-3 alkyl group. US 6,784,315 describes a stilbene derivative type IV crystal form of formula (VI) as above, however in its hydrochloride form,,as well as a process for its preparation. Finally, (Z)-N-[2-methoxy-5-[2-(3,4,5-trimethoxyphenyl)vinyl]phenyl]-L-serinamide, also known as AVE8062 or ombrabulin, is described in EP 731 085 and can be prepared according to the process disclosed in WO 03/084919. However, none of the prior art documents describes crystallization step of ombrabulin in the form of its free base. US 5,674,906 merely describes a process which comprises a step of purification through silica-gel column chromatography and Monk et al. disclose a process which comprises a step of purification by thin layer chromatography (TLC). Importantly, neither US 5,674,906, nor Monk et al. describe in which physical form ombrabulin base may obtained, let alone whether a crystalline form may exist. Hence, the present invention concerns the obtention of a new crystal form of ombrabulin, as its free base form. Indeed, it is known that the identification of new crystal forms of active ingredient useful for preventing and/or treating cancers and /or tumors may be particularly interesting. It is also known that the ability of a substance to exist in mo e tiiitn one crystal form is defined as crystal polymorphism and its different crystal forms are called polymorphs. In general, crystal polymorphism is due to the ability of a compound to change its molecular conformation or to form different inter-and or intra-molecular interactions, particularly hydrogen bonds, which is reflected in different atom aiTangements in the crystal lattice of different polymorphs. Thus, polymorphs of a compound can differ notably from each other by different energies in their crystal lattices and, therefore, generally have specific physical properties in the solid state such as crystal morphology, density, melting point, colour, chemical and physical stability, hygroscopy, solubility, dissolution rate, granular properties.... In other words, polymorphic forms of the same compound can exhibit different behaviors in terms of formulation, therapeutic activity and chemical and physical stability. Unexpectedly, the inventors have discovered that the compound of formula (VI), as its free base form, can exist in a crystal form which is hereinafter referred to as crystal Form I. Thus, the present invention provides a novel crystal form of (2S)-2-amino-3-hydroxy-N-[2-methoxy∼2-[(1 Z)-2-(3,4,5-trimethoxyphenyl)-ethenyl]phenyl]propanamide of formula (VI)

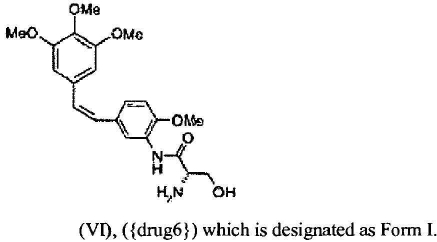

More particularly, said crystal Form I of compound of formula (VI) ({drug6a}) has at least one of the following characteristics:
(a) an X-ray (λCu) powder diffraction pattern with peaks at about 3.6, 7.1, 10.5, 11.2, 12.2 and 17.9 ±0.2 degrees 2-theta,
(b) a melting point (onset) around 104°C.

Another aspects of the present invention are processes for the preparation of said crystal Form I of (2S)-2-amino-3-hydroxy-N-[2-methoxy-2-[(1Z)-2-(3,4,5-trimethoxyphenyl)ethenyl]phenyl] propanamide. Thus, the present invention is directed to a particular process for preparing crystal Form I of (2S)-2-amino-3-hydroxy-N-[2-methoxy-2-[(1Z)-2-(3,4,5-trimethoxyphenyl)ethenyl]phenyl]propanamide comprising at least the step a) consisting to put in contact the hydrochloride salt of (2S)-2-amino-3-hydroxy-N-[2-methoxy-2-[(1Z)-2-(3,4,5-trimethoxyphenyl)ethenyl]phenyl]propanamide of formula (VI"):

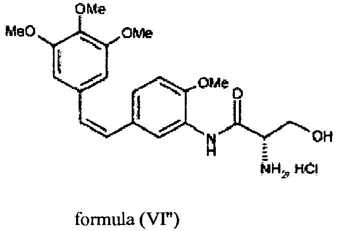

with a solvent like isopropyl acetate and in the presence of a base, in conditions appropriate to promote the crystallization of said (2S)-2-amino-3-hydroxy-N-[2-methoxy-2-[(1Z)-2-(3.4,5-trimethoxyphenyl)ethenyl]phenyl] propanamide crystal Form 1. Said compound of formula (VI") can be prepared by conventional methods of organic synthesis practiced by those skilled in the art. For example, the steps to obtain said compound (VI") in which the starting materials are nitromethoxybenzaldehyde and trimethoxybenzylphosphonium bromide (or chloride) are described in WO 2009/118474. The above-mentioned base (step a)) is advantageously sodium hydroxide. By "conditions appropriate to promote the crystallization"as defined above, it is meant that the process according to the invention comprises: b) a step of collecting the organic phase, c) a step of partially distilling the isopropyl acetate, d) a step of cooling the resulting medium below room temperature. By "partially distilling" in step c), it is meant a distillation of the isopropyl acetate so as to remove a part of the solvent (i.e. the removal of the solvent is not complete). Advantageously, the distillation removes at least 20% of said solvent, more advantageously at least half of the solvent. The step d) of cooling the medium below room temperature is advantageously carried out down to a temperature of about 5°C, advantageously down to 5°C ± 5°C. The step d) of cooling the medium below room temperature is advantageously followed by a step e) of maintaining the medium at the temperature achieved in the preceding step, for a duration of at least about 1 hour, advantageously for 1 to 2 hours. The step d) of cooling the medium below room temperature may be preceded by a step d') of cooling the medium at about room temperature, advantageously at about 20°C, such as 20°C ± 5°C. The reaction medium is advantageously maintained at such a temperature for a few to several hours, such as about 12 hours. The process according to the instant invention advantageously comprises a final step f) comprising the filtration, washing and drying of the crystal Form I of (2S)-2-amino-3-hydroxy-N-i2-methoxy-2-[(1Z)-2-(3,4,5-trimethoxyphenyl)ethenyl]phenyl] propanamide obtained as a result of the preceding steps. The washing is advantageously earned out with isopropyl acetate. The process according to the invention can be carried out in two variants: a first variant consists in preparing and isolating the hydrochloride form of (2S)-2-amino-3-hydroxy-N-[2-methoxy-2-[(1Z)-2-(3,4,5-trimethoxyphenyl)ethenyl]phenyl] propanamide (compound (VI")), and then carrying out the above-defined crystallization steps so as to obtain the compound (VI), Form I; a second variant is carried out without the prior isolation of the hydrochloride form of compound (VI) (compound (VI")), and consists in carrying out in situ the crystallization steps defined above, on a non-isolated intermediate (compound (VI")) obtained starting from intermediate (Z)-(Ib) as defined below. In a specific embodiment, the process for preparing crystal Form I of (2S)'2-amino-3∼hydroxy-rl-[2-memoxy-2-[(1Z)-2-(3,4,5-trim.ethoxyphenyl)ethenyl]phenyl] propanamide may therefore comprise the preliminary step of synthesizing the hydrochloride salt of (2S)-2-ammo-3-hydroxy-N-[2-methoxy-2-[(1Z)-2-(3,4,5-trimethoxyphenyl)ethenyl] phenyl]propanamide, the crystallization step a) being carried out in situ by using a solvent like isopropyl acetate and applying the above defined steps to the so obtained hydrochloride derivative. As known in the art, the synthesis of said hydrochloride derivative of (2S)-2-amino-3-hydroxy-N-[2-methoxy-2-[(1Z)-2-(3,4,5-trimethoxyphenyl)ethenyl]phenyl] propanamide may be carried out starting from the protected intermediate (Z)-(Ib), wherein PG represents a protecting group such as boc (tert-butoxycarbonyl):

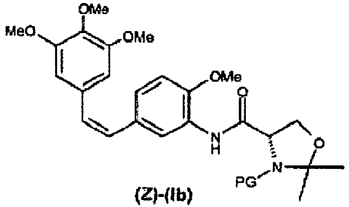

The processes according to the instant invention are particularly advantageous over the processes of preparation of ombrabulin base disclosed in the prior art since they do not comprise any purification steps by chromatography in order to recover the crystals of the compound of formula (VI), and since they provide said compound of formula (VI) Form I in good yields and with excellent chemical purity (higher than or equal to 99.5%). By "good yields"in the present invention, it is meant that said compound of formula (VI). Form I, is obtained with a yield higher than or equal to 56% starting from the protected intermediate (Z)-(Ib), advantageously higher than or equal to 77% starting from the compound of formula (VI").

In a preferred aspect, the invention provides crystal Form 1 of (2S)-2-amino-3-hydroxy-N-[2-methoxy-2-[(1Z)-2-(3,4,5-trimethoxyphenyl)ethenyl]phenyl] propanamide as defined herein substantially free of any other polymorph(s). In a further preferred aspect, the crystal Form I of (2S)-2-amino-3-hydroxy-N-[2-methoxy-2-[(1Z)-2-(3,4,5-trimethoxyphenyl)-ethenyl]phenyl]pi panamide as herein defined is substantially free of impurities. By "substantially free", it is meant that the crystal Form I comprises less than 10%, preferably less than 2%, more preferably less than 0.5%, of any other polymorph(s) or impurity or impurities. In another preferred embodiment, said crystal Form I of compound of formula (VI) has an X-ray powder diffraction diagram substantially identical to that of Figure 1 of WO2013/084150. In another preferred embodiment, said crystal Form I of compound of formula (VI) has thermo-gravimetric analysis data substantially identical to that of Figure 2 of WO2013/084150 in which no weight loss is observed from room temperature up to 170°C (i.e. up to a temperature far beyond 104°C which is the compound melting temperature). Thus said crystal Form I is an anhydrous compound. In another preferred embodiment, said crystal Form I of compound of formula (VI) has a water sorption/desorption 25°C isotherm substantially identical to that of Figure 3 of WO2013/084150 in which no significant water uptake is observed. Said crystal form is thus not hygroscopic. Said crystal Form I is a stable anhydrous and non-hygroscopic crystal solid form. These characteristics are particularly advantageous for the processes of formulation, for the stability of said compound and thus for the conditions of storage. The instrumentation and method are described in the Experimental part below. In a further aspect, said compound of formula (VI) Form I according to the invention is useful for obtaining different addition salts of said compound with a pharmaceutically acceptable acid, for example the hydrochloride salt. Using the compound of formula (VI) Form I according to the invention as an intermediate for obtaining different addition salts provides the advantages of a synthesis route devoid of purification steps by chromatography, and of using an intermediate in base form with excellent purity and optimal storage conditions (stability, non-hygroscopicity). Said compound of formula (VI) Form I can advantageously be used directly to prepare formulation, by extemporaneous formation of an addition salt of said compound with a pharmaceutically acceptable acid, for example hydrochloride salt, by addition of the corresponding acid, for example hydrochloric acid.

The present invention also relates to said crystal Form I of (2S)-2-amino-3-hydroxy-N-[2∼methoxy∼2-[(1Z)-2-(3,4,5-trimethoxyphenyl)ethenyi]phenyl]propanamide as a medicament. In a further aspect, the present invention provides, said crystal Form I of (2S)-2-amino-3-hydroxy-N-[2-methoxy-2-[(1Z)-2-(3,4,5-trimethoxyphenyl)ethenyl] phenyl] propanamide for its use for preventing and/or treating cancers and/or tumors. Another aspect of the present invention is the use of said crystal Form I of (2S)-2-amino-3-hydi xy-N-[2-methoxy-2-[(1Z)-2-(3,4,5-tnmethoxyphenyl)ethenyl] phenyl] propanamide for preventing and/or treating cancers and/or tumors. Another aspect of the present invention is the use of said crystal Form I of (2S)-2-amino-3-hydroxy-N-[2-methoxy-2-[(1Z)-2-(3,4,5-trimethoxyphenyl)ethenyl] phenyl] propanamide for the manufacture of a medicament useful for preventing and/or treating cancers and/or tumors. Said, crystal Form I can advantageously be used directly as an Active Pharmaceutical Ingredient (API) to prepare formulations. Thus, the present invention also relates to a pharmaceutical composition comprises said compound of fonnula (A) Form I and also at least one pharmaceutically acceptable excipient. All components of the present compositions must be pharmaceutically acceptable. As used herein, a "pharmaceutically acceptable"component is one that is suitable for use with humans and/or other animals without undue adverse side effects (such as toxicity, irritation and allergic response) commensurate with a reasonable benefit/risk ratio. The compositions of the present invention are generally administered to patients, which include, but are not limited to, mammals, for example, humans, by conventional routes known in the art. The present invention further relates to the use of the pharmaceutical compositions of the invention for preventing and/ r treating cancer and/or tumors. For example, the cancer may be chosen among sarcoma, ovarian cancer and non-small cell lung cancer. The tumor may be a solid tumor. Figure 1 of WO2013/084150 is an X-ray Powder Diffractogram of crystal Form I of (2S)-2-amino-3-hydroxy-N-[2-methoxy-2-[(1Z)-2-(3,4,5-trimethoxyphenyl)ethenyl] phenyl]propanamide of the present invention. Figure 2 of WO2013/084150 is Thenno-Gravimetric Analysis data of crystal Form I of (2S)-2-amino-3-hydroxy-N-[2-methoxy-2-[(1Z)-2-(3,4,5-trimethoxyphenyl)ethenyl]phenyl] propanamide of the present invention. Figure 3 of WO2013/084150 shows water sorption/desorption isotherms recorded at 25°C of ciystal Form I of (2S)-2-amino-3-hydroxy-N-[2-methoxy-2-[(1Z)-2-(3,4,5-trimethoxyphenyi)ethenyl] phenyl]propanamide of the present invention.

Example 1: Preparation of crystal Form I of (2S)-2-amino-3-hydroxy-N-[2-methoxy-2-[(1Z)-2-(3,4,5-trimethoxyphenyl)-ethenyl]phenyl]propanamide of formula (VI) via the isolation of its hydrochloride form. This synthesis can be illustrated by scheme 1 as shown below:

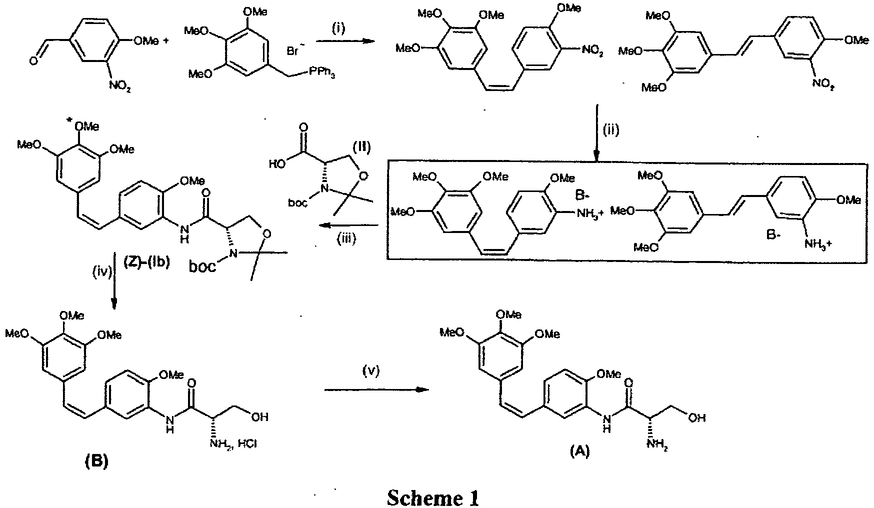

The steps (i) to (iv) as described in Scheme 1 have already been described in WO 2009/118474. Step (iv'): At a temperature of 10-20°C, a solution of sodium hydroxide of 1 N (55.2 ml, 55.2 mmoles) is poured into a mixture comprising isopropyl acetate (400 ml), water (150 ml) and (2S)-2-amino-3-hydroxy-N-[2-methoxy-2-[(1Z)-2-(3,4,5-trimethoxyphenyl)-ethenyl]phenyl] propanamide hydrochloride of formula (VI") (20.0 g, 45.6 mmoles) so as to reach a pH equal to 12 in the aqueous phase. The organic phase is collected, washed twice with 200 ml of water, and then partially concentrated. 200 ml of isopropyl acetate are then distilled at 35°C under 110 mbar. The resulting medium is cooled down to 20+5 °C and maintained at this temperature during 12 hours. The so obtained white suspension is then cooled down to 5+5°C during 1 to 2 hours before being filtered. The wet product on filter is washed twice with 20 ml of isopropyl acetate before being dried in vacuum (3.4.2 g, 77%) in order to obtain the product of formula (VI). The so obtained product of formula (VI) has a chemical purity higher than or equal to 99.5% as determined by High-Performance Liquid Chromatography (HPLC).

Example 2: Preparation of crystal Form I of (2S)-2-amino-3-hydroxy-N-[2-methoxy-2-[(1Z)-2-(3,4,5-trimethoxyphenyl)ethenyl]phenyl]propanamide of formula (VI) without the isolation of its hydrochloride form. This synthesis can be illustrated by scheme 2 as shown below:

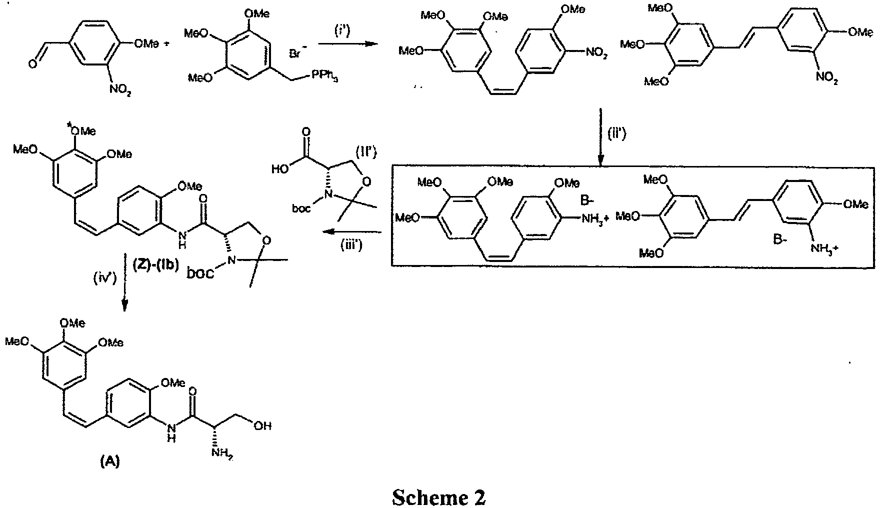

The steps (i') to (iii') are the same as the steps (i) to (iii) as described above in scheme 1 and thus are also described in WO 2009/118474. Step(iv'): TEA (32.0 ml, 0.230 mole) is poured into a medium comprising dichloromethane (DCM) (300 ml), compound (Z)-(Ib) (30 g, 0.085 mole) and compound of formula (IF) with Boc being the protective group (L-serine-N-BOC-acetonide; 25.1 g, 0.102 mole) at 20°C. Then, a solution of propanephosphonic acid anhydride (T3P) in the DCM (92.4 g, 0.145 mole) is added to the reaction medium. The medium is set and maintained at reflux during about 1 hour. The reaction medium is then cooled down to 20-25°C and then water (300 ml) is added. After decantation, the organic phase is washed with a solution of sodium bicarbonate at 6% (300 ml, 0.213 mole). After decantation, the organic phase is washed again with water (300 ml). After partial concentration, methanolic hydrochloride (114 ml, 0.682 mole) and methanol (114 ml) are added. The reactive medium is stirred up for about 4-5 hours at 20-25°C. Water (180 ml) is added to the medium, and then phases are decanted. The organic phase is then further extracted with water (120 ml). Aqueous phases are put together, and isopropyl acetate (390 ml, 13 volumes) and sodium hydroxide (69 ml, 0.690 mole) are added thereto. The decanted organic phase is washed twice with 240 ml of water. Charcoal is added to the organic phase and stirred at 25°C during 1 hour, and then filtered. The isopropyl acetate is partially distilled until 10 volumes under vacuum. The obtained slurry is cooled down to 5+5°C, maintained at this temperature during 1 hour, and then Filtered. The wet product on filter is washed twice with 25 ml of isopropyl acetate before being dried under vacuum at 40°C (19.2 g, 56 %) in order to obtain the product of formula (VI). The so obtained product of formula (VI) has a chemical purity higher than or equal to 99.5% as determined by HPLC.

Said crystal Form I according to the invention (samples obtained according to the process of scheme 1) was characterized by several physical methods such as X-ray Powder Diffraction, Differential Scanning Calorimetry, Thenno-Gravimetric Analysis, Water-Activity isotherm measurement, as shown below. X-Ray Powder Diffraction (XRPD): XRPD experiments are carried out on a Braker AXS D8 powder diffractometer using the Bragg-Brentano parafocusing geometry. A thin layer of the product is deposited on a single-crystal silicon wafer, cut out according to Si (510) crystallographic orientation that, by systematic extinction, impedes any Bragg reflection. A sealed copper anode X-ray tube (λCuKₐmean 1.54184 A wavelength) running at 35 kV and 40 mA levels is used. A Lynx-Eye detector completes the setup. Diagrams are recorded in the following conditions: a 1.5 to 40.0° scan in angle 20, 10 and 100 seconds counting time per degree in 20, according to the amount of powder to be analyzed, and ambient conditions of pressure, temperature and relative humidity. The recording of a reference XRPD diagram of said Form I is carried out on a powder gently ground with agate mortar and pestle in order to reduce preferred orientation effects. The so obtained reference XRPD diagram for a sample of Form I is shown in Figure 1 of WO2013/084150. According to Figure 1 of WO2013/084150, the main peaks have 2-theta angles (± 0.2 °) of 3.6, 7.1, 10.5, 11.2, 12.2 and 17.9 degrees 2-theta, more precisely of 3.6,. 7.1, 10.0, 10.5, 11.2, 12.2, 13.3, 14.3, 17.9, 18.7, 18.9 and 21.2 degrees 2-theta (λCuKₐmean 1.54184A). Differential Scanning Calorimetry (DSC): Analyses are carried out under a nitrogen stream with a PerkinElmer Pyris Diamond analyzer. The calorimeter is also temperature-calibrated with indium and lead (onset temperatures of 429.8 K and 600.7 K respectively). Energy calibration is done with a certified indium standard (melting enthalpy of 28.7 J/g). A mechanical compressor (Intracooler II) is used to obtain and equilibrate the temperature program: from 290 to 465 K at a rate of 5 K/min under a constant nitrogen stream of 30 mL/min. Between 1.5 and 5 mg of the product to be analyzed is placed in a semi-open aluminum sample pan. An endo thermic peak is recorded (onset around 104°C). This phenomenon corresponds to the melting of the sample. Hence, said Form I according to the invention exhibits a melting point (onset) around 104°C, advantageously 104°C ± 2°C. Thermo-Gravimctrie Analysis (TGA): Analyses are carried out using a TG209C Netzsch Instrument. A sample mass of 5 to 10 mg is deposited in an aluminium crucible. The TGA analysis is conducted under a dry nitrogen stream at 10 mL/min. The sample is heated from 25 to 270 °C at a rate of 5°C/min. Figure 2 of WO2013/084150 shows thermo-gravimetric analysis data of said Form I. No weight loss is observed from room temperature up to 170°C (i.e. up to a temperature far beyond 104°C which is the compound melting temperature). Thus said crystal Form I is an anhydrous compound. Water-Activity isotherm (DVS): All experiments are performed on a DVS-1 automated gravimetric vapor sorption analyser (Surface Measurement Systems Ltd., London, UK). The DVS-1 measures the uptake and loss of vapor gravimetrically using a Cahn D200 recording ultra-microbalance with a mass resolution of ±0.1 µg. A controlled relative humidity is generated by mixing different proportions of dry and water saturated carrier gas streams (monitored by mass flow conti llers). The temperature is maintained constant, ± 0.1 °C, by enclosing the entire system in a temperature-controlled incubator. A sample size between 4 and 10 mg is used. Prior to being exposed to any water vapor the samples were dried at 0% relative humidity (RH) to remove any surface water present and establish a dry, baseline mass. Next, the samples are exposed to an increasing relative humidity raised by a step of 5% RH from 0% to 90% RH. At each stage, the sample mass is allowed to reach equilibrium before the relative humidity is increased or decreased (considering that equilibrium is established when dm/dt ratio (m = mass; t = time) does not exceed the value of 3.3 10"4 mg/s during 30 minutes). If equilibrium state is not reached, the change in relative humidity takes place automatically after 600 minutes. From the complete moisture sorption and desorption profile an isotherm is calculated using the DVS Advanced Analysis Suite v3.6. All experiments are performed at 25°C. Figure 3 of WO2013/084150 shows water sorption/desorption isotherm recorded at 25°C. At 90% RH a total water uptake of less than 0.15% (w/w) is observed. A slight and usual hysteresis between the sorption and desorption steps is observed from 50% to 80% RH. In addition after DVS experiment no solid phase change was observed as confirmed by XRPD. Thus at 25°C said Form 1 is non-hygroscopic and physically stable over the entire range of relative humidity (0% to 90% RH).

### PREFERRED EMBODIMENTS CONCERNING ASPECTS (I), (II), (III), (IV), (V), AND (VI) OF THE INVENTION

The invention relates to an administration unit comprising (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug31}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {dwg5e}, or {drug5f}; or(vi) {drug6a}. Preferably, the administration unit according to the invention comprises a therapeutically effective amount of (i) {drug 1}, (ii) {drug2}, (iii) {drug3}, (iv) {drug4}, (v) {drug5}, or (vi) {drug6}, wherein at least 10 % by weight of said therapeutically effective amount of (i) {drug1}, (ii) {drugs2}, (iii) {drug3}, (iv) {drug4}, (v) {drug5}, or (vi) {drug6} are present as (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug31}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}. Preferably, at least 20 % by weight, or at least 30 % by weight, or at least 40 % by weight, or at least 50 % by weight, or at least 60 % by weight, or at least 70 % by weight, or at least 80 % by weight, or at least 90 % by weight of said therapeutically effective amount of (i) {drug1}, (ii) {drug2}, (iii) {drug3}, (iv) {drug4}, (v) {drug5}, or (vi) {drug6} are present as (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}. Preferably, at least 95 % by weight, or at least 96 % by weight, or at least 97 % by weight, or at least 98 % by weight, or at least 99 % by weight, or at least 99.5 % by weight, or at least 99.8 % by weight, or at least 99.9 % by weight of said therapeutically effective amount of (i) {drug1}, (ii) {drug2}, (iii) {drug3}, (iv) {drug4}, (v) {drug5}, or (vi) {drug6} are present as (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or(vi) {drug6a}.

The total content of (i) {drug1}, (ii) {drug2}, (iii) {drug3}, (iv) {drug4}, (v) {drug5}, or (vi) {drug6} as well as the relative content of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} can be determined by standard analysis known to the skilled artisan. Suitable methods include but are not limited to thermal analysis, HPLC and XRPD.

In a preferred embodiment of the administration unit according to the invention, not more than 90 % by weight of said therapeutically effective amount of (i) {drug1}, (ii) {drug2}, (iii) {drug3}, (iv) {drug4}, (v) {drug5}, or (vi) {drug6} are amorphous. Preferably, not more than 80 % by weight, or not more than 70 % by weight, or not more than 60 % by weight, or not more than 50 % by weight, or not more than 40 % by weight, or not more than 30 % by weight, or not more than 20 % by weight, or not more than 10 % by weight of said therapeutically effective amount of (i) {drug1}, (ii) {drug2}, (iii) {drug3}, (iv) {drug4}, (v) {drug5}, or (vi) {drug6} are amorphous. Preferably, not more than 5 % by weight, or not more than 4 % by weight, or not more than 3 % by weight, or not more than 2 % by weight, or not more than 1 % by weight, or not more than 0.5 % by weight, or not more than 0.2 % by weight, or not more than 0.1 % by weight of said therapeutically effective amount of (i) {drug1}, (ii) {drug2}, (iii) {drug3}, (iv) {drug4}, (v) {drug5}, or (vi) {drug6} are amorphous.

The total content of (i) {drug1}, (ii) {drug2}, (iii) {drug3}, (iv) {drug4}, (v) {drug5}, or (vi) {drug6} as well as the relative content of amorphous (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}. {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} can be determined by standard analysis known to the skilled artisan. Suitable methods include but are not limited to thermal analysis, HPLC and XRPD (see e.g. K.D. Harris, Powder diffraction crystallography of molecular solids, Top Curr Chem., 2012, 315:133-77).

In a preferred embodiment of the administration unit according to the invention, not more than 90 % by weight of said therapeutically effective amount of (i) (drug1), (ii) {drug2}, (iii) {drug3}, (iv) {drug4}, (v) {drug5}, or (vi) {drug6} are polymorphs of (i) {drug1}, (ii) {drug2}, (iii) {drug3}, (iv) {drug4}, (v) {drug5}, or (vi) {drug6} other than (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}. Preferably, not more than 80 % by weight, or not more than 70 % by weight, or not more than 60 % by weight, or not more than 50 % by weight, or not more than 40 % by weight, or not more than 30 % by weight, or not more than 20 % by weight, or not more than 10 % by weight of said therapeutically effective amount of (i) {drug1}, (ii) {drug2}, (iii) {drug3}, (iv) {drug4}, (v) {drug5}, or (vi) {drug6} are polymorphs of (i) {drug1}, (ii) {drug2}, (iii) {drug3}, (iv) {drug4}, (v) {drug5}, or (vi) {drug6} other than (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}. Preferably, not more than 5 % by weight, or not more than 4 % by weight, or not more than 3 % by weight, or not more than 2 % by weight, or not more than 1 % by weight, or not more than 0.5 % by weight, or not more than 0.2 % by weight, or not more than 0.1 % by weight of said therapeutically effective amount of (i) {drug1}, (ii) {drug2}, (iii) {drug3}, (iv) {drug4}, (v) {drug5}, or (vi) {drug6} are polymorphs of (i) {drug1}, (ii) {drug2}, (iii) {drug3}, (iv) {drug4}, (v) {drug5}, or (vi) {drug6} other than (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or(vi) {drug6a}.

The total content of (i) {drug1}, (ii) {drug2}, (iii) {drug3}, (iv) {drug4}, (v) {drug5}, or (vi) {drug6} as well as the relative content of polymorphs other than (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} can be determined by standard analysis known to the skilled artisan. Suitable methods include but are not limited to thermal analysis, HPLC and XRPD (see e.g. N. Chieng, T. Rades, J.Aaltonen, An overview of recent studies on the analysis of pharmaceutical polymorphs, J Pharm Biomed Anal. 2011, 25:55(4):618-44; R. Hilfiker, Polymorphism, Wiley-VCH, 1st ed. 2006; H.G. Brittain, Polymorphism in Pharmaceutical Solids (Drugs and the Pharmaceutical Sciences), Informa Healthcare, 2nd ed. 2009).

In a preferred embodiment of the administration unit according to the invention, therapeutically effective amount of (i) {drug1}, (ii) {drug2}, (iii) {drug3}, (iv) {drug4}, (v) {drug5}, or (vi) {drug6} is 0.1 µg to 2 g of (i) {drug1}, (ii) {drug2}, (iii) {drug3}, (iv) {drug4}, (v) {drug5}, or (vi) {drug6}. Preferably, therapeutically effective amount of (i) {drug1}, (ii) {drug2}, (iii) {drug3}, (iv) {drug4}, (v) {drug5}, or (vi) {drug6} is 1 µg to 2.5 µg of (i) {drug1}, (ii) {drug2}, (iii) {drug3}, (iv) {drug4}, (v) {drug5}, or (vi) {drug6}, or 2.5 µg to 5 µg of (i) {drug1}, (ii) {drug2}, (iii) {drug3}, (iv) {drug4}, (v) {drug5}, or (vi) {drug6}, or 5 µg to 10 µg of (i) {drug1}, (ii) {drug2}, (iii) {drug3}, (iv) {drug4}, (v) {drug5}, or (vi) {drug6}, or 10 µg to 25 µg of (i) {drug1}, (ii) {drug2}, (iii) {drug3}, (iv) {drug4}, (v) {drug5}, or (vi) {drug6}, or 25 µg to 50 µg of (i) {drug1}, (ii) {drug2}, (iii) {drug3}, (iv) {drug4}, (v) {drug5}, or (vi) {drug6}, or 50 µg to 100 µg of (i) {drug1}, (ii) {drug2}, (iii) {drug3}, (iv) {drug4}, (v) {drug5}, or (vi) {drug6}, or 100 µg to 250 µg of (i) {drug1}, (ii) {drug2}, (iii) {drug3}, (iv) {drug4}, (v) {drug5}, or (vi) {drug6}, or 250 µg to 500 µg of (i) {drug1}, (ii) {drug2}, (iii) {drug3}, (iv) {drug4}, (v) {drug5}, or (vi) {drug6}, or 500 µg to 1 mg of (i) {drug1}, (ii) {drug2}, (iii) {drug3}, (iv) {drug4}, (v) {drug5}, or (vi) {drug6}, or 1 mg to 2.5 mg of (i) {drug1}, (ii) {drug2}, (iii) {drug3}, (iv) {drug4}, (v) {drug5}, or (vi) {drug6}, or 2.5 mg to 5 mg of (i) {drug1}, (ii) {drug2}, (iii) {drug3}, (iv) {drug4}, (v) {drug5}, or (vi) {drug6}, or 5 mg to 10 mg of (i) {drug1}, (ii) {drug2}, (iii) {drug3}, (iv) {drug4}, (v) {drug5}, or (vi) {drug6}, or 10 mg to 25 mg of (i) {drug1}, (ii) {drug2}, (iii) {drug3}, (iv) {drug4}, (v) {drug5}, or (vi) {drugs6}, or 25 mg to 50 mg of (i) {drug1}, (ii) {drug2}, (iii) {drug3}, (iv) {drug4}, (v) {drug5}, or (vi) {drug6}, or 50 mg to 100 mg of (i) {drug1}, (ii) {drug2}, (iii) {drug3}, (iv) {drug4}, (v) {drug5}, or (vi) {drug6}, or 100 mg to 250 mg of (i) {drug1}, (ii) {drug2}, (iii) {drug3}, (iv) {drug4}, (v) {drug5}, or (vi) {drug6}, or 250 mg to 500 mg of (i) {drug1}, (ii) {drug2}, (iii) {drug3}, (iv) {drug4}, (v) {drug5}, or (vi) {drug6}, or 500 mg to 1 g of (i) {drug1}, (ii) {drug2}, (iii) {drug3}, (iv) {drug4}, (v) {drug5}, or (vi) {drug6}, or 1 g to 1.25 g of (i) {drug1}, (ii) {drug2}, (iii) {drug3}, (iv) {drug4}, (v) {drug5}, or (vi) {drug6}, or 1.25 g to 1.5 g of (i) {drug1}, (ii) {drug2}, (iii) {drug3}, (iv) {drug4}, (v) {drug5}, or (vi) {drug6}, or 1.5 g to 1.75 g of (i) {drug1}, (ii) {drug2}, (iii) {drug3}, (iv) {drug4}, (v) {drug5}, or (vi) {drug6}, or 1.75 g to 2 g of (i) {drug1}, (ii) {drug2}, (iii) {drug3}, (iv) {drug4}, (v) {drug5}, or (vi) {drug6}.

Preferably, the administration unit according to the invention comprises 0.1 µg to 2 g of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}. Preferably, it comprises 1 µg to 2.5 µg of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, (drug3l), {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 2.5 µg to 5 µg of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 5 µg to 10 µg of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or(vi) {drug6a}, or 10 µg to 25 µg of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 25 µg to 50 µg of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 50 µg to 100 µg of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 100 µg to 250 µg of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 250 µg to 500 µg of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or(vi) {drug6a}, or 500 µg to 1 mg of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, (drug3l), {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 1 mg to 2.5 mg of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 2.5 mg to 5 mg of (i) {drug1a}; (ii) {drug2a}, {drug2b}, {drug2c}, {drug2d}, or {drug2e}; (iii) {drug3a}, {drug3b}, {drug3e}, {drug3d}, {drug3e}, {drug3f}, {drug3g}, {drug3h}, {drug3i}, {drug3j}, {drug3k}. {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 5 mg to 10 mg of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 10 mg to 25 mg of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 25 mg to 50 mg of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 50 mg to 100 mg of (i) {drug1a}; (ii) {drug2a}, {drug2b}, {drug2c}, {drug2d}, or {drug2e}; (iii) {drug3a}, {drug3b}, (drug3c), {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, (drug5d), {drug5e}, or {drug5f}; or (vi) {drug6a}, or 100 mg to 250 mg of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or(vi) {drug6a}, or 250 mg to 500 mg of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 500 mg to 1 g of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 1 g to 1.25 g of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 1.25 g to 1.5 g of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or(vi) {drug6a}, or 1.5 g to 1.75 g of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 1.75 g to 2 g of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or(vi) {drug6a}. Preferably, the administration unit according to the invention contains (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} as substantially the only form of (i) {drug1}, (ii) {drug2}, (iii) {drug3}, (iv) {drug4}, (v) {drug5}, or (vi) {drug6}, i.e. preferably contains neither non-crystalline forms of (i) {drug1}, (ii) {drug2}, (iii) {drug3}, (iv) {drug4}, (v) {drug5}, or (vi) {drug6} nor crystalline forms other than (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) (drug5a), {drug5b}, {drug5c}, {drug5d}, {drug5e}, or {drug5f}; or(vi) {drug6a}.

The administration unit according to the invention and its constituents, i.e. (i) {drug1}, (ii) {drug2}, (iii) {drug3}, (iv) {drug4}, (v) {drug5}, or (vi) {drug6} and (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, respectively, as well as pharmaceutical excipients, can be characterized by analytic methods that are known to the skilled artisan and described in Ph. Eur. and USP (see e.g. H.G. Brittain, S.J. Bodanowich, D.E. Bugay, J. DeVoncentis, G. Lewen, A.W. Newman, Physical characterization of pharmaceutical solids, Pharm Res. 1991, 8(8):963-73; D.E. Bugay, Characterization of the solid-state: spoectroscopic techniques, Adv Drug Deliv Rev., 2001, 48(1):43-65; P.A. Tishmack, D.E. Bugay, S.R. Byrn, Solid-state nuclear magnetic resonance spectroscopy-pharmaceutical application, J Pharm Sci, 2003, 92(3):441-74; C.J. Lee, C.J. Strachan, P.J. Manson, T. Rades, Characterization of the bulk properties of pharmaceutical solids using nonlinear optics-a review, J Pharm Pharmacol., 2007, 59(2):241-50); R.A. Storey, Solid State Characterization of Pharmaceuticals, Wiley-Blackwell, 2011).

In a preferred embodiment, the administration unit according to the invention contains (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} in form of particles, wherein the particle size distribution of X90 is about 500 µm or less, i.e. a particle size distribution (preferably by volume) such that at least 90 % of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} has a particle size smaller than 500 µm. Preferably, for (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} the particle size distribution of X90 is about 480 µm or less, or about 460 µm or less, or about 440 µm or less, or about 420 µm or less, or about 400 µm or less, or about 380 µm or less, or about 360 µm or less, or about 340 µm or less, or about 320 µm or less, or about 300 µm or less, or about 280 µm or less, or about 260 µm or less, or about 240 µm or less, or about 220 µm or less, or about 200 µm or less, or about 180 µm or less, or about 160 µm or less, or about 140 µm or less, or about 120 µm or less, or about 100 µm or less, or about 80 µm or less, or about 60 µm or less, or about 40 µm or less, or about 20 µm or less.

In a preferred embodiment, the administration unit according to the invention contains (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} in form of particles, wherein the particle size distribution of X50 is about 400 µm or less, i.e. a particle size distribution (preferably by volume) such that at least 50 % of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or(vi) {drug6a} has a particle size smaller than 400 µm. Preferably, for (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} the particle size distribution of X50 is about 380 µm or less, or about 360 µm or less, or about 340 µm or less, or about 320 µm or less, or about 300 µm or less, or about 280 µm or less, or about 260 µm or less, or about 240 µm or less, or about 220 µm or less, or about 200 µm or less, or about 180 µm or less, or about 160 µm or less, or about 140 µm or less, or about 120 µm or less, or about 100 µm or less, or about 80 µm or less, or about 70 µm or less, or about 60 µm or less, or about 50 µm or less. Preferably, for (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} the particle size distribution of X50 is about 45 µm or less, or about 40 µm or less, or about 35 µm or less, or about 30 µm or less, or about 25 µm or less, or about 20 µm or less, or about 15 µm or less, or about 10 µm or less.

According to USP, the following parameters may be defined based on the cumulative distribution. QR(X) = cumulative distribution of particles with a dimension less than or equal to X [µm] where the subscript R reflects the distribution type: 0 Number, 1 Length, 2 Area, 3 Volume. Therefore, by definition: QR(X) = 0.90 when X = X90; QR(X) = 0.50 when X = X50; and QR(X) = 0.10 when X = X10.

In preferred embodiments, the administration unit according to the invention contains (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {dmg4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} in form of particles having a cumulative distribution by volume basis Q3(X) (X in µm) of Q3(355) <0.50; or Q3(180) <0.50 and Q3(355) ≥0.50; or Q3(125) <0.50 and Q3(180) ≥0.50; or Q3(125) ≥0.50.

In preferred embodiments, the administration unit according to the invention contains (i) {drug1a}; (ii) {drug2a}, {drug2b}, {drug2c}, {drug2d}, or {drug2e}; (iii) {drug3a}, {drug3b}, {drug3c}, (drug3d), {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {dmg4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} in form of particles having a cumulative distribution by volume basis Q3(X) (X in µm) of Q3(268) <0.50 and Q3(355) ≥0.50; or Q3(180) <0.50 and Q3(268) ≥0.50; or Q3(153) <0.50 and Q3(180) ≥0.50; or Q3(125) <0.50 and Q3(153) ≥0.50; or Q3(111) <0.50 and Q3(125) ≥0.50; or Q3(98) <0.50 and Q3(111) ≥0.50; or Q3(98) ≥0.50.

In preferred embodiments, the administration unit according to the invention contains (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {dmg4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} in form of particles having a cumulative distribution by volume basis Q3(X) (X in µm) of Q3(312) <0.50 and Q3(355) ≥0.50; or Q3(268) <0.50 and Q3(312) ≥0.50; or Q3(224) <0.50 and Q3(268) ≥0.50; or Q3(180) <0.50 and Q3(224) ≥0.50; or Q3(167) <0.50 and Q3(180) ≥0.50; or Q3(153) <0.50 and Q3(167) ≥0.50; or Q3(139) <0.50 and Q3(153) ≥0.50; or Q3(125) <0.50 and Q3(139) ≥0.50; or Q3(111) <0.50 and Q3(125) ≥0.50; or Q3(98) <0.50 and Q3(111) ≥0.50; or Q3(84) <0.50 and Q3(98) ≥0.50; or Q3(84) ≥0.50.

The indicated particle size properties are determined by laser-diffraction method, in particular low angle laser light scattering, i.e. Fraunhofer diffraction. Alternatively, the particle size properties can be also determined by microscopy (e.g. electron microscopy or scanning electron microscopy). The powder fineness is preferably determined in accordance with USP35 <811>. The results of the particle size distribution determined by different techniques can be correlated with one another. Preferably, the administration unit according to the invention is solid, semisolid or liquid.

Preferably, the administration unit according to the invention is for administration once daily, or twice daily, or thrice daily, or four times daily, or five times daily, or six times daily.

Preferably, the administration unit according to the invention is monolithic or multiparticulate.

In a preferred embodiment of the administration unit according to the invention, (i) {drug1a}; (ii) {drug2a}, {drug2b}, {drug2c}, {drug2d}, or {drug2e}; (iii) {drug3a}, {drug3b}, {drug3c), {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} is homogeneously distributed over the administration unit.

Excipient composition and homogeneity can be determined by standard analysis known to the skilled artisan. Suitable methods include but are not limited to near-infrared chemical imaging.

In another preferred embodiment of the administration unit according to the invention, (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} is not homogeneously distributed over the administration unit.

In a preferred embodiment, the administration unit according to the invention provides controlled release of (i) {drug1}. (ii) {drug2}, (iii) {drug3}, (iv) {drug4}, (v) {drug5}, or (vi) {drug6}. Preferably, the administration unit according to the invention comprises a controlled release matrix or a controlled release coating.

In a preferred embodiment, under in vitro conditions (paddle apparatus, 75 rpm, 900 mL artificial gastric juice, pH 1.2) the administration unit according to the invention has released after 1 hour not more than 90 % of (i) {drug1}, (ii) {drug2}, (iii) {drug3}, (iv) {drug4}, (v) {drug5}, or (vi) {drug6}. Preferably, the administration unit according to the invention has released after 1 hour not more than 80 % of (i) {drug1} (ii) {drug2}, (iii) {drug3}, (iv) {drug4}, (v) {drug5}, or (vi) {drug6}, or not more than 70 % of (i) {drug1}, (ii) {drug2}, (iii) {drug3}, (iv) {drug4}, (v) {drug5}, or (vi) {drug6}, or not more than 60 % of (i) {drug1}, (ii) {drug2}, (iii) {drug3}, (iv) {drug4}, (v) {drug5}, or (vi) {drug6}, or not more than 50 % of (i) {drug1}, (ii) {drug2}, (iii) {drug3}, (iv) {drug4}, (v) {drug5}, or (vi) {drug6}, or not more than 40 % of (i) {drug1}, (ii) {drug2}, (iii) {drug3}, (iv) {drug4}, (v) {drug5}, or (vi) {drug6}, or not more than 30 % of (i) {drug1}, (ii) {drug2}, (iii) {drug3}, (iv) {drug4}, (v) {drug5}, or (vi) {drug6}, or not more than 20 % of (i) {drug1}, (ii) {drug2}, (iii) {drug3}, (iv) {drug4}, (v) {drug5}, or (vi) {drug6}, or not more than 10 % of (i) {drug1}, (ii) {drug2}, (iii) {drug3}, (iv) {drug4}, (v) {drug5}, or (vi) {drug6}.

In a preferred embodiment, under in vitro conditions (paddle apparatus, 75 rpm, 900 mL artificial gastric juice, pH 1.2) the administration unit according to the invention has released after 2 hours not more than 90 % of (i) {drug1}, (ii) {drug2}, (iii) {drug3}, (iv) {drug4}, (v) {drug5}, or (vi) {drug6}. Preferably, the administration unit according to the invention has released after 2 hours not more than 80 % of (i) {drug1} (ii) {drug2}, (iii) {drug3}, (iv) {drug4}, (v) {drug5}, or (vi) {drug6}, or not more than 70 % of (i) {drug1}, (ii) {drug2}, (iii) {drug3}, (iv) {drug4}, (v) {drug5}, or (vi) {drug6}, or not more than 60 % of (i) {drug1}, (ii) {drug2}, (iii) {drug3}, (iv) {drug4}, (v) {drug5}, or (vi) {drug6}, or not more than 50 % of (i) {drug1}, (ii) {drug2}, (iii) {drug3}, (iv) {drug4}, (v) {drug5}, or (vi) {drug6}, or not more than 40 % of (i) {drug1}, (ii) {drug2}, (iii) {drug3}, (iv) {drug4}, (v) {drug5}, or (vi) {drug6}, or not more than 30 % of (i) {drug1}, (ii) {drug2}, (iii) {drug3}, (iv) {drug4}, (v) {drug5}, or (vi) {drug6}, or not more than 20 % of (i) {drug1}, (ii) {drug2}, (iii) {drug3}, (iv) {drug4}, (v) {drug5}, or (vi) {drug6}, or not more than 10 % of (i) {drug1}, (ii) {drug2}, (iii) {drug3}, (iv) {drug4}, (v) {drug5}, or (vi) {drug6}.

In a preferred embodiment, under in vitro conditions (paddle apparatus, 75 rpm, 900 mL artificial gastric juice, pH 1.2) the administration unit according to the invention has released after 4 hours not more than 90 % of (i) {drug1}, (ii) {drug2}, (iii) {drug3}, (iv) {drug4}, (v) {drug5}, or (vi) {drug6}. Preferably, the administration unit according to the invention has released after 4 hours not more than 80 % of (i) {drug1} (ii) {drug2}, (iii) {drug3}, (iv) {drug4}, (v) {drug5}, or (vi) {drug6}, or not more than 70 % of (i) {drug1}, (ii) {drugs2}, (iii) {drug3}, (iv) {drug4}, (v) {drug5}, or (vi) {drug6}, or not more than 60 % of (i) {drug1}, (ii) {drug2}, (iii) {drug3}, (iv) {drugs4}, (v) {drug5}, or (vi) {drug6}, or not more than 50 % of (i) {drug1}, (ii) {drug2}, (iii) {drug3}, (iv) {drug4}, (v) {drug5}, or (vi) {drug6}, or not more than 40 % of (i) {drug1}, (ii) {drug2}, (iii) {drug3}, (iv) {drug4}, (v) {drug5}, or (vi) {drug6}, or not more than 30 % of (i) {drug1}, (ii) {drug2}, (iii) {drug3}, (iv) {drug4}, (v) {drug5}, or (vi) {drug6}, or not more than 20 % of (i) {drug1}, (ii) {drug2}, (iii) {drug3}, (iv) {drug4}, (v) {drug5}, or (vi) {drug6}, or not more than 10 % of (i) {drug1} (ii) {drug2}, (iii) {drug3}, (iv) {drug4}, (v) {drug5}, or (vi) {drug6}.

In a preferred embodiment, under in vitro conditions (paddle apparatus, 75 rpm, 900 mL artificial gastric juice, pH 1.2) the administration unit according to the invention has released after 8 hours not more than 90 % of (i) {drug 1}, (ii) {drugs2}, (iii) {drug3}, (iv) {drug4}, (v) {drug5}, or (vi) {drugs6}. Preferably, the administration unit according to the invention has released after 8 hours not more than 80 % of (i) {drug1} (ii) {drug2}, (iii) {drug3}, (iv) {drug4}, (v) {drug5}, or (vi) {drug6}, or not more than 70 % of (i) {drug1}, (ii) {drug2}, (iii) {drug3}, (iv) {drug4}, (v) {drug5}, or (vi) {drug6}, or not more than 60 % of (i) {drug1}, (ii) {drug2}, (iii) {drug3}, (iv) {drug4}, (v) {drug5}, or (vi) {drug6}, or not more than 50 % of (i) {drug1}, (ii) {drug2}, (iii) {drug3}, (iv) {drug4}, (v) {drug5}, or (vi) {drug6}, or not more than 40 % of (i) {drug1}, (ii) {drug2}, (iii) {drugs3}, (iv) {drug4}, (v) {drug5}, or (vi) {drug6}, or not more than 30 % of (i) {drug1}, (ii) {drug2}, (iii) {drug3}, (iv) {drug4}, (v) {drug5}, or (vi) {drug6}, or not more than 20 % of (i) {drug1}, (ii) {drug2}, (iii) {drug3}, (iv) {drug4}, (v) {drug5}, or (vi) {drug6}, or not more than 10 % of (i) {drug1}, (ii) {drug2}, (iii) {drug3}, (iv) {drug4}, (v) {drug5}, or (vi) {drug6}.

In another preferred embodiment, the administration unit according to the invention provides immediate release of (i) {drug1} (ii) {drug2}, (iii) {drug3}, (iv) {drug4}, (v) {drug5}, or(vi) {drug6}.

In a preferred embodiment, under in vitro conditions (paddle apparatus, 75 rpm, 900 mL artificial gastric juice, pH 1.2) the administration unit according to the invention has released after 0.5 hours at least 10 % of (i) {drug1} (ii) {drug2}, (iii) {drug3}, (iv) {drug4}, (v) {drug5}, or (vi) {drug6}. Preferably, the administration unit according to the invention has released after 0.5 hours at least 20 % of(i) {drug1}, (ii) {drug2}, (iii) {drug3}, (iv) {drug4}, (v) {drug5}, or(vi) {drug6}, or at least 30 % of (i) {drug1}, (ii) {drug2}, (iii) {drug3}, (iv) {drug4}, (v) {drug5}, or (vi) {drug6}, or at least 40 % of (i) {drug1}, (ii) {drug2}, (iii) {drug3}, (iv) {drug4}, (v) {drug5}, or(vi) {drug6}, or at least 50 % of (i) {drug1}, (ii) {drug2}, (iii) {drug3}, (iv) {drug4}, (v) {drug5}, or (vi) {drug6}, or at least 60 % of (i) {drug1}, (ii) {drug2}, (iii) {drug3}, (iv) {drug4}, (v) {drug5}, or (vi) {drug6}, or at least 70 % of (i) {drug1}, (ii) {drug2}, (iii) {drug3}, (iv) {drug4}, (v) {drug5}, or (vi) {drug6}, or at least 80 % of (i) {drug1}, (ii) {drug2}, (iii) {drug3}, (iv) {drug4}, (v) {drug5}, or (vi) {drug6}, or at least 90 % of (i) {drug1}, (ii) {drug2}, (iii) {drug3}, (iv) {drug4}, (v) {drug5}, or (vi) {drug6}.

Preferably, the administration unit according to the invention has a total weight of 10 mg to 3 g. Preferably, the administration unit according to the invention has a total weight of 10 to 25 mg, or 25 mg to 50 mg, or 50 mg to 100 mg, or 100 mg to 250 mg, or 250 mg to 500 mg, or 500 mg to 1000 mg, or 1000 mg to 2500 mg.

In a preferred embodiment, the administration unit according to the invention is for systemic or topical administration. In another preferred embodiment, the administration unit according to the invention is for parenteral administration.

Preferably, the administration unit according to the invention is for oral, buccal, opthalmic, nasal, rectal, transmucosal, or intestinal administration. Preferably, it is for oral administration.

Preferably, the administration unit according to the invention is selected from the group consisting of tablets, capsules, effervescent tablets, orodispersible tablets, dragees, sachets, drops, suspensions, and powders. Preferably, it is a tablet.

Preferably, the administration unit according to the invention is round or oblong; and/or is planar or biconvex.

In a preferred embodiment, the administration unit according to the invention has a round cross-section with a diameter of 1 mm to 20 mm. Preferably, the diameter of the round cross-section is 1 mm to 3 mm, or 3 mm to 5 mm, or 5 mm to 8 mm, or 8 mm to 10 mm, or 10 mm to 13 mm, or 13 mm to 15 mm, or 15 mm to 18 mm, or 18 mm to 20 mm.

In a preferred embodiment, the administration unit according to the invention is oblong and has an aspect ratio of at least 1.1:1. Preferably, the aspect ratio is at least 4:3, or at least 21/2:1, or at least 3:2, or at least 16:10, or at least □:1, or at least 5:3, or at least 16:9.

In a preferred embodiment, the administration unit according to the invention has been manufactured by direct compression. In another preferred embodiment, the administration unit according to the invention has been manufactured by granulation and subsequent compression of granules. Preferably, granulation is wet granulation or dry granulation.

Preferably, the administration unit according to the invention has been compacted at a pressure of 10 MPa to 10,000 MPa. Preferably, the administration unit according to the invention has been compacted at a pressure of 10 MPa to 25 MPa, or 25 MPa to 50 MPa, or 50 MPa to 100 MPa, or 100 MPa to 250 MPa, or 250 MPa to 500 MPa, or 500 MPa to 1000 MPa, or 1000 MPa to 2500 MPa, or 2500 MPa to 5000 MPa, or 5000 MPa to 10,000 MPa.

In a preferred embodiment, the administration unit according to the invention comprises a film coating. Preferably, the film coating is enteric. Suitable enteric coating materials are known to the skilled artisan and include but are not limited to methyl acrylate-methacrylic acid copolymers, cellulose acetate succinate, hydroxy propyl methyl cellulose phthalate, hydroxy propyl methyl cellulose acetate succinate (hypromellose acetate succinate), polyvinyl acetate phthalate (PVAP), methyl methacrylate-methacrylic acid copolymers, sodium alginate and stearic acid.

In a preferred embodiment of the administration unit according to the invention, the film coating comprises a cellulose ether (e.g. hydroxypropylmethyl cellulose) or a synthetic polymer (e.g. polyvinyl alcohol).

In a preferred embodiment of the administration unit according to the invention, the film coating has an average thickness of 0.01 µm to 0.03 µm, or 0.03 to 0.05 µm, or 0.05 to 0.1 µm, or 0.1 to 0.25 µm, or 0.25 to 0.5 µm, or 0.5 to 1.0 µm, or 1.0 µm to 2.5 µm, or 2.5 µm to 5.0 µm, or 5.0 µm to 10 µm, or 10 µm to 25 µm, or 25 µm to 50 µm, or 50 µm to 100 µm, or 100 µm to 250 µm, or 250 µm to 500 µm, or 500 µm to 1000 µm.

In a preferred embodiment, the administration unit according to the invention has a tablet porosity of not more than 90 %. Preferably, it has a tablet porosity of not more than 80 %, or not more than 70 %, or not more than 60 %, or not more than 50 %, or not more than 40 %, or not more than 30 %, or not more than 20 %, or not more than 10 %. Preferably, it has a tablet porosity of not more than 5 %, or not more than 4 %, or not more than 3 %, or not more than 2 %, or not more than 1 %, or not more than 0.5 %, or not more than 0.2 %, or not more than 0.1 %.

The most common strength test in pharmaceutical applications is the diametral compression test, which is used to calculate the radial tensile strength of a tablet (Fell, J.T., Newton, J.M., 1970. Determination of tablet strength by diametral compression test. J. Pharm. Sci. 59,688-691). In order to calculate the radial tensile strength, the stress conditions have to be such that the tablet fails in tension. During radial tensile strength measurements, the fracture occurs through a predetermined diametral cross section of the tablet. Therefore, the radial tensile strength is likely to reflect the average strength of tablet rather than the strength of the weakest plane in the tablet.

In a preferred embodiment, the administration unit according to the invention has a radial tensile strength of 0.1 to 100 MPa. Preferably, it has a radial tensile strength of 0.1 MPa to 0.3 MPa, or 0.3 MPa to 0.5 MPa, or 0.5 MPa to 1.0 MPa, or 1.0 MPa to 2.5 MPa, or 2.5 MPa to 5.0 MPa, or 5.0 MPa to 10 MPa, or 10 MPa to 25 MPa, or 25 MPa to 50 MPa, or 50 MPa to 100 MPa.

Another method for determining mechanical strength is to measure the axial tensile strength. The force necessary to break the tablet is obtained by pulling the tablet parallel to the applied force during the formation of the tablet and this force is then used to calculate the axial tensile strength (Nyström, C., Malmqvist, K., Mazur, J., Alex, W., Hölzer, A.W., 1978. Measurement of axial and radial tensile strength of tablets and their relation to capping. Acta Pharm. Suec. 15, 226-232). During axial tensile strength measurements, the fracture will occur through the weakest plane in the tablet. Consequently, this method allows detection of capping tendencies in a tablet.

In a preferred embodiment, the administration unit according to the invention has an axial tensile strength of 0.1 to 100 MPa. Preferably, it has an axial tensile strength of 0.1 MPa to 0.3 MPa, or 0.3 MPa to 0.5 MPa, or 0.5 MPa to 1.0 MPa, or 1.0 MPa to 2.5 MPa, or 2.5 MPa to 5.0 MPa, or 5.0 MPa to 10 MPa, or 10 MPa to 25 MPa, or 25 MPa to 50 MPa, or 50 MPa to 100 MPa.

In a preferred embodiment, the administration unit according to the invention has an average pore size of 0.001 µm to 1000 µm. Preferably, it has an average pore size of 0.001 µm to 0.003 µm, or 0,003 µm to 0.005 µm, or 0.005 µm to 0.01 µm, or 0.01 µm to 0.025 µm, or 0.025 µm to 0.05 µm, or 0.05 µm to 0.1 µm, or 0.1 µm to 0.25 µm, or 0.25 µm to 0.5 µm, or 0.5 µm to 1 µm, or 1 µm to 2.5 µm, or 2.5 µm to 5 µm, or 5 µm to 10 µm, or 10 µm to 25 µm, or 25 µm to 50 µm, or 50 µm to 100 µm, or 100 µm to 250 µm, or 250 µm to 500 µm, or 500 µm to 1000 µm.

The pore size distribution of a tablet may be assessed by methods that are known to the skilled artisan and include but are not limited to gas adsorption (e.g. Stanley-Wood, N.G., Johansson, M.E., 1980. Variation of intra-and inter-particle porosity with degree of compaction. Analyst 105, 1104-1112; Westermarck, S., Juppo, A.M., Kervinen, L., Yliruusi, J., 1998. Pore structure and surface area of mannitol powder, granules and tablets determined with mercury porosimetry and nitrogen adsorption. Eur. J. Pharm. Biopharm. 46, 61-86) or mercury porosimetry (e.g. Stanley-Wood, N.G., Johansson, M.E., 1980. Variation of intra-and inter-particle porosity with degree of compaction. Analyst 105, 1104-1112; Juppo, A.M., 1996. Relationship between breaking force and pore structure of lactose, glucose and mannitol tablets. Int. J. Pharm.127, 95-102; Westermarck, S., Juppo, A.M., Kervinen, L., Yliruusi, J., 1998. Pore structure and surface area of mannitol powder, granules and tablets determined with mercury porosimetry and nitrogen adsorption. Eur. J. Pharm. Biopharm. 46, 61-86). These techniques are complementary, in that mercury porosimetry can be used to measure larger pores (the lower size limit is about 0.003 µm in diameter) while gas adsorption allows measurement of smaller pores. For further details it is also referred to S. Lowell et al., Characterization of Porous Solids and Powders: Surface Area, Pore Size and Density (Particle Technology Series), Springer, 2010.

In a preferred embodiment, the administration unit according to the invention has a pore size distribution such that at least 10 % of the pores have a pore size of not more than 0.1 µm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not more than 0.1 µm.

In another preferred embodiment, the administration unit according to the invention has a pore size distribution such that at least 10 % of the pores have a pore size of not less than 0.1 µm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not less than 0.1 µm.

In preferred embodiment, the administration unit according to the invention has a pore size distribution such that at least 10 % of the pores have a pore size of not more than 1 µm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not more than 1 µm.

In another preferred embodiment, the administration unit according to the invention has a pore size distribution such that at least 10 % of the pores have a pore size of not less than 1 µm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not less than 1 µm.

In a preferred embodiment, the administration unit according to the invention has a pore size distribution such that at least 10 % of the pores have a pore size of not more than 10 µm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not more than 10 µm.

In another preferred embodiment, the administration unit according to the invention has a pore size distribution such that at least 10 % of the pores have a pore size of not less than 10 µm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not less than 10 µm.

In a preferred embodiment, the administration unit according to the invention has a water content of not more than 5 % by weight, relative to the total weight of the tablet. Preferably, it has a water content of not more than 4 % by weight, or not more than 3 % by weight, or not more than 2 % by weight, or not more than 1 % by weight, or not more than 0.5 % by weight, or not more than 0.1 % by weight, relative to the total weight of the tablet.

In a preferred embodiment, the administration unit according to the invention has a true density of 0.60 to 1.40 g cm-3. Preferably, it has a true density of 0.60 to 0.65 g cm-3, or 0.65 to 0.70 g cm-3, or 0.70 to 0.75 g cm-3, or 0.75 to 0.80 g cm-3, or 0.80 to 0.85 g cm-3, or 0.85 to 0.90 g cm-3, or 0.90 to 0.95 g cm-3, or 0.95 to 1.00 g cm-3, or 1.00 to 1.05 g cm-3, or 1.05 to 1.10 g cm-3, or 1.10 to 1.15 g cm-3, or 1.15 to 1.20 g cm-3, or 1.20 to 1.25 g cm-3, or 1.25 to 1.30 g cm-3, or 1.30 to 1.35 g cm-3, or 1.35 to 1.40 g cm-3.

In a preferred embodiment, the administration unit according to the invention has an apparent density of 0.60 to 1.40 g cm-3. Preferably, it has an apparent density of 0.60 to 0.65 g cm-3, or 0.65 to 0.70 g cm-3, or 0.70 to 0.75 g cm-3, or 0.75 to 0.80 g cm-3, or 0.80 to 0.85 g cm-3, or 0.85 to 0.90 g cm-3, or 0.90 to 0.95 g cm-3, or 0.95 to 1.00 g cm-3, or 1.00 to 1.05 g cm-3, or 1.05 to 1.10 g cm-3, or 1.10 to 1.15 g cm-3, or 1.15 to 1.20 g cm-3, or 1.20 to 1.25 g cm-3, or 1.25 to 1.30 g cm-3, or 1.30 to 1.35 g cm-3, or 1.35 to 1.40 g cm-3.

In a preferred embodiment, the administration unit according to the invention disintegrates within 6000 seconds. Preferably, it disintegrates within 5500 seconds, or within 5000 seconds, or within 4500 seconds, or within 4000 seconds, or within 3500 seconds, or within 3000 seconds, or within 2500 seconds, or within 2000 seconds, or within 1500 seconds, or within 1000 seconds, or within 750 seconds, or within 500 seconds, or within 250 seconds.

In a preferred embodiment, the administration unit according to the invention is a capsule. Preferably, it comprises a multitude of particulates comprising (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}. Preferably, it comprises at least 2 particulates, or at least 3 particulates, or at least 4 particulates, or at least 5 particulates, or at least 6 particulates, or at least 7 particulates, or at least 8 particulates, or at least 9 particulates, or at least 10 particulates.

In a preferred embodiment of the administration unit according to the invention, the capsule material comprises hard gelatine. In a preferred embodiment, the administration unit according to the invention comprises at least 2 different types of particulates that differ from each other in at least one property selected from the group consisting of nature of excipients, content of excipients, content of (i) {drug1a}; (ii) {drug2a}, {drug2b}, {drug2c}, {drug2d}, or {dmg2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or(vi) {drug6a}, content of (i) {drug1}, (ii) {drug2}, (iii) {drug3}, (iv) {drug4}, (v) {drug5}, or (vi) {drug6}, water content, total weight, size, and shape.

In a preferred embodiment, the administration unit according to the invention passes the storage stability test according to Ph. Eur. under accelerated storage conditions at 40°C and 75 % rel. humidity. Preferably, it has a storage stability for at least 3 months, or at least 4 months, or at least 5 months, or at least 6 months, or at least 7 months, or at least 8 months, or at least 9 months, or at least 10 months, or at least 11 months, or at least 12 months according to Ph. Eur. under accelerated storage conditions at 40°C and 75 % rel. humidity.

In a preferred embodiment, the administration unit according to the invention provides the peak plasma level within 0.1 hour to 36 hours after administration. Preferably, it provides the peak plasma level within 0.1 hour to 1 hour, or 1 hour to 2 hours, or 2 hours to 3 hours, or 3 hours to 4 hours, or 4 hours to 5 hours, or 5 hours to 6 hours, or 6 hours to 7 hours, or 7 hours to 8 hours, or 8 hours to 9 hours, or 9 hours to 10 hours, or 10 hours to 11 hours, or 11 hours to 12 hours, or 12 hours to 13 hours, or 13 hours to 14 hours, or 14 hours to 15 hours, or 15 hours to 16 hours, or 16 hours to 17 hours, or 17 hours to 18 hours, or 18 hours to 19 hours, or 19 hours to 20 hours, or 20 hours to 21 hours, or 21 hours to 22 hours, or 22 hours to 23 hours, or 23 hours to 24 hours, after administration.

Preferably, the administration unit according to the invention comprises one or more excipients independently selected from the group consisting of antiadherents, binders, disintegrants, fillers, diluents, flavors, colors, lubricants, glidants, sorbents, surfactants, preservatives and sweeteners.

In a preferred embodiment of the administration unit according to the invention, the antiadherent is selected from the group consisting of silicon dioxide, talc, talc waxes, stearates (e.g. magnesium, calcium and sodium), stearic acid, stearowet, boric acid, sodium chloride, DL-leucine, sodium oleate, sodium benzoate, sodium acetate, sodium lauryl sulfate, and magnesium lauryl sulfate. Magnesium stearate is particularly preferred.

In a preferred embodiment of the administration unit according to the invention, the binder is selected from the group consisting of saccharides and their derivatives; proteins; gums; silicates; and synthetic polymers. Preferably, the binder is a saccharide or a derivative thereof selected from the group consisting of disaccharides (e.g. sucrose, glucose or lactose); polysaccharides and their derivatives (e.g. starch, pregelatinized starch, cellulose, microcrystalline cellulose, polysaccharide acids, cellulose ethers [e.g. methyl cellulose, ethyl cellulose, hydroxyethyl cellulose, hydroxypropyl cellulose, hydroxypropylmethyl cellulose or carboxymethylcellulose sodium]); alginic acid and salts thereof (e.g. sodium alginate); and sugar alcohols (e.g. xylitol, sorbitol or maltitol); or wherein the binder is a protein selected from gelatin; or wherein the binder is a gum (e.g. acacia gum, guar gum, or tragacanth gum); or wherein the binder is a silicate (e.g. magnesium aluminum silicate or bentonite); or wherein the binder is a synthetic polymer selected from the group consisting of polyvinylpyrrolidone, polyvinylpyrrolidone-vinylacetate copolymer, polymethacrylate polymer, polyvinylalcohol, and polyethylene glycol.

In a preferred embodiment of the administration unit according to the invention, the disintegrant is selected from the group consisting of cross-linked polymers (e.g. cross-linked polyvinylpyrrolidone [crospovidone] or cross-linked sodium carboxymethyl cellulose [croscarmellose sodium]), starches (e.g. corn or potato), pregelatinized starch, modified starch (e.g. sodium starch glycolate), algenic acid, alginates (e.g. sodium alginate), carboxymethylcellulose, microcrystalline cellulose, clays (e.g. magnesium aluminum silicate), and gums (e.g. agar, guar, locust bean, karaya, pectin, or tragacanth gum).

In a preferred embodiment of the administration unit according to the invention, the filler (or diluent) is selected from the group consisting of plant cellulose, microcrystalline cellulose, inorganic phosphates (e.g. dibasic calcium phosphate or tricalcium phosphate), inorganic carbonates such as calcium carbonate, inorganic sulfates such as calcium sulfate dihydrate, sugars and sugar alcohols (e.g. lactose, glucose, inositol, mannitol, sorbitol, sucrose, dextrose, maltodextrins, and fructose), magnesium stearate, calcium lactate trihydrate, starch (e.g. corn, wheat, maize, potato or rice starch), and modified starch (e.g. carboxymethyl starch).

In a preferred embodiment of the administration unit according to the invention, the lubricant is hydrophilic or hydrophobic. Preferably, the lubricant is selected from the group consisting of talc, silica, stearin, fatty acids (e.g. stearic acid) or fatty acid derivatives, metal stearates (e.g. magnesium stearate, calcium stearate, or zinc stearate), glyceryl monostearate, glyceryl palmitostearate, glyceryl behenate, sodium lauryl sulfate, magnesium lauryl sulfate, sodium stearyl fumarate, hydrogenated vegetable oil, polyalkylene glycols (e.g. polyethylene glycol), starch, light mineral oil, sodium benzoate, and sodium chloride.

In a preferred embodiment of the administration unit according to the invention, the glidant is selected from the group consisting of fumed silica, cornstarch, talc, and magnesium carbonate.

In a preferred embodiment of the administration unit according to the invention, the surfactant is selected from the group consisting of sodium lauryl sulfate, polyethylene-polypropylene glycol co-polymers, poly(oxyethylene)-poly(oxypropylene)-block-copolymers (e.g. poloxamers).

In a preferred embodiment of the administration unit according to the invention, the preservative is selected from the group consisting of antioxidants (e.g. vitamin A, vitamin C, vitamin E, retinyl palmitate, selenium, ascorbyl palmitate, sodium ascorbate), amino acids (e.g. cysteine or methionine), citric acid and its salts (e.g. sodium citrate), synthetic preservatives (e.g. parabens such as methyl paraben or propyl paraben; butylated hydroxyanisole, butylated hydroxytoluene, monothioglycerol, propyl gallate), hypophosphorous acid, sodium bisulfite, sodium formaldehyde sulfoxylate, and sodium metabisulfite.

In a preferred embodiment, the administration unit according to the invention passes the friability test according to Ph. Eur. Preferably, the weight loss in the friability test according to Ph. Eur. is not more than 2.0 % by weight, or not more than 1.5 % by weight, or not more than 1.0 % by weight, or not more than 0.5 % by weight, or not more than 0.4 % by weight, or not more than 0.3 % by weight, or not more than 0.2 % by weight, or not more than 0.1 % by weight.

In a preferred embodiment, the administration unit according to the invention passes the test uniformity of content of single-dose preparations according to Ph. Eur.

In a preferred embodiment, the administration unit according to the invention does not comprise any active pharmaceutical ingredient other than (i) {drug1}, (ii) {drug2}, (iii) {drug3}, (iv) {drug4}, (v) {drug5}, or (vi) {drug6}.

In a preferred embodiment, the administration unit according to the invention is for storage not above 50 °C. Preferably, it is for storage not above 25 °C.

In a preferred embodiment, the administration unit according to the invention is not identical with any of the administration units that are individualized in the experimental section of WO2013/072327, WO2013/076177, WO2013/076178, WO2013/076179, WO2013/083553, and WO2013/084150, respectively, which is incorporated by reference.

The invention also relates to the administration unit according to the invention for use in the treatment of a disorder or a disease in a mammal. Preferably, the mammal is a human. Preferably, the mammal is an adult.

Preferably, the mammal does not suffer from liver damage and/or kidney damage.

Preferably, the mammal is not pregnant.

Preferably, the mammal is not allergic against (i) {drug1}, (ii) {drug2}, (iii) {drug3}, (iv) {drug4}, (v) {drug5}, or (vi) {drug6}.

In a preferred embodiment, the administration unit according to the invention is administered orally with a liquid. Preferably, the liquid is water, milk, juice or lemonade.

In a preferred embodiment of the administration unit according to the invention, the administered daily dose of (i) {drug1} (ii) {drug2}, (iii) {drug3}, (iv) {drug4}, (v) {drug5}, or (vi) {drug6} is 0.0001 mg/kg body weight to 100 mg/kg body weight. Preferably, the administered daily dose of (i) {drug1}, (ii) {drug2}, (iii) {drug3}, (iv) {drug4}, (v) {drug5}, or (vi) {drug6} is 0.0001 mg/kg body weight to 0.0003 mg/kg body weight, or 0.0003 mg/kg body weight to 0.0005 mg/kg body weight, or 0.0005 mg/kg body weight to 0.001 mg/kg body weight, or 0.001 mg/kg body weight to 0.0025 mg/kg body weight, or 0.0025 mg/kg body weight to 0.005 mg/kg body weight, or 0.005 mg/kg body weight to 0.01 mg/kg body weight, or 0.01 mg/kg body weight to 0.025 mg/kg body weight, or 0.025 mg/kg body weight to 0.05 mg/kg body weight, or 0.05 mg/kg body weight to 0.1 mg/kg body weight, or 0.1 mg/kg body weight to 0.25 mg/kg body weight, or 0.25 mg/kg body weight to 0.5 mg/kg body weight, or 0.5 mg/kg body weight to 1 mg/kg body weight, or 1 mg/kg body weight to 2.5 mg/kg body weight, or 2.5 mg/kg body weight to 5 mg/kg body weight, or 5 mg/kg body weight to 10 mg/kg body weight, or 10 mg/kg body weight to 25 mg/kg body weight, or 25 mg/kg body weight to 50 mg/kg body weight, or 50 mg/kg body weight to 100 mg/kg body weight.

In a preferred embodiment of the administration unit according to the invention, the administered daily dose of (i) {drug1}, (ii) {drug2}, (iii) {drug3}, (iv) {drug4}, (v) {drug5}, or (vi) {drug6} is 0.001 mg to 5000 mg. Preferably, the administered daily dose of (i) {drug1}, (ii) {drug2}, (iii) {drug3}, (iv) {drug4}, (v) {drug5}, or (vi) {drug6} is 0.001 mg to 0.003 mg, or 0.003 mg to 0.005 mg, or 0.005 mg to 0.01 mg, or 0.01 mg to 0.025 mg, or 0.025 mg to 0.05 mg, or 0.05 mg to 0.1 mg, or 0.1 mg to 0.25 mg, or 0.25 mg to 0.5 mg, or 0.5 mg to 1 mg, or 1 mg to 2.5 mg, or 2.5 mg to 5 mg, or 5 mg to 10 mg, or 10 mg to 25 mg, or 25 mg to 50 mg, or 50 mg to 100 mg, or 100 mg to 250 mg, or 250 mg to 500 mg, or 500 mg to 1000 mg, or 1000 mg to 1250 mg, or 1250 mg to 1500 mg, or 1500 mg to 1750 mg, or 1750 mg to 2000 mg.

The invention also relates to a packaging comprising one or more administration units according to the invention.

In a preferred embodiment, the packaging according to the invention comprises a material selected from the group consisting of paper, cardboard, paperboard, metal foil and plastic foil.

In a preferred embodiment, the packaging according to the invention is a blister packaging.

A preferred blister packaging includes but is not limited to PVC-blister, PVDC-blister, PVC/PVDC-blister, moisture-proof packaging material such as aluminium foil blister pack, alu/alu blister, transparent or opaque polymer blister with pouch.

An alternative packaging according to the invention is a polypropylene tube, glass bottle and HDPE bottle optionally containing a child-resistant feature. The primary packaging material may comprise a desiccant such as molecular sieve or silica gel to improve chemical stability of (i) {drug1}, (ii) {drug2}, (iii) {drug3}, (iv) {drug4}, (v) {drug5}, or (vi) {drug6}. Opaque packaging such as colored blister materials, tubes, brown glass bottles or the like can be used to prolong shelflife of (i) {drug1}, (ii) {drug2}, (iii) {drug3}, (iv) {drug4}, (v) {drug5}, or (vi) {drugs6} by reduction of photodegradation.

In a preferred embodiment, the packaging according to the invention comprises a multitude of at least 2, or at least 3, or at least 4, or at least 5, or at least 6, or at least 7, or at least 8, or at least 9, or at least 10 administration units according to the invention.

In a preferred embodiment of the packaging according to the invention, the administration units do not substantially differ in at least one property selected from the group consisting of nature of excipients, content of excipients, content of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or (drug5f); or (vi) {drug6a}, content of (i) {drug1}, (ii) {drug2}, (iii) {drug3}, (iv) {drug4}, (v) {drug5}, or (vi) {drug6}, water content, total weight, release profile, disintegration time, size, shape, porosity, apparent density, true density, average pore size, pore size distribution, axial tensile strength, radial tensile strength, friability, and storage stability. Preferably, the administration units differ in said at least one property by not more than 5 %, or not more than 4 %, or not more than 3 %, or not more than 2 %, or not more than 1 %, or not more than 0.5 %, or not more than 0.1 %. Preferably, all administration units are substantially identical.

The invention also relates to the packaging according to the invention for use in the treatment of a disorder or a disease, wherein one or more administration units of said multitude are administered on a first day, and one or more administration units of said multitude are administered on a second day following said first day.

In a preferred embodiment of the packaging according to the invention, one or more administration units of said multitude are administered on one or more subsequent days following said second day. Preferably, the time interval between the administration of an administration unit and the administration of a subsequent administration unit is not more than 48 hours. Preferably, the time interval is not more than 36 hours, or not more than 24 hours, or not more than 12 hours, or not more than 8 hours, or not more than 6 hours, or not more than 4 hours.

In a preferred embodiment of the packaging according to the invention, the administration units are administered on not more than 30 subsequent days. Preferably, the administration units are administered on not more than 25 subsequent days, or on not more than 20 subsequent days, or on not more than 14 subsequent days, or on not more than 10 subsequent days, or on not more than 7 subsequent days, or on not more than 3 subsequent days.

In a preferred embodiment of the packaging according to the invention, the administered daily dose of (i) {drug1}, (ii) {drug2}, (iii) {drug3}, (iv) {drug4}, (v) {drug5}, or (vi) {drug6} is 0.0001 mg/kg body weight to 100 mg/kg body weight. Preferably, the administered daily dose of (i) {drug1}, (ii) {drug2}, (iii) {drug3}, (iv) {drug4}, (v) {drug5}, or (vi) {drug6} is 0.0001 mg/kg body weight to 0.0003 mg/kg body weight, or 0.0003 mg/kg body weight to 0.0005 mg/kg body weight, or 0.0005 mg/kg body weight to 0.001 mg/kg body weight, or 0.001 mg/kg body weight to 0.0025 mg/kg body weight, or 0.0025 mg/kg body weight to 0.005 mg/kg body weight, or 0.005 mg/kg body weight to 0.01 mg/kg body weight, or 0.01 mg/kg body weight to 0.025 mg/kg body weight, or 0.025 mg/kg body weight to 0.05 mg/kg body weight, or 0.05 mg/kg body weight to 0.1 mg/kg body weight, or 0.1 mg/kg body weight to 0.25 mg/kg body weight, or 0.25 mg/kg body weight to 0.5 mg/kg body weight, or 0.5 mg/kg body weight to 1 mg/kg body weight, or 1 mg/kg body weight to 2.5 mg/kg body weight, or 2.5 mg/kg body weight to 5 mg/kg body weight, or 5 mg/kg body weight to 10 mg/kg body weight, or 10 mg/kg body weight to 25 mg/kg body weight, or 25 mg/kg body weight to 50 mg/kg body weight, or 50 mg/kg body weight to 100 mg/kg body weight.

In a preferred embodiment of the packaging according to the invention, the administered daily dose of (i) {drug1} (ii) {drug2}, (iii) {drug3}, (iv) {drug4}, (v) {drug5}, or (vi) {drug6} is 0.001 mg to 5000 mg. Preferably, the administered daily dose of (i) {drug1}, (ii) {drug2}, (iii) {drug3}, (iv) {drug4}, (v) {drug5}, or (vi) {drug6} is 0.001 mg to 0.003 mg, or 0.003 mg to 0.005 mg, or 0.005 mg to 0.01 mg, or 0.01 mg to 0.025 mg, or 0.025 mg to 0.05 mg, or 0.05 mg to 0.1 mg, or 0.1 mg to 0.25 mg, or 0.25 mg to 0.5 mg, or 0.5 mg to 1 mg, or 1 mg to 2.5 mg, or 2.5 mg to 5 mg, or 5 mg to 10 mg, or 10 mg to 25 mg, or 25 mg to 50 mg, or 50 mg to 100 mg, or 100 mg to 250 mg, or 250 mg to 500 mg, or 500 mg to 1000 mg, or 1000 mg to 1250 mg, or 1250 mg to 1500 mg, or 1500 mg to 1750 mg, or 1750 mg to 2000 mg.

In a preferred embodiment, the packaging according to the invention does not comprise any administration unit comprising an active pharmaceutical ingredient other than (i) {drug1}, (ii) {drug2}, (iii) {drug3}, (iv) {drug4}, (v) {drug5}, or (vi) {drug6}.

The invention also relates to a method for manufacturing an administration unit according to the invention or a packaging according to the invention, comprising the steps:
(a) providing a quantity of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} that exceeds the dose of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}. {drug3m}, {drug3n}, or {drug3o}; (iv) {drug4a}, {drug4b}, {drug4c}, {drug4d}, {drug4e}, {drug4f} , {drug4g}, or {drug4h}; (v) {drug5a}, {drug5b}, {drug5c}, {drug5d}, {drug5e}, or {drug5f}; or (vi) {drug6a} to be contained in a single administration unit by a factor of at least 10;
(b) mixing the quantity of (i) (drug1a); (ii) {drug2a}, {drug2b}, {drug2c}, {drug2d}, or {drug2e}; (iii) {drug3a}, {drug3b}, {drug3c}, {drug3d}, {drug3e}, {drug3f), {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} with one or more pharmaceutical excipients;
(c) dividing the mixture obtained in step (b) in fractions each containing the dose of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} to be contained in a single administration unit;
(d) optionally, forming the fractions obtained in step (c) to administration units; and
(e) optionally, packing the fractions obtained in step (c) and the administration units obtained in step (d), respectively. In a preferred embodiment of the method according to the invention, in step (a) the quantity of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} exceeds the dose of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug31}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} to be contained in a single administration unit by a factor of at least 25, or at least 50, or at least 100, or at least 250, or at least 500, or at least 1000, or at least 2500, or at least 5000, or at least 10,000.

The invention also relates to particles of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} having a particle size distribution X90 of about 500 µm or less, i.e. a particle size distribution (preferably by volume) such that at least 90 % of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} has a particle size smaller than 500 µm. Preferably, for (i) {drug1a}; (ii) {drug2a}, {drug2b}, {drug2c}, {drug2d}, or {drug2e}; (iii) {drug3a}, {drug3b}, {drug3c}, (drug3d), {drug3e}, {drug3f}, {drug3g}, {drug3h}, {drug3i}, {drug3j}. {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} the particle size distribution of X90 is about 480 µm or less, or about 460 µm or less, or about 440 µm or less, or about 420 µm or less, or about 400 µm or less, or about 380 µm or less, or about 360 µm or less, or about 340 µm or less, or about 320 µm or less, or about 300 µm or less, or about 280 µm or less, or about 260 µm or less, or about 240 µm or less, or about 220 µm or less, or about 200 µm or less, or about 180 µm or less, or about 160 µm or less, or about 140 µm or less, or about 120 µm or less, or about 100 µm or less, or about 80 µm or less, or about 60 µm or less, or about 40 µm or less, or about 20 µm or less.

In a preferred embodiment, the particles of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} according to the invention have a particle size distribution X50 of about 400 µm or less, i.e. a particle size distribution (preferably by volume) such that at least 50 % of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, (drug3m), (drug3n), or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} has a particle size smaller than 400 µm. Preferably, for (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} the particle size distribution of X50 is about 380 µm or less, or about 360 µm or less, or about 340 µm or less, or about 320 µm or less, or about 300 µm or less, or about 280 µm or less, or about 260 µm or less, or about 240 µm or less, or about 220 µm or less, or about 200 µm or less, or about 180 µm or less, or about 160 µm or less, or about 140 µm or less, or about 120 µm or less, or about 100 µm or less, or about 80 µm or less, or about 70 µm or less, or about 60 µm or less, or about 50 µm or less. Preferably, for (i) {drug1a}; (ii) {drug2a}, {drug2b}, {drug2c}, {drug2d}, or {drug2e}; (iii) {drug3a}, {drug3b}, {drug3c}, {dmg3d}, {drug3e}, {drug3f}, {drug3g}, {drug3h}, {drug3i}, {drug3j}, {drug3k}, {drug31}, {drug3m}, {drug3n}, or {drug3o}; (iv) {drug4a}, {drug4b}, (drug4c), {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} the particle size distribution of X50 is about 45 µm or less, or about 40 µm or less, or about 35 µm or less, or about 30 µm or less, or about 25 µm or less, or about 20 µm or less, or about 15 µm or less, or about 10 µm or less.

In preferred embodiments, the particles of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) (drug4a), {drug4b}, (drug4c), {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} have a cumulative distribution by volume basis Q3(X) (X in µm) of Q3(355) <0.50; or Q3(180) <0.50 and Q3(355) ≥0.50; or Q3(125) <0.50 and Q3(180) ≥0.50; or Q3(125) ≥0.50.

In preferred embodiments, the particles of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} have a cumulative distribution by volume basis Q3(X) (X in µm) of Q3(268) <0.50 and Q3(355) ≥0.50; or Q3(180) <0.50 and Q3(268) ≥0.50; or Q3(153) <0.50 and Q3(180) ≥0.50; or Q3(125) <0.50 and Q3(153) ≥0.50; or Q3(111) <0.50 and Q3(125) ≥0.50; or Q3(98) <0.50 and Q3(111) ≥0.50; or Q3(98) ≥0.50.

In preferred embodiments, the particles of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug30}; (iv) {drug4a}, {drug4b}, {drug4c}, {drug4d}, {drug4e}, {drug4f}, {drug4g}, or {drug4h}; (v) {drug5a}, {drug5b}, {dnig5c}, {drug5d}, {drug5e}, or {drug5f}; or (vi) {drug6a} have a cumulative distribution by volume basis Q3(X) (X in µm) of Q3(312) <0.50 and Q3(355) ≥0.50; or Q3(268) <0.50 and Q3(312) ≥0.50; or Q3(224) <0.50 and Q3(268) ≥0.50; or Q3(180) <0.50 and Q3(224) ≥0.50; or Q3(167) <0.50 and Q3(180) ≥0.50; or Q3(153) <0.50 and Q3(167) ≥0.50; or Q3(139) <0.50 and Q3(153) ≥0.50; or Q3(125) <0.50 and Q3(139) ≥0.50; or Q3(111) <0.50 and Q3(125) ≥0.50; or Q3(98) <0.50 and Q3(111) ≥0.50; or Q3(84) <0.50 and Q3(98) ≥0.50; or Q3(84) ≥0.50.

The indicated particle size properties are determined by laser-diffraction method, in particular low angle laser light scattering, i.e. Fraunhofer diffraction. Alternatively, the particle size properties can be also determined by microscopy (e.g. electron microscopy or scanning electron microscopy). The powder fineness is preferably determined in accordance with USP35 <811>. The results of the particle size distribution determined by different techniques can be correlated with one another.

Suitable milling and grinding techniques that are suitable for obtaining a specific particle size distribution are known to the skilled artisan (see e.g. N. Rasenack, B.W. Müller, Micron-size drug particles: common and novel micronization techniques, Pharm Dev Technol., 2004, 9(1):1-13; A. Martin, M.J. Cocero, Micronization processes with supercritical fluids: fundamentals and mechanisms, Adv Drug Deliv Rev. 2008, 60(3):339-50; S.C. Gad, Pharmaceutical Manufacturing Handbook: Production and Processes (Pharmaceutical Development Series), Wiley Interscience, 1st ed. 2008; A.J. Hickey, Pharmaceutical Process Engineering (Drugs and the Pharmaceutical Sciences), Informa Healthcare, 2nd ed. 2009; B. Nickerson, Sample Preparation of Pharmaceutical Dosage Forms: Challenges and Strategies for Sample Preparation and Extraction, Springer, 1st ed. 2011; D. Dragoman, Optical Characteriaztion of Solids, Springer, 1st ed. 2010; and D. Schulze; Powders and Bulk Solids: Behavior, Characterization, Storage and Flow, Springer, 2008).

### PREFERRED EMBODIMENTS OF THE INVENTION ACCORDING TO ITEMS 1 TO 137

1. An administration unit comprising (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug31}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or(vi) {drug6a}.
2. The administration unit according to item 1, comprising a therapeutically effective amount of (i) {drug1}, (ii) {drug2}, (iii) {drug3}, (iv) {drug4}, (v) {drug5}, or (vi) {drug6}, wherein at least 10 % by weight of said therapeutically effective amount of (i) {drug1}, (ii) {drug2}, (iii) {drug3}, (iv) {drug4}, (v) {drug5}, or (vi) {drug6} are present as (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {dmg3k}, {drug3l}, {drug3m}, {drug3n}, or {drug30}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or(vi) {drug6a}.
3. The administration unit according to item 2, wherein at least 20 % by weight, or at least 30 % by weight, or at least 40 % by weight, or at least 50 % by weight, or at least 60 % by weight, or at least 70 % by weight, or at least 80 % by weight, or at least 90 % by weight of said therapeutically effective amount of (i) {drug1}, (ii) {drug2}, (iii) {drug3}, (iv) {drug4}, (v) {drug5}, or (vi) {drug6} are present as (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}.
4. The administration unit according to any of items 2 to 3, wherein at least 95 % by weight, or at least 96 % by weight, or at least 97 % by weight, or at least 98 % by weight, or at least 99 % by weight, or at least 99.5 % by weight, or at least 99.8 % by weight, or at least 99.9 % by weight of said therapeutically effective amount of (i) {drug1}, (ii) {drug2}, (iii) {drug3}, (iv) {drug4}, (v) {drug5}, or (vi) {drug6} are present as (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or(vi) {drug6a}.
5. The administration unit according to any of items 2 to 4, wherein not more than 90 % by weight of said therapeutically effective amount of (i) {drug1}, (ii) {drug2}, (iii) {drug3}, (iv) {drug4}, (v) {drug5}, or (vi) {drug6} are amorphous.
6. The administration unit according to item 5, wherein not more than 80 % by weight, or not more than 70 % by weight, or not more than 60 % by weight, or not more than 50 % by weight, or not more than 40 % by weight, or not more than 30 % by weight, or not more than 20 % by weight, or not more than 10 % by weight of said therapeutically effective amount of (i) {drug1}, (ii) {drug2}, (iii) {drug3}, (iv) {drug4}, (v) {drug5}, or (vi) {drug6} are amorphous.
7. The administration unit according to item 5 or 6, wherein not more than 5 % by weight, or not more than 4 % by weight, or not more than 3 % by weight, or not more than 2 % by weight, or not more than 1 % by weight, or not more than 0.5 % by weight, or not more than 0.2 % by weight, or not more than 0.1 % by weight of said therapeutically effective amount of (i) {drug1}, (ii) {drug2}, (iii) {drug3}, (iv) {drug4}, (v) {drug5}, or (vi) {drug6} are amorphous.
8. The administration unit according to any of items 2 to 7, wherein not more than 90 % by weight of said therapeutically effective amount of (i) {drug1}, (ii) {drug2}, (iii) {drug3}, (iv) {drug4}, (v) {drug5}, or (vi) {drug6} are polymorphs of (i) {drug1}, (ii) {drug2}, (iii) {drug3}, (iv) {drug4}, (v) {drug5}, or (vi) {drug6} other than (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or(vi) {drug6a}.
9. The administration unit according to item 8, wherein not more than 80 % by weight, or not more than 70 % by weight, or not more than 60 % by weight, or not more than 50 % by weight, or not more than 40 % by weight, or not more than 30 % by weight, or not more than 20 % by weight, or not more than 10 % by weight of said therapeutically effective amount of (i) {drug1}, (ii) {drug2}, (iii) {drug3}, (iv) {drug4}, (v) {drug5}, or (vi) {drug6} are polymorphs of (i) {drug1}, (ii) {drug2}, (iii) {drug3}, (iv) {drug4}, (v) {drug5}, or (vi) {drug6} other than (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}.
10. The administration unit according to item 8 or 9, wherein not more than 5 % by weight, or not more than 4 % by weight, or not more than 3 % by weight, or not more than 2 % by weight, or not more than 1 % by weight, or not more than 0.5 % by weight, or not more than 0.2 % by weight, or not more than 0.1 % by weight of said therapeutically effective amount of (i) {drug1}, (ii) {drug2}, (iii) {drug3}, (iv) {drug4}, (v) {drug5}, or (vi) {drug6} are polymorphs of (i) {drug1}, (ii) {drug2}, (iii) {drug3}, (iv) {drug4}, (v) {drug5}, or (vi) {drug6} other than (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug30}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}.
11. The administration unit according to any of items 2 to 10, wherein therapeutically effective amount of (i) {drug1}, (ii) {drug2}, (iii) {drug3}, (iv) {drug4}, (v) {drug5}, or (vi) {drug6} is 0.1 µg to 2 g of (i) {drug1}, (ii) {drug2}, (iii) {drug3}, (iv) {drug4}, (v) {drug5}, or (vi) {drug6}.
12. The administration unit according to item 11, wherein therapeutically effective amount of (i) {drug1}, (ii) {drug2}, (iii) {drug3}, (iv) {drug4}, (v) {drug5}, or (vi) {drug6} is 1 µg to 2.5 µg of (i) {drug1}, (ii) {drug2}, (iii) {drug3}, (iv) {drug4}, (v) {drug5}, or (vi) {drug6}, or 2.5 µg to 5 µg of (i) {drug1}, (ii) {drug2}, (iii) {drug3}, (iv) {drug4}, (v) {drug5}, or(vi) {drug6}, or 5 µg to 10 µg of (i) {drug1}, (ii) {drug2}, (iii) {drug3}, (iv) {drug4}, (v) {drug5}, or (vi) {drug6}, or 10 µg to 25 µg of (i) {drug1}, (ii) {drug2}, (iii) {drug3}, (iv) {drug4}, (v) {drug5}, or (vi) {drug6}, or 25 µg to 50 µg of (i) {drug1}, (ii) {drug2}, (iii) {drug3}, (iv) {drug4}, (v) {drug5}, or (vi) {drug6}, or 50 µg to 100 µg of (i) {drug1}, (ii) {drug2}, (iii) {drug3}, (iv) {drug4}, (v) {drug5}, or (vi) {drug6}, or 100 µg to 250 µg of (i) {drug1}, (ii) {drug2}, (iii) {drug3}, (iv) {drug4}, (v) {drug5}, or (vi) {drug6}, or 250 µg to 500 µg of (i) {drug1}, (ii) {drug2}, (iii) {drug3}, (iv) {drug4}, (v) {drug5}, or (vi) {drug6}, or 500 µg to 1 mg of (i) {drug1}, (ii) {drug2}, (iii) {drug3}, (iv) {drug4}, (v) {drug5}, or (vi) {drug6}, or 1 mg to 2.5 mg of (i) {drug1}, (ii) {drug2}, (iii) {drug3}, (iv) {drug4}, (v) {drug5}, or (vi) {drug6}, or 2.5 mg to 5 mg of (i) {drug1}, (ii) {drug2}, (iii) {drug3}, (iv) {drug4}, (v) {drug5}, or (vi) {drug6}, or 5 mg to 10 mg of (i) {drug1}, (ii) {drug2}, (iii) {drug3}, (iv) {drug4}, (v) {drug5}, or (vi) {drug6}, or 10 mg to 25 mg of (i) {drug1}, (ii) {drug2}, (iii) {drug3}, (iv) {drug4}, (v) {drug5}, or (vi) {drug6}, or 25 mg to 50 mg of (i) {drug1}, (ii) {drug2}, (iii) {drug3}, (iv) {drug4}, (v) {drug5}, or (vi) {drug6}, or 50 mg to 100 mg of (i) {drug1}, (ii) {drug2}, (iii) {drug3}, (iv) {drug4}, (v) {drug5}, or (vi) {drug6}, or 100 mg to 250 mg of (i) {drug1}, (ii) {drug2}, (iii) {drug3}, (iv) {drug4}, (v) {drug5}, or (vi) {drug6}, or 250 mg to 500 mg of (i) {drug1}, (ii) {drug2}, (iii) {drug3}, (iv) {drug4}, (v) {drug5}, or (vi) {drug6}, or 500 mg to 1 g of (i) {drug1}, (ii) {drug2}, (iii) {drug3}, (iv) {drug4}, (v) {drug5}, or (vi) {drug6}, or 1 g to 1.25 g of (i) {drug1}, (ii) {drug2}, (iii) {drug3}, (iv) {drug4}, (v) {drug5}, or (vi) {drug6}, or 1.25 g to 1.5 g of (i) {drug1}, (ii) {drug2}, (iii) {drug3}, (iv) {drug4}, (v) {drug5}, or (vi) {drug6}, or 1.5 g to 1.75 g of (i) {drug1}, (ii) {drug2}, (iii) {drug3}, (iv) {drug4}, (v) {drug5}, or (vi) {drug6}, or 1.75 g to 2 g of (i) {drug1}, (ii) {drug2}, (iii) {drug3}, (iv) {drug4}, (v) {drug5}, or (vi) {drug6}.
13. The administration unit according to any of items 1 to 12, comprising 0.1 µg to 2 g of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}.
14. The administration unit according to item 13, comprising 1 µg to 2.5 µg of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug31}, {drug3m}, {drug3n}, or {drug30}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 2.5 µg to 5 µg of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 5 µg to 10 µg of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 10 µg to 25 µg of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug30}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or(vi) {drug6a}, or 25 µg to 50 µg of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 50 µg to 100 µg of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or(vi) {drug6a}, or 100 µg to 250 µg of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 250 µg to 500 µg of (i) {drug1a}; (ii) {drug2a}, {drug2b}, {drug2c}, {drug2d}, or {drug2e}; (iii) {drug3a}, {drug3b}, {drug3c}, {drug3d}, (drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 500 µg to 1 mg of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 1 mg to 2.5 mg of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 2.5 mg to 5 mg of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 5 mg to 10 mg of (i) {drug1a}; (ii) {drug2a}, {drug2b}, {drug2c}, {drug2d}, or {drug2e}; (iii) {drug3a}, {drug3b}, {drug3e}, {drug3d}, {drug3e}, {drug3f}, {drug3g}, {drug3h}, {drug3i}, {drug3j}, {drug3k}, {drug3l}, {drug3m}, {drug3n}, or {drug30}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 10 mg to 25 mg of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 25 mg to 50 mg of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, (drug3l), {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 50 mg to 100 mg of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 100 mg to 250 mg of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 250 mg to 500 mg of (i) {drugla}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 500 mg to 1 g of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 1 g to 1.25 g of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug30}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 1.25 g to 1.5 g of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, (drug3m), {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, or 1.5 g to 1.75 g of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or(vi) {drug6a}, or 1.75 g to 2 g of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug30}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}.
15. The administration unit according to any of items 1 to 14, which is solid, semisolid or liquid.
16. The administration unit according to any of items 1 to 15, which is for administration once daily, or twice daily, or thrice daily, or four times daily, or five times daily, or six times daily.
17. The administration unit according to any of items 1 to 16, which is monolithic or multiparticulate.
18. The administration unit according to any of items 1 to 17, wherein (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} is homogeneously distributed over the administration unit.
19. The administration unit according to any of items 1 to 17, wherein (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} is not homogeneously distributed over the administration unit.
20. The administration unit according to any of items 1 to 19, providing controlled release of (i) {drug1}, (ii) {drug2}, (iii) {drug3}, (iv) {drug4}, (v) {drug5}, or (vi) {drug6}.
21. The administration unit according to item 20, comprising a controlled release matrix or a controlled release coating.
22. The administration unit according to any of items 1 to 19, providing immediate release of (i) {drug1}, (ii) {drug2}, (iii) {drug3}, (iv) {drug4}, (v) {drug5}, or (vi) {drug6}.
23. The administration unit according to any of items 1 to 22, having a total weight of 10 mg to 3 g.
24. The administration unit according to item 23, having a total weight of 10 to 25 mg, or 25 mg to 50 mg, or 50 mg to 100 mg, or 100 mg to 250 mg, or 250 mg to 500 mg, or 500 mg to 1000 mg, or 1000 mg to 2500 mg.
25. The administration unit according to any of items 1 to 24, which is for systemic or topical administration.
26. The administration unit according to any of items 1 to 25, which is for parenteral administration.
27. The administration unit according to any of items 1 to 26, which is for oral, buccal, opthalmic, nasal, rectal, transmucosal, or intestinal administration.
28. The administration unit according to any of items 1 to 27, which is for oral administration.
29. The administration unit according to item 28, which is selected from the group consisting of tablets, capsules, effervescent tablets, orodispersible tablets, dragees, sachets, drops, suspensions, and powders.
30. The administration unit according to any of items 1 to 29, which is a tablet.
31. The administration unit according to item 30, which is round or oblong; and/or which is planar or biconvex.
32. The administration unit according to any of items 30 to 31, having been manufactured by direct compression.
33. The administration unit according to any of items 30 to 31, having been manufactured by granulation and subsequent compression of granules.
34. The administration unit according to item 33, wherein granulation is wet granulation or dry granulation.
35. The administration unit according to any of items 30 to 34, having been compacted at a pressure of 10 MPa to 10,000 MPa.
36. The administration unit according to item 35, having been compacted at a pressure of 10 MPa to 25 MPa, or 25 MPa to 50 MPa, or 50 MPa to 100 MPa, or 100 MPa to 250 MPa, or 250 MPa to 500 MPa, or 500 MPa to 1000 MPa, or 1000 MPa to 2500 MPa, or 2500 MPa to 5000 MPa, or 5000 MPa to 10,000 MPa.
37. The administration unit according to any of items 30 to 36, comprising a film coating.
38. The administration unit according to item 37, wherein the film coating is enteric.
39. The administration unit according to any of items 37 to 38, wherein the film coating comprises a cellulose ether (e.g. hydroxypropylmethyl cellulose) or a synthetic polymer (e.g. polyvinyl alcohol).
40. The administration unit according to any of items 37 to 39, wherein the film coating has an average thickness of 0.01 to 1000 µm.
41. The administration unit according to item 40, wherein the film coating has an average thickness of 0.01 µm to 0.03 µm, or 0.03 to 0.05 µm, or 0.05 to 0.1 µm, or 0.1 to 0.25 µm, or 0.25 to 0.5 µm, or 0.5 to 1.0 µm, or 1.0 µm to 2.5 µm, or 2.5 µm to 5.0 µm, or 5.0 µm to 10 µm, or 10 µm to 25 µm, or 25 µm to 50 µm, or 50 µm to 100 µm, or 100 µm to 250 µm, or 250 µm to 500 μm, or 500 μm to 1000 μm.
42. The administration unit according to any of items 30 to 41, having a tablet porosity of not more than 90 %.
43. The administration unit according to item 42, having a tablet porosity of not more than 80 %, or not more than 70 %, or not more than 60 %, or not more than 50 %, or not more than 40 %, or not more than 30 %, or not more than 20 %, or not more than 10 %.
44. The administration unit according to item 42 to 43, having a tablet porosity of not more than 5 %, or not more than 4 %, or not more than 3 %, or not more than 2 %, or not more than 1 %, or not more than 0.5 %, or not more than 0.2 %, or not more than 0.1 %.
45. The administration unit according to any of items 30 to 44, having a radial tensile strength of 0.1 to 100 MPa.
46. The administration unit according to item 45, having a radial tensile strength of 0.1 MPa to 0.3 MPa, or 0.3 MPa to 0.5 MPa, or 0.5 MPa to 1.0 MPa, or 1.0 MPa to 2.5 MPa, or 2.5 MPa to 5.0 MPa, or 5.0 MPa to 10 MPa, or 10 MPa to 25 MPa, or 25 MPa to 50 MPa, or 50 MPa to 100 MPa.
47. The administration unit according to any of items 30 to 46, having an axial tensile strength of 0.1 to 100 MPa.
48. The administration unit according to item 47, having an axial tensile strength of 0.1 MPa to 0.3 MPa, or 0.3 MPa to 0.5 MPa, or 0.5 MPa to 1.0 MPa, or 1.0 MPa to 2.5 MPa, or 2.5 MPa to 5.0 MPa, or 5.0 MPa to 10 MPa, or 10 MPa to 25 MPa, or 25 MPa to 50 MPa, or 50 MPa to 100 MPa.
49. The administration unit according to any of items 30 to 48, having an average pore size of 0.001 μm to 1000 μm.
50. The administration unit according to item 49, having an average pore size of 0.001 μm to 0.003 μm, or 0,003 μm to 0.005 μm, or 0.005 μm to 0.01 μm, or 0.01 μm to 0.025 μm, or 0.025 μm to 0.05 μm, or 0.05 μm to 0.1 μm, or 0.1 μm to 0.25 μm, or 0.25 μm to 0.5 μm, or 0.5 μm to 1 μm, or 1 μm to 2.5 μm, or 2.5 μm to 5 μm, or 5 μm to 10 μm, or 10 μm to 25 μm, or 25 μm to 50 μm, or 50 μm to 100 μm, or 100 μm to 250 μm, or 250 μm to 500 μm, or 500 μm to 1000 μm.
51. The administration unit according to any of items 30 to 50, having a pore size distribution such that at least 10 % of the pores have a pore size of not more than 0.1 μm.
52. The administration unit according to item 51, having a pore size distribution such that at least 20 %, or at least 30 %, or at least 40 %, or at least 50 %, or at least 60 %, or at least 70 %, or at least 80 %, or at least 90 % of the pores have a pore size of not more than 0.1 μm.
53. The administration unit according to any of items 30 to 52, having a pore size distribution such that at least 10 % of the pores have a pore size of not less than 0.1 μm.
54. The administration unit according to item 53, having a pore size distribution such that at least 20 %, or at least 30 %, or at least 40 %, or at least 50 %, or at least 60 %, or at least 70 %, or at least 80 %, or at least 90 % of the pores have a pore size of not less than 0.1 μm.
55. The administration unit according to any of items 30 to 54, having a pore size distribution such that at least 10 % of the pores have a pore size of not more than 1 μm.
56. The administration unit according to item 55, having a pore size distribution such that at least 20 %, or at least 30 %, or at least 40 %, or at least 50 %, or at least 60 %, or at least 70 %, or at least 80 %, or at least 90 % of the pores have a pore size of not more than 1 μm.
57. The administration unit according to any of items 30 to 56, having a pore size distribution such that at least 10 % of the pores have a pore size of not less than 1 μm.
58. The administration unit according to item 57, having a pore size distribution such that at least 20 %, or at least 30 %, or at least 40 %, or at least 50 %, or at least 60 %, or at least 70 %, or at least 80 %, or at least 90 % of the pores have a pore size of not less than 1 µm.
59. The administration unit according to any of items 30 to 58, having a pore size distribution such that at least 10 % of the pores have a pore size of not more than 10 µm.
60. The administration unit according to item 59, having a pore size distribution such that at least 20 %, or at least 30 %, or at least 40 %, or at least 50 %, or at least 60 %, or at least 70 %, or at least 80 %, or at least 90 % of the pores have a pore size of not more than 10 µm.
61. The administration unit according to any of items 30 to 60, having a pore size distribution such that at least 10 % of the pores have a pore size of not less than 10 µm.
62. The administration unit according to item 61, having a pore size distribution such that at least 20 %, or at least 30 %, or at least 40 %, or at least 50 %, or at least 60 %, or at least 70 %, or at least 80 %, or at least 90 % of the pores have a pore size of not less than 10 µm.
63. The administration unit according to any of items 30 to 62, having a water content of not more than 5 % by weight, relative to the total weight of the tablet.
64. The administration unit according to item 63, having a water content of not more than 4 % by weight, or not more than 3 % by weight, or not more than 2 % by weight, or not more than 1 % by weight, or not more than 0.5 % by weight, or not more than 0.1 % by weight, relative to the total weight of the tablet.
65. The administration unit according to any of items 30 to 64, having a true density of 0.60 to 1.40 g cm-3.
66. The administration unit according to item 65, having a true density of 0.60 to 0.65 g cm-3, or 0.65 to 0.70 g cm-3, or 0.70 to 0.75 g cm-3, or 0.75 to 0.80 g cm-3, or 0.80 to 0.85 g cm-3, or 0.85 to 0.90 g cm-3, or 0.90 to 0.95 g cm-3, or 0.95 to 1.00 g cm-3, or 1.00 to 1.05 g cm-3, or 1.05 to 1.10 g cm-3, or 1.10 to 1.15 g cm-3, or 1.15 to 1.20 g cm-3, or 1.20 to 1.25 g cm-3, or 1.25 to 1.30 g cm-3, or 1.30 to 1.35 g cm-3, or 1.35 to 1.40 g cm-3.
67. The administration unit according to any of items 30 to 66, having an apparent density of 0.60 to 1.40 g cm-3.
68. The administration unit according to item 67, having an apparent density of 0.60 to 0.65 g cm-3, or 0.65 to 0.70 g cm-3, or 0.70 to 0.75 g cm-3, or 0.75 to 0.80 g cm-3, or 0.80 to 0.85 g cm-3, or 0.85 to 0.90 g cm-3, or 0.90 to 0.95 g cm-3, or 0.95 to 1.00 g cm-3, or 1.00 to 1.05 g cm-3, or 1.05 to 1.10 g cm-3, or 1.10 to 1.15 g cm-3, or 1.15 to 1.20 g cm-3, or 1.20 to 1.25 g cm-3, or 1.25 to 1.30 g cm-3, or 1.30 to 1.35 g cm-3, or 1.35 to 1.40 g cm-3.
69. The administration unit according to any of items 30 to 68, disintegrating within 6000 seconds.
70. The administration unit according to item 69, disintegrating within 5500 seconds, or within 5000 seconds, or within 4500 seconds, or within 4000 seconds, or within 3500 seconds, or within 3000 seconds, or within 2500 seconds, or within 2000 seconds, or within 1500 seconds, or within 1000 seconds, or within 750 seconds, or within 500 seconds, or within 250 seconds.
71. The administration unit according to any of items 1 to 29, which is a capsule.
72. The administration unit according to item 71, comprising a multitude of particulates comprising (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}.
73. The administration unit according to any of items 71 to 72, comprising at least 2 particulates, or at least 3 particulates, or at least 4 particulates, or at least 5 particulates, or at least 6 particulates, or at least 7 particulates, or at least 8 particulates, or at least 9 particulates, or at least 10 particulates.
74. The administration unit according to any of items 71 to 73, wherein the capsule material comprises hard gelatine.
75. The administration unit according to any of items 71 to 74, comprising at least 2 different types of particulates that differ from each other in at least one property selected from the group consisting of nature of excipients, content of excipients, content of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, content of (i) {drug1}, (ii) {drug2}, (iii) {drug3}, (iv) {drug4}, (v) {drug5}, or (vi) {drug6}, water content, total weight, size, and shape.
76. The administration unit according to any of items 1 to 75, passing the storage stability test according to Ph. Eur. under accelerated storage conditions at 40°C and 75 % rel. humidity.
77. The administration unit according to item 76, having a storage stability for at least 3 months, or at least 4 months, or at least 5 months, or at least 6 months, or at least 7 months, or at least 8 months, or at least 9 months, or at least 10 months, or at least 11 months, or at least 12 months according to Ph. Eur. under accelerated storage conditions at 40°C and 75 % rel. humidity.
78. The administration unit according to any of items 1 to 77, providing the peak plasma level within 0.1 hour to 36 hours after administration.
79. The administration unit according to item 78, providing the peak plasma level within 0.1 hour to 1 hour, or 1 hour to 2 hours, or 2 hours to 3 hours, or 3 hours to 4 hours, or 4 hours to 5 hours, or 5 hours to 6 hours, or 6 hours to 7 hours, or 7 hours to 8 hours, or 8 hours to 9 hours, or 9 hours to 10 hours, or 10 hours to 11 hours, or 11 hours to 12 hours, or 12 hours to 13 hours, or 13 hours to 14 hours, or 14 hours to 15 hours, or 15 hours to 16 hours, or 16 hours to 17 hours, or 17 hours to 18 hours, or 18 hours to 19 hours, or 19 hours to 20 hours, or 20 hours to 21 hours, or 21 hours to 22 hours, or 22 hours to 23 hours, or 23 hours to 24 hours, after administration.
80. The administration unit according to any of items 1 to 79, comprising one or more excipients independently selected from the group consisting of antiadherents, binders, disintegrants, fillers, flavors, colors, lubricants, glidants, sorbents, surfactants, preservatives and sweeteners.
81. The administration unit according to item 80, wherein the antiadherent is selected from the group consisting of silicon dioxide, talc, talc waxes, stearates (e.g. magnesium, calcium or sodium), stearic acid, stearowet, boric acid, sodium chloride, DL-leucine, sodium oleate, sodium benzoate, sodium acetate, sodium lauryl sulfate, and magnesium lauryl sulfate.
82. The administration unit according to any of items 80 to 81, wherein the binder is selected from the group consisting of saccharides and their derivatives; proteins; gums; silicates; and synthetic polymers.
83. The administration unit according to item 82, wherein the binder is a saccharide or a derivative thereof selected from the group consisting of disaccharides (e.g. sucrose, glucose or lactose); polysaccharides and their derivatives (e.g. starch, pregelatinized starch, cellulose, microcrystalline cellulose, polysaccharide acids, cellulose ethers [e.g. methyl cellulose, ethyl cellulose, hydroxyethyl cellulose, hydroxypropyl cellulose, hydroxypropylmethyl cellulose or carboxymethylcellulose sodium]); alginic acid and salts thereof (e.g. sodium alginate); and sugar alcohols (e.g. xylitol, sorbitol or maltitol); or wherein the binder is a protein selected from gelatin; or wherein the binder is a gum (e.g. acacia gum, guar gum, or tragacanth gum); or wherein the binder is a silicate (e.g. magnesium aluminum silicate or bentonite); or wherein the binder is a synthetic polymer selected from the group consisting of polyvinylpyrrolidone, polyvinylpyrrolidone-vinylacetate copolymer, polymethacrylate polymer, polyvinylalcohol, and polyethylene glycol.
84. The administration unit according to any of items 80 to 83, wherein the disintegrant is selected from the group consisting of cross-linked polymers (e.g. cross-linked polyvinylpyrrolidone [crospovidone] or cross-linked sodium carboxymethyl cellulose [croscarmellose sodium]), starches (e.g. corn or potato), pregelatinized starch, modified starch (e.g. sodium starch glycolate), algenic acid, alginates (e.g. sodium alginate), carboxymethylcellulose, microcrystalline cellulose, clays (e.g. magnesium aluminum silicate), and gums (e.g. agar, guar, locust bean, karaya, pectin, or tragacanth gum).
85. The administration unit according to any of items 80 to 84, wherein the filler is selected from the group consisting of plant cellulose, microcrystalline cellulose, inorganic phosphates (e.g. dibasic calcium phosphate or tricalcium phosphate), inorganic carbonates such as calcium carbonate, inorganic sulfates such as calcium sulfate dihydrate, sugars and sugar alcohols (e.g. lactose, glucose, inositol, mannitol, sorbitol, sucrose, dextrose, maltodextrins, and fructose), magnesium stearate, calcium lactate trihydrate, starch (e.g. corn, wheat, maize, potato or rice starch), and modified starch (e.g. carboxymethyl starch).
86. The administration unit according to any of items 80 to 85, wherein the lubricant is hydrophilic or hydrophobic.
87. The administration unit according to item 86, wherein the lubricant is selected from the group consisting of talc, silica, stearin, fatty acids (e.g. stearic acid) or fatty acid derivatives, metal stearates (e.g. magnesium stearate, calcium stearate, or zinc stearate), glyceryl monostearate, glyceryl palmitostearate, glyceryl behenate, sodium lauryl sulfate, magnesium lauryl sulfate, sodium stearyl fumarate, hydrogenated vegetable oil, polyalkylene glycols (e.g. polyethylene glycol), starch, light mineral oil, sodium benzoate, and sodium chloride.
88. The administration unit according to any of items 80 to 87, wherein the glidant is selected from the group consisting of fumed silica, cornstarch, talc, and magnesium carbonate.
89. The administration unit according to any of items 80 to 88, wherein the preservative is selected from the group consisting of antioxidants (e.g. vitamin A, vitamin C, vitamin E, retinyl palmitate, selenium, ascorbyl palmitate, sodium ascorbate), amino acids (e.g. cysteine or methionine), citric acid and its salts (e.g. sodium citrate), synthetic preservatives (e.g. parabens such as methyl paraben or propyl paraben; butylated hydroxyanisole, butylated hydroxytoluene, monothioglycerol, propyl gallate), hypophosphorous acid, sodium bisulfite, sodium formaldehyde sulfoxylate, and sodium metabisulfite.
90. The administration unit according to any of items 1 to 89, passing the friability test according to Ph. Eur.
91. The administration unit according to item 90, wherein the weight loss in the friability test according to Ph. Eur. is not more than 2.0 % by weight, or not more than 1.5 % by weight, or not more than 1.0 % by weight, or not more than 0.5 % by weight, or not more than 0.4 % by weight, or not more than 0.3 % by weight, or not more than 0.2 % by weight, or not more than 0.1 % by weight.
92. The administration unit according to any of items 1 to 91, passing the test uniformity of content of single-dose preparations according to Ph. Eur.
93. The administration unit according to any of items 1 to 92, not comprising any active pharmaceutical ingredient other than (i) {drug1}, (ii) {drug2}, (iii) {drug3}, (iv) {drug4}, (v) {drug5}, or(vi) {drug6}.
94. The administration unit according to any of items 1 to 93, which is for storage not above 50 °C.
95. The administration unit according to item 94, which is for storage not above 25 °C.
96. The administration unit according to any of items 1 to 95, with the proviso that the administration unit is not identical with any of the administration units that are individualized in the experimental section of WO2013/072327, WO2013/076177, WO2013/076178, WO2013/076179, WO2013/083553, and WO2013/084150, respectively.
97. The administration unit according to any of items 1 to 96 for use in the treatment of a disorder or a disease in a mammal.
98. The administration unit according to item 97, wherein the mammal is a human.
99. The administration unit according to any of items 97 to 98, wherein the mammal is an adult.
100. The administration unit according to any of items 97 to 99 wherein the mammal does not suffer from liver damage.
101. The administration unit according to any of items 97 to 100, wherein the mammal does not suffer from kidney damage.
102. The administration unit according to any of items 97 to 101, wherein the mammal is not pregnant.
103. The administration unit according to any of items 97 to 102, wherein the mammal is not allergic against (i) {drug1}, (ii) {drug2}, (iii) {drug3}, (iv) {drug4}, (v) {drug5}, or(vi) {drug6}.
104. The administration unit according to any of items 97 to 103, which is administered orally with a liquid.
105. The administration unit according to item 104, wherein the liquid is water, milk, juice or lemonade.
106. The administration unit according to any of items 97 to 105, wherein the administered daily dose of (i) {drug1}, (ii) {drug2}, (iii) {drug3}, (iv) {drug4}, (v) {drug5}, or (vi) {drug6} is 0.0001 mg/kg body weight to 100 mg/kg body weight.
107. The administration unit according to item 106, wherein the administered daily dose of (i) {drug 1}, (ii) {drug2}, (iii) {drug3}, (iv) {drug4}, (v) {drug5}, or (vi) {drug6} is 0.0001 mg/kg body weight to 0.0003 mg/kg body weight, or 0.0003 mg/kg body weight to 0.0005 mg/kg body weight, or 0.0005 mg/kg body weight to 0.001 mg/kg body weight, or 0.001 mg/kg body weight to 0.0025 mg/kg body weight, or 0.0025 mg/kg body weight to 0.005 mg/kg body weight, or 0.005 mg/kg body weight to 0.01 mg/kg body weight, or 0.01 mg/kg body weight to 0.025 mg/kg body weight, or 0.025 mg/kg body weight to 0.05 mg/kg body weight, or 0.05 mg/kg body weight to 0.1 mg/kg body weight, or 0.1 mg/kg body weight to 0.25 mg/kg body weight, or 0.25 mg/kg body weight to 0.5 mg/kg body weight, or 0.5 mg/kg body weight to 1 mg/kg body weight, or 1 mg/kg body weight to 2.5 mg/kg body weight, or 2.5 mg/kg body weight to 5 mg/kg body weight, or 5 mg/kg body weight to 10 mg/kg body weight, or 10 mg/kg body weight to 25 mg/kg body weight, or 25 mg/kg body weight to 50 mg/kg body weight, or 50 mg/kg body weight to 100 mg/kg body weight.
108. The administration unit according to any of items 97 to 107, wherein the administered daily dose of (i) {drug1}, (ii) {drug2}, (iii) {drug3}, (iv) {drug4}, (v) {drug5}, or (vi) {drug6} is 0.001 mg to 5000 mg.
109. The administration unit according to item 108, wherein the administered daily dose of (i) {drug1}, (ii) {drug2}, (iii) {drug3}, (iv) {drug4}, (v) {drug5}, or (vi) {drug6} is 0.001 mg to 0.003 mg, or 0.003 mg to 0.005 mg, or 0.005 mg to 0.01 mg, or 0.01 mg to 0.025 mg, or 0.025 mg to 0.05 mg, or 0.05 mg to 0.1 mg, or 0.1 mg to 0.25 mg, or 0.25 mg to 0.5 mg, or 0.5 mg to 1 mg, or 1 mg to 2.5 mg, or 2.5 mg to 5 mg, or 5 mg to 10 mg, or 10 mg to 25 mg, or 25 mg to 50 mg, or 50 mg to 100 mg, or 100 mg to 250 mg, or 250 mg to 500 mg, or 500 mg to 1000 mg, or 1000 mg to 1250 mg, or 1250 mg to 1500 mg, or 1500 mg to 1750 mg, or 1750 mg to 1999 mg.
110. A packaging comprising one or more administration units according to any of items 1 to 109.
111. The packaging according to item 110, comprising a material selected from the group consisting of paper, cardboard, paperboard, metal foil and plastic foil.
112. The packaging according to any of items 110 to 111, which is a blister packaging.
113. The packaging according to any of items 110 to 112, comprising a multitude of at least 2, or at least 3, or at least 4, or at least 5, or at least 6, or at least 7, or at least 8, or at least 9, or at least 10 administration units according to any of items 1 to 109.
114. The packaging according to item 113, wherein the administration units do not substantially differ in at least one property selected from the group consisting of nature of excipients, content of excipients, content of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a}, content of (i) {drug1}, (ii) {drug2}, (iii) {drug3}, (iv) {drug4}, (v) {drug5}, or (vi) {drug6}, water content, total weight, release profile, disintegration time, size, shape, porosity, apparent density, true density, average pore size, pore size distribution, axial tensile strength, radial tensile strength, friability, and storage stability.
115. The packaging according to item 114, wherein the administration units differ in said at least one property by not more than 5 %, or not more than 4 %, or not more than 3 %, or not more than 2 %, or not more than 1 %, or not more than 0.5 %, or not more than 0.1 %.
116. The packaging according to any of items 113 to 115, wherein all administration units are substantially identical.
117. The packaging according to any of items 113 to 116 for use in the treatment of a disorder or a disease, wherein one or more administration units of said multitude are administered on a first day, and one or more administration units of said multitude are administered on a second day following said first day.
118. The packaging according to item 117, wherein one or more administration units of said multitude are administered on one or more subsequent days following said second day.
119. The packaging according to any of items 117 to 118, wherein the time interval between the administration of an administration unit and the administration of a subsequent administration unit is not more than 48 hours.
120. The packaging according to item 119, wherein the time interval is not more than 36 hours, or not more than 24 hours, or not more than 12 hours, or not more than 8 hours, or not more than 6 hours, or not more than 4 hours.
121. The packaging according to any of items 117 to 120, wherein the administration units are administered on not more than 30 subsequent days.
122. The packaging according to item 121, wherein the administration units are administered on not more than 25 subsequent days, or on not more than 20 subsequent days, or on not more than 14 subsequent days, or on not more than 10 subsequent days, or on not more than 7 subsequent days, or on not more than 3 subsequent days.
123. The packaging according to any of items 117 to 122, wherein the administered daily dose of (i) {drug1} (ii) {drug2}, (iii) {drug3}, (iv) {drug4}, (v) {drug5}, or (vi) {drug6} is 0.0001 mg/kg body weight to 100 mg/kg body weight.
124. The packaging according to item 123, wherein the administered daily dose of (i) {drug1}, (ii) {drug2}, (iii) {drug3}, (iv) {drug4}, (v) {drug5}, or (vi) {drug6} is 0.0001 mg/kg body weight to 0.0003 mg/kg body weight, or 0.0003 mg/kg body weight to 0.0005 mg/kg body weight, or 0.0005 mg/kg body weight to 0.001 mg/kg body weight, or 0.001 mg/kg body weight to 0.0025 mg/kg body weight, or 0.0025 mg/kg body weight to 0.005 mg/kg body weight, or 0.005 mg/kg body weight to 0.01 mg/kg body weight, or 0.01 mg/kg body weight to 0.025 mg/kg body weight, or 0.025 mg/kg body weight to 0.05 mg/kg body weight, or 0.05 mg/kg body weight to 0.1 mg/kg body weight, or 0.1 mg/kg body weight to 0.25 mg/kg body weight, or 0.25 mg/kg body weight to 0.5 mg/kg body weight, or 0.5 mg/kg body weight to 1 mg/kg body weight, or 1 mg/kg body weight to 2.5 mg/kg body weight, or 2.5 mg/kg body weight to 5 mg/kg body weight, or 5 mg/kg body weight to 10 mg/kg body weight, or 10 mg/kg body weight to 25 mg/kg body weight, or 25 mg/kg body weight to 50 mg/kg body weight, or 50 mg/kg body weight to 100 mg/kg body weight.
125. The packaging according to any of items 117 to 124, wherein the administered daily dose of (i) {drug1}, (ii) {drug2}, (iii) {drug3}, (iv) {drug4}, (v) {drug5}, or (vi) {drug6} is 0.001 mg to 5000 mg.
126. The packaging according to item 125, wherein the administered daily dose of (i) {drug1}, (ii) {drug2}, (iii) {drug3}, (iv) {drug4}, (v) {drug5}, or (vi) {drug6} is 0.001 mg to 0.003 mg, or 0.003 mg to 0.005 mg, or 0.005 mg to 0.01 mg, or 0.01 mg to 0.025 mg, or 0.025 mg to 0.05 mg, or 0.05 mg to 0.1 mg, or 0.1 mg to 0.25 mg, or 0.25 mg to 0.5 mg, or 0.5 mg to 1 mg, or 1 mg to 2.5 mg, or 2.5 mg to 5 mg, or 5 mg to 10 mg, or 10 mg to 25 mg, or 25 mg to 50 mg, or 50 mg to 100 mg, or 100 mg to 250 mg, or 250 mg to 500 mg, or 500 mg to 1000 mg, or 1000 mg to 1250 mg, or 1250 mg to 1500 mg, or 1500 mg to 1750 mg, or 1750 mg to 2000 mg.
127. The packaging according to any of items 117 to 126, not comprising any administration unit comprising an active pharmaceutical ingredient other than (i) {drug1}, (ii) {drug2}, (iii) {drug3}, (iv) {drug4}, (v) {drug5}, or (vi) {drug6}.
128. A method for manufacturing an administration unit according to any of items 1 to 109 or a packaging according to any of items 110 to 127, comprising the steps: (a) providing a quantity of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} that exceeds the dose of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {dmg4h}; (v) {drug5a}, {drug5b}, {drug5c}, {drug5d}, {drug5e}, or {drug5f}; or (vi) {drug6a} to be contained in a single administration unit by a factor of at least 10; (b) mixing the quantity of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} with one or more pharmaceutical excipients; (c) dividing the mixture obtained in step (b) in fractions each containing the dose of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} to be contained in a single administration unit; (d) optionally, forming the fractions obtained in step (c) to administration units; and (e) optionally, packing the fractions obtained in step (c) and the administration units obtained in step (d), respectively.
129. The method according to item 133, wherein in step (a) the quantity of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} exceeds the dose of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} to be contained in a single administration unit by a factor of at least 25, or at least 50, or at least 100, or at least 250, or at least 500, or at least 1000, or at least 2500, or at least 5000, or at least 10,000.
130. Particles of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} having a particle size distribution X90 of 500 µm or less.
131. The particles of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} according to item 130, having a particle size distribution X90 of 480 µm or less, or 460 µm or less, or 440 µm or less, or 420 µm or less, or 400 µm or less, of 380 µm or less, or 360 µm or less, or 340 µm or less, or 320 µm or less, or 300 µm or less, of 280 µm or less, or 260 µm or less, or 240 µm or less, or 220 µm or less, or 200 µm or less, of 180 µm or less, or 160 µm or less, or 140 µm or less, or 120 µm or less, or 100 µm or less, or 80 µm or less, or 60 µm or less, or 40 µm or less, or 20 µm or less.
132. The particles of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drttg4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} according to any of items 130 to 131, having a particle size distribution X50 of 400 µm or less.
133. The particles of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} according to item 132, having a particle size distribution X50 of 380 or less, or 360 µm or less, or 340 µm or less, or 320 µm or less, or 300 µm or less, of 280 µm or less, or 260 µm or less, or 240 µm or less, or 220 µm or less, or 200 µm or less, 180 µm or less, or 160 µm or less, or 140 µm or less, or 120 µm or less, or 100 µm or less, or 80 µm or less, or 70 µm or less, or 60 µm or less, or 50 µm or less.
134. The particles of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} according to item 133, having a particle size distribution X50 of 45 µm or less, or 40 µm or less, or 35 µm or less, or 30 µm or less, or 25 µm or less, or 20 µm or less, or 15 µm or less, or 10 µm or less.
135. The particles of (i) {drug1a}; (ii) {drug2a}, {drug2b}, {drug2c}, {drug2d}, or {drug2e}; (iii) {drug3a}, {drug3b}, {dnig3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} according to any of items 130 to 134, having a cumulative distribution by volume basis Q3(X) (X in µm) of Q3(355) <0.50; or Q3(180) <0.50 and Q3(355) ≥0.50; or Q3(125) <0.50 and Q3(180) ≥0.50; or Q3(125) ≥0.50.
136. The particles of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} according to any of items 130 to 135, having a cumulative distribution by volume basis Q3(X) (X in µm) of Q3(268) <0.50 and Q3(355) ≥0.50; or Q3(180) <0.50 and Q3(268) ≥0.50; or Q3(153) <0.50 and Q3(180) ≥0.50; or Q3(125) <0.50 and Q3(153) ;:::0.50; or Q3(111) <0.50 and Q3(125) ≥0.50; or Q3(98) <0.50 and Q3(111) ≥0.50; or Q3(98) ≥0.50.
137. The particles of (i) {drug1a}; (ii) {drug2a}, {drug2b}, {drug2c}, {drug2d}, or {drug2e}; (iii) {drug3a}, {drug3b}, {drug3c}, {drug3d}, {drug3e}, {drug3f}, {drug3g}, {drug3h}, {drug3i}, {drug3j}, {drug3k}, {drug3l}, {drug3m}, {drug3n}, or {drug3o}; (iv) {drug4a}, {drug4b}, {drug4c}, {drug4d}, {drug4e}, {drug4f}, {drug4g}, or {drug4h}; (v) {drug5a}, {drug5b}, {drug5c}, {drug5d}, {drug5e}, or {drug5f}; or (vi) {drug6a} according to any of items 130 to 136, having a cumulative distribution by volume basis Q3(X) (X in µm) of Q3(312) <0.50 and Q3(355) ≥0.50; or Q3(268) <0.50 and Q3(312) ≥0.50; or Q3(224) <0.50 and Q3(268) ≥0.50; or Q3(180) <0.50 and Q3(224) ≥0.50; or Q3(167) <0.50 and Q3(180) ≥0.50; or Q3(153) <0.50 and Q3(167) ≥0.50; or Q3(139) <0.50 and Q3(153) ≥0.50; or Q3(125) <0.50 and Q3(139) ≥0.50; or Q3(111) <0.50 and Q3(125) ≥0.50; or Q3(98) <0.50 and Q3(111) ≥0.50; or Q3(84) <0.50 and Q3(98) ≥0.50; or Q3(84) ≥0.50.

## Claims

1. A packaging comprising one or more administration units comprising a sodium salt of (R)-3-[6-amino-pyridin-3-yl]-2-(1-cyclohexyl-1 H-imidazol-4-yl)-propionic acid 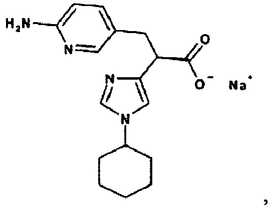 wherein the sodium salt of (R)-3-[6-amino-pyridin-3-yl]-2-(1-cyclohexyl-1 H-imidazol-4-yl)-propionic acid is
a crystalline sodium monohydrate salt, which exhibits in an X-ray powder diffraction pattern measured in transmission with CuKa1 radiation at room temperature a characteristic reflection at degrees 2 theta of 22.2, 16.5 and 6.3, each time ± 0.2 degrees 2 theta; or
a crystalline sodium anhydrate salt, which exhibits in an X-ray powder diffraction pattern measured in transmission with CuKa1 radiation at 110°C a characteristic reflection at degrees 2 theta of 21.4, 16.8 and 6.1, each time ± 0.2 degrees 2 theta.

2. The packaging according to claim 1, wherein the sodium salt of (R)-3-[6-amino-pyridin-3-yl]-2-(1-cyclohexyl-1 H-imidazol-4-yl)-propionic acid is
a crystalline sodium monohydrate salt, which exhibits in an X-ray powder diffraction pattern measured in transmission with CuKa1 radiation at room temperature a characteristic reflection at degrees 2 theta of 22.2, 21.8, 18.1, 16.5, 8.2 and 6.3, each time ± 0.2 degrees 2 theta; or
a crystalline sodium anhydrate salt, which exhibits in an X-ray powder diffraction pattern measured in transmission with CuKa1 radiation at 110°C a characteristic reflection at degrees 2 theta of 22.4, 21.4,16.8,16.4, 8.6 and 6.1, each time ± 0.2 degrees 2 theta.

3. The packaging according to claim 1 or 2, comprising a material selected from the group consisting of paper, cardboard, paperboard, metal foil and plastic foil.

4. The packaging according to any of claims 1 to 3, wherein the admmistration unit is solid, semisolid or liquid.

5. The packaging according to any of claims 1 to 4, wherein the administration unit is for administration once daily, or twice daily, or thrice daily, or four times daily, or five times daily, or six times daily.

6. The packaging according to any of claims 1 to 5, wherein the administration unit provides the peak plasma level within 0.1 hour to 36 hours after administration.

7. The packaging according to any of claims 1 to 6, comprising a multitude of at least 2, or at least 3, or at least 4, or at least 5, or at least 6, or at least 7, or at least 8, or at least 9, or at least 10 administration units according to any of claims 1 to 3.

8. The packaging according to any of claims 1 to 7, wherein all administration units are substantially identical.

9. The packaging according to any of claims 1 to 8, wherein the administration unit is for oral, buccal, opthalmic, nasal, rectal, transmucosal, or intestinal administration.

10. The packaging according to any of claims 7 to 9 for use in the treatment of a disorder or a disease, wherein one or more administration units of said multitude are administered on a first day, and one or more administration units of said multitude are administered on a second day following said first day.

11. The packaging for use according to claim 10, wherein one or more administration units of said multitude are administered on one or more subsequent days following said second day.

12. The packaging for use according to any of claims 10 to 11, wherein the time interval between the administration of an administration unit and the administration of a subsequent administration unit is not more than 48 hours.

13. The packaging for use according to any of claims 1 to 12, wherein the administration unit is for use in the treatment of a disorder or a disease in a mammal.

## Patentansprüche

1. Eine Verpackung, umfassend eine oder mehrere Verabreichungseinheiten, die ein Natriumsalz von (R)-3-[6-Amino-pyridin-3-yl]-2-(1-cyclohexyl-1H-imidazol-4-yl)-propionsäure 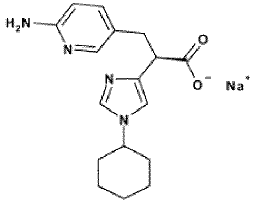 umfassen, wobei das Natriumsalz von (R)-3-[6-Amino-pyridin-3-yl]-2-(1-cyclohexyl-1H-imidazol-4-yl) propionsäure
ein kristallines Natrium Monohydrat Salz ist, welches in einem Röntgenpulverbeugungsmuster gemessen in Transmission mit CuKa1 Strahlung bei Raumtemperatur eine charakteristische Reflexion bei Grad 2 Theta von 22,2, 16,5 und 6,3, jedes Mal ± 0,2 Grad 2 Theta zeigt; oder
ein kristallines Natrium Anhydrat Salz ist, welches in einem Röntgenpulverbeugungsmuster gemessen in Transmission mit CuKa1 Strahlung bei 110 ° C eine charakteristische Reflexion bei Grad 2 Theta von 21,4, 16,8 und 6,1, jedes Mal ± 0,2 Grad 2 Theta zeigt.

2. Die Verpackung nach Anspruch 1, wobei das Natriumsalz von (R)-3-[6-Amino-pyridin-3-yl]-2-(1-cyclohexyl-1H-imidazol-4-yl) propionsäure
ein kristallines Natrium Monohydrat Salz ist, welches in einem Röntgenpulverbeugungsmuster gemessen in Transmission mit CuKa1 Strahlung bei Raumtemperatur eine charakteristische Reflexion bei Grad 2 Theta von 22,2, 21,8, 18,1, 16,5, 8,2 und 6,3, jedes Mal ± 0,2 Grad 2 Theta zeigt; oder
ein kristallines Natrium Anhydrat Salz ist, welches in einem Röntgenpulverbeugungsmuster gemessen in Transmission mit CuKa1 Strahlung bei 110 ° C eine charakteristische Reflexion bei Grad 2 Theta von 22,4, 21,4, 16,8, 16,4, 8,6 und 6,1, jedes Mal ± 0,2 Grad 2 Theta zeigt.

3. Die Verpackung nach Anspruch 1 oder 2, umfassend ein Material ausgewählt aus der Gruppe bestehend aus Papier, Karton, Pappe, Metallfolie und Kunststofffolie.

4. Die Verpackung nach einem der Ansprüche 1 bis 3, wobei die Verabreichungseinheit fest, halbfest oder flüssig ist.

5. Die Verpackung nach einem der Ansprüche 1 bis 4, wobei die Verabreichungseinheit für die Verabreichung einmal täglich, oder zweimal täglich, oder dreimal täglich, oder viermal täglich, oder fünfmal täglich, oder sechsmal täglich ist.

6. Die Verpackung nach einem der Ansprüche 1 bis 5, wobei die Verabreichungseinheit den Spitzenplasmaspiegel innerhalb von 0,1 Stunden bis 36 Stunden nach der Verabreichung liefert.

7. Die Verpackung nach einem der Ansprüche 1 bis 6, umfassend eine Vielzahl von mindestens 2, oder mindestens 3, oder mindestens 4, oder mindestens 5, oder mindestens 6, oder mindestens 7, oder mindestens 8, oder mindestens 9, oder mindestens 10 Verabreichungseinheiten nach einem der Ansprüche 1 bis 3.

8. Die Verpackung nach einem der Ansprüche 1 bis 7, wobei alle Verabreichungseinheiten im Wesentlichen identisch sind.

9. Die Verpackung nach einem der Ansprüche 1 bis 8, wobei die Verabreichungseinheit für die orale, bukkale, ophthalmologische, nasale, rektale, transmukosale oder intestinale Verabreichung ist.

10. Die Verpackung nach einem der Ansprüche 7 bis 9 zur Anwendung bei der Behandlung einer Störung oder einer Krankheit, bei der eine oder mehrere Verabreichungseinheiten der besagten Vielzahl an einem ersten Tag verabreicht werden, und eine oder mehrere Verabreiclmngseinheiten der besagten Vielzahl an eignen zweiten Tag verabreicht werden, welcher auf den ersten Tag folgt.

11. Die Verpackung nach Anspruch 10, wobei eine oder mehrere Verabreichungseinheiten der besagten Vielzahl an einem oder mehreren aufeinanderfolgenden Tagen verabreicht werden, welche dem besagten zweiten Tag folgen.

12. Die Verpackung nach einem der Ansprüche 10 bis 11, wobei das Zeitintervall zwischen der Verabreichung einer Verabreichungseinheit und der Verabreichung einer nachfolgenden Verabreichungseinheit nicht mehr als 48 Stunden beträgt.

13. Die Verpackung nach einem der Ansprüche 1 bis 12, wobei die Verabreichungseinheit zur Anwendung bei der Behandlung einer Störung oder einer Krankheit in einem Säugetier ist.

## Revendications

1. Un emballage comprenant une ou plusieurs unités administratives comprenant un sel de sodium
d'acide (R)-3-acide-[6-amino-pyridine-3-yl]-2-(1-cyclohexyl-1H-imidazol-4-yl)-propionique 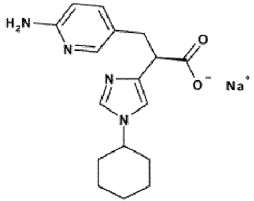 dans lequel le sel de sodium de (R)-3-[6-amino-pyridine-3-yl]-2-(1-cyclohexyl-1H-imidazol-4-yl)-propioniclue est
un sel de sodium monohydraté cristallin qui présente dans un diagramme de diffraction de poudre aux rayons X mesurée en transmission par un rayonnement CuKa1 à température ambiante, une caractéristique de réflexion à des degrés deux thêta de 22,2, 16,5 et 6,3, à chaque fois ± 0,2 degrés 2 thêta; ou
un sel de sodium cristallin, qui présente dans un diagramme de diffraction de poudre aux rayons X mesurée en transmission par un rayonnement CuKa1 à 110 ° C, une caractéristique de réflexion à des degrés 2 thêta de 21,4, 16,8 et 6,1, à chaque fois ± 0,2 degrés 2 thêta.

2. L'emballage selon la revendication 1, dans lequel le sel de sodium de (R)-3-[6-amino-pyridine-3-yl]-2-(1-cyclohexyl-1H-imidazol-4-yl)-propionique est
un sel de sodium monolydraté cristallin qui présente dans un diagramme de diffraction de poudre aux rayons X mesurée en transmission par un rayonnement CuKa1 à température ambiante, une caractéristique de réflexion à des degrés deux thêta de 22,2, 21,8, 18,1, 16,5, 8,2 et 6,3, à chaque fois ± 0,2 degrés 2 thêta; ou
un sel de sodium cristallin, qui présente dans un diagramme de diffraction de poudre aux rayons X mesurée en transmission par un rayonnement CuKa1 à 110 ° C, une caractéristique de réflexion à des degrés 2 thêta de 22,4, 21,4, 16,8, 16,4, 8,6 et 6,1, à chaque fois ± 0,2 degrés 2 thêta.

3. L'emballage selon la revendication 1 ou 2, comprenant un matériau choisi dans le groupe constitué par le papier, une feuille de carton, le carton, une feuille métallique et une feuille en matière plastique.

4. L'emballage selon l'une quelconque des revendications 1 à 3, dans lequel l'unité d'administration est solide, semi-solide ou liquide.

5. L'emballage selon l'une quelconque des revendications 1 à 4, dans lequel l'unité d'administration est pour une administration une fois par jour, ou deux fois par jour, ou trois fois par jour, ou quatre fois par jour, ou cinq fois par jour, ou six fois par jour.

6. L'emballage selon l'une quelconque des revendications 1 à 5, dans lequel l'unité d'administration fournit des taux plasmatiques de pointe à l'intérieur de 0,1 heure à 36 heures après l'administration.

7. L'emballage selon l'une quelconque des revendications 1 à 6, comprenant une multitude d'au moins 2, ou au moins 3, ou au moins 4, ou au moins 5, ou au moins 6, ou au moins 7, ou au moins 8, ou au moins 9, ou au moins 10 unités d'administration selon l'une quelconque des revendications 1 à 3.

8. L'emballage selon l'une quelconque des revendications 1 à 7, dans lequel toutes les unités d'administration sont sensiblement identiques.

9. L'emballage selon l'une quelconque des revendications 1 à 8, dans lequel l'unité d'administration est une administration par voie orale, buccale, ophtalmique, nasale, rectale, transmuqueuse ou intestinale.

10. L'emballage selon l'une quelconque des revendications 7 à 9, pour une utilisation dans le traitement d'un trouble ou d'une maladie, dans lequel une ou plusieurs administrations des unités de ladite multitude sont administrés sur un premier jour, et une ou plusieurs administrations des unités de ladite multitude sont administrées un deuxième jour suivant le premier jour.

11. L'emballage selon la revendication 10, dans lequel une ou plusieurs unités administratives de ladite multitude sont administrées sur un ou plusieurs jours subséquents suivant ledit deuxième jour.

12. L'emballage selon l'une quelconque des revendications 10 à 11, dans lequel l'intervalle de temps entre l'administration d'une unité d'administration et l'administration d'une unité d'administration suivante ne soit pas supérieure de 48 heures.

13. L'emballage selon l'une quelconque des revendications 1 à 12, dans lequel l'unité d'administration est destinée à être utilisé dans le traitement d'un trouble ou d'une maladie chez un mammifère.
